# EUROPEAN PATENT APPLICATION

(11) **EP 1 445 316 A1**
(43) Date of publication of application: **11.08.2004**
(21) Application number: 04008752.0
(22) Date of filing: 01.10.1998
(51) Int. Cl.: C12N 15/12, C12N 15/00, C12N 15/11, C12N 15/63, A61K 38/16, C07K 16/00, C12P 21/02, C12Q 1/68, G01N 33/53, G01N 33/68

(54) **Novel secreted protein**

(30) Priority: 02.10.1997 US 60837 P; 02.10.1997 US 60862 P; 02.10.1997 US 60839 P; 02.10.1997 US 60866 P; 02.10.1997 US 60843 P; 02.10.1997 US 60836 P; 02.10.1997 US 60838 P; 02.10.1997 US 60874 P; 02.10.1997 US 60833 P; 02.10.1997 US 60884 P; 02.10.1997 US 60880 P
(62) Divisional of application: 98950867.6
(71) Applicant: Human Genome Sciences, Inc., Rockville, MD 20850 (US)
(72) Inventor: Ni, Jian, Germantown Maryland 20874 (US); Young, Paul, Gaithersburg Maryland 20878 (US); Rosen, Craig A., Laytonsville Maryland 20882 (US); Florence, Kimberly A., Rockville, MD 20851 (US); Ruben, Steven M., Brookeville, MD 20833 (US); Carter, Kenneth C., North Potomac Maryland 20878 (US); Feng, Ping, Germantown Maryland 20874 (US); Endress, Gregory A., Florence, MA 01062 (US); LaFleur, David W., Washington, D.C. 20015 (US); Shi, Yanggu, Gaithersburg Maryland 20878 (US); Janat, Fouad, Westerly Rhode Island 02891 (US); Duane, D. Roxanne, Bethesda, MD 20817 (US); Greene, John M., Gaithersburg, MD 20878 (US); Ferrie, Ann M., Tewsbury, MA 01876 (US); Yu, Guo-Liang, San Mateo, CA 94403 (US)
(74) Representative: VOSSIUS & PARTNER

(57) **Abstract**

The present invention relates to novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles. including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

In specific embodiments, polypeptides of the invention comprise the following amino acid sequence:
MLMKINFYPLPKPKLHTSISNCLLDISIYKPSSLISITSDLPGLTLKSXNFSPTPM P GQNLVVTSYSSLASSHPCSVCQWIL (SEQ ID NO:215). Polynucleotides encoding these polypeptides are also encompassed by the invention.

This gene is expressed primarily in CD34 positive blood cells.

Therefore, polynucleotides and polypeptides of the invention are useful as reagents for differential identification of the tissue(s) or cell type(s) present in a biological sample and for diagnosis of diseases and conditions which include, but are not limited to, abnormalities of the immune system, in addition to reproductive disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.immune, hematopoeitic, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of diseases and disorders of the immune system. Similarly, the expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:11 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 538 of SEQ ID NO:11, b is an integer of 15 to 552, where both a and b correspond to the positions of nucleotide residues shown in SEQ ID NO: 11, and where the b is greater than or equal to a + 14.

### FEATURES OF PROTEIN ENCODED BY GENE NO: 2

This gene is expressed primarily in healing wound tissue, Hodgkin's lymphoma, and to a lesser extent, in other tissues.

Therefore, polynucleotides and polypeptides of the invention are useful as reagents for differential identification of the tissue(s) or cell type(s) present in a biological sample and for diagnosis of diseases and conditions which include, but are not limited to, proliferative, immune, or hematopoeitic disorders, particularly Hodgekin's lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of Hodgekin's lymphoma and treatment of wounds. Expression within wounded tissue and other cellular sources marked by proliferating cells indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 12 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1420 of SEQ ID NO: 12, b is an integer of 15 to 1434, where both a and b correspond to the positions of nucleotide residues shown in SEQ ID NO:12, and where the b is greater than or equal to a + 14.

### FEATURES OF PROTEIN ENCODED BY GENE NO: 3

The translation product of this gene was shown to have homology to the human M6 membrane glycoprotein which is thought to be important in myelination of central nervous system neurons during development (See Genbank Accession No.bbs|137975).In specific embodiments, polypeptides of the invention comprise the following amino acid sequence: LAPR FAFSQCSLAIMLTLLFQIHFLMILSSNWAYLKDASKMQAYQDIKAKEEQELQDIQ SRSKEQLNSYT (SEQ ID NO:216). Polynucleotides encoding these polypeptides are also encompassed by the invention.

This gene is expressed primarily in fetal brain, and to a lesser extent, in schizophrenic hypothalamus.

Therefore, polynucleotides and polypeptides of the invention are useful as reagents for differential identification of the tissue(s) or cell type(s) present in a biological sample and for diagnosis of diseases and conditions which include, but are not limited to, developmental or neural disorders, particularly neurological and psychogenic disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g.developmental, neural, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neural tissues indicates that polynucleotides and polypeptides corresponding to this gene are useful for diagnosis and treatment of certain neurological psychogenic disorders, including schizophrenia. Moreover, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, mania, dementia, paranoia, obsessive compulsive disorder, panic disorder, learning disabilities, ALS, psychoses , autism, and altered bahaviors, including disorders in feeding, sleep patterns, balance, and preception. In addition, Elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:13 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1867 of SEQ ID NO: 13, b is an integer of 15 to 1881, where both a and b correspond to the positions of nucleotide residues shown in SEQ ID NO:13, and where the b is greater than or equal to a + 14.

### FEATURES OF PROTEIN ENCODED BY GENE NO: 4

It is likely that the open reading frame containing the predicted signal peptide continues in the 5' direction. In specific embodiments, polypeptides of the invention comprise the following amino acid sequence:
IRHEGGGQPFTSXPLEILFFLNGWYNATYFLLELFIFLYKGVLLPYPTANLVLDV V (SEQ ID NO:217), and/or MVHTRCSGHGDQGGELEVSRGLVLRRGRMGITLP LPILECRR VSWADGPGLEDGTHWPYAELLAQMSVLKKSHTAFLRTTCPTN SHWCG (SEQ ID NO:218). Polynucleotides encoding these polypeptides are also encompassed by the invention. The gene encoding the disclosed cDNA is believed to reside on chromosome 11. Accordingly, polynucleotides related to this invention are useful as a marker in linkage analysis for chromosome 11.

This gene is expressed primarily in adult brain.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly neurodegenerative diseases. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g.neural, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 116 as residues: Thr-17 to Lys-25.

The tissue distribution in adult brain indicates that polynucleotides and polypeptides corresponding to this gene are useful for the diagnosis and treatment of neurodegenerative diseases. Moreover, polynucleotides and polypeptides corresponding to this gene are useful for the detection/treatment of behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, Elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 14 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1046 of SEQ ID NO: 14, b is an integer of 15 to 1060, where both a and b correspond to the positions of nucleotide residues shown in SEQ ID NO: 14, and where the b is greater than or equal to a + 14.

### FEATURES OF PROTEIN ENCODED BY GENE NO: 5

The gene encoding the disclosed cDNA is believed to reside on chromosome 5. Accordingly, polynucleotides related to this invention are useful as a marker in linkage analysis for chromosome 5.

This gene is expressed primarily in 12 week old early stage human and infant brain.

Therefore, polynucleotides and polypeptides of the invention are useful as reagents for differential identification of the tissue(s) or cell type(s) present in a biological sample and for diagnosis of diseases and conditions which include, but are not limited to, neural or developmental disorders, particularly neurodegenerative conditions. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g.developmental, neural, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e.. the expression level in healthy tissue or bodily fluid from an individual not having the disorder. Preferred epitopes include those comprising a sequence shown in SEQ ID NO:117 as residues: Phe-20 to Arg-26.

The tissue distribution in neural and developmental tissues indicates that polynucleotides and polypeptides corresponding to this gene are useful for diagnosis and treatment of neurodevelopmental diseases. Moreover, the polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, Elevated expression of this gene product in regions of the brain indicates that it plays a role in normal neural function. Potentially, this gene product is involved in synapse formation, neurotransmission, learning, cognition, homeostasis. or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:15 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1241 of SEQ ID NO: 15, b is an integer of 15 to 1255, where both a and b correspond to the positions of nucleotide residues shown in SEQ ID NO: 15, and where the b is greater than or equal to a + 14.

### FEATURES OF PROTEIN ENCODED BY GENE NO: 6

The translation product of this gene was shown to have homology to the conserved MAP kinase phosphatase which is known to be important as an antagonist in MAP kinase activation (See Genbank Accession No.gil1050849). As such, a role in development or in cellular metabolism may be anticipated. In specific embodiments, polypeptides of the invention comprise the following amino acid sequence: RVIRLTXRANWSSTAVAAALELVDPPGCRNSARVKYCVVYDNNSSTLEILLKD DDDDSDSDGDGKDLVPQAAIEYGRILTRLTHHPVYILKGGYERFSGTYH FLRTQKIIWMPQELDAFQPYPIEIVPGKVFVGNFSQACDPKIQKDLKIKAHV NVSMDTGPFFAGDADKLLHIRIEDSPEAQILPFLRHMCHFIEIHHHLGSVILIFST QGISRSCAAIIAYLMHSNEQTLQRSWAYVKKCKNNMCPNRGLVSQLLEWE KTILGDSTINIMDPLY (SEQ ID NO:219). Polynucleotides encoding these polypeptides are also encompassed by the invention. The gene encoding the disclosed cDNA is believed to reside on chromosome 7. Accordingly, polynucleotides related to this invention are useful as a marker in linkage analysis for chromosome 7.

This gene is expressed primarily in fetal kidney, liver, and spleen.

Therefore, polynucleotides and polypeptides of the invention are useful as reagents for differential identification of the tissue(s) or cell type(s) present in a biological sample and for diagnosis of diseases and conditions which include, but are not limited to, developmental, immune, or haemopoietic disorders. Similarly, polypeptides and antibodies directed to these polypeptides are useful in providing immunological probes for differential identification of the tissue(s) or cell type(s). For a number of disorders of the above tissues or cells, particularly of the haemopoietic system or developing immune system, expression of this gene at significantly higher or lower levels may be routinely detected in certain tissues and cell types (e.g.developmental, renal, immune, hematopoeitic, hepatic, and cancerous and wounded tissues) or bodily fluids (e.g.lymph, bile, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal liver, combined with the homology to a signal transduction regulatory protein indicates that polynucleotides and polypeptides corresponding to this gene are useful for the diagnosis and treatment of hematopoietic disorders involving blood stem cell formation, such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 16 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1022 of SEQ ID NO: 16, b is an integer of 15 to 1036, where both a and b correspond to the positions of nucleotide residues shown in SEQ ID NO: 16, and where the b is greater than or equal to a + 14.

### FEATURES OF PROTEIN ENCODED BY GENE NO: 7

In specific embodiments, polypeptides of the invention comprise the following amino acid sequence:
IRHEFTSEKSWKSSCNEGESSSTSYMHQRSPGGPTKLIEIISDCNWEEDRNKILS ILSQHINSNMPQSLKVGSFIIELASQRKSRGEKNPPVYSSRVXISMPSCQDQ DDMAEKSGSETPDGPLSPGKMEDISPVQTDALDSVRERLHGGKGLPFY AGLSPAGKLVAYKRKPSSSTSGLIQVRIIFNLGIAPLYTPR (SEQ ID NO:220). Polynucleotides encoding these polypeptides are also encompassed by the invention.

This gene is expressed primarily in human fetal heart.

Therefore, polynucleotides and polypeptides of the invention are useful as reagents for differential identification of the tissue(s) or cell type(s) present in a biological sample and for diagnosis of diseases and conditions which include, but are not limited to, developmental or cardiovascular disorders, particularly fetal cardiac defects. Similarly, polypeptides and antibodies directed to these polypeptides are useful in providing immunological probes for differential identification of the tissue(s) or cell type(s). For a number of disorders of the above tissues or cells, particularly of the cardiac system, expression of this gene at significantly higher or lower levels may be routinely detected in certain tissues and cell types (e.g.developmental, cardiac, musculoskeletal, and cancerous and wounded tissues) or bodily fluids (e.g.lymph, amntiotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of fetal cardiac defects. Similarly, expression within fetal tissue indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 17 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1000 of SEQ ID NO: 17, b is an integer of 15 to 1014, where both a and b correspond to the positions of nucleotide residues shown in SEQ ID NO:17, and where the b is greater than or equal to a + 14.

### FEATURES OF PROTEIN ENCODED BY GENE NO: 8

It is likely that the open reading frame containing the predicted signal peptide continues in the 5' direction. In specific embodiments, polypeptides of the invention comprise the following amino acid sequence: CNIEYIRSDKCMFKHELEELRTTI (SEQ ID NO:221). Polynucleotides encoding these polypeptides are also encompassed by the invention. The gene encoding the disclosed cDNA is believed to reside on chromosome 2. Accordingly, polynucleotides related to this invention are useful as a marker in linkage analysis for chromosome 2.

This gene is expressed primarily in fetal cochlea, other fetal tissues, and to a lesser extent in placenta.

Therefore, polynucleotides and polypeptides of the invention are useful as reagents for differential identification of the tissue(s) or cell type(s) present in a biological sample and for diagnosis of diseases and conditions which include, but are not limited to, developmental disorders, particularly of auditory tissues. Similarly, polypeptides and antibodies directed to these polypeptides are useful in providing immunological probes for differential identification of the tissue(s) or cell type(s). For a number of disorders of the above tissues or cells, particularly of the fetal developmental systems, expression of this gene at significantly higher or lower levels may be routinely detected in certain tissues and cell types (e.g.developmental, auditory, and cancerous and wounded tissues) or bodily fluids (e.g.lymph, amniotic fluid, cochlear fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:120 as residues: Met-1 to His-6, Glu-33 to Asn-43.

The tissue distribution within fetal tissue indicates that polynucleotides and polypeptides corresponding to this gene are useful for the diagnosis and treatment of fetal developmental disorders, particularly of auditory tissues. Similarly, expression within fetal tissues and other cellular sources marked by proliferating cells indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:18 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1273 of SEQ ID NO:18, b is an integer of 15 to 1287, where both a and b correspond to the positions of nucleotide residues shown in SEQ ID NO: 18, and where the b is greater than or equal to a + 14.

### FEATURES OF PROTEIN ENCODED BY GENE NO: 9

This gene is expressed primarily in nine week old early stage human.

Therefore, polynucleotides and polypeptides of the invention are useful as reagents for differential identification of the tissue(s) or cell type(s) present in a biological sample and for diagnosis of diseases and conditions which include, but are not limited to, fetal developmental disorders. Similarly, polypeptides and antibodies directed to these polypeptides are useful in providing immunological probes for differential identification of the tissue(s) or cell type(s). For a number of disorders of the above tissues or cells, particularly of the fetal developmental systems, expression of this gene at significantly higher or lower levels may be routinely detected in certain tissues and cell types (e.g.developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:121 as residues: Met-1 to Arg-6.

The tissue distribution in fetal tissue indicates that polynucleotides and polypeptides corresponding to this gene are useful for diagnosis and treatment of some types of fetal developmental disorders. Moreover, the expression within embryonic tissue indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 19 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1091 of SEQ ID NO:19, b is an integer of 15 to 1105, where both a and b correspond to the positions of nucleotide residues shown in SEQ ID NO: 19, and where the b is greater than or equal to a + 14.

### FEATURES OF PROTEIN ENCODED BY GENE NO: 10

This gene is expressed primarily in epididymus.

Therefore, polynucleotides and polypeptides of the invention are useful as reagents for differential identification of the tissue(s) or cell type(s) present in a biological sample and for diagnosis of diseases and conditions which include, but are not limited to, reproductive disorders, particularly male sterility. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues and cell types (e.g.reproductive, cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in epididymus indicates that polynucleotides and polypeptides corresponding to this gene are useful for the diagnosis and treatment of male sterility, and/or could be used as a male contraceptive. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:20 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1075 of SEQ ID NO:20, b is an integer of 15 to 1089, where both a and b correspond to the positions of nucleotide residues shown in SEQ ID NO:20, and where the b is greater than or equal to a + 14.

### FEATURES OF PROTEIN ENCODED BY GENE NO: 11

The translation product of this gene shares sequence homology with a mitotic phosphoprotein which is thought to be important in initiating and coordinating cell division processes.In specific embodiments, polypeptides of the invention comprise the following amino acid sequence: HHQQVPEXDREDSPERCSDXXEEKKARRGRS PKGEFKDEEETVTTKHIHITQATETTTTRHKRTANPSKTIDLGAAAHYTGDKAS PD QNASTHTPQSSVKTSVPSSKSSGDLVDLFDGTSQCNRRXS (SEQ ID NO:222), VSSDSVGGFRYSERYDPEPKSKWDEEWDKNKSAFPFSDKL GELSDKIGSTIDDTISKFRXKIEKTLQKDA ATXXRKRKREEADLPKVNSK MKRRL (SEQ ID NO:223), and/or RQSIFISHRPQRPPQPDTSAQQILPKP LILEQQHITQGTKQVQI R (SEQ ID NO:224). Polynucleotides encoding these polypeptides are also encompassed by the invention.The gene encoding the disclosed cDNA is believed to reside on chromosome 5. Accordingly, polynucleotides related to this invention are useful as a marker in linkage analysis for chromosome 5.

This gene is expressed primarily in placenta, and to a lesser extent in T-cells.

Therefore, polynucleotides and polypeptides of the invention are useful as reagents for differential identification of the tissue(s) or cell type(s) present in a biological sample and for diagnosis of diseases and conditions which include, but are not limited to, spontaneous abortion and in utero developmental problems, in addition to immune disorders, such as autoimmune conditions. Similarly, polypeptides and antibodies directed to these polypeptides are useful in providing immunological probes for differential identification of the tissue(s) or cell type(s). For a number of disorders of the above tissues or cells, particularly of the immune and reproductive systems, expression of this gene at significantly higher or lower levels may be routinely detected in certain tissues and cell types (e.g.developmental, immune, hematopoietic, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:123 as residues: Ser-65 to Gly-71, Ser-155 to Leu-160, Gln-168 to Asp-179, Leu-189 to Pro-196, Gln-210 to Ser-218, Gln-224 to Pro-231, Val-326 to Asp-331.

The tissue distribution in placental tissue combined with the homology to mitotic phosphoprotein indicates that polynucleotides and polypeptides corresponding to this gene are useful for the treatment and diagnosis of diseases that arise in utero due to cell division abnormalities during fetal development. Alternatively, expression within T-cells indicates that the secreted protein may also be used to determine biological activity, to raise antibodies, as tissue markers, to isolate cognate ligands or receptors, to identify agents that modulate their interactions and as nutritional supplements. It may also have a very wide range of biological acitivities. Typical of these are cytokine, cell proliferation/differentiation modulating activity or induction of other cytokines;immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); anti-inflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2817 of SEQ ID NO:21, b is an integer of 15 to 2831, where both a and b correspond to the positions of nucleotide residues shown in SEQ ID NO:21, and where the b is greater than or equal to a + 14.

### FEATURES OF PROTEIN ENCODED BY GENE NO: 12

The translation product of this gene shares sequence homology with murine counterpart of the human TB2/DP1 which is thought to be important in in familial adenomatous polyposis (FAP) disease as one of six genes deleted. Triggering of murine mast cells by IgE plus antigen results in a decrease of TB2/DP1 mRNA up to 60% after 2 h implying a possible role of this gene in regulation of the allergic effector cell. Reverse transcription-polymerase chain reaction (RT-PCR) analysis shows an ubiquitous expression pattern in a number of mouse cell lines and tissues. In specific embodiments, polypeptides of the invention comprise the following amino acid sequence: DQDGLRAVAALTLHQGRQLLYRKFVHPSLSRHEKEIDAYIVQAKE RSYETVLSFGKRGLNIAASAAVQAATXSQGALAGRLRSFSMQDLRSISDAPAPA YHDPLYLEDQVSHRRPPIGYRAGGLQDSDTEDECWSDTEAVPRAPARPRE KPLIRSQSLRVVKXKPPVREGTSRSLKVR TXKKTVPSDVDS (SEQ ID NO:225). Polynucleotides encoding these polypeptides are also encompassed by the invention.

This gene is expressed primarily in T cells,and to a lesser extent, in fetal skin.

Therefore, polynucleotides and polypeptides of the invention are useful as reagents for differential identification of the tissue(s) or cell type(s) present in a biological sample and for diagnosis of diseases and conditions which include, but are not limited to, cancer, particularly familial polyptosis, or other proliferating disorders. Similarly, polypeptides and antibodies directed to these polypeptides are useful in providing immunological probes for differential identification of the tissue(s) or cell type(s). For a number of disorders of the above tissues or cells, particularly of the colon, expression of this gene at significantly higher or lower levels may be routinely detected in certain tissues and cell types (e.g.immune, developmental tissues, integumentary, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 124 as residues: Met-99 to Ala-114.

The tissue distribution in T-cells and fetal skin, combined with the homology to the DP1 gene of the FAP locus indicates that polynucleotides and polypeptides corresponding to this gene are useful for treatment and diagnosis of familial adenomatous polyposis, as well as other cancers. It may also be useful in treating allergic disorders. Expression within fetal tissue and other cellular sources marked by proliferating cells indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1434 of SEQ ID NO:22, b is an integer of 15 to 1448, where both a and b correspond to the positions of nucleotide residues shown in SEQ ID NO:22, and where the b is greater than or equal to a + 14.

### FEATURES OF PROTEIN ENCODED BY GENE NO: 13

The translation product of this gene shares sequence homology with a murine oligodendrocyte-specific protein related to peripheral myelin protein-22 (PMP-22). PMP-22 is important in peripheral myelination and Schwann cell proliferation, and mutations in its gene cause diseases of peripheral nerves. Myelin plays a critical role in nervous system function and alterations in myelin-specific proteins cause a variety of neurologic disorders. The polynucleotide sequence of this gene may have a frame shift. Therefore the preferred signal peptide may reside in a frame other than the associated polynucleotides of the above referenced gene.In specific embodiments, polypeptides of the invention comprise the following amino acid sequence:
LCHRLPGRLQLLGVPVHAGPLWVYSGLPGTHDHRHPPGLPRPLAXHX GPALHQHWGPGALQESQAGGXRRGPPHSGRYLRDGGXLLVRFNITRDFFDPL YPGTKYELGPXLYLGWSASLXSILGGLCLCSACCCGSDEDQPPAPGGP TXLPCP (SEQ ID NO:226). Polynucleotides encoding these polypeptides are also encompassed by the invention.

This gene is expressed primarily in endothelial and T cells.

Therefore, polynucleotides and polypeptides of the invention are useful as reagents for differential identification of the tissue(s) or cell type(s) present in a biological sample and for diagnosis of diseases and conditions which include, but are not limited to, neurological disorders related to myelin abnormalities, in addition to immune or endothelial disorders, particularly vascular conditions. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g.neural, immune, vascular, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in immune cells combined with the homology to an oligodendrocyte-specific protein related to PMP-22 indicates that polynucleotides and polypeptides corresponding to this gene are useful for the diagnosis and treatment of diseases of the nervous system, particularly those involving aberrant myelinization of the nerves, such as ALS and multiple sclerosis, or autoimmune disorders affecting neural tissues. Similarly, polynucleotides and polypeptides corresponding to this gene are useful for the detection/treatment of neurodegenerative disease states. behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, psychoses, autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition. homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1197 of SEQ ID NO:23, b is an integer of 15 to 1211, where both a and b correspond to the positions of nucleotide residues shown in SEQ ID NO:23, and where the b is greater than or equal to a + 14.

### FEATURES OF PROTEIN ENCODED BY GENE NO: 14

The translation product of this gene shares high sequence homology at the nucleotide level with the human G protein-coupled receptor (EBI 1) gene, exon 1. This EBI1 gene is a lymphoid-specific member of the G-protein-coupled receptor family. This receptor, also reported as the Epstein-Barr-induced cDNA EBI1, is expressed in normal lymphoid tissues and in several B- and T-lymphocyte cell lines. While the function and the ligand for EBI1 remain unknown, its sequence and gene structure suggest that it is related to the receptors that recognize chemoattractants, such as interleukin-8, RANTES, C5a, and fMet-Leu-Phe. Like the chemoattractant receptors, EBI1 contains intervening sequences near its 5' end; however, EBI1 is unique in that both of its introns interrupt the coding region of the first extracellular domain. The gene is encoded on human chromosome 17q12-q21.2. None of the other G-protein-coupled receptors has been mapped to this region, but the C-C chemokine family has been mapped to 17q11-q21. The mouse EBI1 cDNA has also been isolated and encodes a protein with 86% identity to the human homolog..

This gene is expressed primarily in spinal cord.

Therefore, polynucleotides and polypeptides of the invention are useful as reagents for differential identification of the tissue(s) or cell type(s) present in a biological sample and for diagnosis of diseases and conditions which include, but are not limited to, inflammatory disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g.neural, immune, skeletal, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to the EBI-1 gene indicates that polynucleotides and polypeptides corresponding to this gene are useful for developing diagnostics and small molecule therapeutics for affecting the action of chemoattractants similar to interleukin-8, RANTES, C5a, and fMet-Leu-Phe. In turn, this could be useful in the treatment of inflammatory diseases such as sepsis, inflammatory bowel syndrome, psoriasis, and rheumatoid arthritis.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1046 of SEQ ID NO:24, b is an integer of 15 to 1060, where both a and b correspond to the positions of nucleotide residues shown in SEQ ID NO:24, and where the b is greater than or equal to a + 14.

### FEATURES OF PROTEIN ENCODED BY GENE NO: 15

This gene is expressed primarily in osteoclastoma, and to a lesser extent, in T cell and fetal liver.

Therefore, polynucleotides and polypeptides of the invention are useful as reagents for differential identification of the tissue(s) or cell type(s) present in a biological sample and for diagnosis of diseases and conditions which include, but are not limited to, osteoclastoma; hematopoietic disorders; immune dysfunction; susceptibility to infection; or osteoporosis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.skeletal tissues, immune or hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in hematopoietic cells and tissues indicates that polynucleotides and polypeptides corresponding to this gene are useful for the diagnosis and/or treatment of disorders of the hematopoietic system. In particular, the elevated expression of this gene product in osteoclastoma indicates that it may play a role particularly in the development of the osteoclast lineage, and thus may be particularly useful in conditions such as osteoporosis and osteopetrosis. Additionally, it may play more generalized roles in hematopoiesis, as evidenced by expression in T cells and fetal liver. Thus, it may also be used to affect the proliferation, survival, activation, and/or differentiation of a variety of hematopoietic lineages. Thus, it may play roles in a variety of disease conditions, including lymphoma/leukemias; defects in immune modulation or immune surveilance; susceptibility to infection; and other hematopoietic disorders.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1043 of SEQ ID NO:25, b is an integer of 15 to 1057, where both a and b correspond to the positions of nucleotide residues shown in SEQ ID NO:25, and where the b is greater than or equal to a + 14.

### FEATURES OF PROTEIN ENCODED BY GENE NO: 16

The translation product of this gene shares sequence homology with bup, a gene locus in mouse of unknown function. Retroviral insertions into this region (that also contains the bmi gene) are frequently correlated with lymphomagenesis (See Genbank Accession No. bbsl125119). The gene encoding the disclosed cDNA is believed to reside on chromosome 10. Accordingly, polynucleotides related to this invention are useful as a marker in linkage analysis for chromosome 10.

This gene is expressed primarily in WI 38 lung fibroblasts, fetal lung, placenta, and to a lesser extent, in T cell lymphoma, fetal liver, and stromal cells.

Therefore, polynucleotides and polypeptides of the invention are useful as reagents for differential identification of the tissue(s) or cell type(s) present in a biological sample and for diagnosis of diseases and conditions which include, but are not limited to, T cell lymphoma, fibrosis, mesenchymal disorders; respiratory disorders; ARDS. Similarly, polypeptides and antibodies directed to these polypeptides are useful in providing immunological probes for differential identification of the tissue(s) or cell type(s). For a number of disorders of the above tissues or cells, particularly of the skeletal, respiratory, and immune systems, expression of this gene at significantly higher or lower levels may be routinely detected in certain tissues and cell types (e.g.skeletal, pulmonary, immune, hematopoietic, and cancerous and wounded tissues) or bodily fluids (e.g.lymph, pulmonary surfactant and sputum, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:128 as residues: Gly-74 to Leu-83, Cys-90 to Arg-96, Glu-103 to Asn-109, Glu-133 to Gln-140, Gln-156 to Pro-164, Lys-183 to Arg-191.

The tissue distribution in lung tissue and cells indicates that polynucleotides and polypeptides corresponding to this gene are useful for the diagnosis and/or treatment of disorders of the lung and, more generally, of mesenchymal cells. Expression of this gene product is elevated in lung, as well as in a cell line derived from lung, suggesting a role in lung function. It is also elevated in mesenchymally-derived cells and tissues such as fibroblasts and endothelium. The expression of this gene also correlates with lymphoma, and it is expressed at hematopoietic sites, such as fetal liver. Thus, it may also play a role in hematopoiesis, either in the survival, proliferation, and/or differentiation of various blood cell lineages.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:26 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 966 of SEQ ID NO:26, b is an integer of 15 to 980, where both a and b correspond to the positions of nucleotide residues shown in SEQ ID NO:26, and where the b is greater than or equal to a + 14.

### FEATURES OF PROTEIN ENCODED BY GENE NO: 17

This gene is expressed primarily in a breast cancer cell line and in Wilm's tumor samples, and to a lesser extent, in apoptotic and helper T cells, as well as activated macrophages.

Therefore, polynucleotides and polypeptides of the invention are useful as reagents for differential identification of the tissue(s) or cell type(s) present in a biological sample and for diagnosis of diseases and conditions which include, but are not limited to, breast cancer; wilm's tumor; hematopoietic disorders; immune dysfunction; acute renal failure. Similarly, polypeptides and antibodies directed to these polypeptides are useful in providing immunological probes for differential identification of the tissue(s) or cell type(s). For a number of disorders of the above tissues or cells, particularly of the breast, kidney, and immune system, expression of this gene at significantly higher or lower levels may be routinely detected in certain tissues or cell types (e.g.breast, reproductive, immune, hematopoietic, and cancerous and wounded tissues) or bodily fluids (e.g.lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in proliferating tissues and cells indicates that polynucleotides and polypeptides corresponding to this gene are useful for the diagnosis and/or treatment of cancer. This gene product is expressed at elevated levels in both breast cancer cells as well as Wilm's tumor. This observation indicates that this gene product may play a role in the control of cell proliferation and/or survival, particularly since it is also observed in apoptotic T cells. Alternately, it may control other aspects of cell behavior or activation, as it is also observed in helper T cells and activated macrophages. Thus, it may play general roles in the immune system as well, either in the control of blood cell survival, proliferation, differentiation, or activation. Thus, this gene product may be useful in controlling immune modulation and immune surveillance as well. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 741 of SEQ ID NO:27, b is an integer of 15 to 755, where both a and b correspond to the positions of nucleotide residues shown in SEQ ID NO:27, and where the b is greater than or equal to a + 14.

### FEATURES OF PROTEIN ENCODED BY GENE NO: 18

This gene is expressed primarily in the synovium.

Therefore, polynucleotides and polypeptides of the invention are useful as reagents for differential identification of the tissue(s) or cell type(s) present in a biological sample and for diagnosis of diseases and conditions which include, but are not limited to, skeletal disorders, particularly joint disorders such as rheumatoid arthritis. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues and cell types (e.g.skeletal, synovium, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in the synovium indicates that the gene and protein product of this gene is useful for diagnosis of disorders of the joints as disregulation of genes encoding proteins secreted from synovial tissues is thought to affect normal function of the joints and may lead to autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 932 of SEQ ID NO:28, b is an integer of 15 to 946, where both a and b correspond to the positions of nucleotide residues shown in SEQ ID NO:28, and where the b is greater than or equal to a + 14.

### FEATURES OF PROTEIN ENCODED BY GENE NO: 19

This gene is expressed primarily in amniotic cells, and to a lesser extent, in chronic lymphocytic leukemia cells of the spleen.

Therefore, polynucleotides and polypeptides of the invention are useful as reagents for differential identification of the tissue(s) or cell type(s) present in a biological sample and for diagnosis of diseases and conditions which include, but are not limited to, developmental or immune disorders, particularly leukemia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.developmental, immune, hematopoietic, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in leukemia cells indicates that polynucleotides and polypeptides corresponding to this gene are useful for the treatment or diagnosis of leukemia and other immune diseases. Similarly. this gene product may be useful in the regulation of the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis. Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 957 of SEQ ID NO:29, b is an integer of 15 to 971, where both a and b correspond to the positions of nucleotide residues shown in SEQ ID NO:29, and where the b is greater than or equal to a + 14.

### FEATURES OF PROTEIN ENCODED BY GENE NO: 20

The translation product of this gene was found to have homology to the human protein, defender against cell death 1 gene, which is a known antagonist of apoptosis (See Genseq Accession No:P46966). The gene encoding the disclosed cDNA is believed to reside on chromosome 14. Accordingly, polynucleotides related to this invention are useful as a marker in linkage analysis for chromosome 14.

This gene is expressed primarily in breast, lung, testes, B cells and T cells.

Therefore, polynucleotides and polypeptides of the invention are useful as reagents for differential identification of the tissue(s) or cell type(s) present in a biological sample and for diagnosis of diseases and conditions which include, but are not limited to, immune or pulmonary disorders, particularly cancer of the breast, lung, testes and B cells. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g.immune, reproductive, pulmonary, and cancerous and wounded tissues) or bodily fluids (e.g.lymph, breast milk, pulmonary surfactant or sputum, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of cancer, particularly of the breast, lung, or in B-cell lymphoma. Similarly, expression within cellular sources marked by proliferating cells, combined with its homology to a conserved regulatory protein of apoptosis indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 994 of SEQ ID NO:30, b is an integer of 15 to 1008, where both a and b correspond to the positions of nucleotide residues shown in SEQ ID NO:30, and where the b is greater than or equal to a + 14.

### FEATURES OF PROTEIN ENCODED BY GENE NO: 21

The translation product of this gene shares sequence homology with human and murine surface glycoprotein which is thought to be important in cell-cell interactions and transducing cellular signals (See Genseq Accession No.gi|2997741).

This gene is expressed primarily in testis.

Therefore, polynucleotides and polypeptides of the invention are useful as reagents for differential identification of the tissue(s) or cell type(s) present in a biological sample and for diagnosis of diseases and conditions which include, but are not limited to, male reproductive diseases or disorders. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues or cell types (e.g.reproductive, immune, and cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:133 as residues: Thr-6 to Leu-11.

The tissue distribution in testes combined with the homology to a conserved cell surface glycoprotein indicates that polynucleotides and polypeptides corresponding to this gene are useful for treating and diagnosis of diseases associated with male reproductive system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 976 of SEQ ID NO:31, b is an integer of 15 to 990, where both a and b correspond to the positions of nucleotide residues shown in SEQ ID NO:31, and where the b is greater than or equal to a + 14.

### FEATURES OF PROTEIN ENCODED BY GENE NO: 22

The translation product of this gene was found to have homology to the human myosin regulatory light chain which is thought to be important in muscle function (See Genbank Accession No.gi|189013). In specific embodiments, polypeptides of the invention comprise the following amino acid sequence:
VDQMFQFASIDVAGNLDYKALSYVITHGEEKEE (SEQ ID NO:227). and/or IRHEAYVILAVCLGG (SEQ ID NO:228). Polynucleotides encoding these polypeptides are also encompassed by the invention. The gene encoding the disclosed cDNA is believed to reside on chromosome 4. Accordingly, polynucleotides related to this invention are useful as a marker in linkage analysis for chromosome 4.

This gene is expressed primarily in lung, testis, and macrophage.

Therefore, polynucleotides and polypeptides of the invention are useful as reagents for differential identification of the tissue(s) or cell type(s) present in a biological sample and for diagnosis of diseases and conditions which include, but are not limited to, cancers and immune disorders, particularly afflicting the pulmonary or reproductive system. Similarly, polypeptides and antibodies directed to these polypeptides are useful in providing immunological probes for differential identification of the tissue(s) or cell type(s). For a number of disorders of the above tissues or cells, particularly of the immue system and male reproductive system, expression of this gene at significantly higher or lower levels may be routinely detected in certain tissues or cell types (e.g.immune, pulmonary, reproductive, and cancerous and wounded tissues) or bodily fluids (e.g.lymph, pulmonary surfactant or sputum, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:134 as residues: Tyr-47 to Phe-54, Arg-144 to Ser-149, Thr-152 to Asp-161, Glu-194 to Asn-203, Glu-242 to Pro-250, Thr-258 to Gly-263, Ala-269 to Gly-274.

The tissue distribution in immune cells and lung tissue indicates that polynucleotides and polypeptides corresponding to this gene are useful for the treatment and diagnosis of diseases of the immune system and male reproductive system. Alternatively, the homology to the conserved myosin regulatory light chain indicates that the protein product of this gene may be useful in the detection, treatment, and/or prevention of a variety of skeletal or cardiac muscle disorders, such as muscular sclerosis. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1117 of SEQ ID NO:32, b is an integer of 15 to 1131, where both a and b correspond to the positions of nucleotide residues shown in SEQ ID NO:32, and where the b is greater than or equal to a + 14.

### FEATURES OF PROTEIN ENCODED BY GENE NO: 23

The translation product of this gene shares sequence homology with potassium channal regulatory subunit which is thought to be important in potassium ion regulation. In specific embodiments, polypeptides of the invention comprise the following amino acid sequence:
WIQRIRHETNPKCSYIPPCKRENQKNLESVMNWQQYWKDEIGS QPFTCYFNQHQRPDDVLLHRTHDEIVLLHCFLWPLVTFVVGVLIVVLTICAKSL AVKAEAMXEAQVLLKGKEACRKQSTEAVLIGTRPPAEPVFPGAGDGQGHD RALRGSSLSGNRNRHNWKTWNLKACIPSAVAMAKGS RS (SEQ ID NO:229). Polynucleotides encoding these polypeptides are also encompassed by the invention. The gene encoding the disclosed cDNA is believed to reside on chromosome 12. Accordingly, polynucleotides related to this invention are useful as a marker in linkage analysis for chromosome 12.

This gene is expressed primarily in the brain.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly neurodegenerative disorders, such as Alzheimers Disease, Parkinsons Disease, or Huntingtons Disease. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g.neural, cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neural tissue combined with the homology to a potassium channal regulatory subunit indicates that polynucleotides and polypeptides corresponding to this gene are useful for the diagnosis and treatment of diseases related to potassium channel malfunction in the brain. Similarly, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1279 of SEQ ID NO:33, b is an integer of 15 to 1293, where both a and b correspond to the positions of nucleotide residues shown in SEQ ID NO:33, and where the b is greater than or equal to a + 14.

### FEATURES OF PROTEIN ENCODED BY GENE NO: 24

The translation product of this gene shares sequence homology with oxidoreductase which is thought to be important in inflammatory reactions.

This gene is expressed primarily in human pancreas tumor.

Therefore, polynucleotides and polypeptides of the invention are useful as reagents for differential identification of the tissue(s) or cell type(s) present in a biological sample and for diagnosis of diseases and conditions which include, but are not limited to, metabolic or immune disorders, particularly proliferative conditions such as pancreas tumor. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g.metabolic tissues, immune, and cancerous and wounded tissues) or bodily fluids (e.g.lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 136 as residues: Ile-72 to Asn-77, Asp-98 to Val-105, Val-210 to Ile-216.

The tissue distribution in pancreatic tissue combined with the homology to oxidoreductase indicates that polynucleotides and polypeptides corresponding to this gene are useful for diagnosis of pancreas tumor and inflammatory diseases. Similarly, expression within cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1000 of SEQ ID NO:34, b is an integer of 15 to 1014, where both a and b correspond to the positions of nucleotide residues shown in SEQ ID NO:34, and where the b is greater than or equal to a + 14.

### FEATURES OF PROTEIN ENCODED BY GENE NO: 25

The translation product of this gene was shown to have homology to the rat TIP120, which is thought to be important in the regulation of basal as well as activated trascriptional metabolism (See Genbank Accession No. gnl|PID|d1014122). Based upon homology to the referenced gene, it is likely that the open reading frame containing the predicted signal peptide continues in the 5' direction. In specific embodiments, polypeptides of the invention comprise the following amino acid sequence:
HYEKVRLQVPIRNSRVDPRVXKFTISDHPQPIDPLLKNCIGDFLKTLEDPDLNVR RVALVTFNSAAHNKPSLIRDLLDTVLPHLYNETKVRKELIREVEMGPFK HTVDDGLDIRKAAFECMYTLLDSCLDRLDIF EFLNHVEDGLKDHYDIK (SEQ ID NO:230). Polynucleotides encoding these polypeptides are also encompassed by the invention.

This gene is expressed primarily in infant brain and various cancers.

Therefore, polynucleotides and polypeptides of the invention are useful as reagents for differential identification of the tissue(s) or cell type(s) present in a biological sample and for diagnosis of diseases and conditions which include, but are not limited to, neural or developmental disorders, particularly cancers. Similarly, polypeptides and antibodies directed to these polypeptides are useful in providing immunological probes for differential identification of the tissue(s) or cell type(s). For a number of disorders of the above tissues or cells, particularly of the nervous or immune system, expression of this gene at significantly higher or lower levels may be routinely detected in certain tissues or cell types (e.g.developmental, neural, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 137 as residues: Ser-41 to Lys-53, Ser-80 to Pro-86, Ile-95 to Ser-110.

The tissue distribution in brain indicates that polynucleotides and polypeptides corresponding to this gene are useful for the treatment and diagnosis of a variety of neural disorders. Similarly, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:35 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1208 of SEQ ID NO:35, b is an integer of 15 to 1222, where both a and b correspond to the positions of nucleotide residues shown in SEQ ID NO:35, and where the b is greater than or equal to a + 14.

### FEATURES OF PROTEIN ENCODED BY GENE NO: 26

It is likely that the open reading frame containing the predicted signal peptide continues in the 5' direction. In specific embodiments, polypeptides of the invention comprise the following amino acid sequence:
IRHEHLRGVQERVNLSAPLLPKEDPIFTYLSKRLGRSIDDIGHLIHEGLQKNTSS WVLYNMASFYWRIKN EPYQVVECA (SEQ ID NO:231). Polynucleotides encoding these polypeptides are also encompassed by the invention.

This gene is expressed primarily in brain, testes and Hodgkins lymphoma.

Therefore, polynucleotides and polypeptides of the invention are useful as reagents for differential identification of the tissue(s) or cell type(s) present in a biological sample and for diagnosis of diseases and conditions which include, but are not limited to, neural, reproductive, or immune disorders, particularly Hodgkins lymphoma. Similarly, polypeptides and antibodies directed to these polypeptides are useful in providing immunological probes for differential identification of the tissue(s) or cell type(s). For a number of disorders of the above tissues or cells, particularly of the immune system expression of this gene at significantly higher or lower levels may be routinely detected in certain tissues or cell types (e.g.neural, reproductive, immune, hematopoietic, and cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:138 as residues: Ser-7 to Asp-13, Gln-93 to Leu-99, Ser-105 to His-122, Arg-125 to Thr-132.

The tissue distribution indicates that polynucleotides and polypeptides corresponding to this gene are useful for the diagnosis and treatment of a variety of immune system disorders. Expression of this gene product in Hodgkins lymphoma indicates a role in the regulation of the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types including reproductive or neural tissues. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 887 of SEQ ID NO:36, b is an integer of 15 to 901, where both a and b correspond to the positions of nucleotide residues shown in SEQ ID NO:36, and where the b is greater than or equal to a + 14.

### FEATURES OF PROTEIN ENCODED BY GENE NO: 27

It is likely that the sequence of this polunucleotide continues upstream of the preferred signal peptide. In specific embodiments, polypeptides of the invention comprise the following amino acid sequence:
EFGTSPHQTCGRRPGTAAGWLLAHSTV (SEQ ID NO:232). Polynucleotides encoding these polypeptides are also encompassed by the invention.

This gene is expressed primarily in epididymus, small intestine, and kidney.

Therefore, polynucleotides and polypeptides of the invention are useful as reagents for differential identification of the tissue(s) or cell type(s) present in a biological sample and for diagnosis of diseases and conditions which include, but are not limited to, reproductive, renal, or gastrointestinal disorders, particularly degenerative kidney disease, congenital digestive disorders, and male infertility. Similarly, polypeptides and antibodies directed to these polypeptides are useful in providing immunological probes for differential identification of the tissue(s) or cell type(s). For a number of disorders of the above tissues or cells, particularly of the uinary, digestive and male reproductive systems, expression of this gene at significantly higher or lower levels may be routinely detected in certain tissues or cell types (e.g.reproductive, urogenital, intestinal, endothelial, and cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 139 as residues: Ala-59 to Thr-68, Glu-72 to Ser-108, Glu-115 to Lys-126.

The tissue distribution in kidney indicates that this gene or gene product could be used in the treatment and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Alternatively, expression within the epididymus indicates that the protein product of this gene may be useful for the detection, treatment, and/or prevention of a variety of reproductive disorders, particularly male infertility. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:37 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 940 of SEQ ID NO:37, b is an integer of 15 to 954, where both a and b correspond to the positions of nucleotide residues shown in SEQ ID NO:37, and where the b is greater than or equal to a + 14.

### FEATURES OF PROTEIN ENCODED BY GENE NO: 28

In specific embodiments, polypeptides of the invention comprise the following amino acid sequence:
NSARDSLNTAIQAWQQNKCPEVEELVFSHFVICNDTQETLRFGQVDTDENILLA SLHSHQYSWRSHKSPQ LLHICIEGWGNWRWSEPFSVDHAGTFIRTIQYRGR TASLIIKVQQLNGVQKQIIICGRQIICSYLSQSIE LKVVQHYIGQDGQAVVREHFD CLTAKQKLPSYILENNELTELCVKAKGDEDWSRDVCLESKAPEYSIVIQVPSS NSSIIYVWCTVLTLEPNSQVQQRMIVFSPLFIMRSHLPDPIIIHLEKRSLGLSETQII PGKGQEKP LQNIEPDLVHHLTFQA (SEQ ID NO:233), NKCPEVEELVFSHF VICNDTQETLRF (SEQ ID NO:234), HICIEGWGNWRWSEPFSVDHAGTFI (SEQ ID NO:235), VVREHFDCLTAKQKLPSYILENNELTE (SEQ ID NO:236), EDWSRD VCLESKAPEYSIVIQVPSSNS (SEQ ID NO:237), and/or IIHLEKRSLGLSETQII PGKGQEKPLQ (SEQ ID NO:238). Polynucleotides encoding these polypeptides are also encompassed by the invention. The gene encoding the disclosed cDNA is believed to reside on chromosome 8. Accordingly, polynucleotides related to this invention are useful as a marker in linkage analysis for chromosome 8.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, disorders of the immune system, particularly immunodefiencies. such as AIDS. Similarly, polypeptides and antibodies directed to these polypeptides are useful in providing immunological probes for differential identification of the tissue(s) or cell type(s). For a number of disorders of the above tissues or cells, particularly of for those of the immune system, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 140 as residues: Met-1 to Gly-8, Thr-33 to Cys-38, Arg-79 to Arg-89.

The tissue distribution in immune cells indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of hematopoietic cell lineages, including blood stem cells. This gene product may be involved in the regulation of cytokine production. antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 876 of SEQ ID NO:38, b is an integer of 15 to 890, where both a and b correspond to the positions of nucleotide residues shown in SEQ ID NO:38, and where the b is greater than or equal to a + 14.

### FEATURES OF PROTEIN ENCODED BY GENE NO: 29

It has been discovered that the translation product of this gene shares homology to a conserved Caenorhabditis elegans protein (See Genbank Accession No gi|577546).In specific embodiments, polypeptides of the invention comprise the following amino acid sequence: LIIQDQTRRCHGLWHLPSLLWPLLWSSGTGLC RNVCRLHGIYHXVLXRVGHAYQTSFRQXVCXXWAADLCGRHEEGIIENTYRL SCNHVFHEFCIRGWCIVGKKQTCPYCKEKVDLKRMFSNPWERPHVM YGQLLDWLRYLVAWQPVIIGVVQGINYILG LE (SEQ ID NO:239), and/or TAFVTFRATRKPLVQTTPRLVYKWFLLIYKISYATGIVGYMAVMFTLFGLNLLF KIKPEDAMDFGISLLFYGLYYGVLERDFAEMCADYMASTIXFXSESGMPT KHLSDSXCAXCGQQIFVDVMKRGSLRTRIGCPAIMSSTSSASVAGASWER SKRVPTAKRR (SEQ ID NO:240). Polynucleotides encoding these polypeptides are also encompassed by the invention.

This gene is expressed primarily in embryonic brain.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly mental retardation of various types, seizures, and mood disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g.neural, developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 141 as residues: Ser-22 to Met-28.

The tissue distribution in neural tissue indicates that polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Alternatively, expression within embryonic tissue indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:39 and may have been publicly available prior to conception of the present invention. Preferably. such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1056 of SEQ ID NO:39, b is an integer of 15 to 1070, where both a and b correspond to the positions of nucleotide residues shown in SEQ ID NO:39, and where the b is greater than or equal to a + 14.

### FEATURES OF PROTEIN ENCODED BY GENE NO: 30

It is likely that the sequence of this polunucleotide continues upstream of the preferred signal peptide. In specific embodiments, polypeptides of the invention comprise the following amino acid sequence:
ATSMKRLSHPSICRTGLPLSQQKRASLL (SEQ ID NO:241). Polynucleotides encoding these polypeptides are also encompassed by the invention. When tested against Jurket cell lines, supernatants removed from cells containing this gene activated NF-kB (Nuclear Factor kB). Thus, it is likely that this gene activates immune cells through various signal transduction pathways. NF-kB is a transcription factor activated by a wide variety of agents, leading to cell activation, differentiation, or apoptosis. Reporter constructs utilizing the NF-kB promoter element are used to screen supernatants for such activity.

This gene is expressed primarily in early stage human embryos.

Therefore, polynucleotides and polypeptides of the invention are useful as reagents for differential identification of the tissue(s) or cell type(s) present in a biological sample and for diagnosis of diseases and conditions which include, but are not limited to, developmental disorders, particularly various types of birth defects and congenital conditions. Similarly, polypeptides and antibodies directed to these polypeptides are useful in providing immunological probes for differential identification of the tissue(s) or cell type(s). For a number of disorders of the above tissues or cells, particularly for those of the developing embryo, expression of this gene at significantly higher or lower levels may be routinely detected in certain developing and, ultimately, adult, tissues or cell types (e.g.developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution within embryonic tissue combined with the detected NF-kB biological activity indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 758 of SEQ ID NO:40, b is an integer of 15 to 772, where both a and b correspond to the positions of nucleotide residues shown in SEQ ID NO:40, and where the b is greater than or equal to a + 14.

### FEATURES OF PROTEIN ENCODED BY GENE NO: 31

This gene is expressed primarily in breast.

Therefore, polynucleotides and polypeptides of the invention are useful as reagents for differential identification of the tissue(s) or cell type(s) present in a biological sample and for diagnosis of breast cancer and related disorders and disease. Similarly, polypeptides and antibodies directed to these polypeptides are useful in providing immunological probes for differential identification of the tissue(s) or cell type(s). For a number of disorders of the above tissues or cells, particularly of the breast lymphatic system, expression of this gene at significantly higher or lower levels may be routinely detected in certain tissues or cell types (e.g.breast, reproductive, endocrine, and cancerous and wounded tissues) or bodily fluids (e.g.lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 143 as residues: Lys-27 to Arg-41.

The tissue distribution in breast tissue indicates that the protein product of this gene may be useful for the detection, treatment, and/or prevention of disorders of the breast or reproductive tissue, particularly cancer. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 773 of SEQ ID NO:41, b is an integer of 15 to 787, where both a and b correspond to the positions of nucleotide residues shown in SEQ ID NO:41, and where the b is greater than or equal to a + 14.

### FEATURES OF PROTEIN ENCODED BY GENE NO: 32

This gene is expressed primarily in osteosarcoma.

Therefore, polynucleotides and polypeptides of the invention are useful as reagents for differential identification of the tissue(s) or cell type(s) present in a biological sample and for diagnosis of various skeletal disorders, paricularly of osteosarcoma and related disorders. Similarly, polypeptides and antibodies directed to these polypeptides are useful in providing immunological probes for differential identification of the tissue(s) or cell type(s). For a number of disorders of the above tissues or cells, particularly of the skeletal and immune systems, expression of this gene at significantly higher or lower levels may be routinely detected in certain tissues or cell types (e.g.immune, skeletal, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:144 as residues: Trp-25 to Pro-33, Gln-88 to Pro-93.

The tissue distribution in skeletal tissue indicates that polynucleotides and polypeptides corresponding to this gene are useful for diagnosis and treatment of a variety of skeletal disorders, such as osteosarcoma. Similarly, the expression of this gene product in osteo tissue would suggest a role in the detection and treatment of disorders and conditions affecting the skeletal system, in particular osteoporosis, bone cancer, as well as, disorders afflicting connective tissues (e.g. arthritis, trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:42 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 638 of SEQ ID NO:42, b is an integer of 15 to 652, where both a and b correspond to the positions of nucleotide residues shown in SEQ ID NO:42, and where the b is greater than or equal to a + 14.

### FEATURES OF PROTEIN ENCODED BY GENE NO: 33

The gene encoding the disclosed cDNA is believed to reside on chromosome 10. Accordingly, polynucleotides related to this invention are useful as a marker in linkage analysis for chromosome 10.

This gene is expressed primarily in microvascular endothelial cells and in fetal liver cells.

Therefore, polynucleotides and polypeptides of the invention are useful as reagents for differential identification of the tissue(s) or cell type(s) present in a biological sample and for diagnosis of diseases and conditions which include, but are not limited to, cardiovascular, hematopoetic, immunological, or developmental disorders. Similarly, polypeptides and antibodies directed to these polypeptides are useful in providing immunological probes for differential identification of the tissue(s) or cell type(s). For a number of disorders of the above tissues or cells, particularly of the hematopoietic system, expression of this gene at significantly higher or lower levels may be routinely detected in certain tissues or cell types (e.g.cardiovascular, hematopoietic, immune, developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal liver indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Alternatively, expression within vascular tissue indicates that polynucleotides and polypeptides corresponding to this gene are useful for the treatment, diagnosis, and/or prevention of a variety of vascular disorders, particularly cardiovascular disease, atherosclerosis, microvascular disease. stroke, embolism, or aneurysm.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:43 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1506 of SEQ ID NO:43, b is an integer of 15 to 1520, where both a and b correspond to the positions of nucleotide residues shown in SEQ ID NO:43, and where the b is greater than or equal to a + 14.

### FEATURES OF PROTEIN ENCODED BY GENE NO: 34

When tested against PC 12 cell lines, supernatants removed from cells containing this gene activated the EGR1 (early growth response gene 1) promoter element. Thus, it is likely that this gene activates sensory neuron cells through the EGR1 signal transduction pathway. EGR1 is a separate signal transduction pathway from Jak-STAT, genes containing the EGR1 promoter are induced in various tissues and cell types upon activation, leading the cells to undergo differentiation and proliferation.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune system disorders, particularly inflammatory disorders such as arthritis and related conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:146 as residues: Pro-18 to Glu-25.

The tissue distribution in immune cells combined with the detected EGR1 biological activity indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:44 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 782 of SEQ ID NO:44, b is an integer of 15 to 796, where both a and b correspond to the positions of nucleotide residues shown in SEQ ID NO:44, and where the b is greater than or equal to a + 14.

### FEATURES OF PROTEIN ENCODED BY GENE NO: 35

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly mental retardation, mood disorders, epilepsy, learning disorders, and dementia. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g.neural, cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neural tissue indicates that polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:45 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1364 of SEQ ID NO:45, b is an integer of 15 to 1378, where both a and b correspond to the positions of nucleotide residues shown in SEQ ID NO:45, and where the b is greater than or equal to a + 14.

### FEATURES OF PROTEIN ENCODED BY GENE NO: 36

This gene is expressed in stage B2 prostate cancer.

Therefore, polynucleotides and polypeptides of the invention are useful as reagents for differential identification of the tissue(s) or cell type(s) present in a biological sample and for diagnosis of diseases and conditions which include, but are not limited to, reproductive disorders, particularly proliferative disorders of the prostate including benigh prostatic hypertrophy. Similarly, polypeptides and antibodies directed to these polypeptides are useful in providing immunological probes for differential identification of the tissue(s) or cell type(s). For a number of disorders of the above tissues or cells, particularly of the glandular or reproductive systems, expression of this gene at significantly higher or lower levels may be routinely detected in certain tissues or cell types (e.g.reproductive, prostate, and cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in proliferate tissues indicates that polynucleotides and polypeptides corresponding to this gene are useful for the diagnosis, and/or treating prostate disease including prostate cancer, or other reproductive conditions such as male infertility. Similarly,expression within cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:46 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 583 of SEQ ID NO:46, b is an integer of 15 to 597, where both a and b correspond to the positions of nucleotide residues shown in SEQ ID NO:46, and where the b is greater than or equal to a + 14.

### FEATURES OF PROTEIN ENCODED BY GENE NO: 37

When tested against U937 cell lines, supernatants removed from cells containing this gene activated the GAS (gamma activating sequence) promoter element. Thus, it is likely that this gene activates myeloid cells through the Jak-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
MIILSCCSLWIYDYLIHPVPSVGHRVCLCCLPESATGRISPLGEGPRKWHGLRR SPEHISLGGLLLSSRLMAFCNLSRAVLPGNRTMETETYQLWASQYQRKWVSRS LSQVQCLRL (SEQ ID NO:242). Polynucleotides encoding these polypeptides are also encompassed by the invention.

This gene is expressed primarily in colorectal tumors.

Therefore, polynucleotides and polypeptides of the invention are useful as reagents for differential identification of the tissue(s) or cell type(s) present in a biological sample and for diagnosis of diseases and conditions which include, but are not limited to, cancers of the colon, rectum or gastrointestinal tract. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues or cell types (e.g.gastrointesinal, and cancerous and wounded tissues) or bodily fluids (e.g.lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:149 as residues: Phe-48 to Cys-54.

The tissue distribution in colorectal tumors indicates that polynucleotides and polypeptides corresponding to this gene are useful for the treatment or diagnosis of tumors of the gastrointestinal tract, particularly of the colon or rectum.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:47 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 586 of SEQ ID NO:47, b is an integer of 15 to 600, where both a and b correspond to the positions of nucleotide residues shown in SEQ ID NO:47. and where the b is greater than or equal to a + 14.

### FEATURES OF PROTEIN ENCODED BY GENE NO: 38

It is likely that the sequence of this polunucleotide continues upstream of the preferred signal peptide. In specific embodiments, polypeptides of the invention comprise the following amino acid sequence:
WIPRAAGIRHEHLSTLDRSVIWSKSILNARCKICRKKGDAENMVLCDGC DRGHHTYCVRPKLKTVPEGDWFCPECRPKQRSRRLSSRQRPSLESDEDVEDSM GGEDDEVDGDEEEGQSE EEEYEVEQXEDDSXEEXEVRXVLXCNKMSQ (SEQ ID NO:243) and/orMRVARYVERKA (SEQ ID NO:244). Polynucleotides encoding these polypeptides are also encompassed by the invention.

This gene is expressed primarily in serum treated smooth muscle.

Therefore, polynucleotides and polypeptides of the invention are useful as reagents for differential identification of the tissue(s) or cell type(s) present in a biological sample and for diagnosis of diseases and conditions which include, but are not limited to, neuromuscular or vascular diseases, such as restenosis stroke, aneurysm, or atherosclerosis. Similarly, polypeptides and antibodies directed to these polypeptides are useful in providing immunological probes for differential identification of the tissue(s) or cell type(s). For a number of disorders of the above tissues or cells, particularly of the muscular and vascular sytems, expression of this gene at significantly higher or lower levels may be routinely detected in certain tissues or cell types (e.g.vascular tissue, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:150 as residues: Ser-46 to Trp-54, Lys-76 to Arg-86.

The tissue distribution in smooth muscle indicates that polynucleotides and polypeptides corresponding to this gene are useful for treating restenosis or muscular responses due to degenerative conditions or injury. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:48 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 897 of SEQ ID NO:48, b is an integer of 15 to 911, where both a and b correspond to the positions of nucleotide residues shown in SEQ ID NO:48, and where the b is greater than or equal to a + 14.

### FEATURES OF PROTEIN ENCODED BY GENE NO: 39

When tested against dermal fibroblast cell lines, supernatants removed from cells containing this gene activated the EGR1 (early growth response gene 1) promoter element. Thus, it is likely that this gene activates fibroblast cells through the EGR1 signal transduction pathway. EGR1 is a separate signal transduction pathway from Jak-STAT, genes containing the EGR1 promoter are induced in various tissues and cell types upon activation, leading the cells to undergo differentiation and proliferation.The gene encoding the disclosed cDNA is believed to reside on chromosome 3. Accordingly, polynucleotides related to this invention are useful as a marker in linkage analysis for chromosome 3.

This gene is expressed in primary dendritic cells, and to a lesser extent, in human amygdala.

Therefore, polynucleotides and polypeptides of the invention are useful as reagents for diagnosis of diseases and conditions which include, but are not limited to, immune or neural disorders. Similarly, polypeptides and antibodies directed to these polypeptides are useful to detect a number of disorders of the above tissues or cells, particularly of the vascular or neural system. Expression of this gene at significantly higher or lower levels may be routinely detected in certain tissues or cell types (e.g.immune, hematopoietic, neural, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 151 as residues: Glu-30 to Gln-42.

The tissue distribution in primary dendritic cells indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Alternatively, expression within the human amygdala indicates the the protein product of this gene may be useful for the treatment and/or diagnosis of a variety of neural disorders, particularly those involving processesing of sensory information, including endocrine disorders as they relate to neural dysfunction. Protein, as well as, antibodies directed against the protein may show utility as a tumor marker and/or immunotherapy targets for the above listed tissues.. Many polynucleotide sequences, such as EST sequences, are publicly available and accessible through sequence databases. Some of these sequences are related to SEQ ID NO:49 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1849 of SEQ ID NO:49, b is an integer of 15 to 1863, where both a and b correspond to the positions of nucleotide residues shown in SEQ ID NO:49, and where the b is greater than or equal to a + 14.

### FEATURES OF PROTEIN ENCODED BY GENE NO: 40

The translation product of this gene shares sequence homology with the human rtvp-1 and glioma pathogenesis protein which are both glioma- specific proteins thought to be important in regulating the activity of extracellular proteases (See Genbank Accession No.gi|1030053 and gi|847722, respectively).In specific embodiments, polypeptides of the invention comprise the following amino acid sequence:
QRWLKHGANQCKFEHNDCLDKSYKCYAAXEXVGENIWLGGIKSFTPRHAITA WYNETQFYDFDSLSCSRV CGHYTQLVWANSFYVGXAXAMCPNLGGASTAI FVCNYGPAGNFANMPPYVRGESCSLCSKEEKCVKNLCKNPFLKPTGRAPQQ TAFNPXQLRFSSSENLLMSFIYKRNSQMLK (SEQ ID NO:245). Polynucleotides encoding these polypeptides are also encompassed by the invention.

This gene is expressed primarily in testes.

Therefore, polynucleotides and polypeptides of the invention are useful as reagents for differential identification of the tissue(s) or cell type(s) present in a biological sample and for diagnosis of diseases and conditions which include, but are not limited to, reproductive disorders, particular those disorders where proteases are thought to regulate the levels of secreted proteins including growth factors. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g.reproductive, testes, and cancerous and wounded tissues) or bodily fluids (e.g.lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 152 as residues: Glu-43 to Asn-49.

The tissue distribution in testes combined with the homology to two conserved glioma-specific proteins indicates that polynucleotides and polypeptides corresponding to this gene are useful for treating diseases of the reproductive system or diseases associated with increased degradation of secreted proteins or growth factors.The secreted protein can also be used to determine biological activity, to raise antibodies, as tissue markers, to isolate cognate ligands or receptors, to identify agents that modulate their interactions and as nutritional supplements. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); anti-inflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:50 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 796 of SEQ ID NO:50, b is an integer of 15 to 810, where both a and b correspond to the positions of nucleotide residues shown in SEQ ID NO:50, and where the b is greater than or equal to a + 14.

### FEATURES OF PROTEIN ENCODED BY GENE NO: 41

It is likely that the sequence of this polunucleotide continues upstream of the preferred signal peptide. In specific embodiments, polypeptides of the invention comprise the following amino acid sequence:
TEGGCALVPNDMESLKQKLVRVLEENLILSEKIQQLEEGAAISIVSGQQSHTYD DLLHKNQQLTMQVACLNQELAQLKKLEKTVAILHESQRSLVVTNEYLL QQLNKEPKGYSGKALLPPEKGHHLGRSSPFGKSTLSSSSPVAHETGQYLIQSV LDAAPEPGL (SEQ ID NO:246) and/or SMVSK (SEQ ID NO:247). Polynucleotides encoding these polypeptides are also encompassed by the invention.The gene encoding the disclosed cDNA is believed to reside on chromosome 16. Accordingly, polynucleotides related to this invention are useful as a marker in linkage analysis for chromosome 16.

This gene is expressed primarily in lung and testes.

Therefore, polynucleotides and polypeptides of the invention are useful as reagents for differential identification of the tissue(s) or cell type(s) present in a biological sample and for diagnosis of diseases and conditions which include, but are not limited to, pulmonary or reproductive diseases such as adult respiratory distress syndrome (ARDS), pulmonary fibrositis or cystic fibrosis, or male infertility. Similarly, polypeptides and antibodies directed to these polypeptides are useful in providing immunological probes for differential identification of the tissue(s) or cell type(s). For a number of disorders of the above tissues or cells, particularly of the respiratory system, expression of this gene at significantly higher or lower levels may be routinely detected in certain tissues or cell types (e.g.reproductive, pulmonary, and cancerous and wounded tissues) or bodily fluids (e.g.lymph, pulmonary surfactant or sputum, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 153 as residues: Ser-36 to Trp-41, Pro-53 to Arg-58.

The tissue distribution in lung tissue indicates that polynucleotides and polypeptides corresponding to this gene are useful for treating disorders of the lung such as pulmonary fibrosis. cystic fibrosis or acute respiratory distress syndrome. Alternatively, the protien product of this gene may also be useful for the treatment and/or diagnosis of a variety of reproductive disorders, particularly male infertility or impotence, including disorders associated with testosterone regulation and secretion.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:51 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 942 of SEQ ID NO:51, b is an integer of 15 to 956, where both a and b correspond to the positions of nucleotide residues shown in SEQ ID NO:51, and where the b is greater than or equal to a + 14.

### FEATURES OF PROTEIN ENCODED BY GENE NO: 42

The translation product of this gene shares sequence homology with metallothioneins which are thought to be important in binding zinc and protecting cells from degeneration.

This gene is expressed primarily in the thyroid.

Therefore, polynucleotides and polypeptides of the invention are useful as reagents for differential identification of the tissue(s) or cell type(s) present in a biological sample and for diagnosis of diseases and conditions which include, but are not limited to, endocrine disorders, particularly hypothyroidism. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g.endocrine, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in endocrine tissue combined with the homology to metallothioneins indicates that polynucleotides and polypeptides corresponding to this gene are useful for treating disorders of the thyroid gland.Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:52 and may have been publicly available prior to conception of the present invention. Preferably. such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 286 of SEQ ID NO:52, b is an integer of 15 to 300, where both a and b correspond to the positions of nucleotide residues shown in SEQ ID NO:52, and where the b is greater than or equal to a + 14.

### FEATURES OF PROTEIN ENCODED BY GENE NO: 43

It is likely that the sequence of this polunucleotide continues upstream of the preferred signal peptide. In specific embodiments, polypeptides of the invention comprise the following amino acid sequence:
NTDWDQTVLIVLRISSTLPVALLRDEVPGWFLKXPEPQLISKELIMLTEV (SEQ ID NO:248). Polynucleotides encoding these polypeptides are also encompassed by the invention.

This gene is expressed primarily in retinoic acid treated HL60 cells

Therefore, polynucleotides and polypeptides of the invention are useful as reagents for differential identification of the tissue(s) or cell type(s) present in a biological sample and for diagnosis of diseases and conditions which include, but are not limited to, immune disorders, particularly in the modulation of the immune response to infectious agents, or for acute or chronic inflammatory responses. Similarly, polypeptides and antibodies directed to these polypeptides are useful in providing immunological probes for differential identification of the tissue(s) or cell type(s). For example, in a number of disorders of the above tissues or cells, particularly of the immune system, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:155 as residues: Pro-42 to Ser-50, Leu-52 to Phe-58, Pro-61 to Gly-73, Pro-76 to Gln-84.

The tissue distribution in HL60 cells indicates that polynucleotides and polypeptides corresponding to this gene are useful for modulating the immune response to an acute or chronic inflammation or to an infection.The secreted protein can also be used to determine biological activity, to raise antibodies, as tissue markers, to isolate cognate ligands or receptors, to identify agents that modulate their interactions and as nutritional supplements. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); anti-inflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:53 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 827 of SEQ ID NO:53, b is an integer of 15 to 841, where both a and b correspond to the positions of nucleotide residues shown in SEQ ID NO:53, and where the b is greater than or equal to a + 14.

### FEATURES OF PROTEIN ENCODED BY GENE NO: 44

This gene is expressed primarily in B-cell lymphoma .

Therefore, polynucleotides and polypeptides of the invention are useful as reagents for differential identification of the tissue(s) or cell type(s) present in a biological sample and for diagnosis of diseases and conditions which include, but are not limited to, immune disorders, such as proliferative compositions of the blood. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:156 as residues: Pro-38 to Asp-47, Ser-64 to Asn-71.

The tissue distribution in immune tissue indicates that polynucleotides and polypeptides corresponding to this gene are useful for diagnosing and treating tumors of the blood including B-Cell lymphomas. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:54 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 620 of SEQ ID NO:54, b is an integer of 15 to 634, where both a and b correspond to the positions of nucleotide residues shown in SEQ ID NO:54, and where the b is greater than or equal to a + 14.

### FEATURES OF PROTEIN ENCODED BY GENE NO: 45

This gene is expressed primarily in cerebellum, and to a lesser extent, in other tissues.

Therefore, polynucleotides and polypeptides of the invention are useful as reagents for differential identification of the tissue(s) or cell type(s) present in a biological sample and for diagnosis of neuronal disorders. Similarly, polypeptides and antibodies directed to these polypeptides are useful in providing immunological probes for differential identification of the tissue(s) or cell type(s). For a number of disorders of the above tissues or cells, particularly of the cerebellum, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 157 as residues: Cys-56 to Ser-63, Met-67 to Leu-73.

The tissue distribution in neural tissue indicates that polynucleotides and polypeptides corresponding to this gene are useful for diagnosis and treatment of neuronal disorders. The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and preception. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:55 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 849 of SEQ ID NO:55, b is an integer of 15 to 863, where both a and b correspond to the positions of nucleotide residues shown in SEQ ID NO:55, and where the b is greater than or equal to a + 14.

### FEATURES OF PROTEIN ENCODED BY GENE NO: 46

The gene encoding the disclosed cDNA is thought to reside on chromosome 14. Accordingly, polynucleotides related to this invention are useful as a marker in linkage analysis for chromosome 14.

This gene is expressed primarily in colon, and to a lesser extent, in other tissues.

Therefore, polynucleotides and polypeptides of the invention are useful as reagents for differential identification of the tissue(s) or cell type(s) present in a biological sample and for diagnosis of gastrointestinal disorders, particularly colon diseases, such ascolon cancer. Similarly, polypeptides and antibodies directed to these polypeptides are useful in providing immunological probes for differential identification of the tissue(s) or cell type(s). For a number of disorders of the above tissues or cells, particularly of the colon, expression of this gene at significantly higher or lower levels may be routinely detected in certain tissues or cell types (e.g., cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 158 as residues: Pro-26 to Asn-32.

The tissue distribution in colon tissues indicates that polynucleotides and polypeptides corresponding to this gene are useful for diagnosis and treatment of colon-related diseases. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:56 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 698 of SEQ ID NO:56, b is an integer of 15 to 712, where both a and b correspond to the positions of nucleotide residues shown in SEQ ID NO:56, and where the b is greater than or equal to a + 14.

### FEATURES OF PROTEIN ENCODED BY GENE NO: 47

This gene is expressed primarily in number of tumor tissues such as chondrosarcoma, synovial sarcoma, and to a lesser extent, in activated monocytes and T cells.

Therefore, polynucleotides and polypeptides of the invention are useful as reagents for differential identification of the tissue(s) or cell type(s) present in a biological sample and for diagnosis of tumorigenesis and hemapoietic disorders. Similarly, polypeptides and antibodies directed to these polypeptides are useful in providing immunological probes for differential identification of the tissue(s) or cell type(s). For a number of disorders of the above tissues or cells, particularly tumors and other proliferate tissues, expression of this gene at significantly higher or lower levels may be routinely detected in certain tissues or cell types (e.g., skeletal, chondrocytes, fibroid, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in proliferative tissues indicates that polynucleotides and polypeptides corresponding to this gene are useful for diagnosis and treatment of cell growth related disorders such as tumorigenesis and hemapoietic diseases. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:57 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 911 of SEQ ID NO:57, b is an integer of 15 to 925, where both a and b correspond to the positions of nucleotide residues shown in SEQ ID NO:57, and where the b is greater than or equal to a + 14.

### FEATURES OF PROTEIN ENCODED BY GENE NO: 48

This gene is expressed primarily in breast tissue and to a lesser extent in other tissues.

Therefore, polynucleotides and polypeptides of the invention are useful as reagents for differential identification of the tissue(s) or cell type(s) present in a biological sample and for diagnosis of breast diseases such as breast cancer. Similarly, polypeptides and antibodies directed to these polypeptides are useful in providing immunological probes for differential identification of the tissue(s) or cell type(s). For a number of disorders of the above tissues or cells, particularly of the breast, expression of this gene at significantly higher or lower levels may be routinely detected in certain tissues or cell types (e.g. breast, cancerous and wounded tissues) or bodily fluids (e.g.. lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in breast tissue indicates that polynucleotides and polypeptides corresponding to this gene are useful for diagnosis and treatment of breast disorders such as breast cancer. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:58 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 587 of SEQ ID NO:58, b is an integer of 15 to 601, where both a and b correspond to the positions of nucleotide residues shown in SEQ ID NO:58, and where the b is greater than or equal to a + 14.

### FEATURES OF PROTEIN ENCODED BY GENE NO: 49

When tested against Jurkat T-cell lines, supernatants removed from cells containing this gene activated the NF-kB assay. Thus, it is likely that this gene initiates cellular activation, differentiation, or apoptosis, as demonstrated by the NF-kB assay results. NF-kB (Nuclear factor kB) is a transcription factor activated by a wide variety of agents, leading to cell activation, differentiation, or apoptosis. Reporter constructs utilizing the NF-kB promoter element are used to screen supernatants for such activity.

This gene is expressed primarily in chondrosarcoma, and to a lesser extent, in other tissues.

Therefore, polynucleotides and polypeptides of the invention are useful as reagents for differential identification of the tissue(s) or cell type(s) present in a biological sample and for diagnosis of chondrosarcoma. Similarly, polypeptides and antibodies directed to these polypeptides are useful in providing immunological probes for differential identification of the tissue(s) or cell type(s). For a number of disorders of the above tissues or cells, particularly chondrosarcoma, expression of this gene at significantly higher or lower levels may be routinely detected in certain tissues or cell types (e.g., chondrocytes. fibroid, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of chondrosarcoma. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:59 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 716 of SEQ ID NO:59, b is an integer of 15 to 730, where both a and b correspond to the positions of nucleotide residues shown in SEQ ID NO:59, and where the b is greater than or equal to a + 14.

### FEATURES OF PROTEIN ENCODED BY GENE NO: 50

This gene is expressed primarily in human embryo and to a lesser extent in other tissues.

Therefore, polynucleotides and polypeptides of the invention are useful as reagents for differential identification of the tissue(s) or cell type(s) present in a biological sample and for diagnosis of diseases and conditions which include, but are not limited to, embryonic or development disorders. Similarly, polypeptides and antibodies directed to these polypeptides are useful in providing immunological probes for differential identification of the tissue(s) or cell type(s). For a number of disorders of the above tissues or cells, particularly of the embryo, expression of this gene at significantly higher or lower levels may be routinely detected in certain tissues or cell types (e.g. embryonic, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in developing tissue indicates that polynucleotides and polypeptides corresponding to this gene are useful for diagnosis and treatment of embryonic development disorders. Embryonic development also involves decisions involving cell differentiation and/or apoptosis in pattern formation. Thus this protein may also be involved in apoptosis or tissue differentiation and could be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:60 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 832 of SEQ ID NO:60, b is an integer of 15 to 846, where both a and b correspond to the positions of nucleotide residues shown in SEQ ID NO:60, and where the b is greater than or equal to a + 14.

### FEATURES OF PROTEIN ENCODED BY GENE NO: 51

The gene encoding the disclosed cDNA is thought to reside on chromosome 9. Accordingly, polynucleotides related to this invention are useful as a marker in linkage analysis for chromosome 9.

This gene is expressed primarily in neuronal tissues, fetal tissues, and a number of cancer tissues and to a lesser extent in some other tissues.

Therefore, polynucleotides and polypeptides of the invention are useful as reagents for differential identification of the tissue(s) or cell type(s) present in a biological sample and for diagnosis of diseases and conditions which include, but are not limited to, neuronal or early developmental disorders, and tumorigenesis. Similarly, polypeptides and antibodies directed to these polypeptides are useful in providing immunological probes for differential identification of the tissue(s) or cell type(s). For a number of disorders of the above tissues or cells, particularly of neuronal tissues, fetal tissues, and some cancer tissues, expression of this gene at significantly higher or lower levels may be routinely detected in certain tissues or cell types (e.g.fetal tissues, brain, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:163 as residues: Met-1 to Ser-6, Gln-59 to Gly-67.

The tissue distribution in neural and fetal tissues indicates that polynucleotides and polypeptides corresponding to this gene are useful for diagnosis and treatment of neuronal disorders, early developmental disorders, and tumorigenesis. Embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:61 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 944 of SEQ IID NO:61, b is an integer of 15 to 958, where both a and b correspond to the positions of nucleotide residues shown in SEQ ID NO:61, and where the b is greater than or equal to a + 14.

### FEATURES OF PROTEIN ENCODED BY GENE NO: 52

This gene is expressed primarily in fetal brain and to a lesser extent in other tissues.

Therefore, polynucleotides and polypeptides of the invention are useful as reagents for differential identification of the tissue(s) or cell type(s) present in a biological sample and for diagnosis of neuronal development disorders. Similarly, polypeptides and antibodies directed to these polypeptides are useful in providing immunological probes for differential identification of the tissue(s) or cell type(s). For a number of disorders of the above tissues or cells, particularly of the fetal brain, expression of this gene at significantly higher or lower levels may be routinely detected in certain tissues or cell types (e.g. brain, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:164 as residues: Ser-25 to Tyr-35.

The tissue distribution in fetal brain indicates that polynucleotides and polypeptides corresponding to this gene are useful for the diagnosis and treatment of neuronal development disorders, fetal deficiencies, and pre-natal disorders. Expression within embryonic tissue and other cellular sources marked by proliferating cells indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:62 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 568 of SEQ ID NO:62, b is an integer of 15 to 582, where both a and b correspond to the positions of nucleotide residues shown in SEQ ID NO:62, and where the b is greater than or equal to a + 14.

### FEATURES OF PROTEIN ENCODED BY GENE NO: 53

When tested against both U937 myeloid and Jurkat T-cell cell lines, supernatants removed from cells containing this gene activated the GAS assay. Thus, it is likely that this gene activates both myeloid cells and T-cells through the Jak-STAT signal transduction pathway. GAS (gamma activating sequence) is a a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells.

This gene is expressed primarily in brain frontal cortex.

Therefore, polynucleotides and polypeptides of the invention are useful as reagents for differential identification of the tissue(s) or cell type(s) present in a biological sample and for diagnosis of neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 165 as residues: Gly-36 to Arg-43, Glu-50 to Glu-58.

The tissue distribution in frontal cortex indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and perception. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:63 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 738 of SEQ ID NO:63, b is an integer of 15 to 752, where both a and b correspond to the positions of nucleotide residues shown in SEQ ID NO:63, and where the b is greater than or equal to a + 14.

### FEATURES OF PROTEIN ENCODED BY GENE NO: 54

This gene is expressed primarily in the endometrium, and to a lesser extent, in other tissues.

Therefore, polynucleotides and polypeptides of the invention are useful as reagents for differential identification of the tissue(s) or cell type(s) present in a biological sample and for diagnosis of reproductive disorders and endometrial diseases such as endometrial tumors. Similarly, polypeptides and antibodies directed to these polypeptides are useful in providing immunological probes for differential identification of the tissue(s) or cell type(s). For a number of disorders of the above tissues or cells, particularly of the endometrium, expression of this gene at significantly higher or lower levels may be routinely detected in certain tissues or cell types (e.g. reproductive, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 166 as residues: Arg-7 to Ser-14, Pro-32 to Leu-39.

The tissue distribution in endometrium indicates that polynucleotides and polypeptides corresponding to this gene are useful for diagnosis and treatment of reproductive disorders, particularly endometrial diseases such as tumors or cancers of the endometrium. Given the tissue distribution, the protein product of this gene may also be useful in the treatment of reproductive disorders. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:64 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 692 of SEQ ID NO:64, b is an integer of 15 to 706, where both a and b correspond to the positions of nucleotide residues shown in SEQ ID NO:64, and where the b is greater than or equal to a + 14.

### FEATURES OF PROTEIN ENCODED BY GENE NO: 55

This gene is expressed primarily in activated T cells, and to a lesser extent, in other tissues.

Therefore, polynucleotides and polypeptides of the invention are useful as reagents for differential identification of the tissue(s) or cell type(s) present in a biological sample and for diagnosis of immune disorders. Similarly, polypeptides and antibodies directed to these polypeptides are useful in providing immunological probes for differential identification of the tissue(s) or cell type(s). For a number of disorders of the above tissues or cells, particularly of activated T-cells, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 167 as residues: Arg-35 to Gly-44.

The tissue distribution in T-cells indicates that polynucleotides and polypeptides corresponding to this gene are useful for diagnosis and treatment of immune disorders. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:65 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 386 of SEQ ID NO:65, b is an integer of 15 to 400, where both a and b correspond to the positions of nucleotide residues shown in SEQ ID NO:65, and where the b is greater than or equal to a + 14.

### FEATURES OF PROTEIN ENCODED BY GENE NO: 56

This gene is expressed primarily in skin.

Therefore, polynucleotides and polypeptides of the invention are useful as reagents for differential identification of the tissue(s) or cell type(s) present in a biological sample and for diagnosis of diseases and conditions relating to skin. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g. skin, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in integumentary tissue indicates that polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Bowen's disease, basal cell carcinoma, squamous cell carcinoma, malignant melanoma, Paget's disease, mycosis fungoides, and Kaposi's sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. Moreover, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Many polynucleotide sequences, such as EST sequences, are publicly available and accessible through sequence databases. Some of these sequences are related to SEQ ID NO:66 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 759 of SEQ ID NO:66, b is an integer of 15 to 773, where both a and b correspond to the positions of nucleotide residues shown in SEQ ID NO:66, and where the b is greater than or equal to a + 14.

### FEATURES OF PROTEIN ENCODED BY GENE NO: 57

This gene is expressed primarily in human fetal kidney.

Therefore, polynucleotides and polypeptides of the invention are useful as reagents for differential identification of the tissue(s) or cell type(s) present in a biological sample and for diagnosis of renal disorders. Similarly, polypeptides and antibodies directed to these polypeptides are useful in providing immunological probes for differential identification of the tissue(s) or cell type(s). For a number of disorders of the above tissues or cells, particularly of the urinary system, expression of this gene at significantly higher or lower levels may be routinely detected in certain tissues or cell types (e.g. developmental, renal, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal kidney indicates that this gene or gene product could be used in the treatment and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:67 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 633 of SEQ ID NO:67, b is an integer of 15 to 647, where both a and b correspond to the positions of nucleotide residues shown in SEQ ID NO:67, and where the b is greater than or equal to a + 14.

### FEATURES OF PROTEIN ENCODED BY GENE NO: 58

This gene is expressed primarily in human fetal dura mater.

Therefore, polynucleotides and polypeptides of the invention are useful as reagents for differential identification of the tissue(s) or cell type(s) present in a biological sample and for diagnosis of disorders related to central nervous system. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in dura mater indicates that polynucleotides and polypeptides corresponding to this gene are useful for the diagnosis and/or treatment of disorders of the brain and nervous system. Elevated expression of this gene product within the dura mater indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Many polynucleotide sequences, such as EST sequences, are publicly available and accessible through sequence databases. Some of these sequences are related to SEQ ID NO:68 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 661 of SEQ ID NO:68, b is an integer of 15 to 675, where both a and b correspond to the positions of nucleotide residues shown in SEQ ID NO:68, and where the b is greater than or equal to a + 14.

### FEATURES OF PROTEIN ENCODED BY GENE NO: 59

The translation product of this gene shares sequence homology with human beta-galactosidase (GLB1) mRNA. The gene encoding the disclosed cDNA is thought to reside on chromosome 3. Accordingly, polynucleotides related to this invention are useful as a marker in linkage analysis for chromosome 3.

This gene is expressed primarily in activated human neutrophil, and to a lesser extent in breast, kidney and gallbladder tissue.

Therefore, polynucleotides and polypeptides of the invention are useful as reagents for differential identification of the tissue(s) or cell type(s) present in a biological sample and for diagnosis of diseases and conditions which include, but are not limited to, immune, renal, metabolic or reproductive disorders, such as neutropenia and neutrophilia. Similarly, polypeptides and antibodies directed to these polypeptides are useful in providing immunological probes for differential identification of the tissue(s) or cell type(s). For a number of disorders of the above tissues or cells, particularly of the disorders relating to hemopoietic system, expression of this gene at significantly higher or lower levels may be routinely detected in certain tissues or cell types (e.g. immune, cancerous and wounded tissues) or bodily fluids (e.g., lymph, bile, breat milk. serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of immune disorders. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:69 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 875 of SEQ ID NO:69, b is an integer of 15 to 889, where both a and b correspond to the positions of nucleotide residues shown in SEQ ID NO:69, and where the b is greater than or equal to a + 14.

### FEATURES OF PROTEIN ENCODED BY GENE NO: 60

This gene is expressed primarily in human fetal kidney.

Therefore, polynucleotides and polypeptides of the invention are useful as reagents for differential identification of the tissue(s) or cell type(s) present in a biological sample and for diagnosis of renal disorders. Similarly, polypeptides and antibodies directed to these polypeptides are useful in providing immunological probes for differential identification of the tissue(s) or cell type(s). For a number of disorders of the above tissues or cells, particularly of the urinary system, expression of this gene at significantly higher or lower levels may be routinely detected in certain tissues or cell types (e.g. renal, developmental, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 172 as residues: Arg-27 to Asn-38, His-41 to Ser-54.

The tissue distribution in fetal kidney indicates that this gene or gene product could be used in the treatment and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:70 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 874 of SEQ ID NO:70, b is an integer of 15 to 888, where both a and b correspond to the positions of nucleotide residues shown in SEQ ID NO:70, and where the b is greater than or equal to a + 14.

### FEATURES OF PROTEIN ENCODED BY GENE NO: 61

This gene is expressed primarily in human frontal cortex of an epileptic person.

Therefore, polynucleotides and polypeptides of the invention are useful as reagents for differential identification of the tissue(s) or cell type(s) present in a biological sample and for diagnosis of epilepsy. Similarly, polypeptides and antibodies directed to these polypeptides are useful in providing immunological probes for differential identification of the tissue(s) or cell type(s). For a number of disorders of the above tissues or cells, particularly of the PNS and CNS, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in frontal cortex indicates that polynucleotides and polypeptides corresponding to this gene are useful for diagnosis and treatment of epilepsy. Furthermore, the tissue distribution indicates that polynucleotides and polypeptides corresponding to this gene are useful for the diagnosis and/or treatment of disorders of the brain and nervous system. Elevated expression of this gene product within the frontal cortex of the brain indicates that it may be involved in neuronal survival; synapse formation; conductance; neural differentiation. etc. Such involvement may impact many processes, such as learning and cognition. It may also be useful in the treatment of such neurodegenerative disorders as schizophrenia; ALS; or Alzheimer's. Many polynucleotide sequences, such as EST sequences, are publicly available and accessible through sequence databases. Some of these sequences are related to SEQ ID NO:71 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 782 of SEQ ID NO:71, b is an integer of 15 to 796, where both a and b correspond to the positions of nucleotide residues shown in SEQ ID NO:71, and where the b is greater than or equal to a + 14.

### FEATURES OF PROTEIN ENCODED BY GENE NO: 62

This gene is expressed primarily in human frontal cortex in a person with Schizophrenia.

Therefore, polynucleotides and polypeptides of the invention are useful as reagents for differential identification of the tissue(s) or cell type(s) present in a biological sample and for diagnosis of neural conditions, particularly schizophrenic disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph. serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 174 as residues: Pro-49 to Gly-54.

The tissue distribution in frontal cortex indicates that polynucleotides and polypeptides corresponding to this gene are useful for the diagnosis and/or treatment of disorders of the brain and nervous system. Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Many polynucleotide sequences, such as EST sequences, are publicly available and accessible through sequence databases. Some of these sequences are related to SEQ ID NO:72 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 518 of SEQ ID NO:72, b is an integer of 15 to 532, where both a and b correspond to the positions of nucleotide residues shown in SEQ ID NO:72, and where the b is greater than or equal to a + 14.

### FEATURES OF PROTEIN ENCODED BY GENE NO: 63

This gene is expressed primarily in hemangiopericytoma.

Therefore, polynucleotides and polypeptides of the invention are useful as reagents for differential identification of the tissue(s) or cell type(s) present in a biological sample and for diagnosis of diseases and conditions which include, but are not limited to, benign disorders related to pericytes and endothelium-lined vessels. Similarly, polypeptides and antibodies directed to these polypeptides are useful in providing immunological probes for differential identification of the tissue(s) or cell type(s). For a number of disorders of the above tissues or cells, particularly of the nonmalignant character of neoplasm relating to pericytes and endothelial vessels, expression of this gene at significantly higher or lower levels may be routinely detected in certain tissues or cell types (e.g. blood vessels, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of hemangiopericytoma. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:73 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 532 of SEQ ID NO:73, b is an integer of 15 to 546, where both a and b correspond to the positions of nucleotide residues shown in SEQ ID NO:73, and where the b is greater than or equal to a + 14.

### FEATURES OF PROTEIN ENCODED BY GENE NO: 64

This gene is expressed primarily in hemangiopericytoma, and to a lesser extent in human colon.

Therefore, polynucleotides and polypeptides of the invention are useful as reagents for differential identification of the tissue(s) or cell type(s) present in a biological sample and for diagnosis of diseases and conditions which include, but are not limited to, benign disorders related to pericytes and endothelium-lined vessels. Similarly, polypeptides and antibodies directed to these polypeptides are useful in providing immunological probes for differential identification of the tissue(s) or cell type(s). For a number of disorders of the above tissues or cells, particularly of the nonmalignant character of neoplasm relating to pericytes and endothelial vessels, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 176 as residues: Lys-39 to Glu-45.

The tissue distribution indicates that polynucleotides and polypeptides corresponding to this gene are useful for diagnosis and treatment of hemangiopericytoma. Protein, as well as. antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:74 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 701 of SEQ ID NO:74, b is an integer of 15 to 715, where both a and b correspond to the positions of nucleotide residues shown in SEQ ID NO:74, and where the b is greater than or equal to a + 14.

### FEATURES OF PROTEIN ENCODED BY GENE NO: 65

This gene is expressed primarily in glioblastoma, and to a lesser extent in B-cell lymphoma and anergic T-cells.

Therefore, polynucleotides and polypeptides of the invention are useful as reagents for differential identification of the tissue(s) or cell type(s) present in a biological sample and for diagnosis of diseases and conditions which include, but are not limited to, disorders related to neuroglial and ependymal cells, as well as the immune system, including tumors. Similarly, polypeptides and antibodies directed to these polypeptides are useful in providing immunological probes for differential identification of the tissue(s) or cell type(s). For a number of disorders of the above tissues or cells, particularly of the central nervous system or immune system, expression of this gene at significantly higher or lower levels may be routinely detected in certain tissues or cell types (e.g. brain, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in glioblastoma indicates that polynucleotides and polypeptides corresponding to this gene are useful for diagnosis and treatment of neural cell disorders. Furthermore, the tissue distribution indicates that the translation product of this gene is useful for the treatment and/or detection of tumors of the brain and immune system, such as glioblastomas and B-cell lymphomas. Many polynucleotide sequences, such as EST sequences, are publicly available and accessible through sequence databases. Some of these sequences are related to SEQ ID NO:75 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 392 of SEQ ID NO:75, b is an integer of 15 to 406, where both a and b correspond to the positions of nucleotide residues shown in SEQ ID NO:75, and where the b is greater than or equal to a + 14.

### FEATURES OF PROTEIN ENCODED BY GENE NO: 66

This gene is expressed primarily in skin.

Therefore, polynucleotides and polypeptides of the invention are useful as reagents for differential identification of the tissue(s) or cell type(s) present in a biological sample and for diagnosis of diseases and conditions relating to skin. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g. skin, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 178 as residues: Pro-27 to Pro-40.

The tissue distribution in integumentary tissue indicates that polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses. Bowen's disease, basal cell carcinoma, squamous cell carcinoma, malignant melanoma, Paget's disease, mycosis fungoides, and Kaposi's sarcoma), injuries and inflammation of the skin (i.e.wounds, rashes, prickly heat disorder, psoriasis, dermatitis), atherosclerosis. uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. Moreover, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Many polynucleotide sequences, such as EST sequences, are publicly available and accessible through sequence databases. Some of these sequences are related to SEQ ID NO:76 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 528 of SEQ ID NO:76, b is an integer of 15 to 542, where both a and b correspond to the positions of nucleotide residues shown in SEQ ID NO:76, and where the b is greater than or equal to a + 14.

### FEATURES OF PROTEIN ENCODED BY GENE NO: 67

This gene is expressed primarily in brain frontal cortex.

Therefore, polynucleotides and polypeptides of the invention are useful as reagents for differential identification of the tissue(s) or cell type(s) present in a biological sample and for diagnosis of diseases and conditions which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 179 as residues: Gly-27 to Pro-34, Tyr-59 to Arg-65.

The tissue distribution in frontal cortex indicates that polynucleotides and polypeptides corresponding to this gene are useful for the diagnosis and/or treatment of disorders of the brain and nervous system. Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Many polynucleotide sequences, such as EST sequences, are publicly available and accessible through sequence databases. Some of these sequences are related to SEQ ID NO:77 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 406 of SEQ ID NO:77, b is an integer of 15 to 420, where both a and b correspond to the positions of nucleotide residues shown in SEQ ID NO:77, and where the b is greater than or equal to a + 14.

### FEATURES OF PROTEIN ENCODED BY GENE NO: 68

This gene is expressed primarily in human frontal cortex of a person exhibiting Schizophrenia.

Therefore, polynucleotides and polypeptides of the invention are useful as reagents for differential identification of the tissue(s) or cell type(s) present in a biological sample and for diagnosis of neural conditions, particularly Schizophrenic disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in frontal cortex indicates that polynucleotides and polypeptides corresponding to this gene are useful for the diagnosis and/or treatment of disorders of the brain and nervous system. Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Many polynucleotide sequences, such as EST sequences, are publicly available and accessible through sequence databases. Some of these sequences are related to SEQ ID NO:78 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 451 of SEQ ID NO:78, b is an integer of 15 to 465, where both a and b correspond to the positions of nucleotide residues shown in SEQ ID NO:78, and where the b is greater than or equal to a + 14.

### FEATURES OF PROTEIN ENCODED BY GENE NO: 69

This gene is expressed primarily in glioblastoma.

Therefore, polynucleotides and polypeptides of the invention are useful as reagents for differential identification of the tissue(s) or cell type(s) present in a biological sample and for diagnosis of diseases and conditions which include, but are not limited to, disorders related to neuroglial and ependymal cells, including cancers. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in glioblastoma indicates that polynucleotides and polypeptides corresponding to this gene are useful for diagnosis and treatment of neural cell disorders. Furthermore, given the tissue distribution, the translation product of this gene may be useful for the intervention or detection of tumors of the brain, such as glioblastomas. Many polynucleotide sequences, such as EST sequences, are publicly available and accessible through sequence databases. Some of these sequences are related to SEQ ID NO:79 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 876 of SEQ ID NO:79, b is an integer of 15 to 890, where both a and b correspond to the positions of nucleotide residues shown in SEQ ID NO:79, and where the b is greater than or equal to a + 14.

### FEATURES OF PROTEIN ENCODED BY GENE NO: 70

This gene is expressed primarily in human fetal brain.

Therefore, polynucleotides and polypeptides of the invention are useful as reagents for differential identification of the tissue(s) or cell type(s) present in a biological sample and for diagnosis of diseases and conditions which include, but are not limited to, immune, growth, or neurologic disorders. Similarly, polypeptides and antibodies directed to these polypeptides are useful in providing immunological probes for differential identification of the tissue(s) or cell type(s). For a number of disorders of the above tissues or cells, particularly of the CNS and immune system, expression of this gene at significantly higher or lower levels may be routinely detected in certain tissues or cell types (e.g. brain, immune, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:182 as residues: Lys-13 to Asn-19, Asn-27 to Asn-35.

The tissue distribution in fetal brain indicates that polynucleotides and polypeptides corresponding to this gene are useful for the detection and treatment of disorders of the central nervous system and immune system. The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and perception. In addition, the gene or gene product may also play a role in the treatment and/or detection of developmental disorders associated with the developing embryo or sexually-linked disorders . Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:80 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 456 of SEQ ID NO:80, b is an integer of 15 to 470, where both a and b correspond to the positions of nucleotide residues shown in SEQ ID NO:80, and where the b is greater than or equal to a + 14.

### FEATURES OF PROTEIN ENCODED BY GENE NO: 71

This gene is expressed primarily in human epithelioid sarcoma, and to a lesser extent in breast cancer and adrenal gland tumors.

Therefore, polynucleotides and polypeptides of the invention are useful as reagents for differential identification of the tissue(s) or cell type(s) present in a biological sample and for diagnosis of diseases and conditions which include, but are not limited to, disorders related to epithelium, and cancer. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g., integumentary, fibroid, epithelial, reproductive, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in epithelial sarcoma indicates that polynucleotides and polypeptides corresponding to this gene are useful for diagnosis and treatment of epithelial disorders. Furthermore, the tissue distribution indicates that polynucleotides and polypeptides corresponding to this gene are useful for the detection, treatment, and/or prevention of various endocrine disorders and cancers, particularly Addison's disease, Cushing's Syndrome, and disorders and/or cancers of the pancrease (e.g. diabetes mellitus), adrenal cortex, pituitary (e.g., hyper-, hypopituitarism), thyroid (e.g. hyper-, hypothyroidism), parathyroid (e.g. hyper-,hypoparathyroidism), and hypothallamus. Protein, as well as, antibodies directed against the protein may show utility as a tumor marker and/or immunotherapy targets for the above listed tissues.Many polynucleotide sequences, such as EST sequences, are publicly available and accessible through sequence databases. Some of these sequences are related to SEQ ID NO:81 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1076 of SEQ ID NO:81, b is an integer of 15 to 1090, where both a and b correspond to the positions of nucleotide residues shown in SEQ ID NO:81, and where the b is greater than or equal to a + 14.

### FEATURES OF PROTEIN ENCODED BY GENE NO: 72

When tested against U937 cell lines, supernatants removed from cells containing this gene activated the GAS (gamma activating sequence) promoter element. Thus, it is likely that this gene activates myeloid cells through the Jak-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells.

This gene is expressed primarily in brain-medulloblastoma.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly proliferative conditions such as brain-medulloblastoma. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues or cell types (e.g.neural, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:184 as residues: Asp-18 to His-25, Phe-55 to Tyr-69.

The tissue distribution in brain-medulloblastoma indicates that polynucleotides and polypeptides corresponding to this gene are useful for diagnosis and intervention of brain-medulloblastoma or other tumors. Additionally, the peptide may act in nerve tissue development and functions.Protein, as well as, antibodies directed against the protein may show utility as a tumor marker and/or immunotherapy targets for the above listed tissues Many polynucleotide sequences, such as EST sequences, are publicly available and accessible through sequence databases. Some of these sequences are related to SEQ ID NO:82 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 684 of SEQ ID NO:82, b is an integer of 15 to 698, where both a and b correspond to the positions of nucleotide residues shown in SEQ ID NO:82, and where the b is greater than or equal to a + 14.

### FEATURES OF PROTEIN ENCODED BY GENE NO: 73

In specific embodiments, polypeptides of the invention comprise the following amino acid sequence:
VAESTEEPAGSNRGQYPEDSSSDGLRQREVLRNLSSPGWENISR (SEQ ID NO:249). Polynucleotides encoding these polypeptides are also encompassed by the invention.

This gene is expressed primarily in chronic lymphocytic leukemia.

Therefore, polynucleotides and polypeptides of the invention are useful as reagents for differential identification of the tissue(s) or cell type(s) present in a biological sample and for diagnosis of diseases and conditions which include, but are not limited to, hemapoietic or immune disorders, particularly leukemic diseases . Similarly, polypeptides and antibodies directed to these polypeptides are useful in providing immunological probes for differential identification of the tissue(s) or cell type(s). For a number of disorders of the above tissues or cells, particularly of the hemapoietic system, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in lymphocytic leukemia indicates that polynucleotides and polypeptides corresponding to this gene are useful for diagnosis and intervention of leukemic diseases and hemapoietic disorders. Similarly, expression within hematopoietic cells indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include ) bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:83 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 854 of SEQ ID NO:83, b is an integer of 15 to 868, where both a and b correspond to the positions of nucleotide residues shown in SEQ ID NO:83, and where the b is greater than or equal to a + 14.

### FEATURES OF PROTEIN ENCODED BY GENE NO: 74

It is likely that the open reading frame containing the predicted signal peptide continues in the 5' direction. In specific embodiments, polypeptides of the invention comprise the following amino acid sequence:
AREPLGLTQDPLVFGMTSFLQTSSPIPNSC (SEQ ID NO:250). Polynucleotides encoding these polypeptides are also encompassed by the invention. The gene encoding the disclosed cDNA is believed to reside on chromosome 11. Accordingly, polynucleotides related to this invention are useful as a marker in linkage analysis for chromosome 11.

This gene is expressed primarily in endothelial cells and in brain.

Therefore, polynucleotides and polypeptides of the invention are useful as reagents for differential identification of the tissue(s) or cell type(s) present in a biological sample and for diagnosis of diseases and conditions which include, but are not limited to, hematopoetic and neurological disorders. Similarly, polypeptides and antibodies directed to these polypeptides are useful in providing immunological probes for differential identification of the tissue(s) or cell type(s). For a number of disorders of the above tissues or cells, particularly of the vascular and nervous systems, expression of this gene at significantly higher or lower levels may be routinely detected in certain tissues or cell types (e.g.immune, hematopoietic, neural, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 186 as residues: Ser-34 to Ser-39.

The tissue distribution in neural tissue indicates that polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival.Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:84 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 615 of SEQ ID NO:84, b is an integer of 15 to 629, where both a and b correspond to the positions of nucleotide residues shown in SEQ ID NO:84, and where the b is greater than or equal to a + 14.

### FEATURES OF PROTEIN ENCODED BY GENE NO: 75

It is likely that the open reading frame containing the predicted signal peptide continues in the 5' direction. In specific embodiments, polypeptides of the invention comprise the following amino acid sequence: FQAPASARTACSTLL (SEQ ID NO:251). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and hematopoetic disorders. Similarly, polypeptides and antibodies directed to these polypeptides are useful in providing immunological probes for differential identification of the tissue(s) or cell type(s). For a number of disorders of the above tissues or cells, particularly of the immune and hematopoetic systems, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:187 as residues: Val-24 to Ser-29, Ser-53 to Ala-59, Glu-69 to Met-74.

The tissue distribution predominantly in neutrophils indicates that the gene could be important for the treatment or detection of immune or hematopoietic disorders including arthritis, asthma, immunodeficiency diseases, leukemia, transplant rejection, and microbial infections.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:85 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 823 of SEQ ID NO:85, b is an integer of 15 to 837, where both a and b correspond to the positions of nucleotide residues shown in SEQ ID NO:85, and where the b is greater than or equal to a + 14.

### FEATURES OF PROTEIN ENCODED BY GENE NO: 76

It is likely that the open reading frame containing the predicted signal peptide continues in the 5' direction. In specific embodiments, polypeptides of the invention comprise the following amino acid sequence:
AQPSPCPSCLAHSWPPFRLLSLPPPAGASLGDGRVCS (SEQ ID NO:252), and/or HSLPPALPAWLTPGHPSDSSLCLLQLAPHLVMAVSVPWPLPEXLGFSCCHCVS LTGPHAGFSYHFLHPAEPRAWQHQSSVVGMSRKQASFSMAQKGVCHLG KSXKRGSKKASCPXYPSFSK (SEQ ID NO:253). Polynucleotides encoding these polypeptides are also encompassed by the invention.

This gene is expressed primarily in endothelial cells.

Therefore, polynucleotides and polypeptides of the invention are useful as reagents for differential identification of the tissue(s) or cell type(s) present in a biological sample and for diagnosis of diseases and conditions which include, but are not limited to. integumentary or vascular disorders. Similarly, polypeptides and antibodies directed to these polypeptides are useful in providing immunological probes for differential identification of the tissue(s) or cell type(s). For a number of disorders of the above tissues or cells, particularly of the hematopoeitic and vascular systems, expression of this gene at significantly higher or lower levels may be routinely detected in certain tissues or cell types (e.g.cardiovascular, immune, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene in endothelial cells indicates that be useful in the treatment and detection of hematopoietic, immune and/or vascular disorders, particularly atherosclerosis, embolism, stroke, or aneurysm.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:86 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 889 of SEQ ID NO:86, b is an integer of 15 to 903, where both a and b correspond to the positions of nucleotide residues shown in SEQ ID NO:86, and where the b is greater than or equal to a + 14.

### FEATURES OF PROTEIN ENCODED BY GENE NO: 77

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 189 as residues: Gly-33 to Asn-44.

The tissue distribution in neutrophils indicates that polynucleotides and polypeptides corresponding to this gene are useful for the diagnosis and treatment of hematopoietic and immune disorders including: anemias, auto-immunities, immunodeficiencies (e.g. AIDS), immuno-supressive conditions (transplantation) and leukemias. In addition this gene product may be applicable in conditions of general microbial infection, arthritis, inflammation or cancer. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:87 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 711 of SEQ ID NO:87, b is an integer of 15 to 725, where both a and b correspond to the positions of nucleotide residues shown in SEQ ID NO:87, and where the b is greater than or equal to a + 14.

### FEATURES OF PROTEIN ENCODED BY GENE NO: 78

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of hematopoietic and immune disorders including: anemias, auto-immunities, immunodeficiencies (e.g. AIDS), immuno-supressive conditions (transplantation) and leukemias. In addition this gene product may be applicable in conditions of general microbial infection, arthritis, inflammation or cancer. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:88 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 592 of SEQ ID NO:88, b is an integer of 15 to 606, where both a and b correspond to the positions of nucleotide residues shown in SEQ ID NO:88, and where the b is greater than or equal to a + 14.

### FEATURES OF PROTEIN ENCODED BY GENE NO: 79

This gene is expressed primarily in hematopoetic cells including neutrophils, T-cells and activated monocytes.

Therefore, polynucleotides and polypeptides of the invention are useful as reagents for differential identification of the tissue(s) or cell type(s) present in a biological sample and for diagnosis of diseases and conditions which include, but are not limited to, hematopoeitic and immune disorders. Similarly, polypeptides and antibodies directed to these polypeptides are useful in providing immunological probes for differential identification of the tissue(s) or cell type(s). For a number of disorders of the above tissues or cells, particularly of the hematopoetic and immune systems, expression of this gene at significantly higher or lower levels may be routinely detected in certain tissues or cell types (e.g.immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene predominantly in hematopoietic cell types indicates that the gene could be important for the treatment or detection of immune or hematopoietic disorders including arthritis, asthma, immunodeficiency diseases and leukemia. Morever, this gene would also be useful for the treatment and diagnosis of other hematopoetic related disorders such as anemia, pancytopenia, leukopenia, or thrombocytopenia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:89 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1128 of SEQ ID NO:89, b is an integer of 15 to 1142, where both a and b correspond to the positions of nucleotide residues shown in SEQ ID NO:89, and where the b is greater than or equal to a + 14.

### FEATURES OF PROTEIN ENCODED BY GENE NO: 80

It is likely that the open reading frame containing the predicted signal peptide continues in the 5' direction. In specific embodiments, polypeptides of the invention comprise the following amino acid sequence: IGIRVWYYRNQKNSKQMWIKCLGS (SEQ ID NO:254). Polynucleotides encoding these polypeptides are also encompassed by the invention. The gene encoding the disclosed cDNA is believed to reside on chromosome 1. Accordingly, polynucleotides related to this invention are useful as a marker in linkage analysis for chromosome 1.

This gene is expressed primarily in endothelial cells.

Therefore, polynucleotides and polypeptides of the invention are useful as reagents for differential identification of the tissue(s) or cell type(s) present in a biological sample and for diagnosis of diseases and conditions which include, but are not limited to, integumentary or vascular disorders. Similarly, polypeptides and antibodies directed to these polypeptides are useful in providing immunological probes for differential identification of the tissue(s) or cell type(s). For a number of disorders of the above tissues or cells, particularly of the vascular and hematopoetic systems, expression of this gene at significantly higher or lower levels may be routinely detected in certain tissues or cell types (e.g.vascular, integumentary, cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in vascular tissue indicates that the protein product of this gene may be useful in the treatment, and/or prevention of a variety of vascular conditions such as atherosclerosis, aneurysm, stroke, or embolism. As the gene is expressed in endothelial cells, it may also be of importance in the treatment and detection of hematopoietic, and/or immune disorders. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:90 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between I to 582 of SEQ ID NO:90, b is an integer of 15 to 596, where both a and b correspond to the positions of nucleotide residues shown in SEQ ID NO:90, and where the b is greater than or equal to a + 14.

### FEATURES OF PROTEIN ENCODED BY GENE NO: 81

The translation product of this gene shares sequence homology with the bile acid CoA:amino acid N-acyltransferase (BAT) which is thought to be important as a liver enzyme that catalyzes the conjugation of bile acids with glycine or taurine (See Genbank Accession No.gnl|PID|e307059).

This gene is expressed primarily in hepatocellular tumor.

Therefore, polynucleotides and polypeptides of the invention are useful as reagents for differential identification of the tissue(s) or cell type(s) present in a biological sample and for diagnosis of diseases and conditions which include, but are not limited to, liver diseases and hepatocellular carcinoma. Similarly, polypeptides and antibodies directed to these polypeptides are useful in providing immunological probes for differential identification of the tissue(s) or cell type(s). For a number of disorders of the above tissues or cells, particularly of the hepatocellular carcinoma, expression of this gene at significantly higher or lower levels may be routinely detected in certain tissues or cell types (e.g.hepatic, and cancerous and wounded tissues) or bodily fluids (e.g.lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:193 as residues: Thr-55 to Gln-66, Asp-85 to Glu-92, Pro-125 to Ser-130, Gly-146 to Ala-154, Leu-170 to Lys-177.

The tissue distribution in hepatocellular tumor and homology to bile acid CoA:amino acid N-acyltransferase (BAT) indicates that polynucleotides and polypeptides corresponding to this gene are useful for diagnosis and intervention of hepatocellular tumor, particularly as a new molecular prognostic marker in hepatocellular carcinoma patients, following hepatic resection. Moreover, the protein product of this gene would also be useful for the detection and treatment of other liver disorders and cancers (e.g. hepatoblastoma, jaundice, hepatitis, liver metabolic diseases and conditions that are attributable to the differentiation of hepatocyte progenitor cells). The protein may also be useful in developmental abnormalities, fetal deficiencies, pre-natal disorders and various would-healing models and/or tissue trauma.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:91 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 619 of SEQ ID NO:91, b is an integer of 15 to 633, where both a and b correspond to the positions of nucleotide residues shown in SEQ ID NO:91, and where the b is greater than or equal to a + 14.

### FEATURES OF PROTEIN ENCODED BY GENE NO: 82

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, bone, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene in bone marrow indicates that the gene could be important for the treatment or detection of immune or hematopoietic disorders including arthritis, asthma, immunodeficiency diseases, leukemia, and also in treatement of cancer patients with a depleted immune system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:92 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 711 of SEQ ID NO:92, b is an integer of 15 to 725, where both a and b correspond to the positions of nucleotide residues shown in SEQ ID NO:92, and where the b is greater than or equal to a + 14.

### FEATURES OF PROTEIN ENCODED BY GENE NO: 83

When tested against K562 leukemia cell lines, supernatants removed from cells containing this gene activated the ISRE assay. Thus, it is likely that this gene activates leukemia cells through the Jak-STAT signal transduction pathway. The ISRE (interferon-sensitive responsive element) is a promoter element found upstream in many genes involved in the Jak-STAT pathway. The Jak-STAT pathway is a large, signal transduction pathway involved in the differentiation and proliferation of cells. Therefore, activation of the Jak-STAT pathway, reflected by the binding of the ISRE element, can be used to indicate proteins involved in the proliferation and differentiation of cells.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immunologically mediated disorders. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neurophils indicates that polynucleotides and polypeptides corresponding to this gene are useful for the diagnosis and treatment of hematopoietic and immune disorders including: anemias, auto-immunities, immunodeficiencies (e.g. AIDS), immuno-supressive conditions (transplantation) and leukemias. In addition this gene product may be applicable in conditions of general microbial infection, inflammation or cancer. Protein, as well as, antibodies directed against the protein may show utility as a tumor marker and/or immunotherapy targets for the above listed tissues.Many polynucleotide sequences, such as EST sequences, are publicly available and accessible through sequence databases. Some of these sequences are related to SEQ ID NO:93 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 587 of SEQ ID NO:93, b is an integer of 15 to 601, where both a and b correspond to the positions of nucleotide residues shown in SEQ ID NO:93, and where the b is greater than or equal to a + 14.

### FEATURES OF PROTEIN ENCODED BY GENE NO: 84

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic disorders. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:196 as residues: Trp-22 to Trp-35, Ser-42 to Gly-50.

The tissue distribution of this gene predominantly in neutrophils indicates that the gene could be important for the treatment or detection of immune or hematopoietic disorders including arthritis, asthma, immunodeficiency diseases, leukemia, transplant rejection, and microbial infections. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:94 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 678 of SEQ ID NO:94, b is an integer of 15 to 692, where both a and b correspond to the positions of nucleotide residues shown in SEQ ID NO:94. and where the b is greater than or equal to a + 14.

### FEATURES OF PROTEIN ENCODED BY GENE NO: 85

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immunological disorders. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:197 as residues: Asn-51 to Asn-69.

The tissue distribution in neutrophils indicates that polynucleotides and polypeptides corresponding to this gene are useful for the diagnosis and treatment of hematopoietic and immune disorders including: anemias, auto-immunities, immunodeficiencies (e.g. AIDS), immuno-supressive conditions (transplantation) and leukemias. In addition this gene product may be applicable in conditions of general microbial infection, inflammation or cancer. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:95 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 991 of SEQ ID NO:95, b is an integer of 15 to 1005, where both a and b correspond to the positions of nucleotide residues shown in SEQ ID NO:95, and where the b is greater than or equal to a + 14.

### FEATURES OF PROTEIN ENCODED BY GENE NO: 86

This gene is expressed primarily in brain medulloblastoma.

Therefore, polynucleotides and polypeptides of the invention are useful as reagents for differential identification of the tissue(s) or cell type(s) present in a biological sample and for diagnosis of diseases and conditions which include, but are not limited to, cancer, neurodegenerative diseases and behavioural disorders. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues or cell types (e.g. brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates that polynucleotides and polypeptides corresponding to this gene are useful for the diagnosis and treatment of cancers of the brain, such as medulloblastomas. Furthermore, the tissue distribution also indicates that the translation product of this gene is useful for the detection and/or treatment of neurodegenerative disease states and behavioural disorders such as Alzheimers Disease, Parkinsons Disease, Huntingtons Disease, schizophrenia, mania, dementia, paranoia, obsessive compulsive disorder and panic disorder. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:96 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 598 of SEQ ID NO:96, b is an integer of 15 to 612, where both a and b correspond to the positions of nucleotide residues shown in SEQ ID NO:96, and where the b is greater than or equal to a + 14.

### FEATURES OF PROTEIN ENCODED BY GENE NO: 87

This gene is expressed primarily in brain, bone marrow, lung, and to a lesser extent, in other tissues.

Therefore, polynucleotides and polypeptides of the invention are useful as reagents for differential identification of the tissue(s) or cell type(s) present in a biological sample and for diagnosis of diseases and conditions which include, but are not limited to, disorders of the brain and lungs. Similarly, polypeptides and antibodies directed to these polypeptides are useful in providing immunological probes for differential identification of the tissue(s) or cell type(s). For a number of disorders of the above tissues or cells, particularly of the immune, CNS, and pulmonary systems expression of this gene at significantly higher or lower levels may be routinely detected in certain tissues or cell types (e.g. brain, lung, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in bone marrow indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the tissue distribution in brain tissue indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and perception. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:97 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 656 of SEQ ID NO:97, b is an integer of 15 to 670, where both a and b correspond to the positions of nucleotide residues shown in SEQ ID NO:97, and where the b is greater than or equal to a + 14.

### FEATURES OF PROTEIN ENCODED BY GENE NO: 88

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of a variety of immune system disorders. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:98 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 605 of SEQ ID NO:98, b is an integer of 15 to 619, where both a and b correspond to the positions of nucleotide residues shown in SEQ ID NO:98. and where the b is greater than or equal to a + 14.

### FEATURES OF PROTEIN ENCODED BY GENE NO: 89

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune system disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of a variety of immune system disorders. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:99 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 689 of SEQ ID NO:99, b is an integer of 15 to 703, where both a and b correspond to the positions of nucleotide residues shown in SEQ ID NO:99, and where the b is greater than or equal to a + 14.

### FEATURES OF PROTEIN ENCODED BY GENE NO: 90

This gene is expressed primarily in neutrophils. It is likely that a frame shift exists in the sequence, and these are easily resolved by those skilled in the art using known molecular biology techniques.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune system disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of a variety of immune system disorders. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 100 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 748 of SEQ ID NO: 100, b is an integer of 15 to 762, where both a and b correspond to the positions of nucleotide residues shown in SEQ ID NO: 100, and where the b is greater than or equal to a + 14.

### FEATURES OF PROTEIN ENCODED BY GENE NO: 91

Contact of cells with supernatant containing the expressed product of this gene increases the permeability of the plasma membrane of astrocytes to calcium. Thus, it is likely that the product of this gene is involved in a signal transduction pathway that is initiated when the product binds a receptor on the surface of the astrocytes. Thus, polynucleotides and polypeptides of this gene have uses which include, but are not limited to, activating astrocytes.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune system disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g.. serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:203 as residues: Met-1 to Glu-6.

The tissue distribution in neutrophils indicates that polynucleotides and polypeptides corresponding to this gene are useful for the diagnosis and treatment of a variety of immune system disorders. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as. antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:101 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 636 of SEQ ID NO:101, b is an integer of 15 to 650, where both a and b correspond to the positions of nucleotide residues shown in SEQ ID NO:101, and where the b is greater than or equal to a + 14.

### FEATURES OF PROTEIN ENCODED BY GENE NO: 92

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune system disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:204 as residues: Ile-4 to Cys-9, Ser-36 to Asp-49, Ile-107 to Ile-115.

The tissue distribution in neurophils indicates that polynucleotides and polypeptides corresponding to this gene are useful for the diagnosis and treatment of hematopoietic and immune system disorders including: anemias, auto-immunities, immunodeficiencies (e.g. AIDS), immuno-supressive conditions (transplantation) and leukemias. In addition this gene product may be applicable in conditions of general microbial infection, arthritis, inflammation or cancer. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO: 102 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 346 of SEQ ID NO:102, b is an integer of 15 to 360, where both a and b correspond to the positions of nucleotide residues shown in SEQ ID NO: 102, and where the b is greater than or equal to a + 14.

### FEATURES OF PROTEIN ENCODED BY GENE NO: 93

This gene is expressed primarily in hemangiopericytoma.

Therefore, polynucleotides and polypeptides of the invention are useful as reagents for differential identification of the tissue(s) or cell type(s) present in a biological sample and for diagnosis of diseases and conditions which include, but are not limited to, hemangiopericytoma. Similarly, polypeptides and antibodies directed to these polypeptides are useful in providing immunological probes for differential identification of the tissue(s) or cell type(s). For a number of disorders of the above tissues or cells, particularly of the capillaries and arterioles, expression of this gene at significantly higher or lower levels may be routinely detected in certain tissues or cell types (e.g. circulatory, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:205 as residues: Thr-46 to Asp-52.

The tissue distribution in hemangiopericytoma indicates that polynucleotides and polypeptides corresponding to this gene are useful for the diagnosis and intervention of hemangiopericytoma or other pericyte related diseases. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:103 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 803 of SEQ ID NO: 103, b is an integer of 15 to 817, where both a and b correspond to the positions of nucleotide residues shown in SEQ ID NO: 103, and where the b is greater than or equal to a + 14.

### FEATURES OF PROTEIN ENCODED BY GENE NO: 94

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, hematopoetic and immune disorders. Similarly, polypeptides and antibodies directed to these polypeptides are useful in providing immunological probes for differential identification of the tissue(s) or cell type(s). For a number of disorders of the above tissues or cells, particularly of the hematopoetic and immune systems, expression of this gene at significantly higher or lower levels may be routinely detected in certain tissues or cell types (e.g. immune, bone, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene in bone marrow indicates that the gene could be important for the treatment or detection of immune or hematopoietic disorders including arthritis, asthma, immunodeficiency diseases, leukemia, and also in the treatement of cancer patients with a depleted immune system. The polypeptides or polynucleotides are also useful to enhance or protect proliferation, differentiation, and functional activation of hematopoietic progenitor cells (e.g., bone marrow cells), useful in treating cancer patients undergoing chemotherapy or patients undergoing bone marrow transplantation. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 104 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 867 of SEQ ID NO: 104, b is an integer of 15 to 881, where both a and b correspond to the positions of nucleotide residues shown in SEQ ID NO: 104, and where the b is greater than or equal to a + 14.

### FEATURES OF PROTEIN ENCODED BY GENE NO: 95

The gene encoding the disclosed cDNA is thought to reside on chromosome 4. Accordingly, polynucleotides related to this invention are useful as a marker in linkage analysis for chromosome 4.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and hematopoetic disorders. Similarly, polypeptides and antibodies directed to these polypeptides are useful in providing immunological probes for differential identification of the tissue(s) or cell type(s). For a number of disorders of the above tissues or cells, particularly of the hematopoetic and immune systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene in neutrophils indicates that the gene could be important for the treatment or detection of immune or hematopoietic disorders including arthritis, asthma, immunodeficiency diseases, leukemia, transplant rejection, and microbial infections. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene pr Protein, as well as, antibodies directed against the protein may show utility as a tumor marker and/or immunotherapy targets for the above listed tissues.duct may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 105 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 641 of SEQ ID NO: 105, b is an integer of 15 to 655, where both a and b correspond to the positions of nucleotide residues shown in SEQ ID NO: 105, and where the b is greater than or equal to a + 14.

### FEATURES OF PROTEIN ENCODED BY GENE NO: 96

This gene is expressed primarily in osteosarcoma.

Therefore, polynucleotides and polypeptides of the invention are useful as reagents for differential identification of the tissue(s) or cell type(s) present in a biological sample and for diagnosis of diseases and conditions which include, but are not limited to, osteosarcoma and other cancers. Similarly, polypeptides and antibodies directed to these polypeptides are useful in providing immunological probes for differential identification of the tissue(s) or cell type(s). For a number of disorders of the above tissues or cells, particularly of bone, expression of this gene at significantly higher or lower levels may be routinely detected in certain tissues or cell types (e.g. bone, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in osteosarcoma indicates that polynucleotides and polypeptides corresponding to this gene are useful for the detection and treatment of: fracture and trauma, osteoporosis, osteosarcoma, osteoclastoma, chondrosarcoma, regulation of ossification and osteonecrosis, arthritis, tendonitis, chrondomalacia and inflammation. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 106 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 592 of SEQ ID NO:106, b is an integer of 15 to 606, where both a and b correspond to the positions of nucleotide residues shown in SEQ ID NO: 106, and where the b is greater than or equal to a + 14.

### FEATURES OF PROTEIN ENCODED BY GENE NO: 97

This gene is expressed primarily in salivary gland and osteosarcoma.

Therefore, polynucleotides and polypeptides of the invention are useful as reagents for differential identification of the tissue(s) or cell type(s) present in a biological sample and for diagnosis of diseases and conditions which include, but are not limited to, osteosarcoma and other cancers, as well as digestive disorders. Similarly, polypeptides and antibodies directed to these polypeptides are useful in providing immunological probes for differential identification of the tissue(s) or cell type(s). For a number of disorders of the above tissues or cells, particularly of bone and the digestive system, expression of this gene at significantly higher or lower levels may be routinely detected in certain tissues or cell types (e.g., cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in osteosarcoma indicates that polynucleotides and polypeptides corresponding to this gene are useful for the detection and treatment of bone-related disorders and conditions, such as: fracture and trauma, osteoperosis, osteosarcoma, osteoclastoma, chondrosarcoma, regulation of ossification and osteonecrosis, arthritis, tendonitis, chrondomalacia and inflammation. In addition, the expression in salivary gland suggest a possible role for this gene product in the detection and treatment of digestive disorders. Many polynucleotide sequences, such as EST sequences, are publicly available and accessible through sequence databases. Some of these sequences are related to SEQ ID NO: 107 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 643 of SEQ ID NO: 107, b is an integer of 15 to 657, where both a and b correspond to the positions of nucleotide residues shown in SEQ ID NO: 107, and where the b is greater than or equal to a + 14.

### FEATURES OF PROTEIN ENCODED BY GENE NO: 98

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues or cell types (e.g. immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of hematopoietic and immune disorders including: anemias, auto-immunities, immunodeficiencies (e.g. AIDS), immuno-supressive conditions (transplantation) and leukemias. In addition this gene product may be applicable in conditions of general microbial infection, arthritis, inflammation or cancer. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 108 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 591 of SEQ ID NO: 1 08, b is an integer of 15 to 605, where both a and b correspond to the positions of nucleotide residues shown in SEQ ID NO: 108, and where the b is greater than or equal to a + 14.

### FEATURES OF PROTEIN ENCODED BY GENE NO: 99

This gene is expressed primarily in breast lymph node.

Therefore, polynucleotides and polypeptides of the invention are useful as reagents for differential identification of the tissue(s) or cell type(s) present in a biological sample and for diagnosis of diseases and conditions which include, but are not limited to, breast cancer and other immune diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g., lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in breast lymph node indicates that polynucleotides and polypeptides corresponding to this gene are useful for the diagnosis and intervention of breast cancer and other immune diseases. Expression of this gene product in lymph nodes indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 109 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 490 of SEQ ID NO: 109, b is an integer of 15 to 504, where both a and b correspond to the positions of nucleotide residues shown in SEQ ID NO:109, and where the b is greater than or equal to a + 14.

### FEATURES OF PROTEIN ENCODED BY GENE NO: 100

This gene is expressed primarily in T-cell lymphoma, and to a lesser extent, in human thymus tissue.

Therefore, polynucleotides and polypeptides of the invention are useful as reagents for differential identification of the tissue(s) or cell type(s) present in a biological sample and for diagnosis of diseases and conditions which include, but are not limited to, T-cell lymphoma and immune diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, thymus, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cell lymphoma indicates that polynucleotides and polypeptides corresponding to this gene are useful for the diagnosis and intervention of T-cell lymphomas and other immune diseases. Expression of this gene product in the thymus, as well as in T-cell lymphomas, indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.. Many polynucleotide sequences, such as EST sequences, are publicly available and accessible through sequence databases. Some of these sequences are related to SEQ ID NO:110 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 756 of SEQ ID NO:110, b is an integer of 15 to 770, where both a and b correspond to the positions of nucleotide residues shown in SEQ ID NO: 110, and where the b is greater than or equal to a + 14.

### FEATURES OF PROTEIN ENCODED BY GENE NO: 101

This gene is expressed primarily in chronic lymphocytic leukemia.

Therefore, polynucleotides and polypeptides of the invention are useful as reagents for differential identification of the tissue(s) or cell type(s) present in a biological sample and for diagnosis of diseases and conditions which include, but are not limited to, immune disorders, particularly chronic lymphocytic leukemia. Similarly, polypeptides and antibodies directed to these polypeptides are useful in providing immunological probes for differential identification of the tissue(s) or cell type(s). For a number of disorders of the above tissues or cells, particularly of the hemapoietic system expression of this gene at significantly higher or lower levels may be routinely detected in certain tissues or cell types (e.g. immune, cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in chronic lymphocytic leukemia indicates that polynucleotides and polypeptides corresponding to this gene are useful for the diagnosis and intervention of leukemia diseases or hemapoietic disoders. Expression of this gene product in spleen indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.Many polynucleotide sequences, such as EST sequences, are publicly available and accessible through sequence databases. Some of these sequences are related to SEQ ID NO:111 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 737 of SEQ ID NO:111, b is an integer of 15 to 751, where both a and b correspond to the positions of nucleotide residues shown in SEQ ID NO: 111, and where the b is greater than or equal to a + 14.

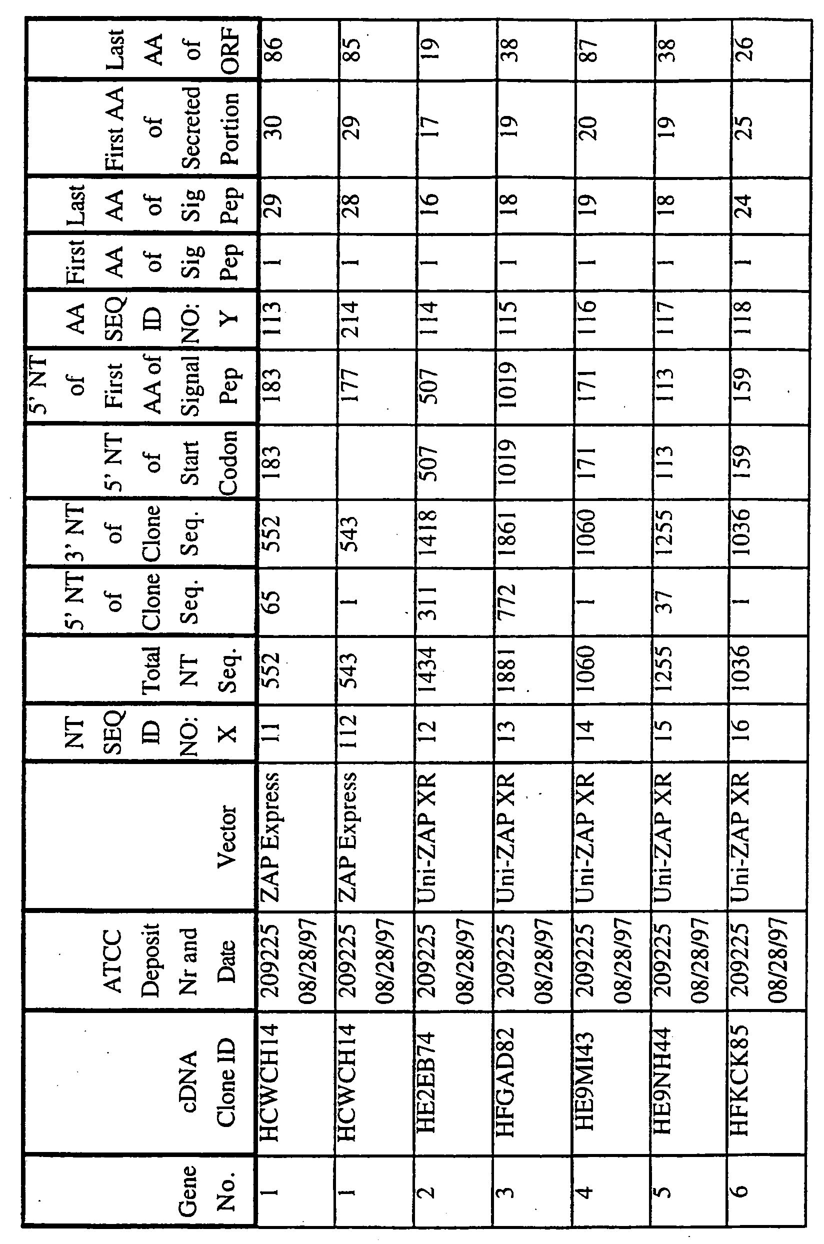

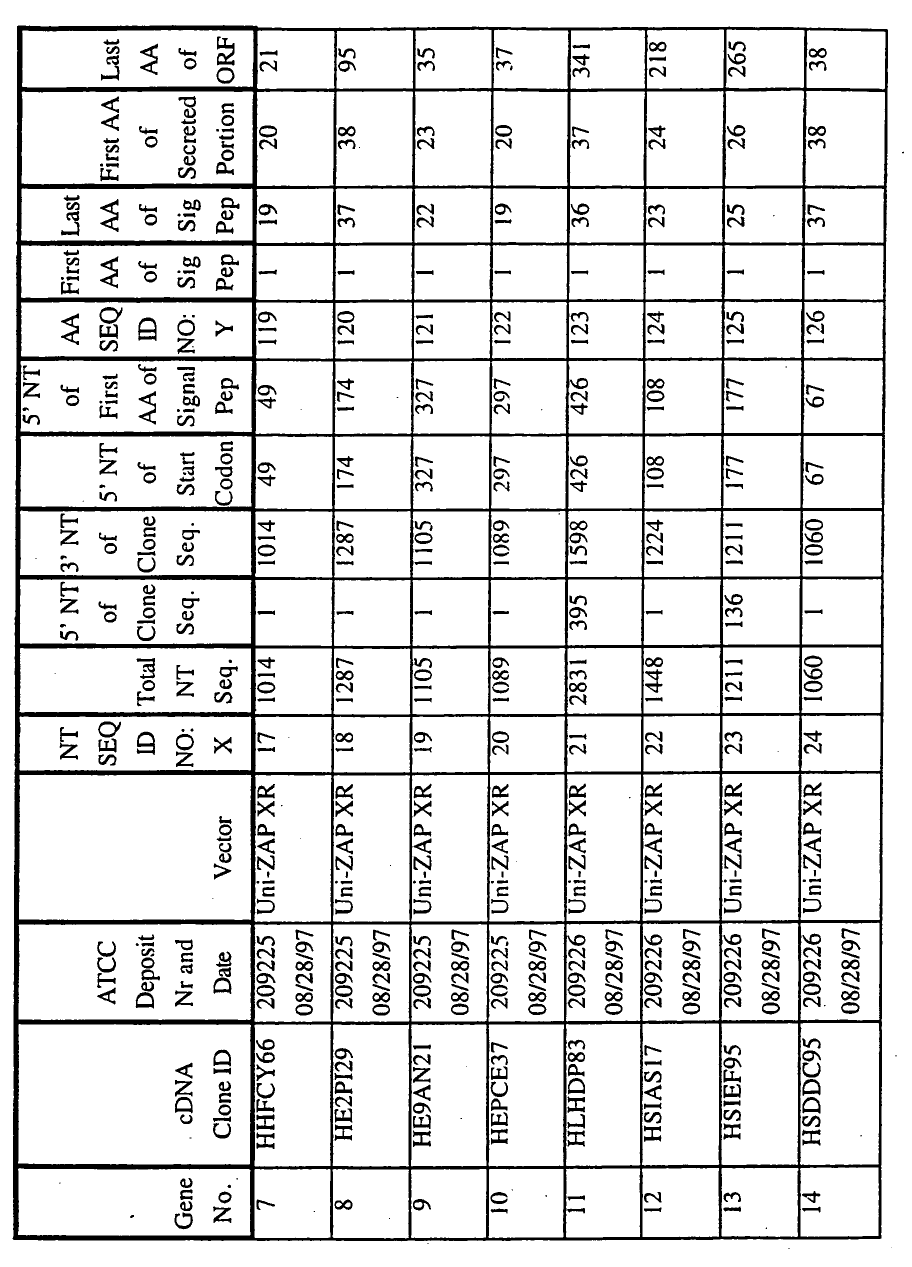

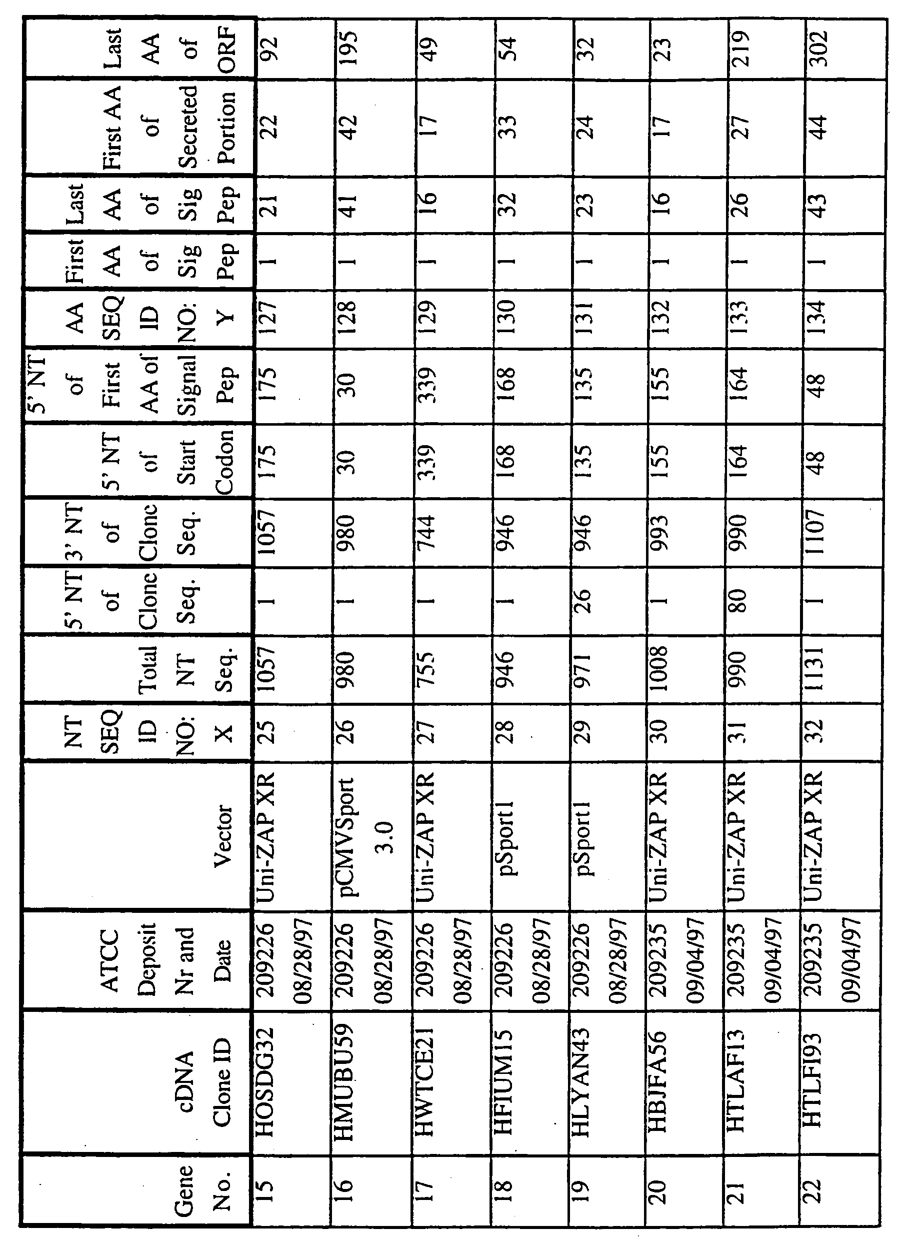

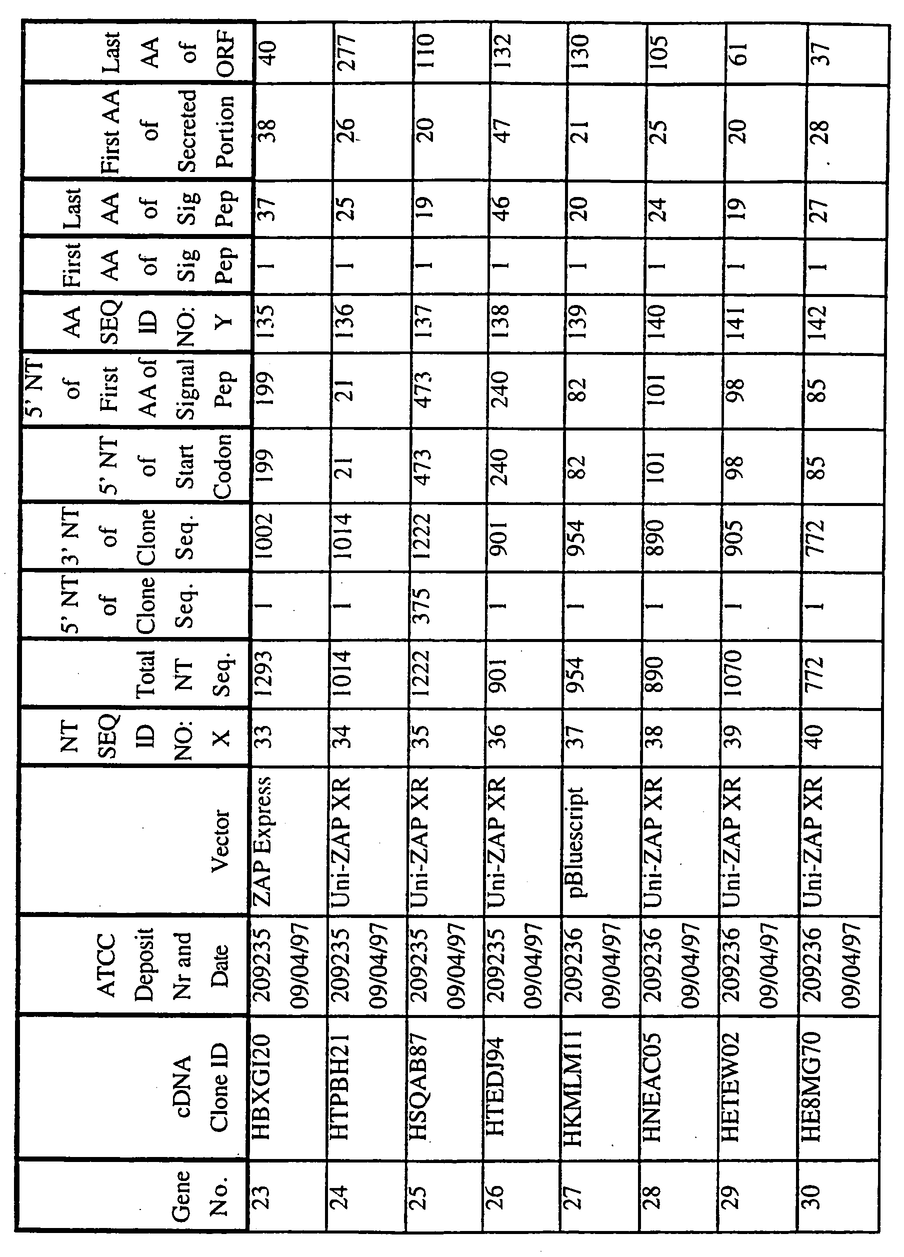

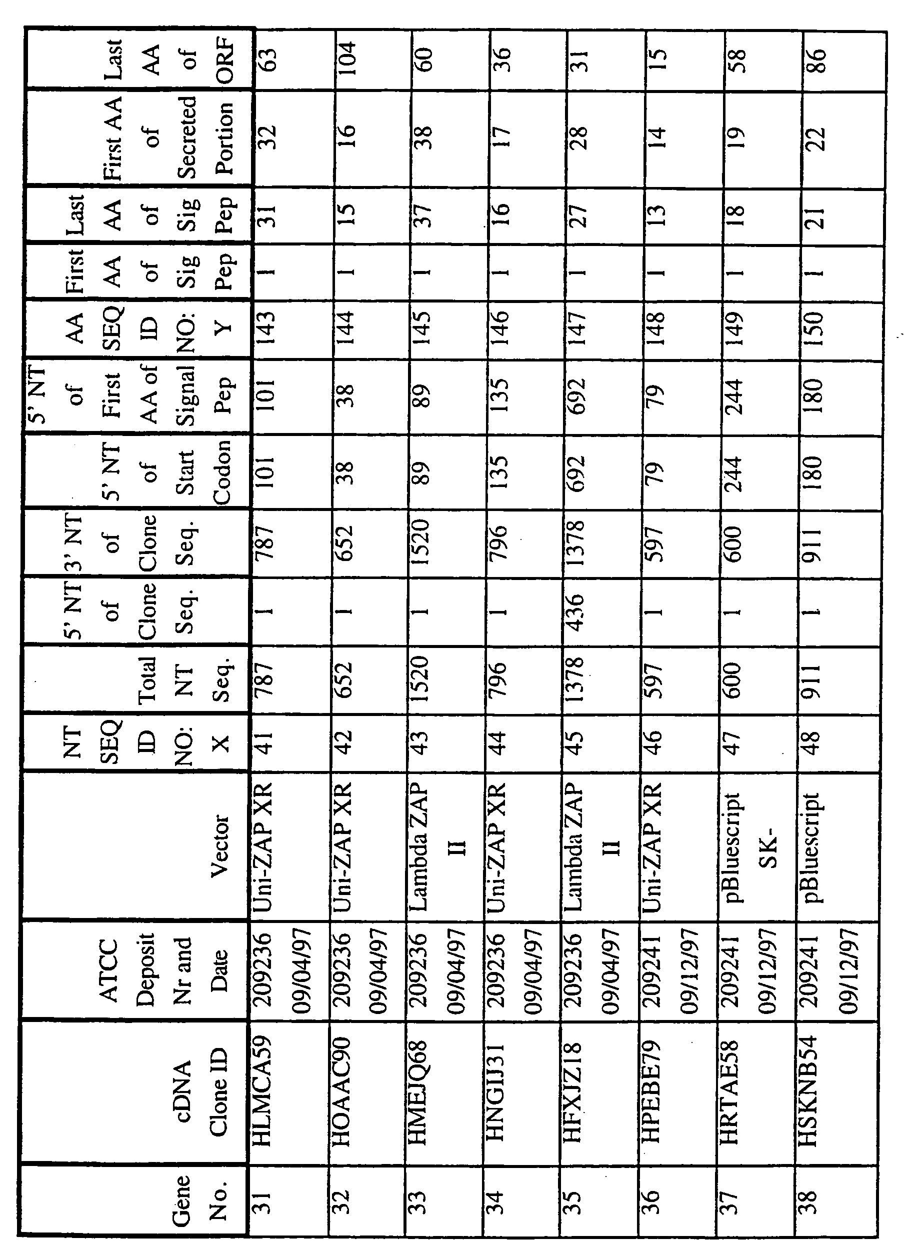

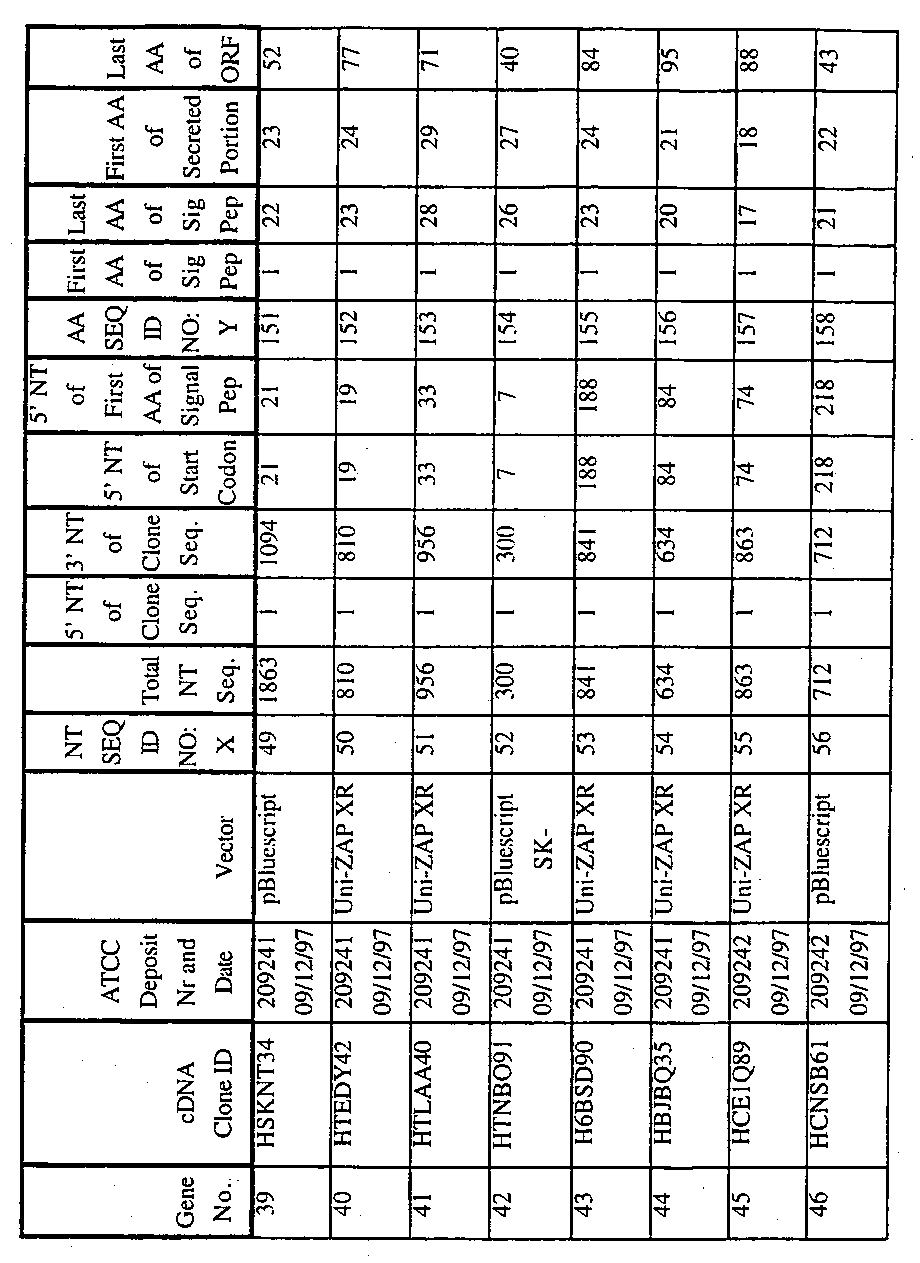

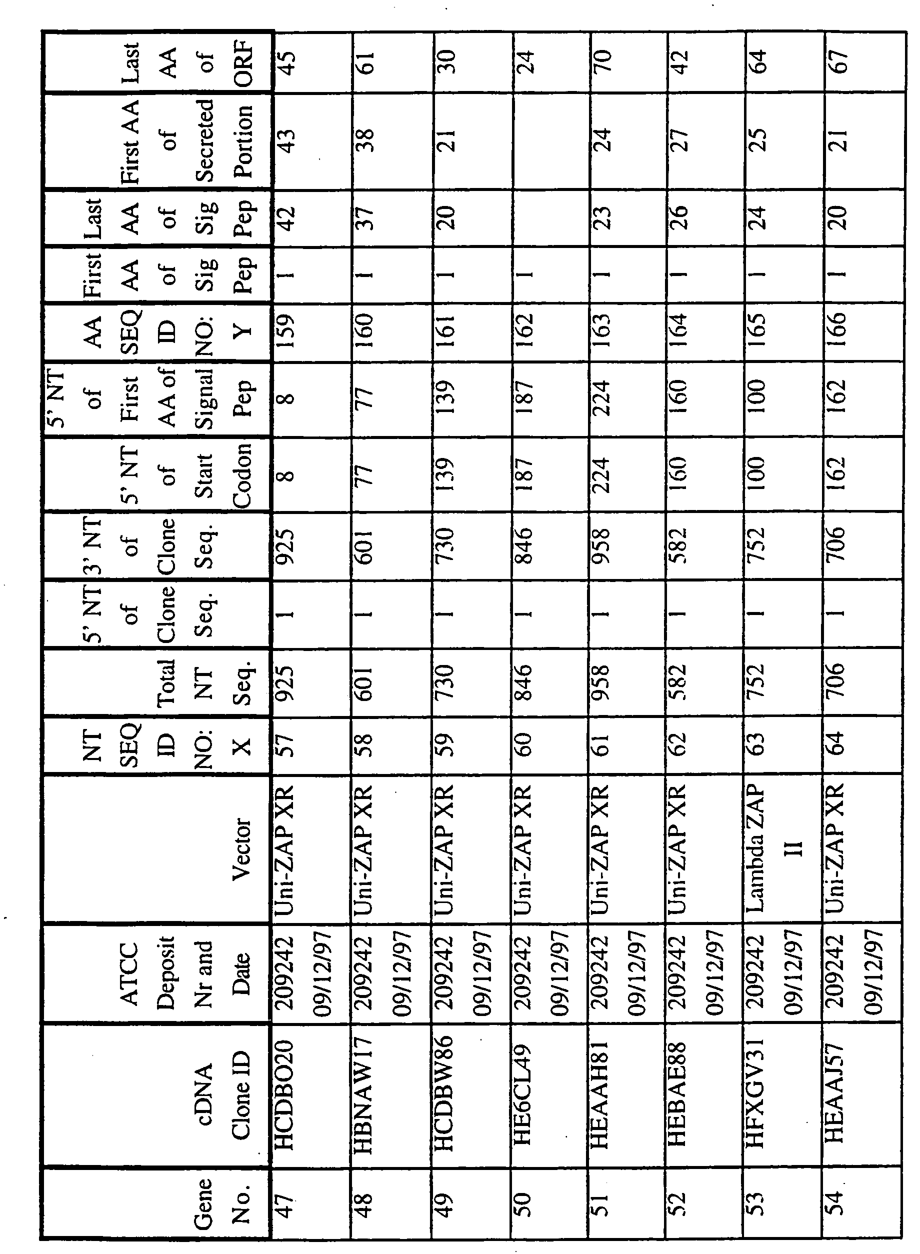

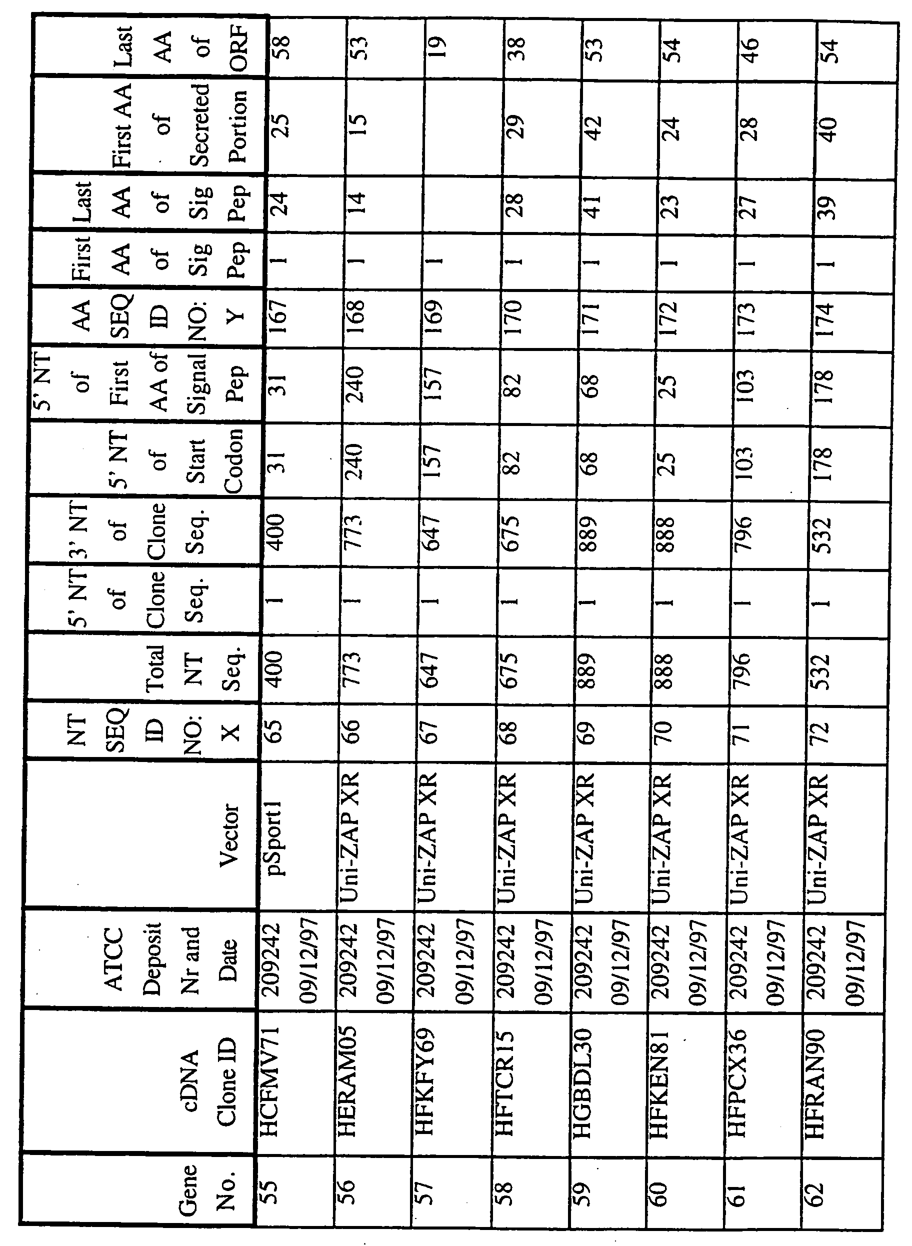

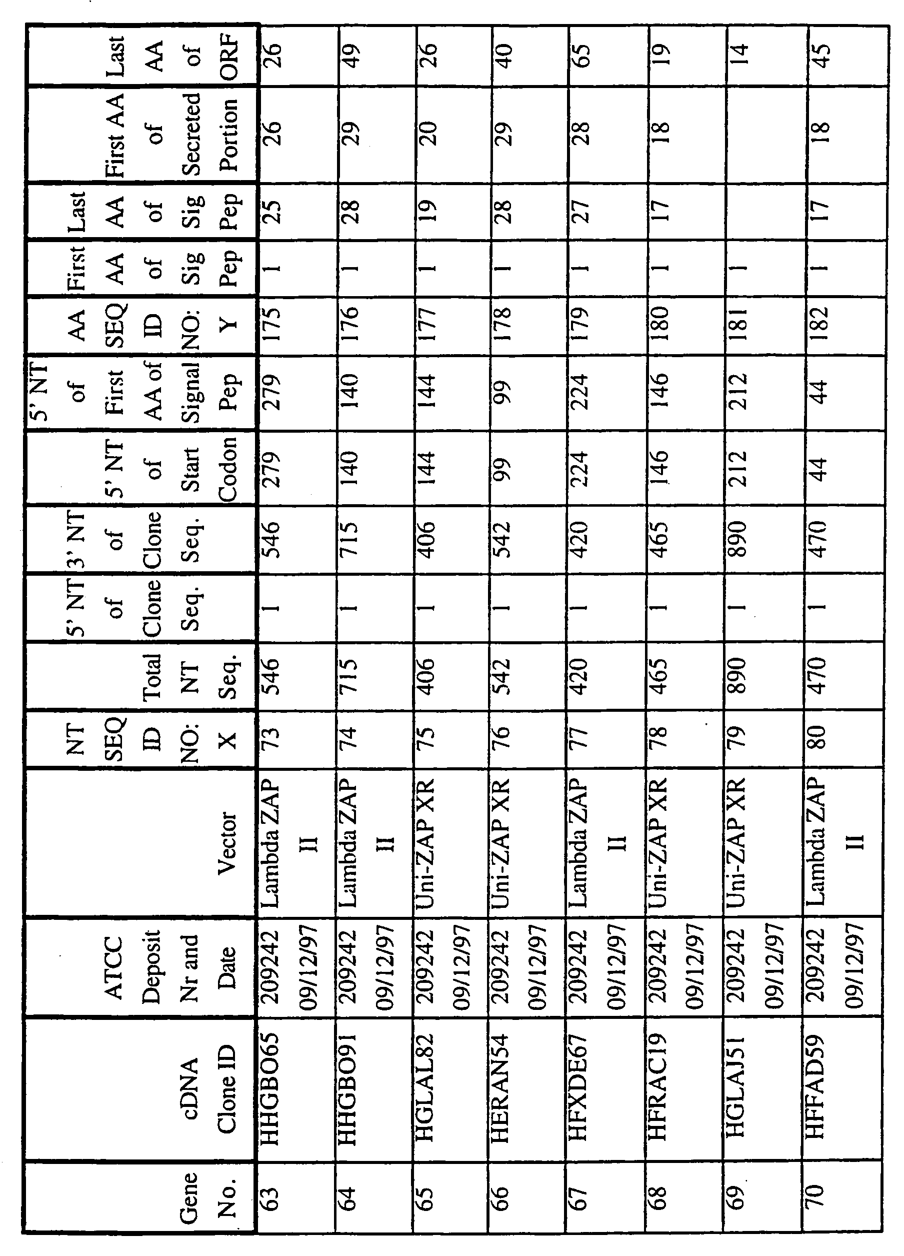

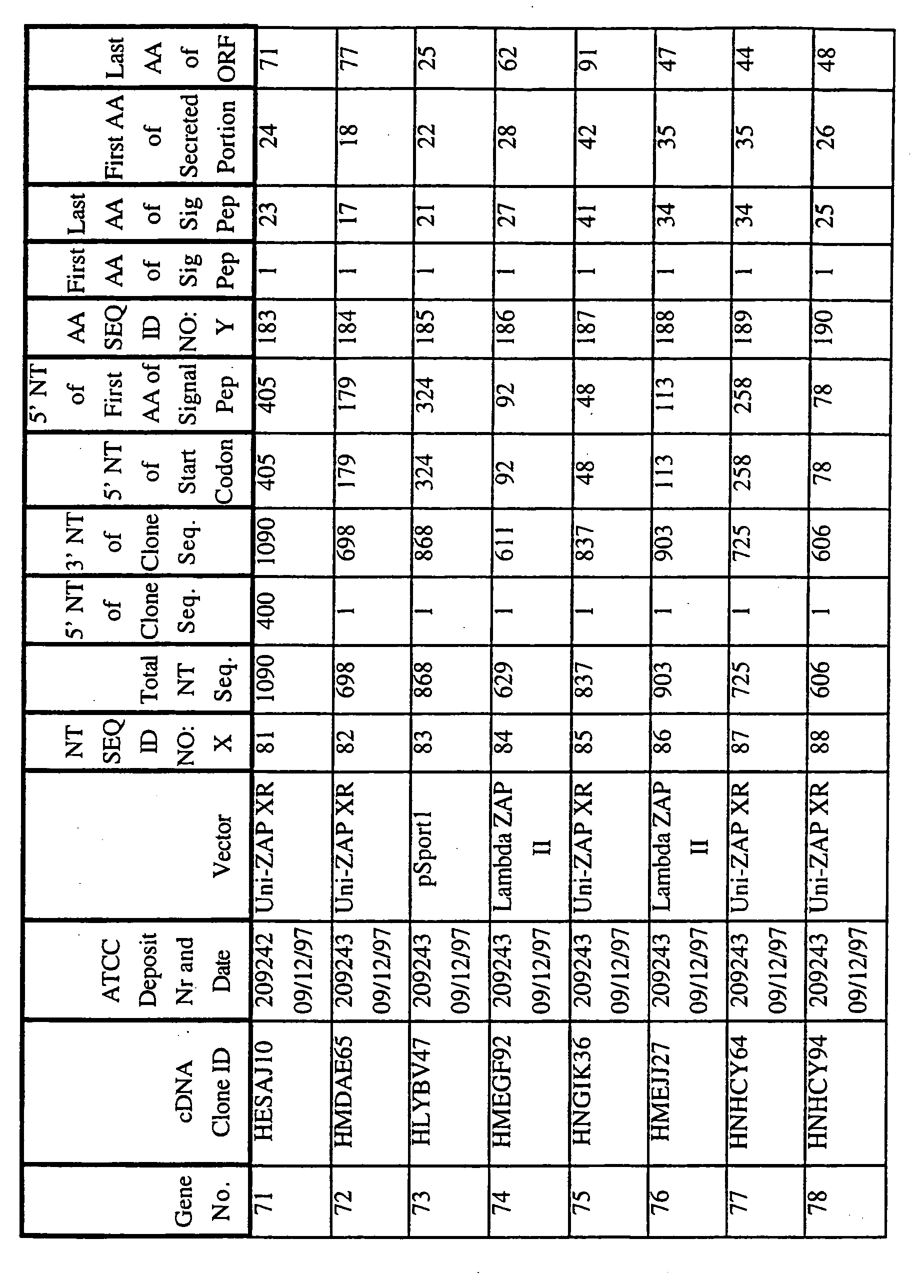

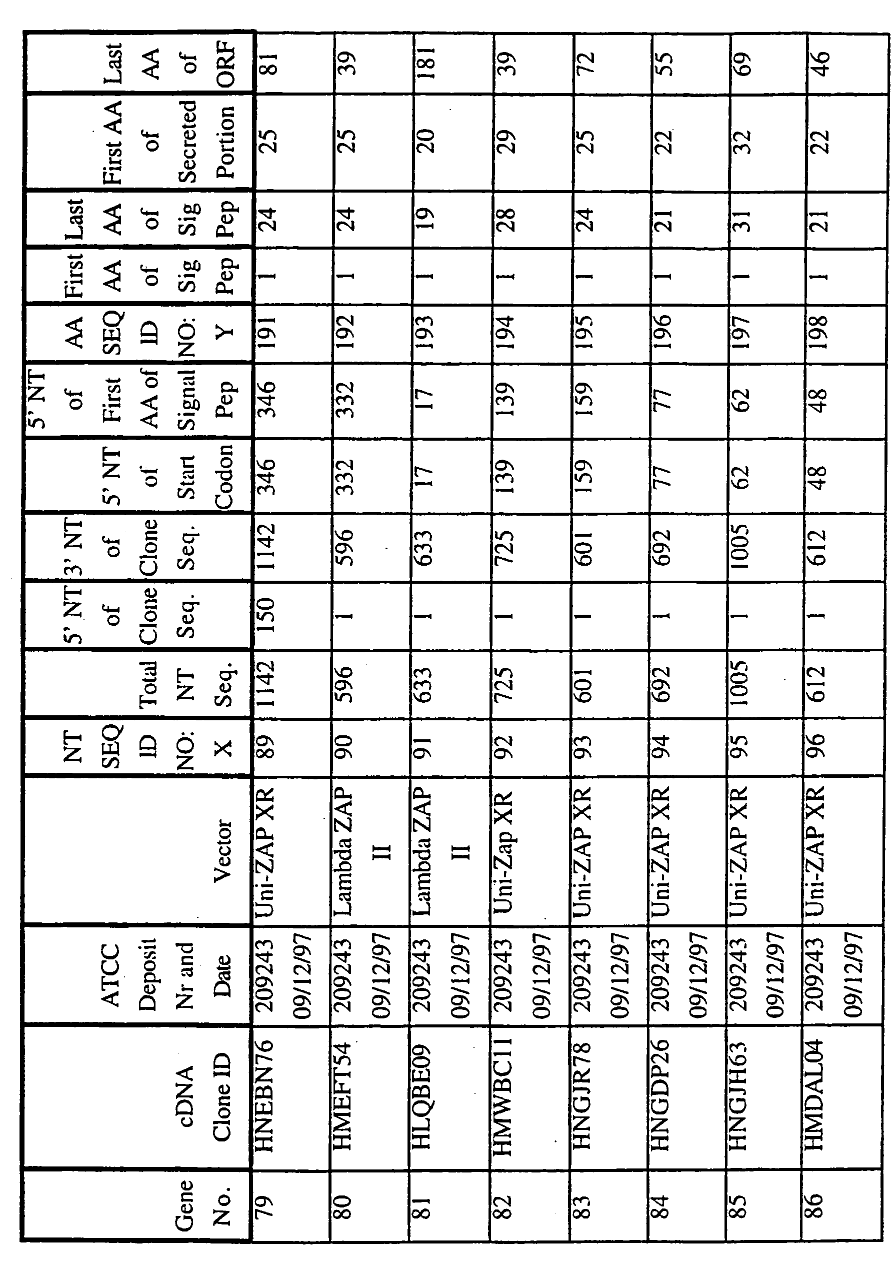

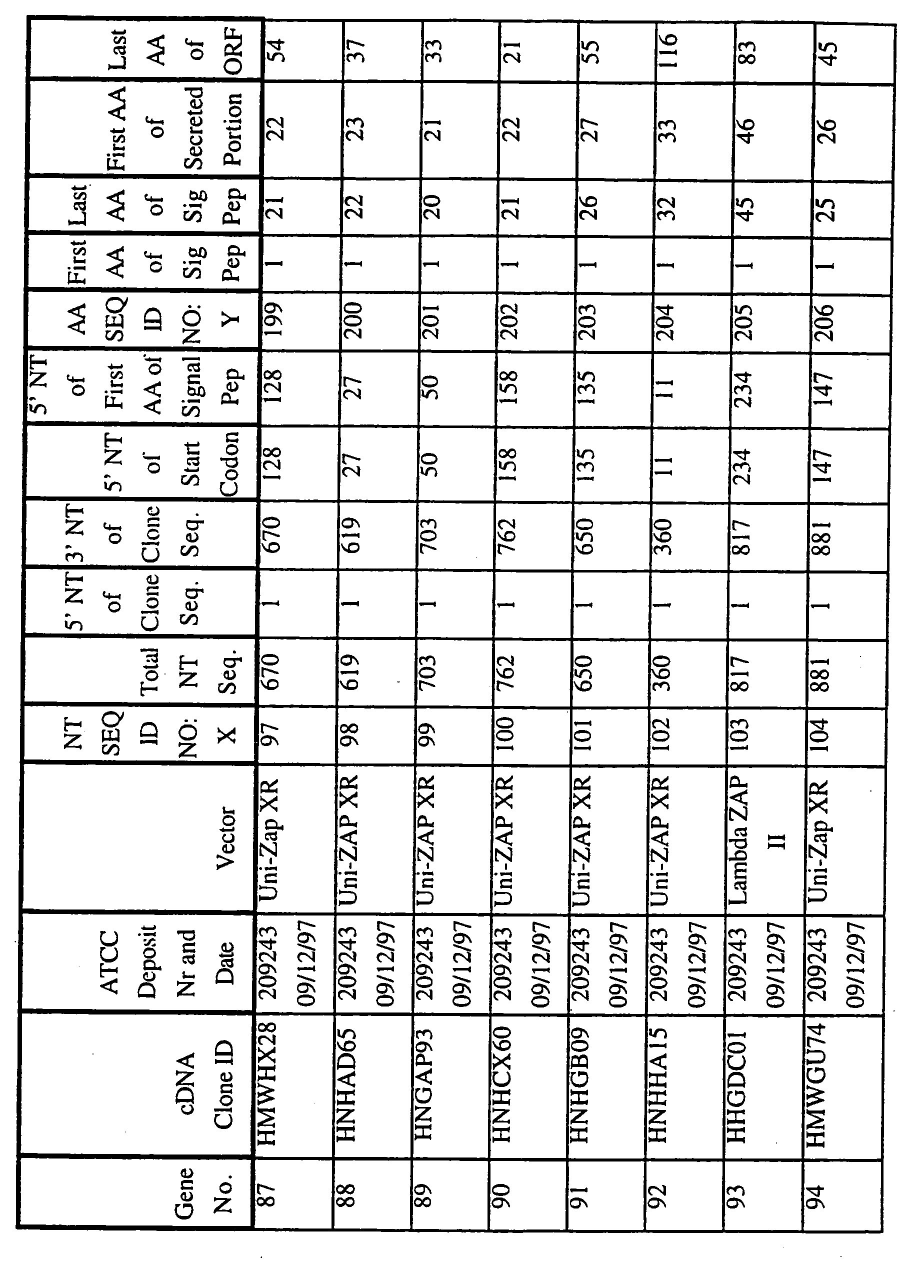

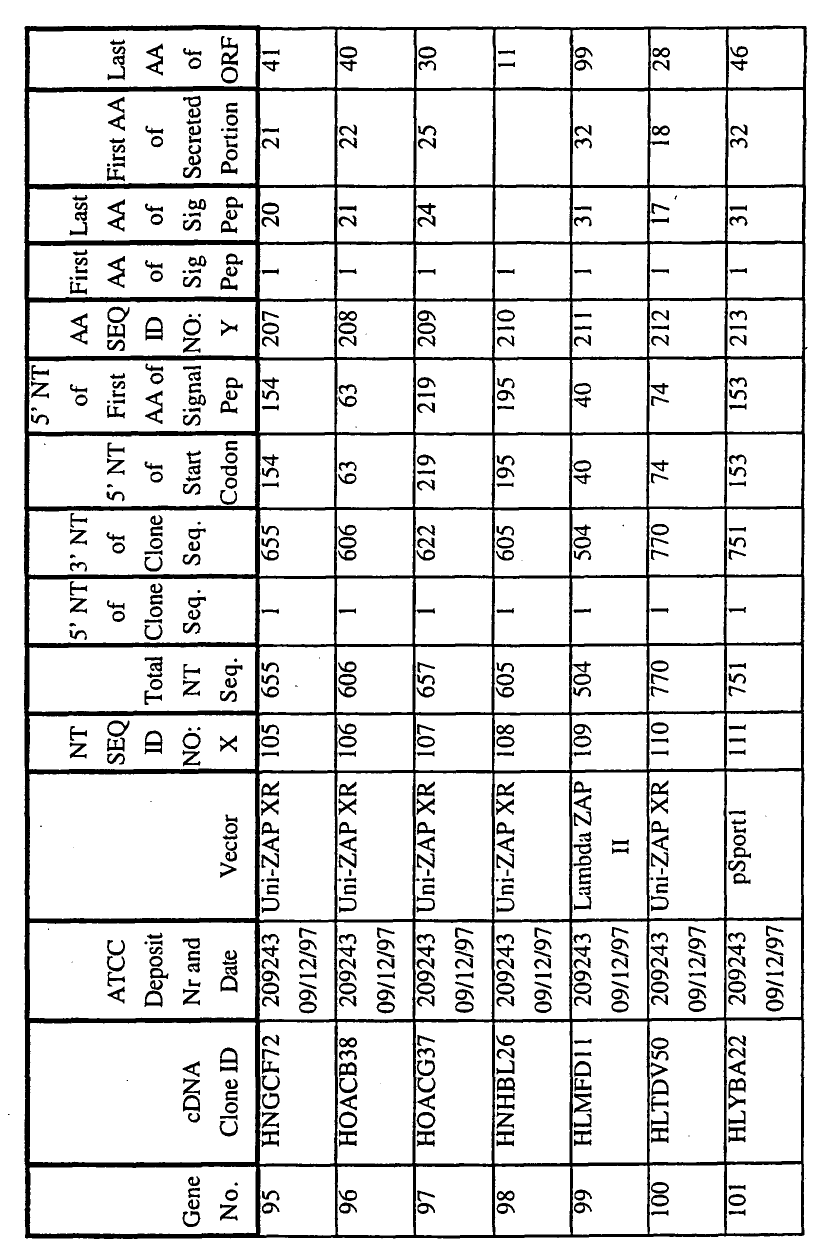

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification, such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as. for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated. the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3. pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers. since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York ( 1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979): Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton. FL (1988).) Triple helix formation optimally results in a shut-off of RNA transcription from DNA. while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene. are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine. and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example. by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include. but are not limited to neoplasms located in the: abdomen. bone. breast. digestive system. liver, pancreas. peritoneum. endocrine glands (adrenal. parathyroid. pituitary. testicles. ovary. thymus. thyroid). eye. head and neck. nervous (central and peripheral). lymphatic system, pelvic. skin. soft tissue, spleen. thoracic. and urogenital.

Similarly, other hyperproliferative disorders can also be treated or detected by a polynucleotide or polypeptide of the present invention. Examples of such hyperproliferative disorders include, but are not limited to: hypergammaglobulinemia. lymphoproliferative disorders. paraproteinemias. purpura. sarcoidosis. Sezary Syndrome. Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells. infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include. but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover. parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related). Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate. and attract cells, leading to the regeneration of tissues. (See. Science 276:59-87 (1997).) The regeneration of tissues could be used to repair, replace, or protect tissue damaged by congenital defects, trauma (wounds. bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome. and other tendon or ligament defects. A further example of tissue regeneration of non-healing wounds includes pressure ulcers, ulcers associated with vascular insufficiency, surgical, and traumatic wounds.

Similarly. nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders. head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body. such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus. a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist). increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast. Drosophila, or *E*. *coli*. Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention: and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics. such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1: and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128. 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+. SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01 % (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for I min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT 1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth. CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M 15/rep4 (Qiagen, Inc.) which contains multiple copies of the plasmid pREP4. which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8. the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4° C or frozen at -80° C.

In addition to the above expression vector, the present invention further includes an expression vector comprising phage operator and promoter elements operatively linked to a polynucleotide of the present invention. called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI. or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polyneptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E*. *coli* fermentation. the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin. Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5. 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autograplur californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 ( 1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and ) optionally, can be dephosphorylated using calf intestinal phosphatase, using routine procedures known in the art. The DNA is then isolated from a 1% agarose gel using a commercially available kit ("Geneclean" BIO 101 Inc., La Jolla. Ca.).

The fragment and the dephosphorylated plasmid are ligated together with T4 DNA ligase. *E*. *coli* HB 101 or other suitable *E. coli* hosts such as XL-1 Blue (Stratagene Cloning Systems, La Jolla. CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego. CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra*. An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours. 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include. for example, vectors such as pSVL and pMSG (Pharmacia. Uppsala, Sweden), pRSVcat (ATCC 37152), pSV2dhfr (ATCC 37146), pBC12MI (ATCC 67109), pCMVSport 2.0. and pCMVSport 3.0. Mammalian host cells that could be used include, human Hela, 293, H9 and Jurkat cells, mouse NIH3T3 and C 127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978): Hamlin, J. L. and Ma, C., Biochem. et Biophys. Acta, 1097:107-143 (1990); Page, M. J. and Sydenham. M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, Xbal and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1 % agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E*. *coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner. Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM. 100 nM, 200 nM. 400 nM, 800 nM). Clones growing at the highest concentrations of methotrexate are then transferred to new 6-well plates containing even higher concentrations of methotrexate (1 µM, 2 µM, 5 µM, 10 mM. 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however. it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention: however, it is preferable to employ the parent myeloma cell line (SP2O), available from the ATCC. After fusion. the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 ( 1986): Cabilly et al., U.S. Patent No. 4.816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells. to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O: 0.050 mg/L of Fe(NO₃)₃-9H₂O; 0.417 mg/L of FeSO₄-7H₂O; 311.80 mg/L of Kcl: 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite: 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four. using a 300ul multichannel pipetter. aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2. Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo. PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE ISRE |
| IFN-g | | + | + | - | 1 | GAS (IRF1>Lys6>IFP) |
| Il-10 | + | ? | ? | - | 1,3 | |
| | | | | | | |

| gp 130 family | | | | | | |
|---|---|---|---|---|---|---|
| IL-6 (Pleiotrohic) | + | + | + | ? | 1,3 | GAS (IRF1>Lys6>IFP) |
| Il-11 (Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |
| | | | | | | |

| g-C family | | | | | | |
|---|---|---|---|---|---|---|
| IL-2 (lymphocytes) | - | + | - | + | 1,3,5 | GAS |
| IL-4 (lymphocytes) | - | + | - | + | 6 | GAS (IRFI = IFP >> Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |
| | | | | | | |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |
| | | | | | | |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |
| | | | | | | |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase. alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance. SRE. IL-2. NFAT, or Osteocalcin promoters can be substituted, alone or in combination (e.g., GAS/NF-KB/EGR. GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast). HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20.000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1 %Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish. using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H 10, and H 11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCI, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR 1 (early growth response gene 1), is induced in various tissues and cell types upon activation. The promoter of EGR 1 is responsible for such induction. Using the EGR1 promoter linked to reporter molecules, activation of cells can be assessed.

Particularly, the following protocol is used to assess neuronal activity in PC12 cell lines. PC 12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin. and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an Xhol site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium. sodium. pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford.MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above. this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally. rectally, parenterally, intracistemally. intravaginally. intraperitoneally, topically (as by powders, ointments, gels, drops or transdermal patch), bucally, or as an oral or nasal spray. "Pharmaceutically acceptable carrier" refers to a non-toxic solid. semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3.218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients. carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts. laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties.

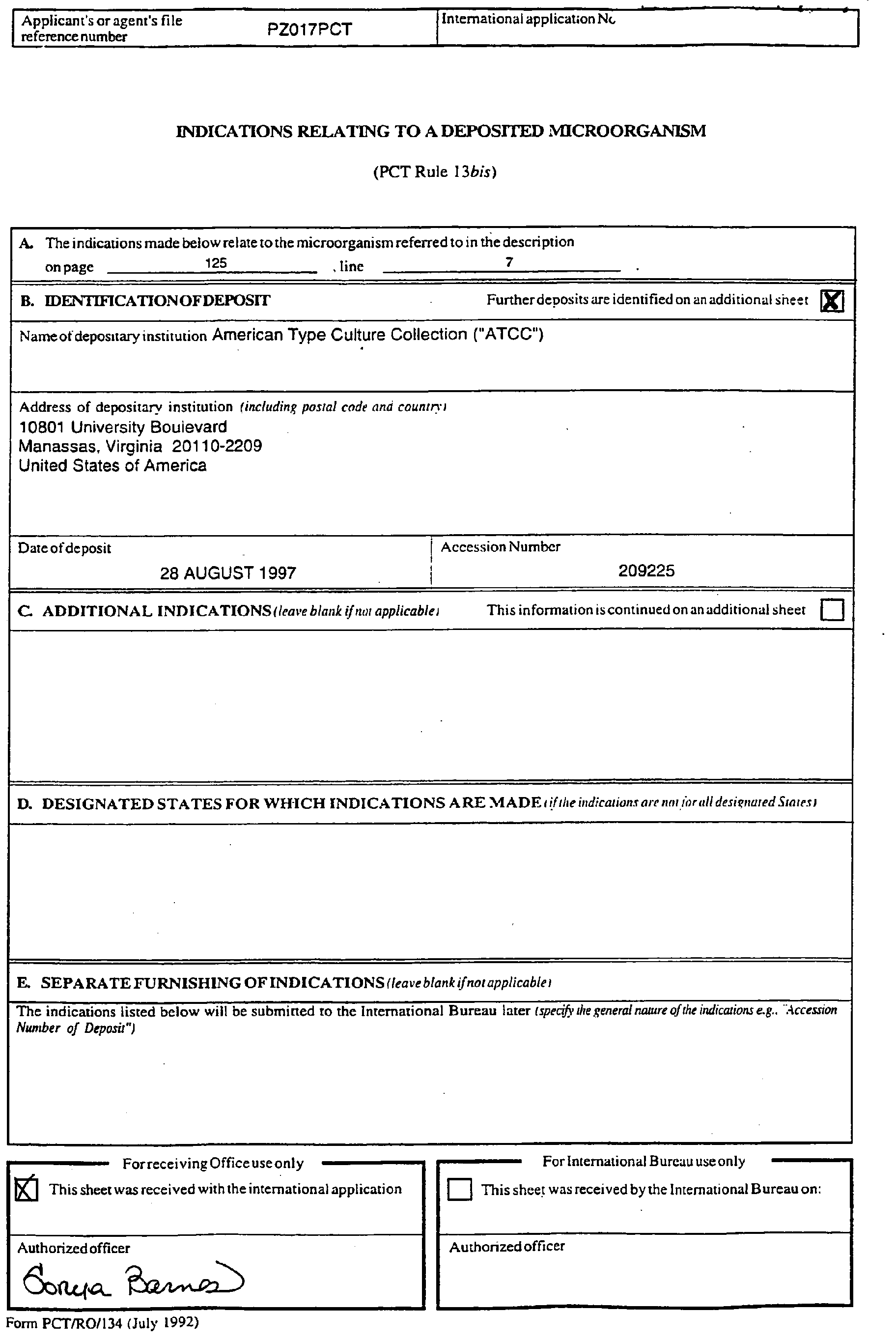
Applicant's or agent's file reference number | PZ017PCT | International application No.

INDICATIONS RELATING TO A DEPOSITED MICROORGANISM

(PCT Rule 13bis)

A. The indications made below relate to the microorganism referred to in the description
on page 125 , line 7 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
28 AUGUST 1997 | 209225

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[X] This sheet was received with the international application | [ ] This sheet was received by the International Bureau on:

Authorized officer | Authorized officer


Form PCT/RO/134 (July 1992)

Applicant's or agent's file reference number | PZ017PCT | International application N

INDICATIONS RELATING TO A DEPOSITED MICROORGANISM

(PCT Rule 13bis)

A. The indications made below relate to the microorganism referred to in the description
on page 126 , line 15 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
28 AUGUST 1997 | 209226

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:

Authorized officer | Authorized officer
Sonya Barnes

Form PCT/RO/134 (July 1992)

Applicant's or agent's file reference number | PZ017PCT | International application No.

INDICATIONS RELATING TO A DEPOSITED MICROORGANISM

(PCT Rule 13bis)

A. The indications made below relate to the microorganism referred to in the description
on page 127 , line 17 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
04 SEPTEMBER 1997 | 209235

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:
Authorized officer | Authorized officer
Sonya Barnes |

Form PCT/RO/134 (July 1992)

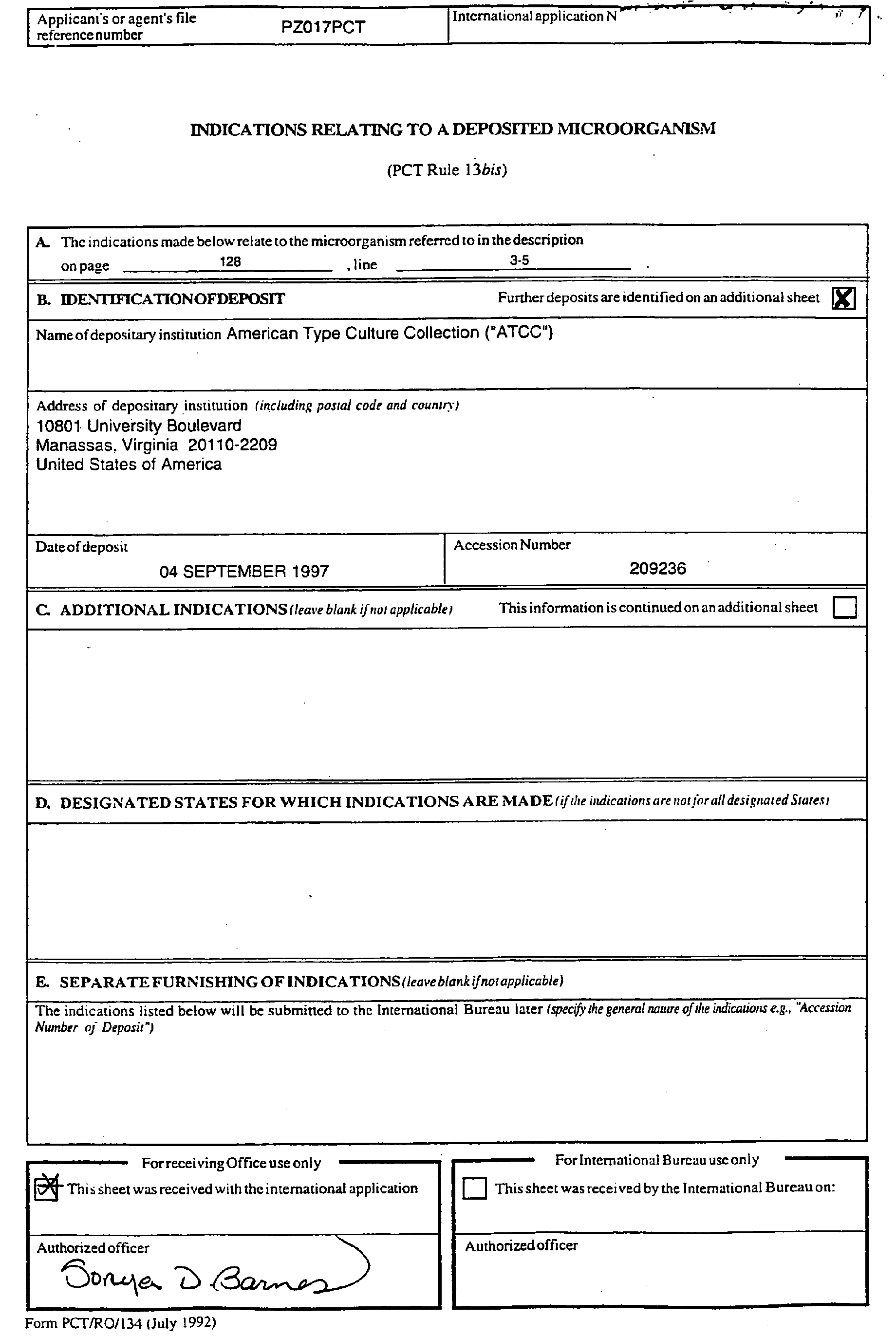
Applicant's or agent's file reference number | PZ017PCT | International application N

INDICATIONS RELATING TO A DEPOSITED MICROORGANISM

(PCT Rule 13bis)

A. The indications made below relate to the microorganism referred to in the description
on page 128 , line 3-5 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
04 SEPTEMBER 1997 | 209236

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[X] This sheet was received with the international application | [ ] This sheet was received by the International Bureau on:

Authorized officer | Authorized officer

Form PCT/RO/134 (July 1992)

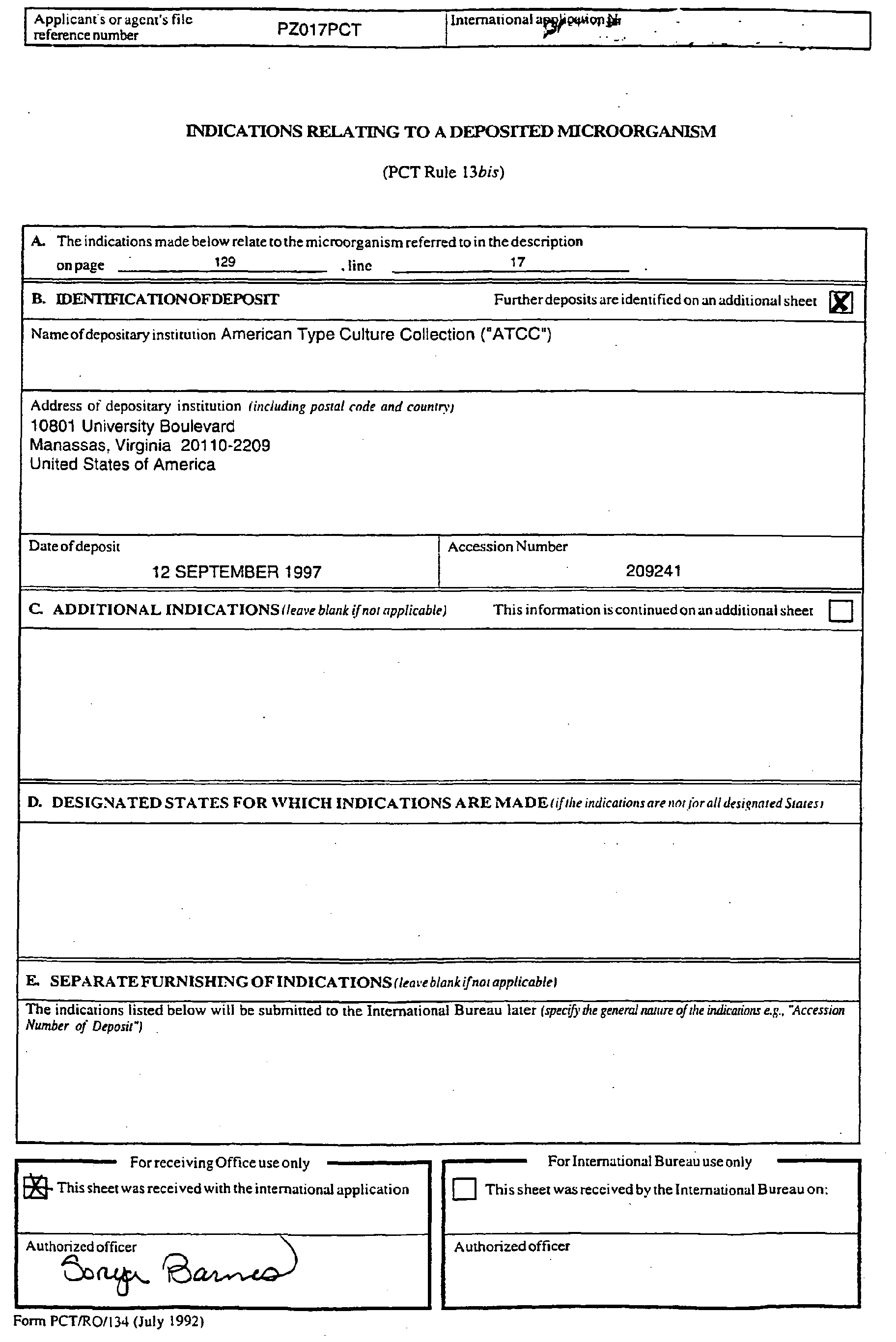
Applicant's or agent's file reference number | PZ017PCT | International application No.

INDICATIONS RELATING TO A DEPOSITED MICROORGANISM

(PCT Rule 13bis)

A. The indications made below relate to the microorganism referred to in the description
on page 129 , line 17 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
12 SEPTEMBER 1997 | 209241

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:
Authorized officer | Authorized officer
 |

Form PCT/RO/134 (July 1992)

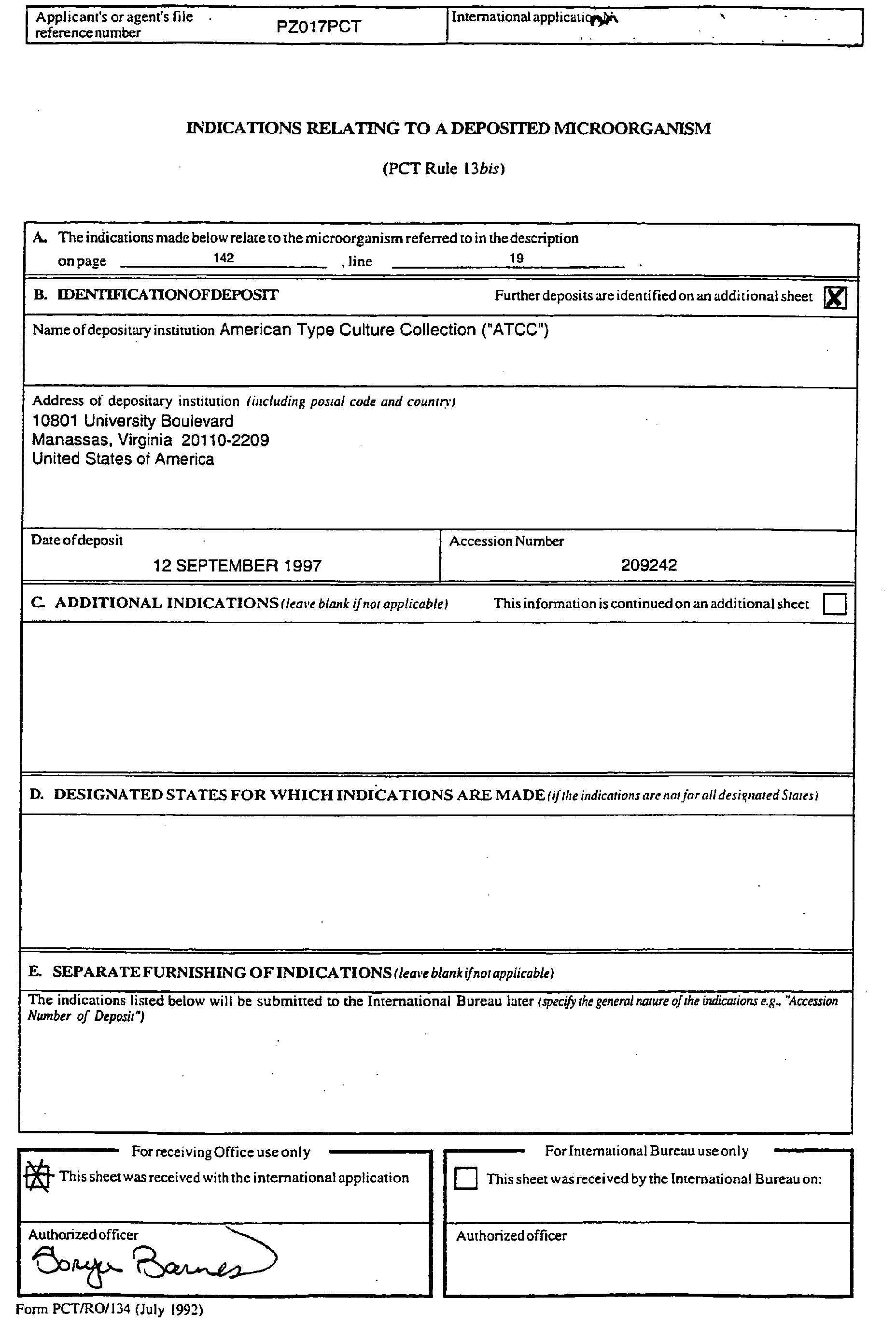
Applicant's or agent's file reference number | PZ017PCT | International application No.

INDICATIONS RELATING TO A DEPOSITED MICROORGANISM

(PCT Rule 13bis)

A. The indications made below relate to the microorganism referred to in the description
on page 142 , line 19 .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
12 SEPTEMBER 1997 | 209242

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[X] This sheet was received with the international application | [ ] This sheet was received by the International Bureau on:

Authorized officer | Authorized officer
 |

Form PCT/RO/134 (July 1992)

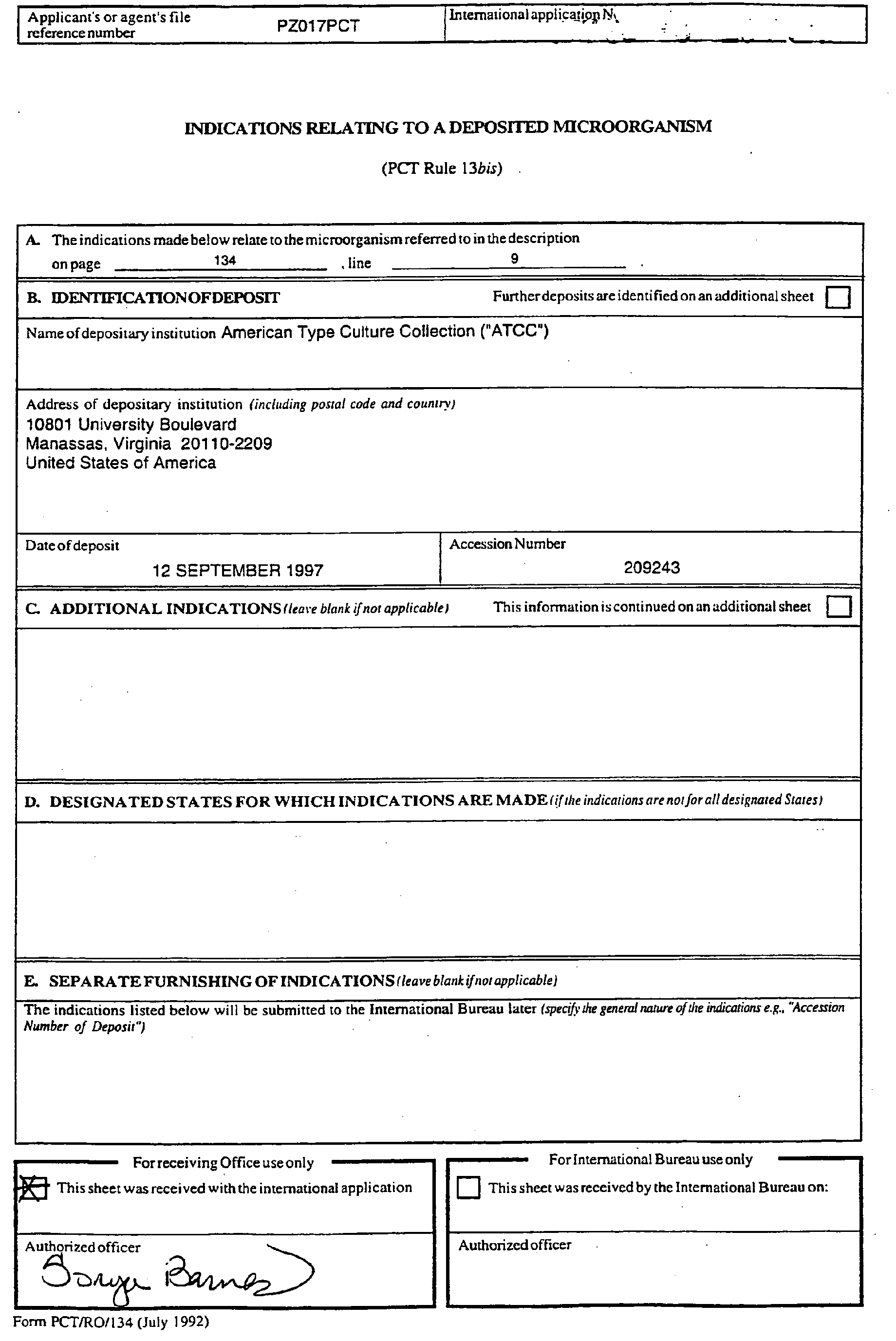
Applicant's or agent's file reference number | PZ017PCT | International application N[?]

INDICATIONS RELATING TO A DEPOSITED MICROORGANISM

(PCT Rule 13bis)

A. The indications made below relate to the microorganism referred to in the description
on page 134 , line 9 .

B. IDENTIFICATION OF DEPOSIT | Further deposits are identified on an additional sheet [ ]

Name of depositary institution American Type Culture Collection ("ATCC")

Address of depositary institution (including postal code and country)
10801 University Boulevard
Manassas, Virginia 20110-2209
United States of America

Date of deposit | Accession Number
12 SEPTEMBER 1997 | 209243

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[X] This sheet was received with the international application | [ ] This sheet was received by the International Bureau on:

Authorized officer | Authorized officer

Form PCT/RO/134 (July 1992)

## Claims

1. A nucleic acid molecule comprising a polynucleotide having a nucleotide sequence selected from the group consisting of:
(a) a polynucleotide having the entire nucleotide sequence of SEQ ID NO: 23;
(b) a polynucleotide having the entire cDNA sequence contained in clone HSIEF95 included in ATCC Deposit No. 209226 which is hybridizable to SEQ ID NO: 23;
(c) a polynucleotide fragment of SEQ ID NO: 23;
(d) a polynucleotide fragment of the cDNA sequence contained in clone HSIEF95 included in ATCC Deposit No. 209226, which is hybridizable to SEQ ID NO: 23;
(e) a polynucleotide encoding a polypeptide fragment of SEQ ID NO: 125;
(f) a polynucleotide encoding a polypeptide fragment encoded by the cDNA sequence contained in clone HSIEF95 included in ATCC Deposit No. 209226, which is hybridizable to SEQ ID NO: 23;
(g) a polynucleotide encoding a polypeptide fragment of SEQ ID NO: 125 or a polypeptide fragment encoded by the cDNA sequence contained in clone HSIEF95 included in ATCC Deposit No. 209226, having biological activity;
(h) a polynucleotide encoding a polypeptide fragment of SEQ ID NO: 125 or a polypeptide fragment encoded by the cDNA sequence contained in clone HSIEF95 included in ATCC Deposit No. 209226, which is secreted;
(i) a polynucleotide encoding a full length polypeptide of any one of SEQ ID NO: 125 or being encoded by the cDNA sequence contained in clone HSIEF95 included in ATCC Deposit No. 209226;
(j) a polynucleotide encoding a polypeptide domain of SEQ ID NO: 125;
(k) a polynucleotide encoding a polypeptide domain encoded by the cDNA sequence contained in clone HSIEF95 included in ATCC Deposit No. 209226, which is hybridizable to SEQ ID NO: 23;
(l) a polynucleotide encoding a polypeptide epitope of SEQ ID NO: 125;
(m) a polynucleotide encoding a polypeptide epitope encoded by the cDNA sequence contained in clone HSIEF95 included in ATCC Deposit No. 209226, which is hybridizable to SEQ ID NO: 23;
(n) a polynucleotide which is a variant of SEQ ID NO: 23;
(o) a polynucleotide encoding a variant of SEQ ID NO: 125;
(p) a polynucleotide which is an allelic variant of SEQ ID NO: 23;
(q) a polynucleotide encoding an allelic variant of SEQ ID NO: 125;
(r) a polynucleotide which is a species homologue of SEQ ID NO: 23.
(s) a polynucleotide which encodes a species homologue of SEQ ID NO: 125;
(t) a polynucleotide which is at least 90% identical to a polynucleotide as defined in any one of (a) to (s);
(u) a polynucleotide encoding a polypeptide having an amino acid sequence at least 95% identical to the amino acid sequence of a polypeptide encoded by a polynucleotide of any one of (a) to (t);
(v) the polynucleotide of any one of (a) to (u), wherein the polynucleotide fragment comprises a nucleotide sequence encoding a secreted protein; and
(w) a polynucleotide capable of hybridizing under stringent conditions to any one of the polynucleotides specified in (a) to (v), wherein said polynucleotide does not hybridize under stringent conditions to a nucleic acid molecule having a nucleotide sequence of only A residues or of only T residues;
or the complementary strand of polynucleotide (a) to (w).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. A gene corresponding to the cDNA sequence of SEQ ID NO: 23 or encoding the polypeptide having the amino acid sequence of SEQ ID NO: 125.

5. A vector comprising the nucleic acid molecule of any one of claims 1 to 3 or the gene of claim 4.

6. A method of making a recombinant host cell comprising introducing the nucleic acid molecule of any one of claims 1 to 3, the gene of claim 4 or the vector of claim 5.

7. A recombinant host cell containing the nucleic acid molecule of claims 1 to 3, the gene of claim 4 or the vector of claim 5 or produced by the method of claim 6.

8. The recombinant host cell of claim 7 which expresses a polypeptide encoded by the nucleic acid molecule of any one of claims 1 to 3 or the gene of claim 4.

9. A method of making a polypeptide encoded by the nucleic acid molecule of any one of claims 1 to 3 or the gene of claim 4 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3, the gene of claim 4 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3, the gene of claim 4 or the antibody of claim 11.

23. A method for the production of a pharmaceutical composition comprising the steps of the method of claim 14 and (d) formulating the compound identified in step (c) in a pharmaceutically acceptable form.


<110> Rosen et al.
      Human Genome Sciences, Inc.

<120> 101 Human Secreted Proteins

<130> PZ017.PCT

<140> Unassigned
<141> 1998-10-01

<150> 60/060,837
<151> 1997-10-02

<150> 60/060,862
<151> 1997-10-02

<150> 60/060,839
<151> 1997-10-02

<150> 60/060,866
<151> 1997-10-02

<150> 60/060,843
<151> 1997-10-02

<150> 60/060,836
<151> 1997-10-02

<150> 60/060,838
<151> 1997-10-02

<150> 60/060,874
<151> 1997-10-02

<150> 60/060,833
<151> 1997-10-02

<150> 60/060,884
<151> 1997-10-02

<150> 60/060,880
<151> 1997-10-02

<160> 254

<170> PatentIn Ver. 2.0


<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180

tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733

<210> 2
<211> 5
<212> PRT
<213> Homo sapiens

<220>
<221> Site
<222> (3)
<223> Xaa equals any of the twenty naturally ocurring L-amino acids

<400> 2
Trp Ser Xaa Trp Ser
1               5

<210> 3
<211> 86
<212> DNA
<213> Homo sapiens

<400> 3
gcgcctcgag atttccccga aatctagatt tccccgaaat gatttccccg aaatgatttc 60
cccgaaatat ctgccatctc aattag 86

<210> 4
<211> 27
<212> DNA
<213> Homo sapiens

<400> 4
gcggcaagct ttttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120
gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg                                    32


<210> 7
<211> 31
<212> DNA
<213> Homo sapiens

<400> 7
gcgaagcttc gcgactcccc ggatccgcct c                                     31


<210> 8
<211> 12
<212> DNA
<213> Homo sapiens

<400> 8
ggggactttc cc                                                          12


<210> 9
<211> 73
<212> DNA
<213> Homo sapiens

<400> 9
gcggcctcga ggggactttc ccggggactt tccggggact ttccgggact ttccatcctg      60
ccatctcaat tag                                                         73


<210> 10
<211> 256
<212> DNA
<213> Homo sapiens

<400> 10
ctcgagggga ctttcccggg gactttccgg ggactttccg ggactttcca tctgccatct      60
caattagtca gcaaccatag tcccgcccct aactccgccc atcccgcccc taactccgcc     120
cagttccgcc cattctccgc cccatggctg actaattttt tttatttatg cagaggccga     180
ggccgcctcg gcctctgagc tattccagaa gtagtgagga ggcttttttg gaggcctagg     240
cttttgcaaa aagctt                                                     256


<210> 11
<211> 552
<212> DNA
<213> Homo sapiens

<220>
<221> SITE
<222> (186)

<223> n equals a,t,g, or c

<400> 11
ggcacgagct tgttcttatg ggctttatat gtcatctata tgttgatgaa aataaatttc 60
tatcccttgc ccaagcctaa acttcatact agcatatcca actgcctact ggacatctcc 120
atttataagc ctagtagcct aataagcata acctcagact taccaggcct cacactgaag 180
tcatgnaact tcagcccaac ccccatgcca gggcaaaacc ttgttgttac ctcttattcc 240
tctcttgcct catcccatcc atgttcagtc tgtcagtgga tcctgtgagt ccagtcttga 300
ggatagttcc aggatctgat cacttctcac tgcctctttt gctgccacca cctctggcct 360
ggataattgc agcagcctcc cagttagcct tgctgtgtcc atccttgttt tccccttctg 420
tctgctctca acagaggagc tagtgattct cttaggacag aataaatcat ttaggttttc 480
ttcacatggt cctgaagaag cttcctacct cactcagtgt aaaaaccaaa aaaaaaaaaa 540
aaaaaaactc ga 552

<210> 12
<211> 1434
<212> DNA
<213> Homo sapiens

<400> 12
cattaaactc tttttatcgg gaatagtatg atattttcaa tgtcactcca ttcatgttga 60
tttggagctg acagttattt tgtgtaagca gagatttaat tttatattga aagtcagtgc 120
aaaattatga ataggatata ctaataaata caaagtaata acaaaagtca aagcagtgtt 180
ctaaataaaa attctgggtt ccttaaaaat tattttaaat ttatcttgaa atagttttct 240
tagattaatc tcaggatatg agaaagtcaa ttaagtgtga gtaaagttag tatcattaaa 300
caaattgtct attaaatgca mgagtggtaa tatacagaat ttatcaggca ttaccaagtc 360
taggcacata taggaaatgc agcactcaga atggtttcaa tgtagtagtt gatgcttgta 420
aggtagggga gcttattcag acatagtaga tagtttctct aatgctgtst caattgctgg 480
cctttggcta cctgtacttc cscattatgg cagcccattc agtcttgagt tttcttctct 540
ggacacctta tgctctgaaa tcatgagcga ggctgattca attggtgatt tgggtagaaa 600
gcagtatgtt ttgctgacat taagatgtag gttatagata ggtttagcct ttaagtgtat 660
gtttttatac tttaaaataa gaaatataac cttttaagct attccacctc ctcccccagc 720
ctatctcaaa ctggtggaat atatggagag atcttgaaag aagtaaaata aaccttcact 780
gctccactcc aggtgaatcc gcccactccc actgacctag tagaatttgt aatttaatac 840
ttaccttcta tttctgaaat cagttgtgaa ctgttgcctt atgttcagar gtttaagaac 900
ctcmgtgaat tcatttttta aaatctgcta ttctgagaag cattgaatga attcttaaca 960
agaagactca tctgtagctg tttgctgact cctatgagcc ccataagggt tctgtgctta 1020
gcattaacaa aataaggttt ataggtaaag ccaatgtatt aatttttttt tgcatggagg 1080
gctttaaaat ttgtgctctt tttcatattt tattcatatt caatttatgg tttgtaactg 1140
ctttttaggg agataattat atgttataaa ttagttttgg ggggaataat tgtgcaaaga 1200
ggataattta atttacgtgc ttctgttatt cagaataaag agagaagact acgctgcata 1260
ttcaagagtt gtaccttaac attggtgaaa cattttttct aagattttca aaaggaatat 1320
gtgtaaattg agaaatcata accactgtcc taacttggta aacaaactgt tcttaaataa 1380
agtatttaat gattttaaaa aaaaaaaaaa aaaaaaaaaa aaaaaaaaaa aaaa 1434

<210> 13
<211> 1881
<212> DNA
<213> Homo sapiens

<220>
<221> SITE
<222> (70)
<223> n equals a,t,g, or c

<220>

<221> SITE
<222> (126)
<223> n equals a,t,g, or c

<220>
<221> SITE
<222> (1860)
<223> n equals a,t,g, or c

<400> 13
atttttcctt ttccttaaca atacctttgg ccattttttt ccagttcact atgtttgtat 60
actaactttn cttcagcctt ttaatgcgaa gcaactagta gagcatgctt tcaggatctg 120
acagcnctgc tagtagagcg aagtatttat taatacagaa ttaaccttmg cccctttaaa 180
gtcaagtctg tctaatctaa ctagcgcctc gctttgcctt ctcacaatgc tcactagcca 240
tcatgctcac ccttctcttc cagatccact tcctcatgat actgtcttct aactgggctt 300
acttaaagga tgcgagcaaa atgcaggctt accaggatat caaagcaaag gaagaacagg 360
aactgcaaga tatccagtct cggtcaaaag aacaactcaa ttcttacaca taaatgtttg 420
ccagagtgtt tcggccgacg tatttacagc tctgacaaat catcagacag ctgctctgca 480
gtacagatgt gtatcccacc aaactaatgt agatgtacaa acacttcact gtctgtctca 540
agctgctggg atgtatctct aggaaaacct tccagtgggt aaatcttttt ctttagaaca 600
aatattggag gtttcatgtt agccatttta aaaggcaaca ctttgacaaa atgatcgttc 660
atactttggg aatttgtggc atgttcacat ttattgctag ggcaattcta ccaagacact 720
caatggaata tgtcacactc cttaataggg acctgtgact ccttaataag gacctgtgac 780
atgcccagca tcaagggata agaccgtaaa ttcacatata tgccatctgt cctcaagtgt 840
tatctacata ggaaataaaa tggaattgat gtaaagttcc atttctgaca gctgacattt 900
attaaacttt ggatcaaaga taatgtgatt cttatgattg atttctcaaa ctagcttttc 960
cctcccaagt ccaggaccca ttaatttcct gagccaatca gaaatatatt tttcaataat 1020
gctaaaatta gctacaattc tgctgaccct actattaaag aatctggatg ctggactcac 1080
tgacaagctt tccagaagca attttataac agatttcatt ttaacaaaat actgatccaa 1140
ttttcattat tcttgagaaa tgtcagcttt gccttaatga gtatttgctt taaatttcta 1200
agaatttata tcataactag agacccaaat atctttcaca gaattttgtt ccataaatgt 1260
ttttcttaat tattaagaag tgttacctta ttaaaatgac caccattcta aaccattttt 1320
cagtggtctg gatacgaagt ttacagtttc ataccaacta tctaaaacct aattgcaaat 1380
tgaccacaga cctctaacct cctactttta tagacttgaa tacttaagta atttaaatta 1440
gggttggtat ttcatttttt tcttatctaa atcttagttt cctggaataa taaagtttga 1500
tgttcagcaa gagaactgct tgagtttaag ccattttcaa aagaaacttg ccttttacat 1560
tattgtgttc cagaacatta agtgactgta ggtactgggt attagtgatg gtaaactttg 1620
tgttgctctt tatgaaatga tccatataac tgttgggtgc atcagtgctt ttcaaagggg 1680
ctgcttacta tagggttaac tatgtatatt cattgttaag agttaacttg tggtttggct 1740
gttycctgga ttttataaca tacatgtgca gaaatgtatt caaatgaaag gaagcatacc 1800
tttatcaaga tgctattaaa attgaacatc aagtataaaa aaaaaaaaaa aaaaaaattn 1860
ctgcggccga caagggaatt c 1881

<210> 14
<211> 1060
<212> DNA
<213> Homo sapiens

<400> 14
gaattcggca cgagggtgga ggacaaccgt ttacctccrc cccgctggaa atcctgttct 60
ttctgaacgg gtggtataat gctacctatt tcctgctgga acttttcata tttctgtata 120
aaggtgtcct gctaccatat ccaacagcta acctagtact ggatgtggtg atgctcctcc 180
tttatcttgg aattgaagta attcgcctgt tttttggtac aaagggaaac ctctgccagc 240
gaaagatgcc actcagtatt agcgtggcct tgaccttccc atctgccatg atggcctcct 300
attacctgct gctgcagacc tacgtactcc gcctggaagc catcatgaat ggcatcttgc 360
tcttcttctg tggctcagag cttttacttg aggtgctcac cttggctgct ttctccagta 420
tggacasgat ttgaagtaca gaatttcagc cagcagccca tcaggctgac accacacata 480

ttgcttctgg tactttagcc acaccagtga gaattggtgg ggcaagttgt cctgagaaag 540
gctgtgtggc ttttcttcag cacagacatt tgggcaagca actcagcata aggccagtgg 600
gtaccatctt ctaaaccagg accatcagcc caagagactc ttctacactc cagtataggg 660
aggggcaagg ttattcccat cctgcccctt ctcagaacca gtcccctgct gacctcaagt 720
tctcctcctt gatcaccgtg gccagagcat ctcgtgtgga ccatctaggc tccttgggct 780
tcaagcagga cctgagccac atgctccctg tacgagctgt gctatacctg tcccacatga 840
gcacggagag cctcatgttg gtgggtttcc agagtgatgt gaaagcctct caccccaatc 900
ctcggagact gagttccaca acttttttag tagctcatag tgttattttt ctactctctt 960
catgaaacta actttatttt ataataaata trtatttcct gttgtggggg aaaaaaaaaa 1020
aaaaaaactt cgaggggggg caccggtacc caatcgaccc 1060

<210> 15
<211> 1255
<212> DNA
<213> Homo sapiens

<400> 15
ttcccaactt tctgccacac ttaaattacg ttcctccatt tcagttttgt cttttctgtc 60
taaagttcag tcaaagagta tcaaaaaatt atgtttcagc tagactggtg taatgtataa 120
gtttttgtat cttgtattag aggatttcgt agcttttatt agaggctcat ttccacctca 180
gcatacaaga tcgttagtct tttggcatgt gtgccaatta gaatactaaa gcaagtccaa 240
gcacattttt ctcttctcac gtttctaata agtgttaggg actttgcctc ttttacttac 300
cacgtcccca aaagtgtcag gtagacatgt cacaaatggc tctgtagaga gccatgggaa 360
gagagaggag gtggatgtgg aacataaagg gttcagaaac tccagaagag gagtgggttt 420
tggatagaag catttgagga cagctgctcc aaagccttat gtgtatgatg aaacttaacc 480
acggggaaga gactcttcag tagcctgttc tgtctggtga tttttatttt aagtgaacct 540
ttggatctat ctttaactct ctttattgtg agtctaaatt ccaattctgc agcagatcag 600
taaactcaca gtattttccc tgtggaaatc tattcaataa ggaaaccaag acaggataat 660
aaaatttaaa aaaaaaacaa ctttgaattc ccctgcctag gtcttccagt tgttttccag 720
cacatacctc aggtatgact ttgctagcyg gggacaaaat tagcaccttc cgawtctcta 780
gtccaaatga actttgtgct aaataaaaaa ttattatact acataataaa gttacagaya 840
gcaggaaatg caagagctag gagattccta gattatatct gccaagcaaa taccttaaac 900
atccacctga aatcctacta ccccctcttc tgagataatt tgcccagccc ttctcttccc 960
acacactcac tcaatgtcac ccccttctaa tccccaaaac tgtttttgtg gtctttgtag 1020
cctatagtag ttttctcaca tctttccccc tagacttttc tgtttttcag tttcagacaa 1080
aaaaactctt cagctttttc cagtgtgtct ccttaacagt aactttacca cttgaaatct 1140
tatttcatag aaaaactaaa ttggtgtgga aaggctgcac acaataaagt tatattatta 1200
tccatgaaaa aaaaaaaaaa aaaaaaaaaa aaaaaaaaaa aaaaaaaaaa ctcga 1255

<210> 16
<211> 1036
<212> DNA
<213> Homo sapiens

<400> 16
gcgcgtaata cgactcacts atagggcgaa ttggagctcc accgcggtgg cggccgctct 60
agaactagtg gatcccccgg gctgcaggaa ttcggcacga gtgaagtact gcgtggtgta 120
tgataacaac agcagcaccc tggagatact cttaaaagat gatgatgatg attcagactc 180
tgatggtgat ggcaaagatc ttgtgcctca agcagccatt gagtatggca ggatcctgac 240
ccgcctcacc caccaccccg tctacatcyt gaaagggggc tatgagcgct tctcaggcac 300
gtaccacttt ctccggaccc agaagatcat ctggatgcct caggaactgg atgcatttca 360
gccatacccc attgaaattg tgccagggaa ggtcttcgtt ggcaatttca gtcaagcctg 420
tgaccccaag attcagaagg acttgaaaat caaagcccat gtcaatgtct ccatggatac 480
agggcccttt tttgcaggcg atgctgacaa gcttctgcac atccggatag aagattcccc 540
ggaagcccag attcttccct tcttacgcca catgtgtcac ttcattgaaa ttcaccatca 600
ccttggctct gtcattctga tcttttccac ccagggtatc agccgcagtt gtgccgccat 660

catagcctac ctcatgcata gtaacgagca gaccttgcag aggtcctggg cctatgtcaa 720
gaagtgcaaa aacaacatgt gtccaaatcg gggattggtg agccagctgc tggaatggga 780
gaagactatc cttggagatt ccatcacaaa catcatggat ccgctctact gatcttctcc 840
gaggcccacc gaagggtact gaagagcctc acctgggggc atttgtgggg tggagggcca 900
gagtgtgtat acccaggctt gtctggaagg agaaggcctt tgctgcctga aagtcwmaaa 960
aaaaaaaaaa aaaackcgag gggggcccgg gtacccagct tttgttccct ttagtgaggg 1020
ttaatttgcg cgtccg 1036

<210> 17
<211> 1014
<212> DNA
<213> Homo sapiens

<400> 17
gaattcggca cgagtttaca tcagaaaaga gctggaagtc ttcctgcaat gaaggagaat 60
cctcttctac ttcttatatg catcaraggt cacctggtgg tcccaccaaa ctgattgaga 120
tcatctcaga ctgcaactgg gaggaagatc ggaacaagat tttragcatc ttatcccagc 180
acatcaatag caacatgcca caatcactta aggtgggcag cttcatcatt gagttggctt 240
ctcagcgaaa gagccggggt gagaagaacc ctcctgttta ttcttctcgt gtgamaatct 300
ctatgccatc atgtcaagac caagatgata tggctgagaa atctggatca gagactcctg 360
atggtccatt gtcccctggg aaaatggagg atatctctcc tgtgcagaca gatgccctgg 420
attcagtgag ggagagatta catggaggca aaggtctgcc tttttatgca gggctttctc 480
ctgcagggaa gcttgtggcc tataaacgta aacccagttc aagtacatct gggcttatcc 540
aggtgagaat tatctttaat ctgggtatag cacctttgta tacacctagg tagtatcatg 600
atttttcaga gccctttatg gtcctgatat cctttatctt gacatttcct gggaactggg 660
tgacaaaatt attatctctt tttgtaatag gcctagttta gatgcatacc tagagtgaat 720
ttttgtcaca tttatgaaca gaaacgtaga gccttgtatt agttttaatt ttctttctaa 780
tcttcccaga aagttgctct tcataaactt tattgcctgc aggctctagt gatactttga 840
caataaagca agggtaatca gggattcagt ctagctcttg gaatttatta ttagcagata 900
ggtttcaaaa caaaaccatg gttagaacgg taggtgtaag ggaagatgaa aattgactta 960
aagataggca atatatgttt agaaacttgg ggaaaaaaaa aaaaaaaaac tcga 1014

<210> 18
<211> 1287
<212> DNA
<213> Homo sapiens

<220>
<221> SITE
<222> (1282)
<223> n equals a,t,g, or c

<400> 18
gaattcggca cgagatttac taaaatgatg taataaataa catgttaata gactcaagct 60
ttaccttatg aaattgatgt atttttacca gttatttcta atgtaacatt gaatatataa 120
gatctgacaa atgtatgttt aaacatgaat tagaagagtt gagaactacc attatgtata 180
gggattctca tagtgtcttg gcccttaatt ggaaagttgt ggcaacttta aagtactttt 240
tactgtatgt tataattctt tataacttag agagagacaa tggtcactca aactatgaga 300
actatgaatt aggagataaa agtttaaatt tgttgttgtt ttataacagt atgtacaagt 360
tagttttccc ttatatattt acgttttcaa gtttttaaat ctcatcatat acatccatac 420
tctataaaat gttttatatt caaagaactg taaaatccta aacattagtt ttcactattg 480
aaattgtttt ttaaagatag gcataaatag ttgtccttag acttattcat acaaatatag 540
tcatttactt ctatgtagtt tgagattctg agagttattc caactttatg aagattgatt 600
tcaatgtgcc tgctaagtcc taaaagattc agaaagaaaa tttatatatt attgatttaa 660
atatcatcct ttaaatatgt tgtataacat tcaatatagt ttatgtatca gtgattgtat 720
tttattctga atgcatgatc tcaagcctta actactataa tctttttctg cccctcagaa 780

attgaataac ctaaccaaga tgcctttagg ggatgcccta agtaaatgta atttcagatt 840
tcagggtttt tttttttcc tctctaagtg ttccttccct ttcttctcct gctctccatc 900
atgttatgga gaccagtgag gaaccagtgt taacttggtg acaatgtgac agctggtgct 960
ttatctaagc tccgttttct atttcttggg aatgctttat tgtggaaact gcttcagata 1020
cttaaattga atcataactt gcttctgtaa attgcgtaaa gacaacaaac tgattttagt 1080
ttgaaaagtt tatcttttac ttgtaaacct tgtttgccag ttaccttccg aaagctgtgt 1140
aaagagttat ttttaacaaa gtcttaacaa tatatgttac tttttagata ctatagaaaa 1200
taataaatat aacctgtaaa ccacaaaaaa aaaaaaaaaa aaactcgagg gggggcccgg 1260
tacccaatcg csgwgtgatg gngctat 1287

<210> 19
<211> 1105
<212> DNA
<213> Homo sapiens

<400> 19
gaattcggca cgagtggcaa cacaagcacc tagctcagag atcttgaaga atgaaatgag 60
attatgtaaa taacaactta ccacagtgct tggcacatag taagtgctca atgtcagcta 120
tgattattat tattcccttc ttaacacaca aagaaggagg ggatccaaaa ataacagtgt 180
gccacagttt gaaaggcatt tatttgatct tgtctctaaa tttccatttt acatgtagca 240
cttacccggt ggaagtgaaa atacagtgaa cgctaaaaag ccctgtgtct ctcggtggtg 300
tctggacaac cctggcaact cggaacatga aggagagaac aagaattccc tgtgcttttc 360
cttttcttct tttccaaaca cgtgtgcaga cttcccctgc atttcagccc caccctcttt 420
attttactgc ctaatctata aaggaggatt aacagcagca cgctgctttg gcatagagca 480
gattctgggt gaggacctgt aggtagagtt taatgaatac aattttctag gactgtgagt 540
gcatattttt agctccatgc tgggcttcag cgttggctct tgagacagat gaacagactc 600
tttgatcaga cttgggtgtt gctccaagaa gaacttttct cagaaagtcg ttaggaaaaa 660
aaattgtctt ctgttgccct tattcctaat gtgcactcta tagattcaga ttccagataa 720
cttgtcctga tctcagtaaa ttaattgcat tgcaacattg agttacacca ctgtggaaag 780
aaaaagtact tctgggcagg aacagatcca ctttctcaca aaagagaatg gctggtgttc 840
aagtgtgtgg ttgccatcct ttcccttttg agagtagggt agaggtagtt aaccttcctg 900
ggggaggttt ggcctagaca acatcataga cactatatcc cccctggagt taccaaacaa 960
taaaactgct tcctttgcca aacacaaaga atggtctgga gttggatatt agcaaacagc 1020
aaaccacata aagaagacaa aaaaaaaaaa aaaaaaaaaa ctcgaggggg ggcccgtacc 1080
caatcgcctg tgatgtatcg tatac 1105

<210> 20
<211> 1089
<212> DNA
<213> Homo sapiens

<400> 20
gaattcggca cgaggagaag atcgctcaca agagtttgaa cataagctgg accacaaagg 60
atagagtaaa tgtggaaaga tggaaaagaa aaaaagaaac ctacaaacac cagatatgta 120
gcccaaaagc ccagcttcta taacttgttc atggctaccg tacatagaag cacccaggac 180
tgcaatccct tttgtataca agtttctttt ctttctgagc caagtcaaga aacctgaaaa 240
ctataaggca ggaaaaaaga agaagattaa grttatccat gatttcatca ctcgggatga 300
ccagtgttat tgtactattt atcttaaaag tgtttttcaa atatttttct acaacatcat 360
ttttaaatgc ttgcatacat tttatacata aatgtaaact agttaactaa ttcctctatt 420
gctggaattt taagatgtct ctaaatgata taaacaatat ttcaaatttt gtgattggga 480
atgtggattc tagaatatga gtgtcaaggt ccaagatttg tctccactgt ttgttaggtg 540
aattgcataa actctataaa ctcagtttcc tactttaaaa aacagaagtg tgtcagtgac 600
agtggtgtat gcctgtagtc ctagctattc tagaggcaga ggggagagga tcacttgagt 660
ccaggagttt aaagctgtag tgtgccatga tctcacctgt gaatagccac tgcactccag 720
cctagacaac acagtgagac ctcatctcta aaaaagaaaa tagggggcta ggcgtggtgt 780
tacgcctgta atcccagcac tttgggaggc tgaggcaggt ggatcacgtg gtcaggagtt 840

cgagaccagc ctggccaaca tggtgaaacc ccgtctctac caaaaataca aaaattagct 900
gggtgtggag gtgcatgcct ataatcccag ctactcagga ggctgaggca ggagaatcgc 960
ttgaacccgg gaggcggtgg ttgcagtgag cgaagatagt gccattgcac tccagcctgg 1020
gtgacagggt gagactctgt ctcaaaaaaa aaaaaaaaaa aaaaaaaaaa aaaaaaaaaa 1080
aaactcgta 1089

<210> 21
<211> 2831
<212> DNA
<213> Homo sapiens

<220>
<221> SITE
<222> (182)
<223> n equals a,t,g, or c

<220>
<221> SITE
<222> (219)
<223> n equals a,t,g, or c

<400> 21
gggtttcctc agacagtgtt ggaggattca gatacagtga aagatatgat cctgagccca 60
aatcaaaatg ggatgaggag tgggataaaa acaagagtgc ttttccattc agtgataaat 120
taggtgagct gagtgataaa attggaagca caattgatga caccatcagc aagttccgga 180
gnaagataga gaagactctc cagaaagatg cagcgacana atkgaggaaa agaaagcgag 240
aagaggcaga tctcccaaag gtgaattcaa agatgaagag gagactgtga cgacaaagca 300
tattcatatc acacaggcca cagagaccac cacaaccaga cacaagcgca cagcaaatcc 360
ttccaaaacc attgatcttg gagcagcagc acattacaca ggggacaaag caagtccaga 420
tcagaatgct tcaacccaca cacctcagtc ttcagttaag acttcagtgc ctagcagcaa 480
gtcatctggt gaccttgttg atctgtttga tggcaccagc cagtgcaaca ggaggwtcag 540
ctgatttatt cggaggattt gctgactttg gctcagctgc tgcatcaggc agtttccctt 600
cccaagtaac agcaacaagt gggaatggag actttggtga ctggagtgcc ttcaaccaag 660
ccccatcagg ccctgttgct tccagtggcg agttctttgg cagtgcctca cagccagcgg 720
tagaacttgt tagtggctca caatcagctc taggcccacc tcctgctgcc tcaaattctt 780
cagacctgtt tgatcttatg ggctcgtccc aggcaaccat gacatcttcc cagagtatga 840
atttctctat gatgagcact aacactgtgg gacttggttt gcctatgtca agatcacagc 900
ctttgcaaaa tgttagcaca gtgctgcaga agcctaatcc tctctataat cagaatacag 960
atatggtcca gaaatcagtc agcaaaacct tgccctctac ttggtctgac cccagtgtaa 1020
acatcagcct agacaactta ctacctggta tgcagccttc caaaccccag cagccatcac 1080
tgaatacaat gattcagcaa cagaatatgc agcagcctat gaatgtgatg actcaaagtt 1140
ttggagctgt gaacctcagt tctccatcga acatgcttcc tgtccggccc caaactaatg 1200
ctttgatagg gggacccatg cctatgagca tgcccaatgt gatgactggc accatgggaa 1260
tggcccctct tggaaatact ccgatgatga accagagcat gatgggcatg aacatgaaca 1320
tagggatgtc cgctgctggg atgggcttga caggcacaat gggaatgggc atgcccaaca 1380
tagccatgac ttctggaact gtgcaaccca agcaagatgc ctttgcaaat ttcgccaatt 1440
ttagcaaata agagattgta aaagaagcag attgaatgaa gaatttttag ctgtgcagat 1500
aggtgatgtt gggatggaaa atgctaatca actacccttt cttttatcaa gtaattaaaa 1560
taaatctaca taaagaacca aaaaggctgt tttataaaag tgaaatatcc agtatttcag 1620
agggccaggc aagagcactt cagatgaggc agtcaaaatc atttttttcc rgtgaggata 1680
gaccacaagt gggtggtgag accattgaaa gcctttatca actgaagagt ccatttaaca 1740
gcataatttg tgggaagact ggaatagggc tgaataaatg tgtttgaatc tctaatttta 1800
tactttcttt tcctgaggaa cttgattttt ctgtccctgg atcgccttgt cataattggg 1860
tctgttcctt ttactaccac tcttgagtcc atatatgaaa tcattaaagt tggatgatca 1920
gtttttttata aaaatatata tttttgtcca agaaaaaaaa aagcatacat atgtgattat 1980
ggctaaatca aaggtaactg gaatgtatat acttttgcta atgttccagc aacactgcta 2040
ttatactatc caaattttta ttgtaacaaa acctctttaa gcaattggtg attgccatgg 2100

gacttttccc atgtcttctg ctgtaattat cctgtgcaga actaggaaga aatttttttc 2160
aggactgctc tatggtttcc tttaaaagaa aaaaacttct gtttgttttt agcagtcatt 2220
atttacaatt tgcagtgatt aacttggcaa ggcttccttc cgtgtttatc cctgtagcca 2280
tcatttaagt caggaacagt cagaaaaata tttattttat tttttttttg ggtgtctgca 2340
aaggtaaaaa tccattaaaa ccttaagtta aatataaatg ttacaactca atgtttgctt 2400
ttagatttta tacagtattt gttttgtttt ggttttgagt gtatataatg cagcattagc 2460
aatatggttc caatagagga gttaaatata tattgttaaa ggagacctgt agcagtcaaa 2520
gattttattg atttaatgac aaaggaaatt aatgaaaatg tttttgtttt tctgctgtaa 2580
ttctgcatta agctcacatg aaaatcayga ttctagagtt tggaatgcaa aattaattgt 2640
tttaccctca agctgggaat atttttcaaa ataaatacta taatatagat atcaaattat 2700
tacctcccca tgttatgttg aaaatttttt tattaaattg ataaaacttt atttccatta 2760
tattcataat gttctgttat acataacatt aaaatgttca ttaaaaaaaa aaaaaaaaaa 2820
ctcgagacta g 2831

<210> 22
<211> 1448
<212> DNA
<213> Homo sapiens

<220>
<221> SITE
<222> (1422)
<223> n equals a,t,g, or c

<220>
<221> SITE
<222> (1434)
<223> n equals a,t,g, or c

<400> 22
gaattcggca cgagcaactg ccctgatcac cccccgtccc agcccttgag tgaacgtcct 60
tctgagcggc ttcctggggt cctccccacg tcccaaaggc cggcaagatg gtgtcctgga 120
tgatctgtcg cctggtggtg ctggtgtttg ggatgctgtg tccagcttat gcttcctata 180
aggctgtgaa gaccaagaac attcgtgaat atgtgcggtg gatgatgtac tggattgttt 240
ttgcactctt catggcagca gagatcgtta cagacatttt tatctcctgg ttccctttct 300
actatgagat caagatggcc ttcgtgctgt ggctgctctc accctacacc aagggcgcca 360
gctgctttac cgcaagtttg tccacccgtc cctgtcccgc catgagaagg agatcgacgc 420
gtacatcgtg caggccaagg agcgcagcta cgagaccgtg ctcagcttcg ggaagcgggg 480
cctcaacatt gccgcctccg ctgctgtgca ggctgccacc aakagtcagg gggcgctggc 540
cggcaggctg cggagcttct ccatgcagga cctgcgctcc atctctgacg cacctgcccc 600
tgcctaccat gaccccctct acctggagga ccaggtgtcc caccggaggc cacccattgg 660
gtaccgggcc gggggcctgc aggacagcga caccgaggat gagtgttggt cagatactga 720
ggcagtcccc cgggcgccag cccggccccg agagaarccc ctaatccgca gccagagcct 780
gcgtgtggtc aagargaagc caccggtgcg ggarggcacc tcgcgctccc tgaaggttcg 840
gacgargaaa aagactgtgc cctcagacgt ggacagctag ggtctgctgc atctgccccc 900
ttcttacctc gtgccctgca kggctccagg gctatttgga gggaccttgg gctgcacatc 960
tggcctgcct gcaccagctg cctgggcycc accctcctga ctcctgctga tggttaaggg 1020
ccgggagcag atgctgccaa ggccacatgc agggatgcac ccacaatgta ccaaagcagg 1080
ctgggcccag ggttctattt attgccttgc tctgccctct cccttccccg gttgtgggac 1140
aagagccctc cctgaacccc tgcaaccctc cctgaacccc tgcaaatgaa accaaacgtc 1200
cacctgggtg tgttcattcc ttcctgtcct tcaaagtact tgatagcctt tcataaggcc 1260
tggcacatgt gtcctggttg tgtgtgtgtg tgttggtgag tgaggtcagg tttgcgagtg 1320
ttttgataaa taaatacata aaggggcaaa aaaaaaaaaa aaaaaaaaaa aacaaaaaaa 1380
aaaaaaaaaa aaaaaaaaaa aaaaaaaaaa aaaaaaaaaa anaaaaaaaa aaanaaaaaa 1440
aaaaaggg 1448

<210> 23
<211> 1211
<212> DNA
<213> Homo sapiens

<220>
<221> SITE
<222> (131)
<223> n equals a,t,g, or c

<220>
<221> SITE
<222> (915)
<223> n equals a,t,g, or c

<400> 23
agagaaagtg gagacggacc tgagcccgag ggagaggcag gcagaggctg aggctgattc 60
caccccagcc tgcctgggac aaccctcctt agccgcagcc ccttccagtt ccctgagggg 120
ttctgcccct nccccctctc tgggggcacc aaccccccag ggtcctgcat cccaccatgt 180
cgatggctgt ggaaaccttt ggcttcttca tggcaactkt ggggctgctg atgctggggg 240
tgactctgcc aaacagctac tggcgagtgt ccactgtgca cgggaacgtc atcaccacca 300
acaccatctt cgagaacctc tggtttagct gtgccaccga ctccctgggc gtctacaact 360
gctgggagtt cccgtccatg ctggccctct ctgggtatat tcaggcctgc cgggcactca 420
tgatcaccgc catcctcctg ggcttcctcg gcctcttgct argcatakcg ggcctgcgct 480
gcaccaacat tgggggcctg gagctctcca ggaaagccaa gctggcggcc amcgcagggg 540
ccctccacat tctggccggt atctgcggga tggtggcmat ctcctggtac gcttcaacat 600
cacccgggac ttcttcgacc ccttgtaccc cggaaccaag tacgagytgg gccccgscct 660
ctacctgggg tggagcgcct cactgwtctc catcctgggt ggcctctgcc tctgctccgc 720
ctgctgctgc ggctctgacg argaccagcc gccagcgccc ggcggsccta ccargctccc 780
gtgtccgtga tgcccgtcgc cacctcggac caagaaggcg acagcagctt tggcaaatac 840
ggcagaaacg cctacgtgta gcarctctgg cccgtgggsc ccgctgtctt cccactgccc 900
caaggarapg ggacntggcc ggggcccatt cccctatagt aacctcaggg gccggccacg 960
ccccgctccc gtagccccgc cccggccacg gccccgtgtc ttgcactctc atggcccctc 1020
caggccaaga amtgctcttg ggaagtcgca tatctcccct ctgaggctgg atccctcatc 1080
ttctgaccct gggttctggg ctgtgmaggg gacggtgtcc ccgcacgttt gtattgtgta 1140
taaatacatt cattaataaa tgcatattgt gaccgttaaa aaaaaaaaaa aaaaaaaaaa 1200
aaaaaactcg a 1211

<210> 24
<211> 1060
<212> DNA
<213> Homo sapiens

<220>
<221> SITE
<222> (453)
<223> n equals a,t,g, or c

<220>
<221> SITE
<222> (1045)
<223> n equals a,t,g, or c

<400> 24
gccacttctt ccaaatacag tagatgtgtc tgctgtgtat ttatacaaca tcctgaacta 60
cttaacatgc tgtttattta cttgtttgta ttccccatta gaataggctc tgagaaagca 120
aagactgtat ctgtcttgct tatcattgta tccctgacag ctcgcccact ggctggcttt 180

taataagcac accataaata tttacttgaa atactcattt ttaaaatgaa cagatgaatg 240
aatgatagat ggatggtgga tggcattatg tagctaaaaa ttgtgtcctg tctctaccta 300
tttttgaaga ccatccttta gtttgcgttt cctgccatgt ttgaggggcc tttttttggt 360
ccataactct tgtcttttat tcaaattaaa acaccgaaca aaagcacatt cgattattgr 420
ccatgrggtt ttttattcyg ctgtcagtgt canccycmtg tctaaatccc cyggggtcaa 480
acttacatat atctggatag cccttttkga tgacgatggt agtctaattt gtgtgttatg 540
tgctcttgaa atgttttgct gtaaagacac tagaactgaa ttttgcttta ttgccaatga 600
tgatgaatgt taaaaaaaac aactcagtaa cattcaaacc aatttccaag tttgttcttc 660
agccagagga acttgcacac tgactttttg taaaggtagc agatttattg tgttgtaatt 720
catacaccat aaaattcacc attttaaagt ttccaattta gtggttttta gtatgtttac 780
agagtcatgc aaccatcacc acagtatcat tgcaggatgt ttttatcatc cctcaaagaa 840
atccagaccc acaggaggct gaggcaggag aatcgcttga acccggaagg cggaggtttc 900
agtgagtccg agatagcgcc actgcactcc agcctagtga cagagcaaga ctctgtctca 960
aaaaaaaaaa aaaaaaaaaa aaaaaaaaaa aaaaaaaaaa aaaaaaaaac tcgaaggggg 1020
ggcccggtac ccaatcgtcc ctatnatgag tcgtattaca 1060

<210> 25
<211> 1057
<212> DNA
<213> Homo sapiens

<220>
<221> SITE
<222> (348)
<223> n equals a,t,g, or c

<400> 25
   gaattcggca cgagcggcac gagattttag gtaaatgacg aagggaatgt ggtgaatgtc 60
   actgtccaga gccataaatc agacaaaacc atacatagca tgctgaaaaa cttttgtaat 120
   ggaacaccca acaaatgaca cctaacctgt ctgtgatcca acaagtccga taacatgctg 180
   ctgtatttgt attctctggg aatctcagta ttaataattt catttcccac aaattctagc 240
   attcatgtaa ggaaaaacat ggctaatcaa tatcttaaag gggcaatctt tcagagcagt 300
   ggttttcaaa gtgtggccgg acagcattgg cagcatctta atctcctngg gactttgtta 360
   aaaatgcaaa ttctcagccc caccctagtc ctactgaatt gggaaactgg cgtgggaccc 420
   agcagtcttt gttttaacat gttctccaag tgattctgat gcctgttcaa acttgggaaa 480
   cacttttaga gcacttgagg aacctaaaag atgactggtt cagcattttg tgtggtagat 540
   aagaaagaaa ttatcacaaa aaatcagaaa tgaacagtga gagaaaaata ggaccccaga 600
   cagtttatac cttccatttg ctgttttaaa agtgtgagcc tgccaagtca acaagtatgc 660
   ctttagcgca catgtaaata gcctgcactt cctaaatctc gtgtggcctc ccatggttac 720
   attcttcaaa ggtwaactga gttgagagga agattcagca tttaaaagag aagggttgaa 780
   aaagattgtg tgtgtgtgtg tgtgtgtgtt taattggccc agggttactt aaataaatca 840
   taaccatttt gccacattct gtaactgttt agctaaggtc aaattaagtt taccctatgg 900
   attttgtttc atcttttgtt tcgtgtatat actgtttgcc tttttcataa aaatcttgga 960
   tttgttatat attgttcctg ttatttttga catctttgct attgtaaata aattactatt 1020
   ttgttttaag ttaaaaaaaa aaaaaaaaaa acwcgta 1057

<210> 26
<211> 980
<212> DNA
<213> Homo sapiens

<400> 26
tcgacccacg cgtccgcggc gcgctcacaa tggagctctc ggagtctgtg cagaaaggct 60
tccagatgct ggcggatccc cgctccttcg actccaacgc cttcacgctt ctcctccggg 120
cggcattcca gagtctgctg gacgcccagg cggacgaggc cgtgttagat catccagact 180
tgaaacatat cgacccagtg gttttaaaac attgtcatgc agcagctgca acttacatac 240

tagaggcagg aaagcaccga gctgacaagt caactctaag cacttatcta gaagactgta 300
aatttgacag agagcgaata gaactgtttt gcacggaata tcagaataat aagaattccc 360
tagaaatcct actgggaagt ataggcagat ctctccctca tataacggat gtttcttggc 420
gcttggaata tcagataaag accaatcaac ttcataggat gtacagacct gcatatttgg 480
tgaccttaag tgtacagaac actgattccc catcctatcc agagattagt tttagttgca 540
gcatggaaca attacaggac ttggtgggga aacttaaaga tgcttcgaaa agcctggaaa 600
gagcaactca gttgtaactt ggggaagtta acgatccgcc cgagtgcaga ggaaaaccag 660
aaacgccttg ccttcagctg aaccaccgtt tgtgcgagct ggatgtcctt ttcagtagaa 720
aagaattttc cttttgaatt tataccattc atcaattttg acactttaaa aacgtgtgaa 780
agggttaaga gggaaagata ctgcccaagt atttgaatcg tttagtagta actgtccatt 840
tatcctattt tgatcttttt caagtcttct gaaaggaagt agacagtatt acaccctgaa 900
taaataaggt gttgttttcc acaaaaaaaa aaaaaaaaaa aaaaaaaaaa aaaaaaaaaa 960
aaaaaaaaaa aagggcggcc 980

<210> 27
<211> 755
<212> DNA
<213> Homo sapiens

<220>
<221> SITE
<222> (748)
<223> n equals a,t,g, or c

<400> 27
gaattcggca cgagattgtg cacatgtacc ctaaaactta agatgtaata ataataaaat 60
aaaataaaat aaattaaaaa ataaaaataa aaacarattt aatgataggg tacttaatga 120
aagtwttggt ggtccttgaa tgacgtattt tacactacat atgtacctac ttttctattc 180
tcctcctcag atgggaaagg tctagataaa ctggcctcta tcccgcagct cttctccaca 240
atggttaaga acagttcaac acggaggacc agcagtaaat gacctttaaa aagtgtaata 300
ataactattg cccaaaataa tcttattaat catagaaaat ggcttctatt cttctgctcc 360
ttgttctgtc acacagctgt tgctgtaaaa acacttgttt acaggttcta tgtaattttg 420
actcagtcca taatctctcc accctaattt taaaaattat catcagggtg gatgtgctag 480
tatactaaga aacatctgtt aatattattt attttcttta tttaatcttt ttcatagatt 540
cacttgtttt aaaatatctt aggtttataa tctctttgca aagctcaata aatcatttta 600
acagctaaaa ataaaaactt aaaaatgaac tccagataaa tatgaagatt caaaactatg 660
tggaatctct gcccccctct taatactcac caataaattc tacttcctgt cmaaaaaaaa 720
aaaaaaaaaa aaaaaaaaaa aaaaaaanaa aaaaa 755

<210> 28
<211> 946
<212> DNA
<213> Homo sapiens

<220>
<221> SITE
<222> (5)
<223> n equals a,t,g, or c

<220>
<221> SITE
<222> (23)
<223> n equals a,t,g, or c

<400> 28
tcgcnactat agggaactgg tcnctgcagg tccggtcgga attccgggtc gacccacgcg 60

tccggtaaat gttttatgtg ttcgcctact gatcccattc gttgcttcta ttgtaaatat 120
ttgtcatttg tatttattat ctctgtgttt tccccctaag gcataaaatg gtttactgtg 180
ttcatttgaa cccatttact gatctctgtt gtatattttt catgccactg ctttgttttc 240
tcctcagaag tcgggtagat agcatttcta tcccatccct cacgttattg gaagcatgca 300
acagtattta ttgctcaggg tcttctgctt aaaactgagg aaggtccaca ttcctgcaag 360
cattgattga gacatttgca caatctaaaa tgtaagcaaa gtagtcatta aaaatacacc 420
ctctacttgg gctttatact gcatacaaat ttactcatga gccttccttt gaggaaggat 480
gtggatctcc aaataaagat ttagtgttta ttttgagctc tgcatcttaa caagatgatc 540
tgaacacctc tcctttgtat caataaatag ccctgttatt ctgaagtgag aggaccaagt 600
atagtaaaat gctgacatct aaaactaaat aaatagaaaa caccaggcca gaactatagt 660
catactcaca caaagggaga aatttaaact cgaaccaagc aaaaggcttc acggaaatag 720
catggaaaaa caatgcttcc agtggscact tcctaaggag gaacaacccc gtctgatctc 780
agaattggca ccacgtgagc ttgctaagtg ataatatctg tttctactac ggatttaggc 840
aacaggacct gtacattgtc acattgcatt atttttcttc aagcgttaat aaaagtttta 900
aataaaaaaa aaaaaaaaaa aaaaaaaaaa aaaaaaaagg gcggcc 946

<210> 29
<211> 971
<212> DNA
<213> Homo sapiens

<400> 29
gcttctatcc atttattcaa gcacatattg gtcacctact gtgtgcctgg cactcatgtc 60
acaaagataa gttcctgatt cggtacactt actgagcacc tgctgtgtgc agggagctga 120
gctatgggat gggaatggga gtaaacaagg tacttttyac ttttttcttt ttttcctcac 180
tgctagacgg tgtgggaact tctcactcat tggcttcttt cccacacacc tgaagagcac 240
tgactgtgtg ccgggcacta gtgatacaaa agagtgtgac agttgttcag tctgcatttt 300
cgatcatggg ctacatgccg agtgctgggg cacagagatg aacaagatcg gttccttcac 360
ttcttcatgc cacaagtgtt tattgagcac ctgtgtgcca ggcctcacag actcccagtt 420
gggttgaaga atggttgact gagtttgatt cttcctgtac cctcggtcgt ctgagctgtg 480
tgcagacaac atccccccac cacccaagag ggagggtagc tcttccgcca ccaggggcaa 540
gcacaggtcc tggtggcccc acgccacatg ttagcccccc tggagggggc gccagttgga 600
gacgggggct gggtgtccct ggcccactcc cggtcccctg tgctttacct ccttgccctt 660
gtgtctcagg tgtggtccct gcctgcttga tgaagttgct ctgttcaagc ctttggtggg 720
atcatgtgtt tgggggcttt taggggaccc agctgcactg gggcactgcc cgtggcctgg 780
gtaggacatt tcccagcaag ggctggagga gttgccgtgc cttcagcctg aatcgaatgt 840
cagaaccagc cagcggtgct tcaccctctt ggggataact tgcttagttt tttaataaat 900
gttcctggtt ggttttcaca gcaaaaaaaa aaaaaaaaaa aaaaaaaaaa aaaaaaaaaa 960
aaaaaaaaaa g 971

<210> 30
<211> 1008
<212> DNA
<213> Homo sapiens

<220>
<221> SITE
<222> (421)
<223> n equals a,t,g, or c

<400> 30
   gcggcacgag ctggaggtca ctttccaacc agagctgtgc tggagtccag taggtgggag 60
   gctgtgcttt gagggactaa aggaagcctg tatcttgtgg tgagggttcc acctcacaag 120
   ttacagatct cagttccatt tggctctagc agcaatgtgg ccacttctgt tgcggttact 180
   ctttcttcac ctttttcttg ccaaaaataa acttatcttt aaatgaaaac taaattattt 240
   cttatatttt ggtcctttgt tatagctgag attgggaatt tttctttctt tcttgaatcc 300
   ttacttccct accctgcctc cccaccaatg gaaatctgtg cttcataagc attttagatt 360
   ccagaaagct ctttaggtta aactacaacc ctctcacctc aaagaatttg tgggccaggg 420
   naagtcagtg acttatgtga agtcttgcgg ctaattaatg gtagagctgg agttaggaca 480
   catgtctcac agttcctagt tcgttttgct ttgatgtgct tgaaattcag ttttgacatt 540
   aatttttctg gatactactc ccataaaatg ttctttgaaa aatacttgct tctttctagt 600
   ttttctcgcc tggtttaaat attgtcctga gtgtgggaac cccataactg tcttgtgggt 660
   tagaatttag atggaaggat ttggggccct gtctctagta tcataagaca tttaaccttg 720
   ctgctttttt cttctaggtt cactctttga atttcctgga taagagttct ggagatggca 780
   gcttattgga cacatggatt ttcttcagat ttgcacttac tgctagctct gctttttatg 840
   caggagaaaa gcccagagtt cactgtgtgt cagaacaact ttctaacaaa catttattaa 900
   tccagcctct gcctttcatt aaatgtaacc ttttgccttc caaattaaag aactccatgc 960
   cactcctcaa aaaaaaaaaa aaaaaaaaaa aaaaaaaaaa aaaaaaaa 1008

<210> 31
<211> 990
<212> DNA
<213> Homo sapiens

<400> 31
aattcggcac gagtggacaa ccatcaggga gccaggacac agaggggcag agcaagtcag 60
cattggcgcc ccttcctcag atccctatca tcttgggaaa cagtagccca gaggttcagg 120
aagatgttaa cttaaatgtt cggggtgccc cagtctgttc agcatggctg aaatccacac 180
tccgtattct tccttgaaga aactgttatc tttactcaat ggcttcgtgg ctgtgtctgg 240
catcatccta gttggcctgg gcattggtgg taaatgtgga ggggcctctc tgacgaatgt 300
cctcgggctg tcctccgcat acctccttca cgttggcaac ctgtgcctgg tgatgggatg 360
catcasggta ctgcttggct gtgccgggtg gtatggagcg actaaagaga gcagaggcac 420
gytcttgttt gttggagatg tggccttgga acacamcttc gtgaccctga ggaagaatta 480
cagaggttac aacgagccag acgactattc tacacagtgg aacttggtca tggagaagct 540
aaagtgctgt ggggtgaata actacacaga tttttctggc tcttccttcg aaatgacaac 600
gggccacacy taccccagga gttgctgtaa atccatcgga agtgtgtcct gtgacggacg 660
cgatgtgtct ccaaacgtca tccaccagaa gggctgtttc cataaactcc taaaaatcac 720
caagactcag agcttcaccc tgagtgggag ctctctggga gctgcagtga tacagttgcc 780
aggaattctt gccactttgc tgctgtttat caagctgggc tgacacccag gcctggagaa 840
gatgagacac ctgggcccat ctggctgctg gagattcagt ctcagtttta tttctctgtg 900
gcactcactg cttctggagg ggagactgtt aataaaagat ttgggaaaaa aaaaaaaaaa 960
aaaaaaaaaa aaaaaaaaaa aaaaactcga 990

<210> 32
<211> 1131
<212> DNA
<213> Homo sapiens

<400> 32
   gaattcggca cgaggcctat gtcatcctgg ctgtgtgctt ggggggaatg atcgggatct 60
   ctgccagctt ctcagccctc ctggagcaga tcctctgtgc aagcggccac tccagtgggt 120
   tttccggcct ctgtggcgct ctcttcatca cgtttgggat cctgggggca ctggctctcg 180
   gcccctatgt ggaccggacc aagcacttca ctgaggccac caagattggc ctgtgcctgt 240
   tctctctggc ctgcgtgccc tttgccctgg tgtcccagct gcagggacag acccttgccc 300
   tggctgccac ctgctcgctg ctcgggctgt ttggcttctc ggtgggcccc gtggccatgg 360
   agttggcggt cgagtgttcc ttccccgtgg gggagggggc tgccacaggc atgatctttg 420
   tgctggggca ggccgaggga atactcatca tgctggcaat gacggcactg actgtgcgac 480
   gytcggagcc gtccttgtcc acctgccagc aggggaggga tccacttgac tggacagtgt 540
   ctctgctgct gatggccggc ctgtgcacct tcttcagctg catcctggcg gtcttcttcc 600
   acaccccata ccggcgcctg caggccgagt ctggggagcc cccctccacc cgtaacgccg 660
   tgggcggcgc agactcaggg ccgggtgtgg accgaggggg agcaggaagg gctggggtcc 720
   tggggcccag cacggcgact ccggagtgca cggcgagggg ggcctcgcta gaggacccca 780
   gagggcccgg gagcccccac ccagcctgcc accgagcgac tccccgtgcg caaggcccag 840
   cagccaccga cgcgccctcc cgccccggca gactcgcagg cagggtccaa gcgtccaggt 900
   ttattgaccc ggctgggtct cactcctcct tctcctcccc gtgggtgatc acgtagctga 960
   gcgccttgta gtccaggttg cccgccacat cgatggaggc gaactggaac atctggtcca 1020
   cctgcgggcg ggggcgaaag ggctccttgc gggctccggg agcgaattac aagcgcgcac 1080
   ctgcagcggc cccgggtgtg gtttctgcgc cgcgggaggg ggagctgtgc c 1131

<210> 33
<211> 1293
<212> DNA
<213> Homo sapiens

<220>
<221> SITE
<222> (1)
<223> n equals a,t,g, or c

<220>
<221> SITE
<222> (7)
<223> n equals a,t,g, or c

<220>
<221> SITE
<222> (8)
<223> n equals a,t,g, or c

<220>
<221> SITE
<222> (25)
<223> n equals a,t,g, or c

<220>
<221> SITE
<222> (396)
<223> n equals a,t,g, or c

<220>
<221> SITE
<222> (1271)
<223> n equals a,t,g, or c

<400> 33
naagganncc aaaccgcaga aagtnacccg tcacgtaaag ggaacaaaag cctggaggta 60
gcgcgcctgc aggtcgacac tagtggatcc aaagaattcg gcacgagacc aaccccaagt 120
gctcctatat ccctccctgt aagagagaaa atcagaagaa tttggaaagt gtcatgaatt 180
ggcaacagta ctggaaagat gagattggtt cccagccatt tacttgctat tttaatcaac 240
atcaaagacc agatgatgtg cttctgcatc gcactcatga tgagattgtc ctcctgcatt 300
gcttcctctg gcccctggtg acatttgtgg tgggcgttct cattgtggtc ctgaccatct 360
gtgccaagag cttggcggtc aaggcggaag ccatgnaaga agcgcaagtt ctcttaaagg 420
ggaaggaggc ttgtagaaag caaagtacag aagctgtact catcggcacg cgtccacctg 480
cggaacctgt gtttcctggc gcaggagatg gacagggcca cgacagggct ctgagaggct 540
catccctcag tggcaacaga aacaggcaca actggaagac ttggaacctc aaagcttgta 600
ttccatctgc tgtagcaatg gctaaagggt caagatctta gctgtatgga gtaactattt 660
cagaaaaccc tataagaagt tcattttctt tcaaaagtaa cagtatatta tttgtacagt 720
gtagtataca aaccattatg atttatgcta cttaaaaata ttaaaataga gtggtctgtg 780
ttattttcta tttccttttt tatgcttaga acaccagggt ttaaaaaaaa aaaaaargtg 840

aggacatctg ggtctcattt gcttctgcta ggttaaactt ttacttgaca acaaggattc 900
ctgctgaagt ctgaacctta ctgtgtaacc ctcagtttcc actattaaag agtatctttt 960
gacgtctgct tggaaaatga atagtatact ggtaactcag tctccagtca cctctgtgtc 1020
tcttaagcaa gagattctaa aagattggga aaacatatcc tccaamacct gcctttgcct 1080
aaccattatt tttcaccaga ttacttctta agagagggag gtgattctga agaaggcttc 1140
tatctcaaaa agcactgggc ttccttattc atctgttctt gttgtttttg acggagttaa 1200
aaaagtttgt gtgcaataca atataaatga tgtgaaggac actcttaaaa aaaaaaaaaa 1260
aaaaaaaaat ngctgcggcc gacaagggaa ttc 1293

<210> 34
<211> 1014
<212> DNA
<213> Homo sapiens

<400> 34
ggcacgaggt cagccagaac atgtctttca acctgcaatc atcaaagaaa ctgttcattt 60
tcttaggaaa atcactgttt agtcttctgg aggctatgat ttttgcctta ctcccaaagc 120
cacggaagaa cgttgctggt gaaatagtcc tcatcacagg tgctggaagt ggactcggaa 180
ggctcttagc cttgcagttt gcccggctgg gatctgttct tgttctctgg gatatcaata 240
aggaggggaa tgaggaaaca tgtaagatgg ctcgggaagc tggagccaca agagtgcacg 300
cctatacctg cgattgcagc caaaaggaag gagtgtatag agtagccgac caggttaaaa 360
aagaagtcgg cgatgtttcc atcctaatca acaatgccgg aatcgtaaca ggcaaaaagt 420
tccttgactg tccagatgag cttatggaaa agtcatttga tgtgaatttc aaagcacatt 480
tatggactta taaagccttt ctacctgcta tgattgctaa tgaccatgga catttggttt 540
gcatttcaag ttcagctgga ttaagtggag taaatgggct ggcagattac tgtgcaagta 600
aatttgcagc ctttgggttt gctgaatctg tatttgtaga aacatttgtc caaaaacaaa 660
aggggatcaa aaccacgatt gtgtgcccct tttttataaa aactggaatg tttgaaggtt 720
gtactacagg ctgtccttct ctgttgccaa ttctggaacc aaaatatgca gttgaaaaaa 780
tagtagaagc tattctacaa gaaaaaatgt acttgtatat gccaaaagtt gttatacttc 840
atgatgtttc ttaaaaaggt aattacatca gcttctatta cttccctaac atgccagtct 900
acagttttac tcccaaatcc cacccaggaa aaagccactt twaaaaatac ctgataaatt 960
aaaattcatt aatttaattc taaaaaaaaa aaaaaaaaaa aaaaaaaaaa aaaa 1014

<210> 35
<211> 1222
<212> DNA
<213> Homo sapiens

<220>
<221> SITE
<222> (4)
<223> n equals a,t,g, or c

<220>
<221> SITE
<222> (52)
<223> n equals a,t,g, or c

<220>
<221> SITE
<222> (78)
<223> n equals a,t,g, or c

<400> 35
actnatcttg aggtgacact atgagaaggt acgcctgcag gtaccgatcc gnaattcccg 60
ggtcgaccca cgcgtccnga aatttacaat ttctgaccat ccacaaccta ttgatccact 120

gttaaagaac tgcataggtg atttcctaaa aactttggaa gacccagatt tgaatgtgag 180
aagagtagcc ttggtcacat ttaattcagc agcacataac aagccatcat taataaggga 240
tctattggat actgttcttc cacatcttta caatgaaaca aaagttagaa aggagcttat 300
aagagaggta gaaatgggtc catttaaaca tacggttgat gatggtctgg atattagaaa 360
ggcagcattt gagtgtatgt acacacttct agacagttgt cttgatagac ttgatatctt 420
tgaatttcta aatcatgttg aagatggttt gaaggaccat tatgatatta agatgctgac 480
atttttaatg ttggtgagac tgtctaccct ttgtccaagt gcagtactgc agaggttgga 540
ccgacttgtt gagccattac gtgcaacatg tacaactaag gtaaaggcaa actcagtaaa 600
gcaggagttt gaaaaacaag atgaattaaa gcgatctgcc atgagagcag tagcagcact 660
actaaccatt ccagaagcag agaagagtcc actgatgagt gaattccagt cacagatcag 720
ttctaaccct gagctggcgg ctatctttga aagtatccag aaagattcat catctactaa 780
cttggaatca atggacacta gttagatgtt tgttcaccat ggggaccatt acatatgacc 840
atacaatgca ctgaattgac aggttaatca taagacatgg aaagagaagt gtctaaaagc 900
ttcaaaatgt tccacttttt tttccttcat ggagactgtt tgtttggctt tcttccattg 960
ttgtttttgt agcatttatt tcagaaatgt gtatttccat aatccagagg ttgtaaaacc 1020
actagtgttt tagtggttac agcaacattt gaaatggaaa ctaaaagtta ggattttatg 1080
gagtatggag atagggtcca gtatctattt accctgtaat gtttaggatt aaaatgttaa 1140
aattttgtga ccatgaattt ctttctttta taaattttct catttaaaaa tcaaaaaaaa 1200
aaaaaaaaaa aaaaaaactc ga 1222

<210> 36
<211> 901
<212> DNA
<213> Homo sapiens

<220>
<221> SITE
<222> (895)
<223> n equals a,t,g, or c

<400> 36
gaattcggca cgagcacttg agaggtgtac aggagagagt taatctttct gcacctctgc 60
tacctaaaga agacccaatc ttcacatatt tatctaaacg gttaggaagg agtatagatg 120
acataggtca cctcattcat gaaggcctac agaagaacac ttcctcgtgg gtactgtata 180
acatggcttc attttactgg agaattaaga atgagccata tcaggtagta gaatgtgcca 240
tgcgagcact tcacttctct tccaggcaca ataaagacat tgccctggtc aacctggcaa 300
acgttctaca cagagcacac ttctctgctg atgctgctgt cgtggtccat gcagctctgg 360
atgacagtga cttcttcacc agctattaca ctttggggaa tatatatgca atgcttgggg 420
aatataacca ctcagtgctc tgttatgacc acgctttgca ggccagacct gggtttgagc 480
aagctataaa gaggaagcat gctgtcctat gtcagcaaaa actggagcag aaattggagg 540
ctcagcatag atctctccag cgaacactga atgagttaaa agagtatcaa aagcagcatg 600
accactacct gagaccagga aatcctagaa aaacataaac tgattcagga ggagcaaatc 660
ttaagaaata tcattcatga gactcagatg gcaaaagarg cacaattagg aaatcatcag 720
atatgccgac tggtcaacca gcagcatagt ttacattgcc agtgggamca gcctgtwcgc 780
tatcatcgtg gagatatctt tgaaaatgtg gactatgttc argtcttttt cttggtccar 840
tctaattctt ataaacgttt gctttataaa gattttttaa aactttaaaa aaacngcacg 900
a 901

<210> 37
<211> 954
<212> DNA
<213> Homo sapiens

<400> 37
gaattcggca cgagcccaca ccaaacctgt ggacgccgac ccgggaccgc cgctggctgg 60
ctgctggctc actcgaccgt catggagacc ctgggggccc ttctggtgct ggagtttctg 120

ctcctctccc cggtggaggc ccagcaggcc acggagcatc gcctgaagcc gtggctggtg 180
ggcctggctg cggtagtcgg cttcctgttc atcgtctatt tggtcttgct ggccaaccgc 240
ctctggtgtt ccaaggccag ggctgaggac gaggaggaga ccacgttcag aatggagtcc 300
aacctatacc aggaccagag tgaagacaag agagagaaga aagaggccaa ggagaaagaa 360
gagaagagga agaaggagaa aaagacagca aaggaaggag agagcaactt gggactggat 420
ctggaggaaa aagagcccgg agaccatgag agagcaaaga gcacagtcat gtgaagattc 480
ctggctgcct cttccaggca gtcccccaga gatgcctctt ctgcccccta aaagcagtgc 540
cctggacttg aagcccgtga aatgactcca tctgggattc agaatacagt gttctcaagt 600
gaagaaggct tggaacccac cccacctccc tcattggggg ctctctgggc aaacatggtt 660
ttcatgcacc cctcttcctg agcttggtcc ctgcctggtg attcttctta tactcggaga 720
gcatccctgg ttgaggagac acccgcaatc ctccacgatc tcatggctcc acctgcttct 780
ccccactgcc tgatttcttt tctctctgcc tgatgtctac tgaacagaac ttcccctctc 840
ccatgcaccc actgccagct gagagctgct tcccaatggc ctgcattaaa gcattcgtaa 900
cagccaaaaa aaaaaaaaaa aaaaaaaaaa aaaaaaaaaa aaaaaaaaac tcga 954

<210> 38
<211> 890
<212> DNA
<213> Homo sapiens

<400> 38
aattcggcac gagattcact aaacactgca atacaagctt ggcaacagaa caaatgccct 60
gaggtagagg agttggtctt cagccatttt gtgatctgta atgacacaca ggagacactg 120
cggtttggcc aggtggatac tgatgaaaat attctgctgg cgagtctcca cagtcaccag 180
tacagctggc gctctcacaa atccccacag ctgttacaca tctgtattga aggttggggc 240
aactggcgtt ggtcagagcc tttcagtgtg gaccatgccg ggacttttat tagaacaatt 300
cagtacaggg gtcgaactgc ttctctcatc atcaaggttc agcaactcaa tggagtacaa 360
aaacagatta tcatctgtgg aagacagatc atctgtagtt acttgtctca aagcatagaa 420
ctaaaagtcg ttcagcatta cattggtcaa gatggacaag ctgtagttcg ggaacatttt 480
gactgcctca cagccaaaca gaaattgcct tcgtacatac tagaaaacaa tgaactgacg 540
gagctgtgtg tgaaggccaa aggagatgaa gactggtcaa gagatgtgtg cctggaatcc 600
aaagcccctg agtacagcat tgtcattcag gtgccatctt caaacagttc cattatttat 660
gtctggtgca cagttttgac tttagaaccc aactctcaag tgcaacaacg aatgattgtg 720
ttcagccctc tttttatcat gaggagtcat cttccagacc ccattatcat acatttggag 780
aaaaggagtc tgggattgag tgaaacacaa attattccag gaaaagggca ggaaaaacca 840
ctgcaaaaca tagaacctga ccttgtacat cacctgacat tccaagcaag 890

<210> 39
<211> 1070
<212> DNA
<213> Homo sapiens

<220>
<221> SITE
<222> (1016)
<223> n equals a,t,g, or c

<220>
<221> SITE
<222> (1026)
<223> n equals a,t,g, or c

<220>
<221> SITE
<222> (1043)
<223> n equals a,t,g, or c

<400> 39
acagcctttg ttaccttccg agccacccga aaacctctag tacagacaac cccaaggttg 60
gtttataagt ggttcctgct aatctataaa atcagctatg ccactggcat tgttggctac 120
atggctgtca tgtttaccct ctttggtctt aacttattat tcaagatcaa accagaagat 180
gccatggact ttggcatctc ccttctcttc tatggcctct actatggagt tctggaacgg 240
gactttgcag aaatgtgtgc agactacatg gcatctacca targgttcta sagcgagtcg 300
ggcatgccta ccaaacatct ttcagacagt ktgtgtgctk tktgtgggca gcagatcttt 360
gtggacgtca tgaagagggg atcattgaga acacgtatag gctgtcctgc aatcatgtct 420
tccacgagtt ctgcatccgt ggctggtgca tcgtgggaaa gaagcaaacg tgtccctact 480
gcaaagagaa ggtagacctc aagaggatgt tcagcaatcc ctgggagagg cctcacgtca 540
tgtatgggca actgctggac tggcttcgat acttggtagc ctggcagcct gtcatcattg 600
gtgtagtcca aggcatcaac tacatcctgg gcctggaata gtgatgaaga gcatcagtgg 660
aaaacccacc ccacacgcca tggacctcag ggcactctcc tccctgccca caaagacctc 720
ctgggtggga aagactcaaa ggggcgcttg ggccactcag gacccctccg gctgtgtcgg 780
actggggagg gatatgatgg agagccagcc agtggggctg kcagcagtgg ggggcttttt 840
aaaagaaaac tattttgatg aatatattta aaaaaccttt ttttattgtg gagcatagga 900
attgcccccc tccaggcttc accctccctg cctaagcagg ttgggggcag agccatgaca 960
tttttggttt aaaggagcct tctcatctct ggccgagaac actgctgggc tcccangtag 1020
ctgaangcct cagcccaycc atncccttct tccctgtgtg gggctcaagc 1070

<210> 40
<211> 772
<212> DNA
<213> Homo sapiens

<400> 40
gcaaccagta tgaaaaggct ttctcatcca agtatctgca gaactggtct cccactaagc 60
caacaaaaga gagcatctct tctcatgaag gctacactca aattattgcc aacgatcgtg 120
gtcatctact gccttctgtg ccccgttcca aggcaaatcc ttggggttcc ttcatgggca 180
cctggcaaat gcctctgaag ataccccctg ctcgggtgac cctgacctcc cgtacaactg 240
ctggtgctgc ctccctcacc aaatggatac agaaaaatcc tgatttactc caaggcctcc 300
aatgggctgt gtcctgaaat cttaggcaag ccccatgatc cagacagtca gaagaaactc 360
agaaagaagt ctatcacaaa gactgtacaa caagcacgaa gtccaaccat attccaagct 420
ccccagctgc caacctcaat tccccagatg aactccaaag ctcacamccc tctgcaggtc 480
atactccagg tccccaaaga ccagccaaat yctaagagcc cacctggrag tccacgtatg 540
ctagaactct gggcagggcc taatctagct gaggtccaga aatacaaacc tggaacttca 600
tatggaccaa gtggccacac actgaaaaac ccgtatagcg actcagtgaa ataaacaaga 660
gcccccagtc agaactgtga aacagggaaa ttttggggtg gsagtaaaag saaatttgga 720
aaataaactt ttttttgttg aatcttttaa aaaaaaaaaa aaaaaactcg ta 772

<210> 41
<211> 787
<212> DNA
<213> Homo sapiens

<220>
<221> SITE
<222> (444)
<223> n equals a,t,g, or c

<220>
<221> SITE
<222> (506)
<223> n equals a,t,g, or c

<400> 41
ggtggtgtgc gccacccaga ggctctctgt gggtccctag tggggaaaat gactcctccc 60
cacctacagt cttggtcagc agccccactg agctgtgttc atgttgactt ccagctccaa 120
ccttatctcc tgggtcctgc cagagttgtc ctctctgttg tgggttttct tgttctggaa 180
aaggcagtgt ggtgactggg cgggccggaa gaccaggtcc agggtctcag gagttgtcac 240
taatttccca ctccattccc cttcactccg ttacagctcc tttttggaat gaggggacga 300
tgctcaggaa gagaggaggt attggaaagg aaagagaccc cttcatcttc ctttttagcc 360
ctgctcaacc tggctggcta tttctgggag ggccctttag agttgctgtg ggcctctgcc 420
tatgtctgtg cagggcatag gcantgcaca sacagttgcc acacccaggg tggamaaatc 480
cccatggtgg ccttgtctgc tgtcanttgc ataggaaatc tgataaccta agattttttt 540
ttatttttta ttttgagaca gagtcttgct ctgtccccca agttggagtg caatggcatg 600
atcttggctc actgctacct ccaatcctgg atttgagcta ctcaggaggc tgaggtcagg 660
ggaatcgctg gaacgcggga ggcggagctt gcagtgagcc gagatcatgt cactgccctc 720
cagcctgggc gacacagtga gactccatct caaaaaaaaa aacaaaaaaa aaaaaaaaaa 780
actcgta 787

<210> 42
<211> 652
<212> DNA
<213> Homo sapiens

<220>
<221> SITE
<222> (392)
<223> n equals a,t,g, or c

<400> 42
   aattcggcac agggggccca ccacacccgg cctgtacatg ctgttttgca tcttgcttta 60
   tacgttgggg agtgccagat gtcaccatct ttcgttcttc ctctggggct ggtcaaatcc 120
   ccctgagaaa actcctctgg cctcctggcg gggggtgaag gccaggctgc cagggccagg 180
   ctgccagctt ctgggagctg caggggcaga ggcagggagc tgtcaggcat tcagccagca 240
   agacgcactc agtacccact tggggttcag aatccccctc cctcatcttc agatgggcca 300
   gatgtcccca aagccagcgg cccctttctg tttcaccctg tctacagaat aaacccccag 360
   tcactggggg tgggggaaga gtaaggggag angggaaacg agatttggag gtctagctgc 420
   tgctgaaaca gccctcagtt cgtctttatt ttgccttctg caaaactggc ctggtgttgc 480
   cagctccttt tgaggacttt gctamcggtt ctcagcatcc ctcaattgct ggcttaggat 540
   tcatgggttt ttaggggtgg ggtgggatta gcatgtccag ctgctttcca gtttccaaag 600
   ttctgtccct atcatattgc ctctgattta aaaaaaaaaa aaaaaaactc ga 652

<210> 43
<211> 1520
<212> DNA
<213> Homo sapiens

<220>
<221> SITE
<222> (799)
<223> n equals a,t,g, or c

<220>
<221> SITE
<222> (928)
<223> n equals a,t,g, or c

<220>
<221> SITE

<222> (937)
<223> n equals a,t,g, or c

<220>
<221> SITE
<222> (945)
<223> n equals a,t,g, or c

<220>
<221> SITE
<222> (974)
<223> n equals a,t,g, or c

<220>
<221> SITE
<222> (1019)
<223> n equals a,t,g, or c

<400> 43
gaattcggca cgagtcaccc ttttcagtga gttagtcgtg acatttctta cactgtgagg 60
gggagtggta attactttac agggaggtat ggggccatgg tgtttgactc ttctttcaac 120
cacttctggg ttttttagtg aaaacctcta tctaacactg atactttcat ttctgttgtc 180
tattgagtca gttaacactg atccatttat ttttcagttc ccaaaatctt gctttgccat 240
tgcttctatt ttattgtctg gggtgtttta acacctgttt gcatttttta cagtcattta 300
gtttccagat tttagtaagg gacagaggga atagatggac tcattcatga tgtagaaaca 360
aatactccct gtcttgtctt acakgaaaaa ttattcttaa actagcctgt cttkgagaac 420
ctgatcaaag tataaaaaat actttttggc ttatttctta gtgagtcamt attccatatt 480
ttgaaggtgt taagaggtat ggtaaaggtg gtacttgaac atttccaagc aaacgtgtga 540
tgaaatctty catcaatgtc ttagcaatgg tatatgattt ttttagtctt agcaatttta 600
gataagtttt tttttgtctt tgtttttttg agacggagtc ttgctctgtc gcctaggcta 660
cagtgtagtg gcgtgatctc ggctcactgc agcctctgcc tccgagcggg gtccagcgat 720
tctcctgcat cagcctcctg ggtagttggg attacaggtg catgccacca cacccaactg 780
atttttgtat ttttagtana gacagggttt caccatcttg gcctgactgg tcccgaactg 840
atctcaggtg atctgcccac ctcgggctcc caaagtgctg ggattacaag cgtgagccac 900
tgcgtggcct gagcactwag ggcgcaanga raagccngta ctggnawtwt tacactactc 960
rgcacargac mggntttaat ctttttcttg ggggacaaga ttggaaaatt gaggtctgna 1020
gcagacctga agagaggcat ccagcaactc tgagattaat tcatcatgat cattcgttat 1080
tgtttggaat tgacgtttag ctgtgttcct cactcagata cgtgcatgat agctgcttgc 1140
taatttggtc ttagctcaca tttcacctag aatgtatggt ctccctctcc cctgcaaaat 1200
atcccactgt tgctaatctg tctgcctcat aatttccatg agattgagca tcttgtttgt 1260
tttgtcacca ctatataaca gcatgttgga aacaaagcag taataaagct agaaaaacca 1320
agcgaataca ctggattaaa aaaaatactg tttcctagaa ttaaagaaat aaatgaggcc 1380
gggcgcagtg gtgcctgtaa tcccagcagt ttgggaggct gaggctagtg gatcatgtgg 1440
ccgagatcgc gtcactgcac tccagtctag caacagagcg ataccttgtt tcttacttaa 1500
aaaaaaaaaa aaaaactcga 1520

<210> 44
<211> 796
<212> DNA
<213> Homo sapiens

<400> 44
ggcacgaggt gacgtgtttc tgcatctgtt gccatgacaa gctccctgct tcacccattg 60
ctgtatcccc agcacctctc tcactgcctg gcaagggaaa gcactcagaa gacgctgaat 120
gaccargtag agtgatgggt tgtacagcac tgttactcct tttccatctc tgtgtcccat 180
gtgaacctta tggcacccat gagaaggagc ttgtaccagg tttatacttt ctagtttaca 240
gatgagaaaa caggatcaga gtggtacaga tattggtcta agtcacagag aaagtgaatt 300

gtaaaagcag aaacagagca caggctgcct gacttctagt ccagtgcttt ttgctcaaat 360
tgcctcttat ttctcaggtt attcttgaaa tggcagatgg ggattctgtt taatgaaaca 420
aaagtgacaa ttctttcttt cttggagaga aggtggagac agggtctcac tctatcacac 480
aggctggagt gcagtggctc aatcatggct cactgcagcc tcaatctcct gggctcaagt 540
gattcttcca ccttagcctc cttgactcac tgggactaca ggtgcacacc accatacctg 600
gctaattttt aaagtttttt gtagagacag ggtctcacta tattgtgcat tctggtcttg 660
aactcctggt cccaagtgat cttcctgcct cggctttcca aagtgctgga attacaggca 720
tcacccccat gcctagcctg aaaattcttt ctatgtcctt aacatcttct ttcccagtat 780
ttctccatcc actcga 796

<210> 45
<211> 1378
<212> DNA
<213> Homo sapiens

<400> 45
gatctctgtg tttacctgta taaatatttt ccctgttctt tttatgactt gtatatttct 60
ggtataggtt tgttgcaaat ggttatttaa tcttgactag gtgagaagtc atagaaattc 120
tcctaatttc aacatctatt tattcatgga tctatattat ttttgtgtgg gagaaaaact 180
tttctattta aagataattt acaaacgatc ataatctctt ttaggtatgt ctatttttac 240
ttgtcaaaaa cacataacat ttacaatagg atattttgaa atgtttattt tagtcctatt 300
atattgacat tgttatgcaa catattccka aaakgttttk gtcttgcaar gctaaatatc 360
aatacccatt aaaaaactat ggaattttac ccatttcctg ggcacttttc aaacaccact 420
ctgttttctc taagagtgta ctggcttcat atatctcata caatctctgt ctttttgtga 480
ctggctcatt ttattttgca caatatcatc aagctttata gttgttagaa tattttctgc 540
tttttaaata ctgggtgata tttaagtatt ttgtatttta gattatatct actgagtaat 600
ttggkgacaa atttgcackg cttttaccta ttggctttca gtaacaatgc tgcaataatk 660
acmggtatgc aaatgaccta tatgatcata tatgtgtaag tttatatatg tgccgcattc 720
tgttctacta gtgtacgttt ttacctttgt actcatacca aattgttaca attctgtagc 780
tctgtaatgt gtttcaaaat cagaaactgt aatgccttca aaattgttta ttttattgca 840
gatttttggg tactttatta tctcttaaga ctttatatac tttgggggtt gctgtttcta 900
tttcttcaaa aatgcatgag aaattkgamc aacattgcat taaatctgta aattacattg 960
agcaggatgg acatcttcac aagattaatt attttaacat ttcaacaagc atgctcaaga 1020
gtgtattgtt ttaatttcta tgtatttgtg aatttttcag ttttttcttc ttactgttct 1080
atactcattt cattttggtc atataaagta atccataaaa atttagtttt aaataatttg 1140
ttaagacttc ttttttggtt taccaggttt tctatcaagg agaatttcgt atgaggtatt 1200
tagaaggctg tttatcatta tgttgttgag tgttctttat gcctctgtta ttaataattg 1260
ttttatactc ccttcaagtc cggtttcttt accaatattt tgtcttttta aaatctttat 1320
tacagaaagt gaagcattaa aatattctac tataaaaaaa aaaaaaaaaa aaactcga 1378

<210> 46
<211> 597
<212> DNA
<213> Homo sapiens

<400> 46
tggcggccgc tctagaacta gtggatcccc cgggctgcag gaattcggca cgagcccggc 60
cgccatcttg ggtcatcgat gagcctcgcc ctgtgcctgg tcccgcttgt gagggaagga 120
cattagaaaa tgaattgatg tgttccttaa aggatgggca ggaaaacaga tcctgttgtg 180
gatatttatt tgaacgggwt tacagatttg aaatgaagtc acaaagtgag cattaccaat 240
gagaggaaaa cagacgagaa aatcttgatg gcttcacaag acatgcaaca aacaaaatgg 300
aatactgtga tgacatgagg cagccaagct ggggaggaga taaccacggg gcagagggtc 360
aggattctgg ccctgctgcc taaactgtgc gttcataacc aaatcatttc atatttctaa 420
ccctcaaaac aaagctgttg taatatctga tctctacggt tccttctggg cccaacattc 480
tccatatatc cagccacact catttttaat atttagttcc cagatctgta ctgtgacctt 540
tctacactgt agaataacat tactcatttt gttcaaaaaa aaaaaaaaaa aactcga 597

<210> 47
<211> 600
<212> DNA
<213> Homo sapiens

<400> 47
agaactagtg atcccccggg ctgcaggaat tcggcacgag gacctctgac catcaggctt 60
ctgggaacca taggctatac ccacaccaca gagcatcgat aaactatttt gatgtttctc 120
ttgctttcag aaagacagct tccaagattc aagcccaggt ggtgccggtc tttttttgga 180
ggtgctaatt aataatttaa cttcatctaa tgataatttt atcttgttgc agtttgtgga 240
tttatgatta tctcatccat ccggtgccta gtgttgggca tagagtgtgt ctctgctgtc 300
tgccagaatc tgctactggg agaatttccc cactgggaga gggacccagg aaatggcatg 360
gtcttagaag gtctcctgaa cacatttcct tgggagggct cctgttatct tcaaggttga 420
tggctttctg caatctctca agggctgttt tgcctggaaa caggacgatg gagacagaga 480
cctatcagct gtgggcatct caatatcagc ggaaatgggt atcaagaagt ctcagccagg 540
tgcagtgctt gcgcctgtaa tcccaacact ttgggaggct gaggtaggta gatcactcga 600

<210> 48
<211> 911
<212> DNA
<213> Homo sapiens

<220>
<221> SITE
<222> (6)
<223> n equals a,t,g, or c

<400> 48
cccgcnggta aagggaacaa aatcgtggag cgccaccggs ggtggcggcc rcgtctagaa 60
ctagtggatc ccccgggctg caggaattcg gcacgagcac ctatccacct tggatcgtag 120
cgtgatatgg tctaaatcta tactgaatgc gcgttgcaag atatgtcgaa agaaaggcga 180
tgctgaaaac atggttcttt gtgatggctg tgataggggt catcatacct actgtgttcg 240
accaaagctc aagactgtgc ctgaaggaga ctggttttgt ccagaatgtc gaccaaagca 300
acgttctaga agactctcct ctagacagag accatccttg gaaagtgatg aagatgtgga 360
agacagtatg ggaggtgagg atgatgaagt tgatggcgat gaagaagaag gtcaaagtga 420
ggaggaagag tatgaggtag aacaagrtga agatgactct cmagaagagg amgaagtcag 480
gtmagtccta amatgcaata aaatgagtca gtaagtctta gttagacaat ttctccacta 540
ttcaaataca aatggaatag ttagggtctg taacttagtt taaaactaat atataggctg 600
gacacggtag cttatgccta taatcccagc actttgggag gctgaggcag gcagatcacc 660
tgaggtcagg agttcgagat cagcctggcc aacatggtga aaccccgtct ctactaaaaa 720
ttgaaaaatt agccaaggtg ttggtggaca tctgtaatcc cagctactcg ggaggctgag 780
gtaggagagc tgcttgaacc cgggagcgga ggttgcagtg aggtaacgga tcacgcmatt 840
gcactycagt ctgggtgaca agagcgagac tccatctcaa aaaaaaaaaa aaaaaaaaaa 900
aaaaaactcg a 911

<210> 49
<211> 1863
<212> DNA
<213> Homo sapiens

<220>
<221> SITE
<222> (172)
<223> n equals a,t,g, or c

<220>
<221> SITE
<222> (1820)
<223> n equals a,t,g, or c

<220>
<221> SITE
<222> (1826)
<223> n equals a,t,g, or c

<220>
<221> SITE
<222> (1833)
<223> n equals a,t,g, or c

<400> 49
gaattcggca cgaggatgat atggacatat gtagcccagt ggcattgtac tttctgctga 60
cagctgcaca cattacagct gtctccaaac ccacagtgat gcttagggaa agaccctgct 120
caggacccag caggtcagca ccccagagca gactgatagg tccgtgggac cnatgttaga 180
gcagaaaatt tgggctcagc acattttact gttagtagag agccaggaaa cgttttctgg 240
gttggggatt ttgtgggatt ttttaatttt tttagtaggt tttgtttaac ctctgtgcag 300
tttgtatgaa tgaattgcta tacatttata aggagccagg gtctggaggg ttgctatcac 360
tttgtccagc ccaaatacct tcctgggcaa ctcctaccat ttgtttgcag ttgcctctac 420
tagctgatgg cagtatgctg gaaagaggtt gtactataaa gagagttctt tccttctact 480
ccagagttgt tgtgtagctt tgccattgaa ccgatcaatt tttaaactct ttaaagaagc 540
agcctggcca acatagtgaa gccccgtctc tactaaaaat acaaaaaatt agctgggcat 600
ggtggtgggc gcctgtagtc ccggctgctt gagaggctca ggcaggagaa tcgcttgaac 660
ctgggagtgg aggttgcggt gagccgagat tgcaccattg tattccaccc cgggtgacag 720
tgcaagactc catctcaaaa aaaaaaaaaa aatttggcat catttacaat ttcatagaat 780
tactgtgaag gcctttctag ttgagatgtt ggggtatttg ggattctaat tgttaacccc 840
agaagaaggt aatttagctt gtatttattt aaaacccatt tagcctttta cttatatctg 900
gtagaattcc agtgatcatc ctaataaggt atatttcaga ataatttttt tttccttcag 960
aataacttag aatcagatgc tataagggct cctaggagca gtgtgaaatt tccgtaaaga 1020
taaatttgaa tgttgtaacc aagtttatat taaaccaaga ggccatttcc aatatgattt 1080
tttgtttctt tttaacttgt taagtcccta agagattaca tgctagggct tgagtcattt 1140
ctattgtaga taatgatggc ccacacagtc accttcaact atccacataa gctaggcttt 1200
ccgcttttgc cacggacagt gtgaccaaga tatttccaga gtaaataacc caccacaacc 1260
ttggtaattc ctcttttctt cttaagctcc aggaagcgaa agcagaagga ctcttttcag 1320
actgccctct gtagcctaca ttgcagcttt ccaaaacagg cagctagcac tgggaaagcc 1380
catgtggtga ccccatattt ttctgaggtt cttcttttcc atggtgttac tttattatca 1440
gaaagtaaat tcagaaaaca ggtcttgccc ttagcagaca agaaccacac cagtttcttg 1500
taaaggtaac ggatacattg ggattcagga gtgacacaga ggtccagccc cagaacttgt 1560
aaggattttg tttgaacact gagcagatgc ctcctccctg ccacccatca cactagttag 1620
ggctggccat gaattctatg ccagagtcac tcctgcagtc tgctagggat gggccttctt 1680
atcccactct cgcacacatc ccagtctagt ctttgccttc acagagtcct ccttgacacc 1740
cctgacttaa tgatagttgc tgttttggag tagrattgat caggtttaag tcatcctgct 1800
caggttgggg catagtgggn tcatgnctgt tantttcagg catttgggga agccaaagtg 1860
gaa 1863

<210> 50
<211> 810
<212> DNA
<213> Homo sapiens

<220>
<221> SITE

<222> (688)
<223> n equals a,t,g, or c

<400> 50
gatcctccac atccttccat ggctctgaag aataaattca gttgtttatg gatcttgggt 60
ctgtgtttgg tagccactac atcttccaaa atcccatcca tcactgaccc acactttata 120
gacaactgca tagaagccca caacgaatgg cgtggcaaag tcaaccctcc cgcggccgac 180
atgaaataca tgatttggga taaaggttta gcaaagatgg ctaaagcatg ggcaaaccca 240
gtgcaaattt gaacataatg actgtttgga taaatcatat aaatgctatg cagctttkga 300
awawgttgga gaaaatatct ggttaggtgg aataaagtca ttcacaccaa gacatgccat 360
tacggcttgg tataatgaaa cccaatttta tgattttgat agtctatcat gctccagagt 420
ctgtggccat tatacacagt tagtttgggc caattcattt tatgtcggtk gtgcarttgc 480
aatgtgtcct aaccttgggg gagcttcaac tgcaatattt gtatgcaact acggacctgc 540
aggaaatttt gcaaatatgc ctccttacgt aagaggagaa tcttgctctc tctgctcaaa 600
agaagagaaa tgtgtaaaga acctctgcaa aaatccattt ctgaagccaa cggggagagc 660
acctcagcag acagccttta atccattnca gcttaggttt tcttcttctg agaatctttt 720
aatgtcattt atatacaaaa gaaattctca aatgttaaaa taaaggaata gtttattgct 780
taaaaaaaaa aaaaaaaaaa aaaaactcga 810

<210> 51
<211> 956
<212> DNA
<213> Homo sapiens

<400> 51
aattcggcac gagctaaagc atggtttcca agatgctaca ggcagcgagc ctctctctag 60
tgacctgggt agtttgcacg gtttggctgg aaaccacagt ccccccatct ctgccagaac 120
cccccatgtg gccactgtcc tcagacagct cctggagctt gtggataagc actggaatgg 180
ctccggctcc ctcctcctca acaagaagtt tctcggtcct gcccgagatt tgcttctgtc 240
tttggtagtc ccggstcctt ctcagccgag gtgttgctca catcctgaag acacgatgaa 300
agcattctgc aggagggagc ttgaactgaa ggaggctgcg cactggtccc taatgacatg 360
gaaagtttga agcaaaaact ggtcagagtg ctggaggaaa acctcatttt gtcagaaaaa 420
attcaacagt tggaggaagg tgctgccatc tcaattgtga gtgggcaaca gtcacatact 480
tatgatgatc ttctgcacaa aaaccaacag ctgaccatgc aggtggcttg cctgaaccag 540
gagcttgccc agctgaaaaa gctggagaag acagttgcca ttctccatga aagtcagaga 600
tccctggtgg taactaatga gtatctgctg cagcagctga ataaggagcc aaaaggttat 660
tccgggaaag cgctcctgcc tcctgagaag ggtcatcatc tggggagatc atcgcccttt 720
gggaaaagca cgttgtcttc ctcctcacca gtggcacatg agactggtca gtatctaata 780
cagagcgtct tggatgctgc cccagagcct ggcttataga gctagcatgg aactcacacc 840
acagcttccc tggtccacag aggstctcac cgccattgca ccagtatggt ggtatgtact 900
cacaaagatt aagaaagaaa tgtattctga ytaaaaaaaa aaaaaaaaaa actcga 956

<210> 52
<211> 300
<212> DNA
<213> Homo sapiens

<400> 52
gaccatatgt tgcaggaagt caaactggac tttttgtggc tactaaattt gcctttaatc 60
ttattgttct caattttgga atcaagtatg aaaatctgca caaatgcaat gtttacaaga 120
actggttgat tctgggaggc atctgctaca gtctcttttt atatggatat gtacatgtcc 180
tattctacaa aaatgattaa agataaaaac atacttgtat cccactgcta ctttagctgt 240
caaatttggt gtttcatcac attaaaagca ataaatcagt agttggtaat gtaaaaaaaa 300

<210> 53

<211> 841
<212> DNA
<213> Homo sapiens

<220>
<221> SITE
<222> (836)
<223> n equals a,t,g, or c

<400> 53
gaagggtcgg ggagatattt ccgttagaca tcgctgaaac acagactggg atcaaactgt 60
gctcatagtc ctaaggatct ccagcaccct gccggtggca ctactgagag acgaggtgcc 120
agggtggttc ctgaaartgc ctgagcccca acttatcagc aaggagctca tcatgctgac 180
agaagtcatg gaggtctggc atggcttagt gatcgcggtg gtgtccctct tcctgcaggc 240
ctgcttcctc accgccatca actacctgct cagcaggcac atgggtaact ggctcagcat 300
cctcttccct cctagtcact ctcagagacc attctcgagc ctccagcagg acagaccctt 360
tggagttccc aaacgtcact caaaaactac cagaggaccc accggccaaa ttccttccca 420
ccgctccccc tccccccaat aactgtatct gggtaatccc cactctgacc tcacctttta 480
accaactatt tctggctgga agtggccatc cacatccgtc tactacccag accttctgcc 540
tagacacagc ttttgcaatg tcctacgagg aagtgctcgt gtaacctggt ctaattaatt 600
ttcttcatcc ctgttaaagg actgaatatg aagaaatgtc cttgaattac aacagaagga 660
aatatggttg gacttagaga ttagtttaaa ttcttgaact gataaacaat agaaggtagt 720
gaagctcggt cctggaaagg catttcaatt agggaaaata aaacaatgct gctttggttg 780
tgctaagaaa aaaaaaaaaa aaaaaaaact cgtagggggg gtcttggtac ccaatngtcc 840
t 841


<210> 54
<211> 634
<212> DNA
<213> Homo sapiens

<400> 54
gattaatccc ctcaaccttc tttctgagtt cccatttcac agatgggtaa aactgaggtt 60
tactcctcgt ctagcttcac tgaatggcag agcccatagc ttgtctttgc ctaatctgct 120
gcataatcat ttcagcaaca actcaaatgc cttttgaggg ttcttgcttc tgtttggtgc 180
cttgtaattt tcaaccatat tttagacact ttaggcctaa tgatctaagg catatggttt 240
ttacccatgg tctgtgggcc cttgagaagc tgagtcctct gaaagaaaat cagaatgttg 300
catgcatctg tattttttgt cttagatttc acttgattct caaatggatc cttgactccc 360
ccaaagttta atttattcaa caaatctttt ttttcctcca tactttttat tctgaaacat 420
attcccccaa tttttaactt ctgaaaaatt tcagacaagt tattggaata gggtagtgag 480
tatctatgaa cctttcatat aggtttactt taaaaaaaat acaagagaca gggtcttgct 540
ctgtggccca ggctagagtg ctatgattgt gccactgcag cctgggtgac agaacaagac 600
cctgtcttta aaaaaaaaaa aaaaaaaact cgta 634


<210> 55
<211> 863
<212> DNA
<213> Homo sapiens

<220>
<221> SITE
<222> (7)
<223> n equals a,t,g, or c

<220>
<221> SITE

<222> (298)
<223> n equals a,t,g, or c

<400> 55
gggcagnagt tccatttctg ccgtggtccc agcagcgtcg ctgtgggtct ggcctgggtt 60
gcgtgtgttt cgtatgtggg ccgtgctccc tgcttggttc ccttttcctg gaacgtgtca 120
ctgcctccct gtctcgctcc gtggacattt ctgggaggtc aggccgtggc cacctggccc 180
cctgttcagg tctgaggctc ccacctgctt aggttcggga agctcaggag tgaggccatg 240
ccctcctcag gacatcccat ccaagccagc catgtccggt gatgggccgc tgcccggnaa 300
agtccttttc cttcttgtaa ctgagaagaa cttgccttga gccacgtcaa gtcccgtccg 360
tcgcagccac tgcccacaag cgtgagtctg ctgtgagcca gcggctccat ggcagggcat 420
cccagcgcca ttcctgcctt cacacacact tgctgccgtt tccctgtgct gggggctgtg 480
cargtctgcc tcggtgtgga cttttctctt aggaaagagc cccaggtcgg ccgagcacgg 540
tggctcatgc ctgtaatccc agcactttgg gaggctgagg cgggcagatc acgaggccaa 600
gagatcaaga caatcctggc caacatggtg aaatcccgtc tctacttttt aagtatttta 660
tacttaaaat ttttgtattt tatacaaaaa ttagcgggct tggtggcaga tgcctgtagt 720
cccagctact cgggaggctg aggcaggaaa atcacttgaa cctgagaggc ggagattgca 780
gtgagccaag atggcgtcca ctgcattcca gcctgggcga cagagcaaga ctctatctca 840
aaaaaaaaaa aaaaaaactc gta 863

<210> 56
<211> 712
<212> DNA
<213> Homo sapiens

<220>
<221> SITE
<222> (20)
<223> n equals a,t,g, or c

<220>
<221> SITE
<222> (44)
<223> n equals a,t,g, or c

<220>
<221> SITE
<222> (56)
<223> n equals a,t,g, or c

<220>
<221> SITE
<222> (128)
<223> n equals a,t,g, or c

<220>
<221> SITE
<222> (625)
<223> n equals a,t,g, or c

<220>
<221> SITE
<222> (692)
<223> n equals a,t,g, or c

<220>
<221> SITE

<222> (699)
<223> n equals a,t,g, or c

<400> 56
tgttgtttgg aattgtggan cggattaaca atttcaccac gggnaaccgg ctttgnccca 60
tggattccgc caaggcccga atttacccct tcactaaagg ggaaccaaaa gctggagctc 120
caccgcgntg gcggccgctc tagaactagt ggatcccccg ggctgcagga ttcggcacga 180
ggtttcctgt cagtgctatt gagattttat tttattaatg tctgcactta gttttacttc 240
ctactttcta cttttattga gagttaaacc tgttgaagtc tcaggttcaa ttcctcaccc 300
tgagcaacct aatgttttat gtcttgttct tcctacattt ggttattgaa actgaagttt 360
taggttacca gatttgatag aagcacataa gactacttac tgctttagtc tcaattatta 420
attgagaaat tatcaattaa caataaggat ttctcttatt tttccccaag ataagttata 480
tatttaaagt gtgttttata gtagaaaggt tttagaatat ttgggttgct acattaattg 540
aaatggcagc tgaagatgtg atttccagcc agggatttat taaaaaaaaa aaaaaaaaac 600
tcgagggggg gccgtaccca atcgncctat agtgagtcgt atacaatcac gggcgtcgtt 660
acacgtcgga ctggaaacct gcgtaccact ancgctgcnc acaccccttc gc 712

<210> 57
<211> 925
<212> DNA
<213> Homo sapiens

<400> 57
gatttaaatg tgttgtttct ttttaaaaac attgaatctg tggttgggtt atttctgtca 60
atttatttgc cttccttgcc aagtcacact ttgcctaatt gatgtcctgt gtgttttcca 120
ttccgttcat gctgaattat cttaggtcaa agaggaaatc atctttctgc ctccaacctt 180
cttacttgcc tctaatcccc tttcttgact cttccaagtc aggattctca ccaaggaagc 240
tatctgcctt ctttgggaat gttgggctta tgaagacttg gagataatgg ggttcatgta 300
ttcagactct ttrgcatwta cagtagagtt tctaatgttg tcagcattcc ctagtgggca 360
gttacaagtt aggttgggat tctaatcata tttatgatas tcacagatta aattgcactt 420
tgtctctgcc ccagtctttg attccctttt ggccagcagt ttttaggtct gtcagtactg 480
cactgcarga atggcagatt ttgggatctc tgctggccag tttgtggcag tggtctggga 540
taagtcatcc ccagtggagg ctctgaaagg tctggtggat aagcttcaag cgttaaccgg 600
caatgagggc cgcgtgtctg tggaaaacat caagcagctg ttgcaatgta agtacccacc 660
cacgttgtct ttatgaggct ggaggggttt ccatgggagt gttgcatttc tgtggttcct 720
tgatatctga gttttcattt agggtggcat gtgatagtgg tggctggtca ccctgttgtt 780
tttcagttga gatatatcgg aggaaccacc cccaataatt caacgtaggt tcttttctat 840
tttccctaag tgtcggctgg tctgagaaat aaagggaaag gatacaaaaa agaaaaaaat 900
aaaaaaaaaa aaaaaaaaaa ctcga 925

<210> 58
<211> 601
<212> DNA
<213> Homo sapiens

<400> 58
gctgccagga attccggcac ggggaacagt gtaatattga agcaaatgct gtataacaac 60
cacctggaag cccctcatgt atctcttttt gaaaacactc ctctctttct ccactctaat 120
gatgaccacc gccttgtctt ttatggtaat cactgttctt tgggttttat tactgcattt 180
attggctaat atatgcatcc ctagaaaatg tagttttgcc tgcttttata taaatggaat 240
attactgcat gcagtctttt gatttgtgat tgttttgctc taaggcttgt aagggtcatc 300
catgttttgc atatagtttg tttattgtca ttgccataga gtaaatcatt gtatgaatat 360
actgcagttt atttactgtt gacatatgtt tcagttgttt ttaactacta ggaaatgcta 420
ctctgtacat tcttgtatat gtaccttggt gcacatatgt atgtttttct agagtatata 480
cagtggcatg ggattgctga attaaaaggt ttgtatatct tatactagaa gataataaaa 540
acttttcctg atggattctg ccaattcaaa aaaaaaaaaa aaaaaaaaaa aaaaaactcg 600

a                                                                 601

<210> 59
<211> 730
<212> DNA
<213> Homo sapiens

<400> 59
   gggagaactt ctttattcac atattgcatt gttttacaaa tggaacctgc gagtctatgg      60
   atgccatctt tttaacatgg tctggaactg aacctacaat atttctgaga aaattgactt     120
   tgcttctttg agaacagcat ggtgagtcta ctatccttga cttttcatca atttgtttca     180
   tcactaaagt atttcaagtt gctgtctacg tcaaggcaag aaattctgta gggtttcagc     240
   tgaaaaatca gaagccacac aggcttgctg gaacacacag ctgcatttcc agctctgatt     300
   ttaaatgtgc wctatctgga tccatattct ggcacaatct gcctcttgtg atgaagatga     360
   aaatggttac cttaaagttc tcttcggtca ggccttcttc agttttagca tctctaatca     420
   ttgcagcaac gtatcgcttc accaggttcc tcataacttc ctgaggcatt ttagaacaag     480
   agtattgata ctcaatgagt aaataaattt cctcctgagt cagttctgaa ggggggactg     540
   cattttattt tagtgaaaat ttcaagacat agtacaagga caacttactt ggtattggtg     600
   atgtcttctc aagttatcag cagctcgcct ctgaaaagga aaaggacatt cctttctggt     660
   tatactgtta tattactatt ctaaaaaata atttattttt ttaatcgaaa aaaaaaaaaa     720
   aaaaactcga                                                            730

<210> 60
<211> 846
<212> DNA
<213> Homo sapiens

<400> 60
ggagtttttt tttcatttta gtttatatta aataacaaat atttattcct gtgaatcagt      60
agtttacaca gataatattg agaggctttc ttgggaattt gaaaggagtc ttcaaatcat     120
cctttccctc agagatgaaa aatattttta aaaaaattac tgtcttgtat atttgatatt     180
ttgaaaatgg cagggaatca acaatttgtt aatctgttgt taagatcagt tatacattca     240
gtggcatact tcttgtctta gaaattggtt gaaattaata ttgctagtga aagtgtggaa     300
atagraacag ttgaaaggaa gacaaatgag aagtggacct tgcttctcat gaggatgctg     360
cagaactaga gtggttgccc agcaggatga aaatctcaat taattgcttg acagagaatt     420
aaaacaaagg caagtggtgc ttttaaaaaa gataaaaata ggtgaatata aagttgaaag     480
gaggccaggt acagtggctc acacctgtaa tcccagcact gtgggagccc aaggtgggtg     540
gatggcctga ggtcaggagt ttgagaccag cctggacaac atggtgaaac gctgtctcta     600
ctaaaaacac aaaaattact tgggcgtggt ggcatacgcc tgtaatcaca gctactccag     660
aggctgaggc aggagaatca cttgaacctg gaaggtagag gttgcagtga gccgagatcg     720
cgyccattac actccagcct gggtgacaag agcaagacta tgtttccaaa aaaaaaaaag     780
caactgaata ttggatagag aggagaaaaa gggcaatgta tcaaaaaaaa aaaaaaaaaa     840
ctcgag                                                                846

<210> 61
<211> 958
<212> DNA
<213> Homo sapiens

<400> 61
   ggcacgagcc ctgcggctcc ttagtcacct ctgatagcag attgagggag gaaaacaggt      60
   aaggcatgag gaaatggcca ggttgggtta acccactggt ttcaaccagt tcaggaatga     120
   ggttatttgg ccatgactgg ctgatcttga gctcaaggat ctgcttcaaa tgcacacagg     180
   cctagttgaa gtttaaaccc cagcaaaaca ttcctccctg taaatggaaa atcctacttc     240
   tacccccacc ctgccctgtt ttttgttttt tttttcccca agatcattag atgtcctcac     300
   ccctcctcac tgcctcctct ctgggacagg ctgggacctt gaggaagata aagccttcct 360
   tgactaccca tcatattcag tgtccctgtt cctcactcag agaggaaggc agaaccagtc 420
   aggcttattt cagtaagttc cacagttcta caagactgca ggaattctcc ttaagggagg 480
   agagcaagca ggtgtggccc cagcttctgg aaatggcaga agagagggtt ttctcattga 540
   atgggggtgg gggctcgtgt gtcctgggaa accccatcag tcccttcatt tcttgagact 600
   caactcctgg gaggagaggg tctcaagagt tgtccctgga aggagggcgg gggcagtctg 660
   catctatttc aggttgtggc tcttggttct aggactctta cttctctggc taagggctca 720
   gcttcttggg acttcaacca tcttctttct gaaagaccaa atctaatgta accagtaacg 780
   tgaggactgc caagtatggc tttgtcccta tgactcagag gagggtttgt cgggcaaatt 840
   caggtggatg aagtatgtgt gtgcgtgtgc atgggagtgt gcgtggactg ggatatcatc 900
   tctacagcct gcaaataaac cagacaaact taaaaaaaaa aaaaaaaaaa aaaaaaaa 958

<210> 62
<211> 582
<212> DNA
<213> Homo sapiens

<220>
<221> SITE
<222> (20)
<223> n equals a,t,g, or c

<220>
<221> SITE
<222> (27)
<223> n equals a,t,g, or c

<220>
<221> SITE
<222> (49)
<223> n equals a,t,g, or c

<400> 62
ccgtttgccg gcccgcctcn tgggacntgg tggtcccccc ccgggcctnc agggattcgg 60
cmcgrgtgca tacatgccta cctatgtata tataaacaaa catttttgta aacagctcag 120
tgaggacttt ggactggcat aaatcatagg aatatgatta tgaggataca tccaattttc 180
agattgggca atgtatacag tttattatca tttctgattt tgggtagagt tagtactaag 240
aacagcattg aagaaaagca gtataacatt aaaattaaga agatttaaaa tacaagagga 300
ttcataacag tcacttttaa aatattgttt tggctttcta ctttggagct gtaattttaa 360
aaaaagaatg aacaggtttt tgtatgaata tgttagaatg actaattata gagcatcttt 420
caactggaat acatgtagat actaacacct ggttgtattt gatgtaattt cagtgcatac 480
agtgtgtgta atctgtatta agtgaaatac ttatgaataa agttgtttct gcattgcaaa 540
aaaaaaaaaa aaaaaaaaaa aaaaaaaaaa aaaaaaactc ga 582

<210> 63
<211> 752
<212> DNA
<213> Homo sapiens

<400> 63
ggcacgaggg gagaggcagg catttgcatt cagtcttgaa ggctgaatag ggcagggtag 60
gcacagtgat tccagagaga agtctttgct cctccatcta tggaaaaact tctcacattg 120
tatttattac tatatgtttc ttactggagt gtctctccta ctggacaggg agcaggttta 180
tttattgctc agtcctcagc ccctggactt aggcagactc atagtagaca tttgggaaat 240
gcttgggaaa gaaaggaggg gaggagagag gaaggactcc atggccatgt ctaaatgccc 300
agcaatgtca tagaggttat gggggtgcag gagaagacac agccctccct ctggcagcta 360

ggatagagcc tagctgctgt taaagacagg cagctcattc ctcacctggg ccaagctgca 420
gctggtcatc tctgcccctt tctccttcca tcttatggga gcttttatgg agtcagaagt 480
gagtgaggca gacctgggag agccctacac tcaggaagaa tgtaggctgc agaaaggaac 540
aggtgtcctg gagttagctc aggaaggtct tgaaggaagg ggttaacyag cagatggcaa 600
cccagtgact tttgttgctc tctgaagcca cagaggaaaa cagtagcaac rrratraaat 660
aaaataaaat aaaaaataat aaaaaagcaa agttcccaag gaaataagat gggggaattc 720
gatatcaagc ttatcgatac cgtcgacctc ga 752

<210> 64
<211> 706
<212> DNA
<213> Homo sapiens

<400> 64
ggaaagaaat ccctactgtg tggcaccagg acctgtgtga cctgcaaggc gcctgttttc 60
ctcaacaaag cctcttttat accacttgct ccccacacca ccctggcccc ttccacttgc 120
tcaaaaacac tgagctcctt ttcactgtgg ggccattgaa tatgctgttt tccctcccta 180
gaaccttttc ctctcattct tcacctgccc aactcatatt tatccatgca gcctcagttt 240
taatggcatt tcctcccagg ccttccaaga ccactctccc tcaggcagct ttcctgacat 300
ctttagcctg cccgctcatg ctctctacct tttttctgta tcaaaatgcc tttgtttgca 360
agtaacagaa ggcctgactt aacctgcctt taaacagtaa ggacacaagt atgcctatgt 420
tattagaggt ctgcaggtaa ggcacgtaaa gggtcatctt tttccagtgt cttcaactcc 480
atttctctga ggttccatca gctacattct gtgccatgac tttatcctca gtgcattttt 540
cagatggtaa tcaaatggct gtaacatgtt cacctctagc tcagcatgat actcagagga 600
agaatagagt tgcttctagg agttttgtga tgagaatgag ggaatttctt tccctggagc 660
ctccagcaag cttgtcatta agtacctcct caggtttctg gctcga 706

<210> 65
<211> 400
<212> DNA
<213> Homo sapiens

<400> 65
tcgacccacg cgtccgccct gcatggcgag atgtcctcct ttcccgggcc acagtgtgtg 60
caactaataa acctcctcca tctcatctgc ccagtgtcgg gtcttgtgtg ttcagccatc 120
accatagccc tcaggcagaa gtccatccct caccaacagg gaagagaggc agtgatcaaa 180
acacctcctc caggaagtct tccctgaagt tcgtagtctg gcttcagtgc cacttcttcc 240
ctgccctcat attcgctaac cgccacttac tgcctggttt tcagcctcac taggatgtgg 300
gccactaagg gccaacatgg tcctacttgc agctgcatta tcagggccta ccataacacc 360
ttccaaatgc ttaaaaaaaa aaaaaaaaaa aagggcggcc 400

<210> 66
<211> 773
<212> DNA
<213> Homo sapiens

<400> 66
gcacaggtat gttttctgat ggcacaggcg aggtcacaga aaagtggatg gcaggcgttg 60
ctgtctgtca gaataacacg aaagtgagag aaggccgctc tttcagaata acaccacaag 120
tgggagaagg ccgctccctc agggctggcc atgaataaat ggggatttct gcctgttytc 180
tccctcccgc ctcactccct tttcctgcag aggcagctcc tgagccattg ccgagcagga 240
tgctagtttt agcatggatt acatttccac cgtgtaaagc ctgctgcatg atgtgcatct 300
tctccagccg cctccttcag caggagargg tttgcacart tgtccaggga arggaaccta 360
ggggcatggc ccaacgggac agaggatttg artccctctg attatgagca ggttaattta 420
aaagtgaaaa ccatggttac ccattgccct ttaaaaamca cccaggggcc gggcacagtg 480

gctcatgcct gtaattccca gcacttttgg aggccgaggc aggcagatca caaggtcagg 540
agatggagac catcctggct aacatggtga aaccccgtct ctactaaaaa agtacaaaaa 600
attagccagg cgtggtggcg ggccgagtag tcccagctgc tcgagaggct gaggcaggag 660
aatggcgtga acctgggagg cggagcttgc agtgagccta gatcgcgcca ctgcactcca 720
gcctgggtga cagagcaaga ctccatctca aaaaaaaaaa aaaaaaactc gta 773

<210> 67
<211> 647
<212> DNA
<213> Homo sapiens

<400> 67
ggcacgaggt ttgatatatt tttttctcat ctttttgctg ttacttatat gtaactatct 60
ttaacaagtt tgagatcttg ttatatattt tcatttgttg ctttataacc atttctctat 120
attactaagt ttaattaagg tctggaattt ttttagatgg tgtatcatgg gtataatatt 180
tatttagttg ttttcctctt gttatattta gattgaggca gtgctacagg ctttaactag 240
agaggtggtt ggctgttcag gactgggagg tggaggacta gcaggaacag aggtatagca 300
ggagagcatg cctactatgg gtataggggc agtaaggaga gcagctgaag cagccaccaa 360
ttaagaaagc gttcaagctc aacacccact acctaaaaaa tcccaaacat ataactgaac 420
tcctcacacc caattggacc aatctatcac cctatagaag aactaatgtt agtataagta 480
acatgaaaac attctcctcc gcataagcct gcgtcagatt aaaacactga actgacaatt 540
aacagcccaa tatctacaat caaccaacaa gtcattatta ccctcactgt caacccaaca 600
caggcatgct cataaggaaa ggttaaaaaa aaaaaaaaaa aactcga 647

<210> 68
<211> 675
<212> DNA
<213> Homo sapiens

<400> 68
ggactactcc attcctctgg atgtaaaatc tacattctct tgcctgaggt ggatacgttt 60
gcttgggttc tgtttaagga gatggggcca gcagtgtgtt tcagggcctg tgaaatgtgt 120
tctctatccg ggcttttgct taatctctgt tttcagtctt gcctatcagt cccactgtcg 180
ggggtacctc gtgtctgagt ctagaacctt tccaggttgc tgtgggacag attagcctcc 240
ttgttctcag tatcccctga cctccacctt tattgctttg ctccatgaat taaccatttc 300
catgtactgt catgtctaat gaagatgaat tctcttctgt tggtaacccc attccttttt 360
tgtaattgtg tgcttataca atgtttattc ttcactgtat ttctattgga gcctcaggac 420
aaagagcaga tggtgagaat ctgtgttcag tgttaagttt tccttctgta agacatgtgc 480
aacttgtgtt tttcactgaa tagatcatgg acttaatgca tatagagcta ctttgttttt 540
catgattgtg ccttcaatta tatgtagaaa tataatttgt gaattgcctg atgaaatttt 600
cctaattttg aattatcttt gcattcctat aataaacact gttagaatgg caaaaaaaaa 660
aaaaaaaaaa ctcga 675

<210> 69
<211> 889
<212> DNA
<213> Homo sapiens

<400> 69
gtacaggtgc atgccactgc acccagctca ttgccttttg ttttgtatgt taaagcagat 60
ttagcccatg aacttggaga cagttttgct gagcagaact tcatctcttg gctttgctgt 120
ttgtttgcct tgtttttttt gttggtttta cttagttttg tttttggagc taacatccat 180
aacttttgct atgtatgata taatcccctg tatgaccctg ggcaagtaac ttaacccatt 240
cagggtccag gttcctctta tgggaaaggg atgcttgata agacactgtt catggttcct 300
tgcagtttac tattatgata gatattcgat gacctaaaaa ttaaaccagt ttcctttttc 360

aaatttaatt tttycgggag gtggaggaag attttcattc cttatggttt gagaaacatc 420
gctttcatac atgtctaggg taaccaagtt ctctaatgaa tggcaatagt gatgtatttt 480
yctwaaatcc ttttctaamc agcattatgg gtttgtgctg taccggacaa cacttcctca 540
agattgcagc aacccagcac ctctctcttc acccctcaat ggagtccacg atcgagcata 600
tgttgctgtg gatggggtaa gaatcgtctc tgaactgtgc ctggcttttc tccactatct 660
tgaaatcaga tgggaggagg cttttttctg ggtgggactg aggaggcaca ctgaagtccc 720
ccaggtcatc ggggctgggc cattgccttt ttccccaccc tgggtagtcg tggacagaag 780
cttgggatgg gatggagagg agagatcgtg ctgtgtgtca tgtctgttgt tcaagtaaat 840
aaaagttgcc ctgacttcaa aaaaaaaaaa aaaaaaaaaa aaaactcga 889

<210> 70
<211> 888
<212> DNA
<213> Homo sapiens

<220>
<221> SITE
<222> (347)
<223> n equals a,t,g, or c

<400> 70
ggcacgagaa ctgccgtcca atctatgagc tgggcccttc cttccctctt ctttcttctt 60
ttctctccct tccttcttcc ttcaggttta actgtgatta ggagatatac caataacagt 120
aataattatt taaaaaacca cacacaccag aaaaacaaaa gacagcagaa aataaccagg 180
tattcttaga gctatagatt tttggtcact tgcttttata gactatttta atactcagca 240
ctagagggag ggagggggag ggaggaggga gcaggcaggt cccaaatgca aaagccagag 300
aaaggcagat ggggtctccg gggctgggca ggggtgggag tggccantgt tggcggttct 360
tagagcagat gtgtcattgt gttcatttag agaagtgggt gaaggttcct gggatcttag 420
gtaaagacta gacgccgcct agtactggtc tctactgtgc tggctcagga gttctgagaa 480
ctggaaggac ttagcctcaa cctgagttct gcacacaccc cttcccctta aggaaggcag 540
ctctgagagg cagcaggact tgatccaaac ccacagtctt gtcctggagg cagcaggggt 600
gaaggtggag ggtccagggc catgaggagc ccccttgcca tcagagcctg gcctaaccac 660
cctcttctct acttacacac acatgcattt tataatagct ctgacccaac ctggccactc 720
tgcagagact gggacagaca ggtgcaggca atgggccctc ccacacccag tcacctacaa 780
ggaattttca aatccacttt taaaacagaa accggtaaat gcgccgtatt gtatatttta 840
tttaaataaa aaaaattcca gcaaaaaaaa aaaaaaaaaa aactcgta 888

<210> 71
<211> 796
<212> DNA
<213> Homo sapiens

<400> 71
gaaaaaaaag aaaaagccaa aaaaaaaaga agaagaagta ccactgctag gatttgaacc 60
cagatctagc tgactcaaga accatgccct atctctgtgt ccatgttgtc accacttaat 120
cacttgtatt ttcccttcag gtttctctgt atgctgtgtt ctctcccaag agtggtcttc 180
caactcaccc ctattaagga agctttccca agccaggagc ttacctttcc gtgcacacat 240
tgaatgatga tcatttgtca ttctgtcttg ccttacaaaa gaggaccagc tccttgagga 300
taggaacctt gtccttatct ccctgttccc ctgtatgggg gccagctcct ggcaggtgca 360
tagtaaataa tgagtgataa acttgttgga aagaccatgc aggaaccaag caactctttt 420
cctctgcctc aatgcagtta gttcaagaac ttactaagaa aagagttgtt ggccaggcac 480
agtggcacag gcctgtaatc ccagcactgt gggagaccaa ggcaggcaaa ttgcttgagc 540
tcaggagttt gagaccagcc tggacaatat ggcgaaaccc catctctatg aaaaattgga 600
aaagtagcca ggcatggtgg catgcacctg tggtcccagc tactttggag gctgaggtgg 660
gcgaatcact ttagyccggg gaggtcgagg atgcagtgag ctgagattgc gccactgaac 720
tccagcttgg gcgacaaaat gagaccctgt ctcaaaaaaa aaaaaaaaag aaaaaaaaaa 780

aaaaaaaaa ctcgta                                                    796

<210> 72
<211> 532
<212> DNA
<213> Homo sapiens

<220>
<221> SITE
<222> (434)
<223> n equals a,t,g, or c

<220>
<221> SITE
<222> (528)
<223> n equals a,t,g, or c

<400> 72
ggcacgagta aaaggtgcca tctatgaatc agaaagtacg cccttaccag acaccgaatc      60
taccagctcc tggacagaac agactaagat acattccaag aagcagtttc tttggagaca     120
gaggcgtaac tgtgcatatg gacaaggttt atatttctgt tcaaagtggc catccatatg     180
cttctaggct tcctttgtct ctggtatcaa gtgtatgtat gtatgtatgt atgtacttat     240
ttatttattt atttattatt ttctcttttt tctctgcccc atatgatctg caagaaaagt     300
gtcaagttta taatgagctc cccaaagcca ccatctgggt agcctcacat ctttttcatc     360
ccctgtgcct cttccctgct tttgtcctac tctagccaga ctcgtgccga agggggggcc     420
ggtamccaat tcgncctata gtgagtcgta ttacaattca ctggccgtcg tttamaaagt     480
cgtgactggg gaaaacctgg sggtacccaa cttwaatcgc cttgaagnaa at             532

<210> 73
<211> 546
<212> DNA
<213> Homo sapiens

<400> 73
ggcacgagct ctccagcacc tccttggaac agatgccctg ctactttaca aggcttgtgg      60
aaaagagaaa gagaacagta gcaaaagcct gtgtagttca tgaatagaag ttagcatcgt     120
agtgagtaag cagtactgat gatctgtgaa atgattctct gtggacttga gcatgctaaa     180
aagatcttga aaaaggaaaa cataaatctt tccaaaacct cacatgaccc ctgtatgctt     240
tcgccttctt gaagctttgg aggagagcat aggtgtggat gaaatggagt cttttaaaag     300
ttgttttggt ttttgttttt gtgtgtgggt ttttaaagag agcatatcct gccacgtaga     360
agaaaatcca gggggtggct gtcctcctac aggaaggagg taaacaagca tttttcctta     420
agggctctat tccctcagcc tcgctccctc gaaggccaca cttggaggcc aggaagttaa     480
tccattaaaa aaaaaaaaaa aaaaaaaaaa aaaaaaaaaa aaaaaaaaaa aaaaaaaaaa     540
ctcgta                                                                546

<210> 74
<211> 715
<212> DNA
<213> Homo sapiens

<400> 74
ggcacgagct ttccctcagt ccaatcttgc aattgctatg tcagtttcag ttcacaataa      60
taccagtgca gacatggctc cttaagattt tctccttttc cctcacgcgg gtcccaattc     120
taaattccca agggctgaca tgattgacat ttgccatagc ctgaggaggg agcatttcct     180
tttgtggtct ttccttggtt tgttttattg ggcagtgaat ggcaagtctg tctgtgtttc     240

tttgcttcac cccaaacacc ttggcaaaaa tgaaagcctt ctaatttagc tgtgtcctcc 300
tttacttatg tcaggaagcc tgagccataa cctttgatta aaaaaatttt tttttgtttt 360
ttgtttttga gacagggtct tgctctgtca cccaggctga aatgcagtgg cacgactgca 420
gctcattgca gccttgacct cactggagtg tagtggcatg actgcagctc actgcagtcc 480
caagtagctg gcacttacag gcaggtgcca ccatgcctgg ctaattttta aattttttgt 540
agaaacaggg tcttgctggc tgggcacggt ggctcacacc tgtaatccca gcactttggg 600
aggccaaagc gggcggatca cgaggtcagg agtttgagac cagcctggcc aacatggtga 660
aatcctgttt ccactaaaaa taccaaaaaa aaaaaaaaaa aaaaaaaaac tcgta 715

<210> 75
<211> 406
<212> DNA
<213> Homo sapiens

<400> 75
aggttttcca gaaagttatc agatcttgct ttcctgatta gcagcagtta gcggggtgga 60
taaaagcacc ccttcagagc aatctcattt ccatttcttt caggccactt attttttcca 120
actttttttc cgtatcttca taaatgtttc actcttcttt gttagtattt cttagtctct 180
tgagtcaaga aatatttact gagtatgatt gcatgcataa gtagtgtgcg ttagagatac 240
gatacctgta agacaccaca gtgctgggta gatccgggtg ccattgtctg ttgccagggc 300
cgaagttggc attttgtaag tgttcgaata agcaccatgc cgtgggataa gaaataaaag 360
tgtgtgcctc atctgtaaaa aaaaaaaaaa aaaactcgag gggggg 406

<210> 76
<211> 542
<212> DNA
<213> Homo sapiens

<220>
<221> SITE
<222> (429)
<223> n equals a,t,g, or c

<220>
<221> SITE
<222> (473)
<223> n equals a,t,g, or c

<220>
<221> SITE
<222> (510)
<223> n equals a,t,g, or c

<220>
<221> SITE
<222> (518)
<223> n equals a,t,g, or c

<400> 76
gatcttaagc atttttaagc acccctggat agctctcaat gacaccctgc gctggctgtc 60
ctggagtcac ctgggggagg gagggaatgg gttgctagat ggtgcatgtc agtaatttgc 120
cttggtgttt gatgacatta agtatattcg cattgttgtg caaccatcac tgccatccat 180
ccacagaacg cctttcctct tgcaaaactg aaactccgta gtcagtaagc aacaactccc 240
cagtccctca tcctccacct cagcctctgg aaaccactag tctactttct atctctgtga 300
gtttgacact ctcagtacct tgtacaggtg gaaccataca gtatttgtct ttttgtgact 360
ggcttatgtc acctagaata gtatcctcga agggggggcc ggtacccaat tcgccctata 420

gtgagtcgna ttacaatcaa tgggccgtcg ttttacaacg tcgtgactgg ggnaaaacct 480
ggcggtaccc aacttaatgg cttgcaggan atcccccntt cggcagtggg gtaataacga 540
ag 542

<210> 77
<211> 420
<212> DNA
<213> Homo sapiens

<400> 77
ggcacgaggg acaagaaggc ctttctctcg agtcggcatg gttccacttc tctgactgca 60
tcgggaatta cctctccttt gggccaaaga caaaaaagaa tgcagacttg tttccaggat 120
gattaaatta cattcagcat attcttcccg agtgcgtccc gtcttagtgg ggtttagagc 180
tgcgttcagg ccagctgggc tccggttacc tctaatgagg atgatgatct ggaggcttag 240
cgataattct gcactgattc tcttgtgcct gcagaacctg tgttggccaa cttggatggc 300
aggggaagat caacagaagg tgccctccac ccacgtcctc ccagcgctca ccttggtcag 360
cctgggggcc aactcgtgcc gaattcgata tcaagcttat cgataccgtc gacctcgtag 420

<210> 78
<211> 465
<212> DNA
<213> Homo sapiens

<220>
<221> SITE
<222> (446)
<223> n equals a,t,g, or c

<400> 78
gattttttcc catcgtggaa cagagtcttg ccaacttata cctctctctg agccttagtc 60
tcctcgtttg taaaatgaga gttaaaatct acctcatgga atcattgcta agattaagca 120
agatatataa gtagagcttg tgcacatggt aggtacttgg agaatgttat ttctccttcc 180
ctcttactca tctggacaag tttaactaga attctaaaca gttaaatatg tatcaatcct 240
ttgtattaaa tatcttggtg gtaaaatgtt aaaatattga tgtgaataac agctggtatt 300
gaatattcaa attaggggaa ctctttcatt gttttaagat aacatctgta catttaatct 360
gtgccatgca ataaaacagc ttttcctgaa aaaaaaaaaa aaaaaaaaaa aaaaaaaaac 420
tcgagggggg gsccggtacc caattngccc tatagtgagt cgtat 465

<210> 79
<211> 890
<212> DNA
<213> Homo sapiens

<400> 79
aggttactta ttgctcctac ttcatatcat atgtggttct acaacctaca ttatcttgtc 60
tatgtctttt aactagctgt gtgttcttac ataagatctg cagaccttgg ttctcaactg 120
caaaagcata ttgattaaat gattactgtt tttacctgca atactttaat tttggatttt 180
gggattaata atgtaaaaaa gactaacata tatgtgggat tacaaaactg ttttgttagc 240
cttcaaacaa ctatgaactg catcaggagc tgtcttatac ttattgttct gctattaata 300
cttaatgcac tgcctgtaaa gagctgattg ctacttaaaa actctgctta aatgaaaaac 360
caaaacataa aagattaaac caaacatact tactctccca tagccctggt ggacagcaac 420
ataaggaggg aaatgtttct gttgatcttt ggcttcaagg attaatacca gatttggata 480
ccggttagtt agataattgg taaggaatcc cataaagttg taaattacat aagcttcata 540
gcattctctg caggtatcca catatattgc aattccggga tatttcaaag ctatccacta 600
tgaaaaagca cagatgttaa agatagttgc agctaagata aaatgaatca ccactccatt 660

catggtactc acaataagct aattttcatg cttgagatgt cttgtcatat acttacatgg 720
gactctctaa aatttatcat tatgagggct atcaatctgt gaaatgaatg cttaaaagca 780
ataaacatct tagatattgg taaacaaaaa caagtgtttg aggggtaaat aatgaataaa 840
gagagaagct aaagtaaaa aaaaaaaaa aaaaaaact cgtaggggg 890

<210> 80
<211> 470
<212> DNA
<213> Homo sapiens

<400> 80
ggcacgaggg aaatcttgca cataggcagg taaataatta taaatggtga agtggattat 60
tctgagctgc ttaattttaa agggaaagag aactttaaac tcttcaacct tttatgctgc 120
taataagagt tccacaatca atagaaatct atcttggcag gcacttcctt ttacccacta 180
gaattttttc ccttgggagt tcacgatccc cagaaactgt gatatgagcc attcaatatt 240
gatgtactaa aacagtgctc tgcttaaata cagtttttca acatacagtc ttggaagaaa 300
caaaatccaa aataaattcc aatagtccag taacaggaat aaagacaact attgcaaatt 360
aaatcttaca gacttatatg aaagctgttg ttaacagctg ggtactagtt atttgaaaag 420
tttctcgtgc cgaattcgat atcaagctta tcgataccgt cgacctcgta 470

<210> 81
<211> 1090
<212> DNA
<213> Homo sapiens

<220>
<221> SITE
<222> (8)
<223> n equals a,t,g, or c

<220>
<221> SITE
<222> (28)
<223> n equals a,t,g, or c

<220>
<221> SITE
<222> (43)
<223> n equals a,t,g, or c

<220>
<221> SITE
<222> (54)
<223> n equals a,t,g, or c

<220>
<221> SITE
<222> (95)
<223> n equals a,t,g, or c

<220>
<221> SITE
<222> (545)
<223> n equals a,t,g, or c

<220>

<221> SITE
<222> (863)
<223> n equals a,t,g, or c

<400> 81
cattgacntc aatgggagtt tgttttgnca cccaaaatcc aangggactt tccnaaattg 60
tcgtaaccaa ctccccccca ttgaccccaa atggncggta ggcgttgtac gggtgggagg 120
tctatataag cagagctcgt ttagtgaacc gtcaagatcc gcctggagac gccatccacg 180
ctgttttgac ctccatagaa agacaccggg accgatccag cctccggact ctagcctagg 240
cttttgcaaa aagctattta ggtgacacta tagaaggtac gmctgcaggt accggtccgg 300
aattcccggg tcgacccacg cgtccgccag cctggaggcc cagacgtggc gcagcgactc 360
ggaggttcgc ctccagcttg cgcatcatct gcggccgggt cccgatgagc ctcctgttgc 420
ctccgctggc gctgctgctg cttctcgcgg cgcttgtggs cccagccamr gccgccactg 480
cctaccggcc ggactggaac cgtctgagcg gcctaacccg cgcccgggta gagacctgcg 540
ggggnatgac agctgaaccg cctaaaggag agkgaaggct ttcgtcacgc aggacattcc 600
attctatcac aamctggtga tgaaacacct ccctggggcc gaccctgagc tcgtgctgct 660
gggccgccgc tacgaggaac tagagcgcat cccactcagt gaaatgaccc gcgaagagat 720
caatgcgcta gtgcaggagc tcggcttcta ccgcaaggcg gcgcccgacg cgcaggtgcc 780
ccccgagtac gtgtgggcgc ccgcgaagcc cccagaggaa acttcggacc acgctgacct 840
gtaggtccgg gggcgcggcg ganctgggac ctacctgcct gagtcctgga gacagaatga 900
agcgctcagc atcccgggaa tacttctctt gctgagagcc gatgcccgtc cccgggccag 960
cagggatggg gttggggagg ttctcccaac cccactttct tccttcccca gctccactaa 1020
attccctcct gccttaaaaa aaaaaaaaaa aaaaaaaaaa aaaaaaaaaa aaaaaaaaaa 1080
aaaaaaaaaa 1090

<210> 82
<211> 698
<212> DNA
<213> Homo sapiens

<400> 82
gtctagttta tgtttttcca ctggacaggg agctccttga ggaccttgtc ttgctcgctg 60
cccccaccct aaaacttgct gtaaagcagt tcctggaaca gagcaggtgc tcagtagtac 120
tggttgcatg aatgaatgaa tgaatgaata ggttttcctc ttttagacac attgggagat 180
gggcctatgg tttcctatgc tcattttgac ccagagattt gtgtcctgtg actcacatcc 240
agacccaaaa cacacacata cacacgcaca cataaataca cacacacaca gacacgtgca 300
cacacagaca cacatgcaca cacacataca cacaccttgg tttgaagaga agagggatgg 360
gaacagacat tctacgcatg cctacagtgc accactgtgc ataggtaact gatgctgtat 420
aagcactcaa ggattatctc catttttagc cagagaaact gaggcttgct ttctgctgtg 480
tctccagtgc ctagcactgt gcctggcata aacatctgct gaactgaatt gcactagatt 540
caagaggctc agaaaacagt tcaaggtcac ccaactagca agttgtggag ccagaatctg 600
tgctcagggc tgttcagtcc ccagccagtg ccgggtagca gccataggca cctgcacaaa 660
ctccagcgac ctcgttaact tccaaacacg gtctcgta 698

<210> 83
<211> 868
<212> DNA
<213> Homo sapiens

<400> 83
cacgcgtccg cggacgcgtg ggcggacgcg tgggcaaaaa tcttaaaagc actttatcat 60
ttcatttccc tgcactgtaa tttttttaaa tgatcaaaaa cggtatcata ccaaggctta 120
cttatattgg aatactattt tagaaagttg tgggctgggt tgtatttata aatcttgttg 180
gtcagatgtc tgcaatgagt aaatttagca ccattatcag gaagctttct caccaatgac 240
aacttcattg gaagatttta atgaaagtgt agcatactct aggaaaaaaa tatgaatatt 300
ttagcatcta tgtattgaaa attatgttga ataaatgtca gactattttt tacataacgt 360

tgcttctgtt taattttgtc acgttcagag gtggggggta ggagatgtaa gcccttgaca 420
gcaaaataat tccttttgct tgatttcaga cagttgcatc agctcctttg ttctgtgttc 480
atgttacact tatttaggtg gctgaatcca cagaggagcc tgctggttct aatcggggac 540
agtatcctga ggattcctca agtgatggtt taaggcaaag ggaagttctt cggaaccttt 600
cttcccctgg atgggaaaac atctcaaggt gagtgttata ataaagatct tggcttatgc 660
aacatgaatg ttcctcgttt gcatcaattt aagaataagg tatgtttaca cgtatataat 720
cagaactttt aaacatacag aattttgctt tataaatagc ttcgctttaa agatctctta 780
tatatttaac ttttcttaat acacagcctt ttagtacaca caaatttaaa aagtaggtaa 840
tgcatatatt gaaaaaaaaa aaaaaaaa 868

<210> 84
<211> 629
<212> DNA
<213> Homo sapiens

<400> 84
ggcacgagaa cctttggggc tgacacaaga tcctttagtg tttgggatga cctctttcct 60
gcagacttct tcccctatcc ctaactcatg catggaaaac gtttgtcagg ctggtttccc 120
gagcctcctg cacctcaaca tcacgctcac ccttttgggt ttagcccagt gttatttagc 180
aaatttctcc agctgcaggg aaggatcaga gcactatctt tttttttttt tttttctcct 240
ggagccagga ctgcacaagg caatggccaa atttagttga attcagccta ccatcctttg 300
ctgatgactc agctctatgc caagtactgg agccacagag atgggtcagt cccagcccct 360
gtcctcagga agcccatggt cagggaaacg ttgtagggat aagtaataga gggcagttgc 420
cttcagggct cctggtggct gctggtccct atggtgcctt gatgtgaatt agaagacggt 480
gccctttcca ggtggattca gacctacact agaacgcaca gctttgggag tgacacacag 540
gttggatttt agcacccctt gccccttggc cagaggtgcc ctgctgcacg gccatacgct 600
gcagcctcga gggacacaca ggccaaagt 629

<210> 85
<211> 837
<212> DNA
<213> Homo sapiens

<220>
<221> SITE
<222> (474)
<223> n equals a,t,g, or c

<400> 85
   gcttccaggc tccagcctct gcccgcactg cttgcagtac cctactcatg tgtcctcttc 60
   atgtgcccct ccccggtcat atgggtccct tctggcccct gcccagctta tactctgtcc 120
   gatcctcaca gtcaccctgt cccctttgct tttctttgct gccactgcag gcccacctca 180
   gcctcctgca cactctcttc agatcagcct cccaatctcc agcgtctgga gtgttctggg 240
   gctgcctgag agagagacat gaatacatgt caccctgcct tcctcacatg taccagaagt 300
   ttgatttttt tttttttttt tgactgagtc ttgctctgtc accaagctgg agtgcagtgg 360
   cacgctcggc tcactgcaac ctccacctcc cgggttgcag cgattctcct gcctcagcct 420
   cccgagtagc tgggattaca ggcatgcacc agcatgccca gctaattttt gtanttttag 480
   tagagacagg gtttcaccat gttggccagg atggktttga tctcttaacc tcgtgatccg 540
   cccgccttgg cctctcaaag tgctggaatt acaggcgtga gccaccacgc ccggccctga 600
   ttattattat tattatttta aacaataatc tgggccaggc acagtggctc acacctgtaa 660
   tcccaacact tttgggaggc ctgaggcagg aggattattg agcccaggaa tttgagacta 720
   gcctgagcaa catagtgaga ccctgtctct acaaaaagta aaaaattagt ccaggcatgg 780
   tggcacatgc ctgtagtccc agctactcag gaggctgaga taggaggatc actcgta 837

<210> 86

<211> 903
<212> DNA
<213> Homo sapiens

<400> 86
ggcacagcct tccccctgcc cttcctgcct ggctcactcc tggccaccct tcagactcct 60
ctctctgcct cctccagctg gcgcctcact tggtgatggc cgtgtctgtt ccatggcccc 120
tcccagaggk acttggtttc tcctgctgtc attgcgtctc ccttacgggg ccgcatgctg 180
ggttttctta ccatttcctg catcctgcag agccgagggc gtggcagcac caatcaagtg 240
tagtaggaat gagtaggaaa caagcatcct tctccatggc acagaargga gtctgtcacc 300
ttggaaagtc aytcaagaga ggatccaaga aagcgtcttg ccctamctac ccctccttta 360
gcaagtgagg atcttcgagg graggggagt ttccaagtca actggtgaca aagccaggat 420
gagaagacac tcccagacca ctgtggctaa tgacacacac tgcccggcca tgccatctgc 480
cagcgctgga ggtggccgct caacacagga aggtcaaggt catgttagca gctcccccac 540
ccagcagggg aaagggaaag acttgcactg gggagcagtt ttatttattt ttatttattt 600
attattaatt atttttagat ggagtcttgc tctgtcaccc aggctgatgc agtggtgaga 660
ttttagttca ctgcaacctc tacctcctgg gttcgagcga ttctcctgcc ttagcctcct 720
gagtagctgg gactataggt gtggtggtgc atgccggtaa tcccagctac tcgggaggct 780
gaggcaggag aatcacttga acctggaagg cggaggttgt ggtgagccga gatcacacca 840
ttgcactcca gcctggacaa caagagtgaa atccgtctca aaaaaaaaaa aaaaaaactc 900
gta 903

<210> 87
<211> 725
<212> DNA
<213> Homo sapiens

<400> 87
aggttctaag cattttgctt gacctgactc atttaatcct cacaaaactc tacaagataa 60
gtatattctc actactttac aggctaaaaa tctgaggcac agaaaagtta ctgaagctcc 120
aaggtcacac tgtgtaccat aagtggaaga gctaggatgc aaacccaggc agccgggttc 180
cagagcagtg ttctaactac taccctctgt tgcctctcat tcatcccatg accttctttt 240
gtcttaccta cactgggatg tgtttgggac atgcattttg cttgttgcta tctcattctt 300
gcagaatgca ttgtacttgc tatttgtgtc tattcacagt tcaggttttg ccaggcaagt 360
acaatgaagg aggagagggg caaaggaatt gagggtgcct acaagggagt agttagagag 420
atggatgtga aatctaagct gggcaaattg agaagtaagg acatgatata ggtgatgggc 480
agtaaaaata tgtaatgtca gcagtttaaa ggactggatg gggcagatat taattggagt 540
tgcaggacta aaggagttca aaatatagga aatgaatacc agagacagag agagggctga 600
agtcaaaatg ttggaggtgg tacttattat taacaacaag gtctagagga tgaccgcaga 660
attggggtcc aaggtgacac atggctgaca gctgtcattg accacactgt aatgcagaac 720
tcgta 725

<210> 88
<211> 606
<212> DNA
<213> Homo sapiens

<400> 88
tggtcccccg ggctgcagat tcggccgaga attacacgaa ttaawttatt catgaggcta 60
catttcattt catatgcatg tttccaggtt gtattctctt gtgcaatctg tgtatgttct 120
ttgtcttatc tttttctatg ggaatatttg ctttttattc acttataaga gcaatgcatg 180
tatcaaggtt agattttaat tttgcaacat attttgtggc ataatcaggt ttaaaatgct 240
tgaagttacc atatatgtaa attttttctt catgttcttt gcatttaagt gactggaaga 300
gttcattcct tccactgaaa tcactgaata actaccttgg ctacttggtg ccaatgatga 360
aggcatcata tttatacccc tcaaaggatt cacagtccag gaagaagcag acaaacgaag 420
actttcataa gtgctatgga gagccaagga accatctcga tctgctggga attcctgggg 480

caggaaactg aggatgggac tgtggtccaa ggaggcagac tctgaccagg ctgggacagg 540
gaaggggagc gttcaggtca aggtggtcgg ccttctgtca gagcatactg cattacagta 600
ctcgta 606

<210> 89
<211> 1142
<212> DNA
<213> Homo sapiens

<220>
<221> SITE
<222> (39)
<223> n equals a,t,g, or c

<400> 89
tgaacagtgc aggtagatac tggactgggg gcagatctna gggagagggg tttaagtagt 60
gggaggacac tggggatagg ggcttggggc tatttacctg ccattttaag tagtttgcta 120
ttttagcagc caacaataac tattggtgct gaataccagc cctgcagtgt agcatgagac 180
aggtccatgc acacatgcat taggaaaaca ccttcatgaa gcaggattct gcctgggctg 240
atgcacacaa cctctatgga gggtgaaaca gtgtttctga agaccgtagt ttgggaaccc 300
ctgacatatg agcaatgccc ccttagataa gctcaagtta caggaatgty tgagggtgga 360
aggtgtggat atgtgctttt gcctgtytcc ctcttacagt gtctggccat ggggcataaa 420
cactacccag cagtaggtag gytggccaag agaagccagc ttgcatcacc agcatcatct 480
agggaatgga atcatggcag taatacgttg cttaggaaac aaaagctcta tggacacatc 540
ttccaccttc tcagtcccag aaaccrtatg tactgtgacc ccgctcayta ggcccagccc 600
tcgggaagag tgtgggccct tgaaaaggga agactgagtg agcaaaatga tgagaaaact 660
acaaaatggg cagaggtcag tctgacacat tcattctctg tcaagctcag gaagtactgg 720
tccctgatct tggagatgct gtgtgagtgg cagggggact cctgctgggt aaatattcta 780
tatgtggatg cctggacagg cccctatccc aggccctgct tgtcagaagc tccccttggg 840
ccgagcgcgg tggctcacac ttgtaatctt ggcactttgg gaggccgagg caggtggatt 900
gcctgagttc aggagttcaa aaccaggctg ggcaacatgg tgaaaccctg tctctactaa 960
aaaaaaacta accaggcgtg gtggtgcatg cctgtaattc cagctactag ggaggctgag 1020
gcaggccaat cacttgaacc caggaggtgg aggttgcagt gagctgagat cacgccactg 1080
cactctagcc tgggcaacag agcgagactc tgtctcaaaa aaaaaaaaaa aaaaaaactc 1140
ga 1142

<210> 90
<211> 596
<212> DNA
<213> Homo sapiens

<220>
<221> SITE
<222> (4)
<223> n equals a,t,g, or c

<220>
<221> SITE
<222> (8)
<223> n equals a,t,g, or c

<220>
<221> SITE
<222> (28)
<223> n equals a,t,g, or c

<220>
<221> SITE
<222> (57)
<223> n equals a,t,g, or c

<220>
<221> SITE
<222> (61)
<223> n equals a,t,g, or c

<400> 90
gganaccngc tttgcccctt ggtttccnca aagctcgaat ttaccctcac taagggnacc 60
naaagctgga gctcccaccg cgttggcggc ccgctctaga actagtggac cccccgggct 120
gcaggaattc ggcacgagtc ctgacctcag gtgatccacc cacctcggct tcccaaagtg 180
ctaggattat aggcttgagc tactgtgccc ggcccatggt gtttttcttt agggctcttc 240
ctacagcctt gagaagtaga taggcatcag agtatggtac tataggaatc agaaaaattc 300
aaaacaaatg tggattaagt gtttaggctc tatgtggctc acgcagccag aatccttaag 360
tctgtgtgtt tctgtgtctc aagactgggc tcacattctg gctttgtcca taacaatgct 420
ctgggatttc agggagttcc ctcatttgta aaatgagggg gtcagagcag gtgatatcca 480
tgtttcttcc ctttctgata ttgttgtctg tggcatattc tttgtatggc gaatttaata 540
aattatatta atgtgtctct ttgaaaaaaa aaaaaaaaaa aaaaaaaaaa ctcgta 596

<210> 91
<211> 633
<212> DNA
<213> Homo sapiens

<400> 91
ggcagagtgt ctctcaatgg cttctttctt gaagggcatc acagccactg tacttatcaa 60
tgcctgtgta gccaacacag tagctcctct acattacaag gatatgatta ttcctaaact 120
tgtcgatgat ctaggaaaag taaaaatcac taagtcagga tttctcactt ttatggacac 180
ttggagcaat ccactggagg aacacaatca ccaaagtctt gttccattgg aaaaggcgca 240
ggtgcccttc ttgtttattg ttggcatgga tgatcaaagc tggaagagtg aattctatgc 300
tcagatagcc tctgaaaggc tacaagctca tgggaaagaa agaccccaga taatctgtta 360
cccagaaact ggtcactgta ttgacccacc ttattttcct ccttctagag cttctgtgca 420
cgctgttttg ggtgaggcaa tattctatgg aggtgagcca aaggctcact caaaggcaca 480
ggtagatgcc tggcagcaaa ttcaaacttt cttccataaa catctcaatg gtaaaaaatc 540
tgtcaagcac agcaaaatat aacattgtag ccacagacca gataccatta ataaaaatcc 600
tattcataaa aaaaaaaaaa aaaaaaactc gta 633

<210> 92
<211> 725
<212> DNA
<213> Homo sapiens

<400> 92
   ggcagagctt ccctagcaat aattactttg cttaatttac ttttttcatt cttgtgcgtt 60
   cctttatatt tcatatatta aatatccatc aacattatat aggggtcttt aaacattatg 120
   taacaagata catattgaat gtattacact gcagcttgcc ttttcatttc agtgttgttt 180
   ttaggtttat ctgtgttgat aagcgttgct gtagttcatt cattttttaa acattgtata 240
   gtatttcatg atgattaaac cacaatttat ttattctcct gttgatagac aattaggatg 300
   ttttcagttt tttgctgtga caaatactcc cgttatgggc attattttgt ctccttttta 360
   catagataca aaagtttccc tacggtatat accaagaaat ggaatttctg agtttttagg 420
   gtatggacat tctcagcttt actagatttt gcctagttca tctccaaaac tgtggtacta 480
   atatactttc ccaccagcag tatataagag ggcctgtttc tccacatctt tgttaaaact 540
   atatattgtc aaatttttaa attttgccaa tctgggccag acactggggc tcacatctgt 600
   aatcctgtaa tcctagcatt ttggaaagca gaggcaagag gatcgcttga ggccaagagt 660
   ttgggaccag cctgggcaac agagcaagac cccgactcta caaaaaaaaa aaaaaaaaac 720
   tcgta 725

<210> 93
<211> 601
<212> DNA
<213> Homo sapiens

<400> 93
tcccccgggc tgcaggaatt cggcacgagg tcggcacgac actgccccaa aatcaaaatg 60
gctcaagtcc actttcaaaa atgtcagtgc tcaccaacag tgggtgaaaa ggctgcctga 120
cccagcttct cagagagcca gtgcctcaaa tccaatgcat ggcaattgct ctgggccccc 180
tggttttaag ctggctttgt tatttgtggc tgacactgga aagcctctgc acaaacaaga 240
tggcaagtga tgagccggtc agtcatcact gccttcccag actctctgaa ccacccttga 300
cattctgcct ggaagcaggg ggcttggtgg aggtgggtga cctcttgaag tcccgggcca 360
ggcctgtgat tctgtaatct ttgctttacc ataattaggg agggaggcag aagagcagga 420
ggagaaacca tttattactt ctctgggatt ttgacagctt ggaaaaagag agagacagag 480
aaacagtcca gagaaggagc cagccacagt gagtttaacc tctcagtaaa ataaaaatgg 540
gctggacgca cctcatcagc tgccctctgt caatacccgg gcccatctgg caggactcgt 600
a 601

<210> 94
<211> 692
<212> DNA
<213> Homo sapiens

<400> 94
ggcacgagct aaaagagcta gtttgagtaa gctgtgtaag acagctgctg ctaaatagaa 60
ccaaattcac ctgcctatgg ccggccaccc agtgttcttt ctgctcatcc acctactgcc 120
cttagacttc agcatgggct ggacccagac cccaggatct aacaactggc gacgaggatg 180
gaaggaggtg agtgggtctt cagcccctga gggctcccgg gacggctacg tggccgcagc 240
atgagctgtg gtacccggtc gcagtggtgc tgcttggatg agccccagtg gaaacatggg 300
aggcagtgta cagatcccct atgagtgtgg agaaggcgct gaatcacctg gaaatgcaca 360
gcattgaaag gaacatacct ttgccagcag agtcagatgg gcatttgcga ctatgctgag 420
ggaaatgaat gcccaatccc tgcaggatgc agcgcaggga ggaggaacct ccgttgcagg 480
cttgcccggt agtccgtcag aaaatagagc atgaacagct gttgggcccc aagaggaggc 540
ccagagaccc cccatcgtgg tggaacacat ttcctatggt gcctgtgtcc ccgctgaatt 600
gagggagtta agcaactaat gtcgccagtt gtgtacagac ttagtgcaag tcattcggga 660
gaaggacatt tgcgcaacct agtcctactc ga 692

<210> 95
<211> 1005
<212> DNA
<213> Homo sapiens

<220>
<221> SITE
<222> (506)
<223> n equals a,t,g, or c

<400> 95
ggcacgagct cgtgccgttg gttttccctc tgtctgttca gtgggttctg aacattcttt 60
gatggctgga tcctactcct ctgacatctt agtgttggca agatcttgga ccctcctcct 120
tctttctgtt ttgaggttgc agaccgttgg ctcatcagtc acactggact cacaggtggg 180

tattatttgg cctgcagttt tcaaaatagg aaatcgtgtt aaaaaacaaa atcaaataaa 240
agaaaaacga caacaacaaa accaaaactg aacttccaat ttatcttgga gaattagcag 300
acctagtaaa atgagttctg tattctcata tggcaataat tttctggagc tgagtacctg 360
cttcttgggt cattcttaat caactcattc tttccaaaca tcttataccc agcctgtgtc 420
attcatttag gtgagctgac aaaggctagt aggaatataa atttatgacc cttagtttat 480
actctcccca gtggatctta tttaantacc cattwaaata ccatatgctt taaaaagtct 540
tctttcataa cattgagtgc acacaatatg ccctgaacta tgtaccagac actggggata 600
cgcggtgaat kacgcaagtc actctacttc caaagaactt accttctata gaggggagac 660
acacacaaca gtgataacat aaagccaaat aatatttggg ctgggcgcag tsgctcatgc 720
ctgtaatccc agcacttcga gaggctgagg cgagcggatc acgaggtcaa gagattgaga 780
ccaacctggc caacatggtg aaatcctgtc tctactaaaa atacaaaaat tagctgggtg 840
tggtggcagg tgcctgtaat cccagctact tgggaggctg aggcaggaga attgcttgaa 900
cctgggaggc gaaggttgca ttgagccgag attgtgccac tgcactccag cctggtgaca 960
gagcgagact ccatctcaaa aaaaaaaaaa aaaaaaaaac tcgta 1005

<210> 96
<211> 612
<212> DNA
<213> Homo sapiens

<400> 96
gggatctgtg taagacaaaa ttaatagctg tatctggagt actctaaatg tggatttata 60
cactaaccta tatattgatc aattcctcta tgcttgcttt ggttttgagc aaattatatt 120
taaataagtt tgttgctagg aatgtcttaa aaagctactc accctttttg ttagaagtaa 180
gtaaatgatt atgtcaggac ctgccattaa cttggtatag tacgaatata tcctcagaat 240
actgataaaa tggtatgtct tgaaacaaat cacaaactgt caatatgttg gtgatgaatt 300
tcttctgttt tcatttggat cagtagtggg gcagttcacc aagtgtgaga tcgacattta 360
atgttttcat gaaatgcaaa cccatcagtg gctaatttgt taaaaaatag atgttgggct 420
tttcttaagg ctaaattgtt cccatttgtt ttagagaaca actcacttag cctatgagtt 480
tatgcaattt ggcagaaagt gaaaacatat ttggaagtat tgaaagtcac tcattgttga 540
tcttttatat tggaatgycc aaggttgcat catcagagtg tcgttatgaa aaaaaaaaaa 600
aaaaaactcg ta 612

<210> 97
<211> 670
<212> DNA
<213> Homo sapiens

<400> 97
gctcgtgccg aactcgtgcc gacgaaaagc tgccaagttg aaaatggacg agtaatcgcc 60
tgctttgatt cattgaaaaa ctaaatctcc atacccactt catccgtgtt tttggcttat 120
gtatgggatg ctagaatggc ctatctccat gtattttgtt gcatttctcc attgcttctt 180
gtgttctggc gggaatcttg gtgattcttt tcaagcacta cctgagctct gtgccaattg 240
ttcctcttct cccagggtgt tgtgctgcgt ggtcatgtct ccacttcctt agccctgtcc 300
attgacagaa ccttgggttc tgtgatggct gcctctaaac ccttgtgaaa gcggggaata 360
ttcctccccc tgctgctaca gttgagcacc gtgctgggta ccatgttgcc ctctacactt 420
gctttcagtt gttaaggctt cccaagcttt ggctgtggct cagtgatcct gctgtcaaaa 480
ccctgaaact ttcctagcct ggacactcag tggtagcagc aggtgttggg atttctccaa 540
gcccctaaga ctctgggagg aagagaatgg ctgtttgaca tagacctcag gagttttcaa 600
agcaccaaga aacctctcca gaagatatgt aaagatttta aagggaaaaa aaaaaaaaaa 660
aaaaactcga 670

<210> 98
<211> 619
<212> DNA

<213> Homo sapiens

<400> 98
gcggcacgag tgatatttca cgtcacatgg ctagtgagtg ggtaggcctc tcttcactta 60
ttacacttct gcttctaagc tgtgttcttt cctgtattac actggaggaa ggagaaaaag 120
aacttgtatt tggtccttga ctgggtggaa tatcctttaa tgtggctgta aggacatggg 180
tagaatactc tggtcaattc atttcttatt taaatagtga caaaggtatg tccatgttaa 240
ccatttctca cttatgcttt atacataagg atggcttata gggaatgttg ctttattata 300
tcacttaaaa tgtttggtca ggcaatagtg actcatgcct ttaatcccag tacttttgaa 360
ggacaagtca ggaggatcgc ttgagaccag gaactcagga ccagcctgga cgacaaaaca 420
ggatctcgtc tctacaaaaa ataaaatagt cgagtgtggt gatgcagtat tgtagtccca 480
gctatttggg aggctgaggt gggagtatcg cttkagacca ggagttcaag gatatagtga 540
atgatgatcg ctccactgca ttccagcctg gacaacaaag caaaacccta tttctaaaaa 600
aaaaaaaaaa aaactcgta 619

<210> 99
<211> 703
<212> DNA
<213> Homo sapiens

<400> 99
gcttggttac gtttatagct tcaacacgcc tctcattkta ggtttataca tgtgtttgct 60
tgctcattta ttttgtcatc atttgctcat tttattacca gttattgagw gcctactgtg 120
taccaggcac tgggcaaggg gcattctgtg agagagggta tggtacctgc gggcttaagt 180
agtccgtggg cttgtgagga aaacgctaga ttagatcttg attactgtaa atgtcaarta 240
tggccaagtg tgggatttcg tggcaggagt gagctttcct ggaatttgtc tttcttgcct 300
caatttgcct gatagtcatt tcatgctagg gatgttttaa agtctctggg gaggccctgc 360
agtgtagagg aaaatgctga tccacaccag aaatgcgaac ctggctctct gcccttgggc 420
aagtcactta accctcctga gcctcagttt ccatctgtca cttagagctg attataccta 480
cttaacaccc aggctttttg tgaggggcat tatctcatta gagataatgt ttttaaaagc 540
tctttgtaaa ttgtgtagca ttcaaatgga agttattgtt atttttatta ttgagtgcct 600
tctaattcaa cactgggata gtaacaaaag aagagagggg ttattatcac ccctcttccc 660
tgtcacgttt agattggggc aaggaaaggt tctcaccctg cga 703

<210> 100
<211> 762
<212> DNA
<213> Homo sapiens

<400> 100
gtttttctcc ttcttagtat cttttgcata tagaaaataa ttactatgaa attatagatt 60
tgacgtgcaa aggctatttc ttgaatttta ttaaaatgca aaaagatgca tccatgtctt 120
ctctaaaagg actgcgtatt cctccacact tggggaaatg cagcttgtgc tatttcacag 180
gctcatcatg cccctttttt ttgccaggac gctggttgat taatgccatg cttggggagt 240
gctccagcca gaaatgaggg ctatcgcctg tggccaataa cagagcagat tctcaataaa 300
catccccttg gtgttacact taatggggct tgcttttcca aactgctccc tttcctgggc 360
tctgagcagc tgagccgaga gctcgtaagc tctgctgccc cagaacattg tgcattcytt 420
gattttgaaa artctttcct gaagsctcct cttgggtcat tggatcagcc caagagcaaa 480
ggatttaaaa gggccaattt gatagggaca gctcatagcc ctgtgtaaga ccactgggca 540
tttttcctgt ttggggaaat ggttactgga ttagcatttt gctgtacagg gcggtctgca 600
agaatgtgtg ctcttgcctg tcctcaaagc aggcttgtga ggagctttct gttcccagcc 660
ctgccatttc ctcccaattg gctgggccag atgctccaga cacagttaat gagatgctga 720
gtgaaacaga gccgctggct cacatggcct cagcctcctc ga 762

<210> 101

<211> 650
<212> DNA
<213> Homo sapiens

<220>
<221> SITE
<222> (497)
<223> n equals a,t,g, or c

<400> 101
ggcacgaggt gtcctgccca ccccagtgcg ggtcagtaga aggccagaag caggggatgg 60
gagaaggcag gtgggagggc gtgacagcgg cgaggatgag gaaggcagcc aggcctgcag 120
gcagccctga gagcatgaag cagaggggtg agcaggttcc cctcctcctg ccacccttgc 180
tcctctctac caggctctgg ccttgctggg gtgtacccac agaatctgta ggctctggcc 240
tagccagaaa gagtgtgggt gcttctcagg gtcataatta ccccatgccc cacagggtgt 300
gagtcactgg tagcagagtc ctccccaatc ccccccagaa gagtgtggtg aaaggcccgg 360
gccactgggg tgtcgagagt gccaggcctg acctactggg ggtggtgtca gtaggggcca 420
tataccctgt tctcamgaca accccaggcc aactcagatt tgtggagcgg ccatcccacc 480
tccttccggc tcttcancct cacaggagcc tggtgggtcg ggaaaactga ggcctagaga 540
ggcaaaatga tgatacaatg aagagtgagt acatgtggaa caccctctgt gcctcacact 600
ccactaagct cctcacacca ttcacttact caggcctcac cggccctcga 650

<210> 102
<211> 360
<212> DNA
<213> Homo sapiens

<400> 102
ggcacagctg atgtttaaaa tacacgaaaa atcttgtaac cctatttggg catatctttt 60
tcttcttctt tttggttttt gtttaatatg gaagtggaca gtgcctctct tgacctctgg 120
aaggccctat gaaaacctga aaccgaggca aggtgacaaa gtctggtcat tcagcactaa 180
gggccgcctc agattacttc tttacttaga aaaacaaaat gttgttgcaa aagattcaga 240
gtcacaaata ttcttcccgg gcctgtcagt ttctgaattc ttagattttt catttaattt 300
agccatcagg gaatttctga gactagaaat acctaggcag aacccaaaca aaatctcgta 360

<210> 103
<211> 817
<212> DNA
<213> Homo sapiens

<400> 103
ggcacgagct caggttgcgg ccggagagaa aggcctgggg accacctgac tctgggccac 60
ccgggcctcc tcaggtcttc ggccagcgct gtcctgccca cggtagttgg ggttccaatg 120
gctgcggctt cttcctgtct gtggcttgga catgccattg gccgcgtctc tatttcctca 180
tctgcgactc gggtgaccac agttctcagt tcaccgtgtt cggtagaggt gacatgaagt 240
gcctggcacc catgtgggtt tccctgtggg attctgaccc gcttcggagc tgcctcctgc 300
tcctcatccc acacttctct gtgtttctca tcctggcggc tgtgtcctgt ctgcccctct 360
caactgcaac acgctggaga ggtcgggacc ctgtcttgct cattatctgt ctactaaaga 420
acctgcaaaa tggaaaaata acaatatgtg ctgaattaat tattagctta aaatttaaaa 480
cttaagtagc atgatttgag tgcagccagc atcacctgcc gtgagatcgg tgctgtctac 540
aggaggatgg agcttttggt gaaccactga gctgggagta gctacgggca cctttaccca 600
gtcccaaaat gtggaacatt tgagtttaaa aagcagaaaa ctctacagtt aaaagccaat 660
attaaggttg agtccattaa tctaaattaa tctgattttt tatttcttta aataaaaaag 720
taatcctatg caatcaaagt taaagttcgt atatggctcc ctatgaggta ctacattccc 780
tgaagtgtca caaaaaaaaa aaaaaaaaaa aaaaaaa 817

<210> 104
<211> 881
<212> DNA
<213> Homo sapiens

<400> 104
ggcacgagta tgactaataa ggtaatctgt ccttgttaac aagcctgtat ttgttatacc 60
tgtacttaaa gtaaaattca aactccttac cctgtcctac aaggctctac ctgatctggg 120
ccctacctca tctctaacat catcttatgc tattttcttt cttgttcacc agagccacac 180
cagctacctt tctgtccctc cttgttagac ttatttctgc tttagagcac ccttgctgct 240
gccaccacct gaaatgcttc tcttctggta ttttattttg gtgagaacac ctggcatgag 300
atctaccctc taacagattt ttaagtgtat aatacagtat tgctgtctgt aggcacaatg 360
ctgcacagca gatctctaga acttaccttg tataactgaa attttatact cattgattag 420
caacagcccc aaattattga aacctccttg aagcctaaat ttcagaaatg ttcaaatgtt 480
ttgaaaatgg atattctgaa ttatcttatt agcatctacc tataattagc actgaaaata 540
gtaatttttt taataaagaa tcagttaagg gccgggtgtg gtcctcacgc ctgtaatccc 600
agcactttgg gaggctgagg cgggaggatc acaaggtcgg gagatcgaga ccatcctggc 660
taacaccgtg aaaccctgtc tctactaaaa aaatacaaaa aaaatcagct gggcgtggtg 720
gcaggtgcca atagtcccag ctacttggga ggctgaggtc aggagaatgg cgtgaaccca 780
ggagggttgc agtgagccaa gttctcgcca ctgcactcca gcctgggcga cagagcgaga 840
ctctgtctca aaaaaaaaaa aaaaaaaaaa aaaactcgta g 881

<210> 105
<211> 655
<212> DNA
<213> Homo sapiens

<400> 105
ggcagagctg gtctcgaact cctgacctca ggtgatctgc ccaccttggc ctcccaaagt 60
gctgggatta caggcataag ccattgcgct cggctgagat tagcaataat taatgtgata 120
tgaaaatatt ttctttttct tcatgacaaa ttcatggcta atactgccag gatttttttg 180
ttgttgccca tattcataat agaaggaaat gctaatatga aaataaagat gtcacttttt 240
ccccaatcca tgcaatttcc ccctaaattg tatccatgac ctacctgagg gggatccatg 300
gactctcagg ttaagacccc tctactgaag ggtagcagag tacagtttca aaattactga 360
ttaagagcgt gggctcacca ggagttcaag cccagccggg gcaacaggat gagacctcat 420
ctttacaaaa aatgaacaaa attaggcatg gtggtgcttg tctgcagtcc cagctacttg 480
ggagactgag ttgagaggat cacttgaggc tgagaggttg agggtgcagt tgagctgaga 540
ttgcaccact gcactccagc ctgagtgaca gagtgagatc ctgactcaaa aaaaaaaaaa 600
aaaaaaaaga aaaaaaaaaa aaaaaaaaag aaaaaaaaaa aaaaaaaaac tcgta 655

<210> 106
<211> 606
<212> DNA
<213> Homo sapiens

<220>
<221> SITE
<222> (9)
<223> n equals a,t,g, or c

<220>
<221> SITE
<222> (19)
<223> n equals a,t,g, or c

<400> 106
ccccccggnc tgccaggant ttcggcacga gtctctctgt caactctatt tgtatttcta 60
taatggaaac tcaaatttgc ctaactcaga ttgtagcact tttcttcctc aggctagtcc 120
taggaaaact cacttgtttt ttgtatggaa aactagtgtt agtagaagcc tttattcttg 180
catagccccc aaatcagctt tttcagctat aatttagtaa gtctaatgtg ttcgactgaa 240
gtactttttt tttgtaataa caagtgaaaa ataatgaaga gtgtgtcctg gcgcatggct 300
cacgcctgta atcccagcac ttcgggaggc cggagcygag gcagcggatc acttgagggt 360
caggagttca agaccagctt gaccaacatg gtgaagtcct gtctctatta aaaatacaaa 420
aattagccag gtgtggtagt gcatgtctgt aatcccagct acttgggagg ctgagacagg 480
agaattgctt ggacctggga ggcggaggtt gcagtgaggt gagattgcgg cattgcactc 540
cagcctggac aacaagagtg aaactttgtc tcaaaaaaaa gaaagaaaaa aaaaaaaaaa 600
actcga 606

<210> 107
<211> 657
<212> DNA
<213> Homo sapiens

<220>
<221> SITE
<222> (634)
<223> n equals a,t,g, or c

<220>
<221> SITE
<222> (650)
<223> n equals a,t,g, or c

<220>
<221> SITE
<222> (655)
<223> n equals a,t,g, or c

<400> 107
gagtttgtra acctatattc acagcattaa ctaatcatga ttcgccccat atttcactgg 60
ttatgctttg gttatcttag aaaagaaccc agggcattta tgaggtaaaa cttgcagggc 120
agattacagg catgagccac cgcgcctaga cttattagtc tttttaatgg ggatgacagc 180
agctgggrtg tatatattcc tgcaggaaag aaaaggaaat ggcttcacat tgctggatgg 240
gagcagtatg tgtgttgttt ctgggtataa tcttcctagc tgcacttttc ccatacattt 300
ctttctacta aaaatcatga aagtttgaat tatagttcct ctcacaggat tgaaagcaag 360
tatcagagga gtcatccatt caaaacacag ttcttccact gcagtatccg atatgttttg 420
tatgtgcgct aggctgtctt ttcattcagt ctacaataca gttcaccagt gtggagacct 480
tttgccctgc ctgatttgtt ttgttttgtt ttactcactc ttttcaatga cttttggttt 540
tggccagtat gaagagtaat ggatgttgga ataccttctg ccagttaaaa aaaaaaaaaa 600
aaaaaaaagg gcggccgctc tagaaggatc caanttaagt aagcgtgtcn ctccnct 657

<210> 108
<211> 605
<212> DNA
<213> Homo sapiens

<400> 108
acgagctgga aatcaatgat cagtcataaa atcagactgg gaaactragg cacagagagg 60
ggcatggatt tgggcattgg tccaggttat gaagcacatc caccagggtg gcctggtgga 120
gttaaaggcc atccctactg ggcaggatgt gctggtgcca gttgggtgag ttcagaggtg 180
gttgggagag agaaatgctc agagctctct gtctgtctac ctgtccctga ctctcagtgc 240

cagcacccac ccaccccatg gtccccactc atccgggagc ttacagcagc ccctccacct 300
ctatccagcc attttctcta gccataacat tggtgactgg caaagtgtcc cagcacaagg 360
cctggcacac agttggtgct tagtgtttgc taaatgaatg aatggattaa taagaacgaa 420
tattgtgcag aaaaagtaaa ttcttctgga cacttccagc ctatatgtgg aggggacaaa 480
gttttttgtt gttgttgttg ttgttgttgt tgtgtttttt gagacagtgt cgttctgttg 540
cccaggctgg agtgcagtgg tgcgatcaca gctcactgca gccttgatct cctcagcctc 600
tcgta 605

<210> 109
<211> 504
<212> DNA
<213> Homo sapiens

<400> 109
ggcacgagcc aacagccgtt ttgaaggtag aggagagaga tgttgtggta tttkttcccc 60
accaccccac tccctgccca ggtgcagttt tggtggtgcc tgtgttgctg ctacatccat 120
ggctcctggt ggggacccct ctcccaaagc tccagctcct gcaatgcttc agtaactgca 180
ctcagctcag gctgttgtag acctagggcc agcagtccca cagtgcctca ccatcgcttg 240
ttccctatgc ctgcccacac atctgtaaat agtcccttca tttcacatcc ttcagttaga 300
ccctttgagt atgccatctg cttccggtca ggacaatgat tgattctatc tgaatcaaac 360
ctgtccttta tttgaacagg acatcaagtc tagaaaaaca agttaacacc ttgagataac 420
aaacaaatcc agaatttggg accatttact agtctggttc tttcaaaggt caatgttata 480
aaaaaaaaaa aaaaaaactc gtag 504

<210> 110
<211> 770
<212> DNA
<213> Homo sapiens

<400> 110
gctaaaattc aacaaggtga gtggccggca gtggaaggct gttgctcatt ctgatttctg 60
ttggctctat ttcatgctaa mccagttttt tttgtttgtt tgtttccact ttataacata 120
tggatttcta tgccacacta cccgtaactt tgaaaaataa ctttaggctg cagttttcag 180
caaacaggac agtccttagc tgccacatag ctcaacataa agtgcacaaa aaacttcacg 240
gtgggacagt gaatcataaa ttcccaaact gacgtgtgtc tacagaacag atgagaactg 300
ttactcagtg tgtatcttag gagcttttct gcagtttcct cacactccgt cacatttaaa 360
atgtggacac ttgtttattt cattagggag gaggcgaggg actaatgtcc accctgccca 420
gagtatttcg aatatcctta gtgaagagga ggaaagcaag aattctgttc taaaggccac 480
caggctaagc actagaatcg cattctcttc ctgtttgtat gtttatgtca gcagttgcca 540
cagatgtgtt aatattgttt tcctggtaga gaattaaggt gttcgttcat ctcaaaacaa 600
atcccgtaac ctgcacacaa aactccagct tcctaatgca aagagaagag aatattgatt 660
ataagctgct tgatattctt tttattccca gcccctcaaa ataccagcct ggaagtctgg 720
acattactaa aatttaccag tctcaaaaaa aaaaaaaaaa aaaactcgag 770

<210> 111
<211> 751
<212> DNA
<213> Homo sapiens

<400> 111
ccacgcgtcc gcggacgcgt gggagtcatc tgtcttaagt tggaaaaaag tttcatatga 60
ttctttccca tttcccctgt attccttttc attataccct cattccttga acagaattgt 120
tattgttttg tttttccatc cacaccccta taatgcaacc ttcctgtgta aattttaggc 180
ttaagctttt ctattcacat acttttatgc tgaggcttgg atttttattt gggctgttag 240
atgcccattt tgacattgac attaggggtt tcaaaccatc cttaaaaggt tagatgtgac 300

ttgcaatgtt attgaacaat ttgatgatcc gggatattat ggctctatga aatctccatg 360
gttcttggag ctagcttgtt tttattctgg gaagaatttt ctagctcccc agcttacggc 420
ctgaatggtt agagtccagc cagtgctgtt tgactttata gttcaaaggg ggtcatttct 480
gtggtcacta tcctatttaa cagtcatgtc atggtatgtc aaggtaggtc atcatacaaa 540
taatctgcat tctgttttga ctgttttatt tttaaaaata atatctcctc cttttaaact 600
ttaaaaaatt tagtaaagtt tagtaaactt tcaaaaattt agtaaaaaat gtagtaaaaa 660
ttcacttcct tcattatgct ttttgaaatc tggctttttt tctcattctt cccctattaa 720
tggttcttaa aaaaaaaaaa aaagggcggc c 751

<210> 112
<211> 543
<212> DNA
<213> Homo sapiens

<220>
<221> SITE
<222> (22)
<223> n equals a,t,g, or c

<220>
<221> SITE
<222> (42)
<223> n equals a,t,g, or c

<220>
<221> SITE
<222> (51)
<223> n equals a,t,g, or c

<400> 112
cgtcgcccgc ttggagggtc gncactagtg gatccaaagg antcggcacg ngctacccct 60
tgccmaagcc taaacttcat actagatatc caactgccta ctggacatct ccatttataa 120
gcctagtagc ctaataagca taacctcaga cttaccaggc ctcacactga agtcatgaac 180
ttcagcccaa cccccatgcc agggcaaaac cttgttgtta cctcttattc ctctcttgcc 240
tcatcccatc catgttcagt ctgtcagtgg atcctgtgag tccagtcttg aggatagttc 300
caggatctga tcacttctca ctgcctcttt tgctgccacc acctctggcc tggataattg 360
cagcagcctc ccagttagcc ttgctgtgtc catccttgtt ttccccttct gtctgctctc 420
aacagaggag ctagtgattc tcttaggaca gaataaatca tttaggtttt cttcacatgg 480
tcctgaagaa gcttcctacc tcactcagtg taaaaaccaa aaaaaaaaaa aaaaaaaact 540
cga 543

<210> 113
<211> 86
<212> PRT
<213> Homo sapiens

<220>
<221> SITE
<222> (2)
<223> Xaa equals any of the naturally occurring L-amino acids

<400> 113
Met Xaa Leu Gln Pro Asn Pro His Ala Arg Ala Lys Pro Cys Cys Tyr
1               5                   10                  15

Leu Leu Phe Leu Ser Cys Leu Ile Pro Ser Met Phe Ser Leu Ser Val

            20                  25                  30

Asp Pro Val Ser Pro Val Leu Arg Ile Val Pro Gly Ser Asp His Phe
        35                  40                  45

Ser Leu Pro Leu Leu Leu Pro Pro Pro Leu Ala Trp Ile Ile Ala Ala
    50                  55                  60

Ala Ser Gln Leu Ala Leu Leu Cys Pro Ser Leu Phe Ser Pro Ser Val
65                  70                  75                  80

Cys Ser Gln Gln Arg Ser
                85

<210> 114
<211> 20
<212> PRT
<213> Homo sapiens

<220>
<221> SITE
<222> (20)
<223> Xaa equals stop translation

<400> 114
Met Ala Ala His Ser Val Leu Ser Phe Leu Leu Trp Thr Pro Tyr Ala
1               5                   10                  15

Leu Lys Ser Xaa
            20

<210> 115
<211> 39
<212> PRT
<213> Homo sapiens

<220>
<221> SITE
<222> (39)
<223> Xaa equals stop translation

<400> 115
Met Leu Lys Leu Ala Thr Ile Leu Leu Thr Leu Leu Leu Lys Asn Leu
1               5                   10                  15

Asp Ala Gly Leu Thr Asp Lys Leu Ser Arg Ser Asn Phe Ile Thr Asp
            20                  25                  30

Phe Ile Leu Thr Lys Tyr Xaa
        35

<210> 116
<211> 88
<212> PRT
<213> Homo sapiens

<220>
<221> SITE
<222> (86)
<223> Xaa equals any of the naturally occurring L-amino acids

<220>
<221> SITE
<222> (88)
<223> Xaa equals stop translation

<400> 116
Met Leu Leu Leu Tyr Leu Gly Ile Glu Val Ile Arg Leu Phe Phe Gly
1               5                   10                  15

Thr Lys Gly Asn Leu Cys Gln Arg Lys Met Pro Leu Ser Ile Ser Val
            20                  25                  30

Ala Leu Thr Phe Pro Ser Ala Met Met Ala Ser Tyr Tyr Leu Leu Leu
        35                  40                  45

Gln Thr Tyr Val Leu Arg Leu Glu Ala Ile Met Asn Gly Ile Leu Leu
    50                  55                  60

Phe Phe Cys Gly Ser Glu Leu Leu Leu Glu Val Leu Thr Leu Ala Ala
65                  70                  75                  80

Phe Ser Ser Met Asp Xaa Ile Xaa
                85


<210> 117
<211> 39
<212> PRT
<213> Homo sapiens

<220>
<221> SITE
<222> (39)
<223> Xaa equals stop translation

<400> 117
Met Tyr Lys Phe Leu Tyr Leu Val Leu Glu Asp Phe Val Ala Phe Ile
1               5                   10                  15

Arg Gly Ser Phe Pro Pro Gln His Thr Arg Ser Leu Val Phe Trp His
            20                  25                  30

Val Cys Gln Leu Glu Tyr Xaa
        35


<210> 118
<211> 27
<212> PRT
<213> Homo sapiens

<220>

<221> SITE
<222> (27)
<223> Xaa equals stop translation

<400> 118
Met Met Met Met Ile Gln Thr Leu Met Val Met Ala Lys Ile Leu Cys
1               5                   10                  15

Leu Lys Gln Pro Leu Ser Met Ala Gly Ser Xaa
            20                  25


<210> 119
<211> 22
<212> PRT
<213> Homo sapiens

<220>
<221> SITE
<222> (13)
<223> Xaa equals any of the naturally occurring L-amino acids

<220>
<221> SITE
<222> (22)
<223> Xaa equals stop translation

<400> 119
Met Lys Glu Asn Pro Leu Leu Leu Leu Ile Cys Ile Xaa Gly His Leu
1               5                   10                  15

Val Val Pro Pro Asn Xaa
            20


<210> 120
<211> 96
<212> PRT
<213> Homo sapiens

<220>
<221> SITE
<222> (96)
<223> Xaa equals stop translation

<400> 120
Met Tyr Arg Asp Ser His Ser Val Leu Ala Leu Asn Trp Lys Val Val
1               5                   10                  15

Ala Thr Leu Lys Tyr Phe Leu Leu Tyr Val Ile Ile Leu Tyr Asn Leu
            20                  25                  30

Glu Arg Asp Asn Gly His Ser Asn Tyr Glu Asn Tyr Glu Leu Gly Asp
        35                  40                  45

Lys Ser Leu Asn Leu Leu Leu Phe Tyr Asn Ser Met Tyr Lys Leu Val
    50                  55                  60

Phe Pro Tyr Ile Phe Thr Phe Ser Ser Phe Leu Ile Ser Ser Tyr Thr
65                  70                  75                  80

Ser Ile Leu Tyr Lys Met Phe Tyr Ile Gln Arg Thr Val Lys Ser Xaa
                85                  90                  95

<210> 121
<211> 36
<212> PRT
<213> Homo sapiens

<220>
<221> SITE
<222> (36)
<223> Xaa equals stop translation

<400> 121
Met Lys Glu Arg Thr Arg Ile Pro Cys Ala Phe Pro Phe Leu Leu Phe
 1               5                  10                  15

Gln Thr Arg Val Gln Thr Ser Pro Ala Phe Gln Pro His Pro Leu Tyr
            20                  25                  30

Phe Thr Ala Xaa
        35

<210> 122
<211> 38
<212> PRT
<213> Homo sapiens

<220>
<221> SITE
<222> (38)
<223> Xaa equals stop translation

<400> 122
Met Thr Ser Val Ile Val Leu Phe Ile Leu Lys Val Phe Phe Lys Tyr
 1               5                  10                  15

Phe Ser Thr Thr Ser Phe Leu Asn Ala Cys Ile His Phe Ile His Lys
            20                  25                  30

Cys Lys Leu Val Asn Xaa
        35

<210> 123
<211> 342
<212> PRT
<213> Homo sapiens

<220>

<221> SITE
<222> (342)
<223> Xaa equals stop translation

<400> 123
Met Leu Gln Pro Thr His Leu Ser Leu Gln Leu Arg Leu Gln Cys Leu
1               5                   10                  15

Ala Ala Ser His Leu Val Thr Leu Leu Ile Cys Leu Met Ala Pro Ala
            20                  25                  30

Ser Ala Thr Gly Gly Ser Ala Asp Leu Phe Gly Gly Phe Ala Asp Phe
        35                  40                  45

Gly Ser Ala Ala Ala Ser Gly Ser Phe Pro Ser Gln Val Thr Ala Thr
    50                  55                  60

Ser Gly Asn Gly Asp Phe Gly Asp Trp Ser Ala Phe Asn Gln Ala Pro
65                  70                  75                  80

Ser Gly Pro Val Ala Ser Ser Gly Glu Phe Phe Gly Ser Ala Ser Gln
                85                  90                  95

Pro Ala Val Glu Leu Val Ser Gly Ser Gln Ser Ala Leu Gly Pro Pro
            100                 105                 110

Pro Ala Ala Ser Asn Ser Ser Asp Leu Phe Asp Leu Met Gly Ser Ser
        115                 120                 125

Gln Ala Thr Met Thr Ser Ser Gln Ser Met Asn Phe Ser Met Met Ser
    130                 135                 140

Thr Asn Thr Val Gly Leu Gly Leu Pro Met Ser Arg Ser Gln Pro Leu
145                 150                 155                 160

Gln Asn Val Ser Thr Val Leu Gln Lys Pro Asn Pro Leu Tyr Asn Gln
                165                 170                 175

Asn Thr Asp Met Val Gln Lys Ser Val Ser Lys Thr Leu Pro Ser Thr
            180                 185                 190

Trp Ser Asp Pro Ser Val Asn Ile Ser Leu Asp Asn Leu Leu Pro Gly
        195                 200                 205

Met Gln Pro Ser Lys Pro Gln Gln Pro Ser Leu Asn Thr Met Ile Gln
    210                 215                 220

Gln Gln Asn Met Gln Gln Pro Met Asn Val Met Thr Gln Ser Phe Gly
225                 230                 235                 240

Ala Val Asn Leu Ser Ser Pro Ser Asn Met Leu Pro Val Arg Pro Gln
                245                 250                 255

Thr Asn Ala Leu Ile Gly Gly Pro Met Pro Met Ser Met Pro Asn Val
            260                 265                 270

Met Thr Gly Thr Met Gly Met Ala Pro Leu Gly Asn Thr Pro Met Met
        275                 280                 285

Asn Gln Ser Met Met Gly Met Asn Met Asn Ile Gly Met Ser Ala Ala
    290                 295                 300

Gly Met Gly Leu Thr Gly Thr Met Gly Met Gly Met Pro Asn Ile Ala
305                 310                 315                 320

Met Thr Ser Gly Thr Val Gln Pro Lys Gln Asp Ala Phe Ala Asn Phe
                325                 330                 335

Ala Asn Phe Ser Lys Xaa
            340

<210> 124
<211> 219
<212> PRT
<213> Homo sapiens

<220>
<221> SITE
<222> (139)
<223> Xaa equals any of the naturally occurring L-amino acids

<220>
<221> SITE
<222> (217)
<223> Xaa equals any of the naturally occurring L-amino acids

<220>
<221> SITE
<222> (219)
<223> Xaa equals stop translation

<400> 124
Met Val Ser Trp Met Ile Cys Arg Leu Val Val Leu Val Phe Gly Met
  1               5                  10                  15

Leu Cys Pro Ala Tyr Ala Ser Tyr Lys Ala Val Lys Thr Lys Asn Ile
             20                  25                  30

Arg Glu Tyr Val Arg Trp Met Met Tyr Trp Ile Val Phe Ala Leu Phe
         35                  40                  45

Met Ala Ala Glu Ile Val Thr Asp Ile Phe Ile Ser Trp Phe Pro Phe
     50                  55                  60

Tyr Tyr Glu Ile Lys Met Ala Phe Val Leu Trp Leu Leu Ser Pro Tyr
 65                  70                  75                  80

Thr Lys Gly Ala Ser Cys Phe Thr Ala Ser Leu Ser Thr Arg Pro Cys
                 85                  90                  95

Pro Ala Met Arg Arg Arg Ser Thr Arg Thr Ser Cys Arg Pro Arg Ser
            100                 105                 110

Ala Ala Thr Arg Pro Cys Ser Ala Ser Gly Ser Gly Ala Ser Thr Leu
        115                 120                 125

Pro Pro Pro Leu Leu Cys Arg Leu Pro Pro Xaa Val Arg Gly Arg Trp
    130                 135                 140

Pro Ala Gly Cys Gly Ala Ser Pro Cys Arg Thr Cys Ala Pro Ser Leu
145                 150                 155                 160

Thr His Leu Pro Leu Pro Thr Met Thr Pro Ser Thr Trp Arg Thr Arg
                165                 170                 175

Cys Pro Thr Gly Gly His Pro Leu Gly Thr Gly Pro Gly Ala Cys Arg
            180                 185                 190

Thr Ala Thr Pro Arg Met Ser Val Gly Gln Ile Leu Arg Gln Ser Pro
        195                 200                 205

Gly Arg Gln Pro Gly Pro Glu Arg Xaa Pro Xaa
    210                 215

<210> 125
<211> 266
<212> PRT
<213> Homo sapiens

<220>
<221> SITE
<222> (15)
<223> Xaa equals any of the naturally occurring L-amino acids

<220>
<221> SITE
<222> (96)
<223> Xaa equals any of the naturally occurring L-amino acids

<220>
<221> SITE
<222> (98)
<223> Xaa equals any of the naturally occurring L-amino acids

<220>
<221> SITE
<222> (119)
<223> Xaa equals any of the naturally occurring L-amino acids

<220>
<221> SITE
<222> (161)
<223> Xaa equals any of the naturally occurring L-amino acids

<220>
<221> SITE
<222> (170)
<223> Xaa equals any of the naturally occurring L-amino acids

<220>
<221> SITE
<222> (189)

<223> Xaa equals any of the naturally occurring L-amino acids

<220>
<221> SITE
<222> (197)
<223> Xaa equals any of the naturally occurring L-amino acids

<220>
<221> SITE
<222> (200)
<223> Xaa equals any of the naturally occurring L-amino acids

<220>
<221> SITE
<222> (230)
<223> Xaa equals any of the naturally occurring L-amino acids

<220>
<221> SITE
<222> (235)
<223> Xaa equals any of the naturally occurring L-amino acids

<220>
<221> SITE
<222> (244)
<223> Xaa equals any of the naturally occurring L-amino acids

<220>
<221> SITE
<222> (245)
<223> Xaa equals any of the naturally occurring L-amino acids

<220>
<221> SITE
<222> (247)
<223> Xaa equals any of the naturally occurring L-amino acids

<220>
<221> SITE
<222> (266)
<223> Xaa equals stop translation

<400> 125
Met Ser Met Ala Val Glu Thr Phe Gly Phe Phe Met Ala Thr Xaa Gly
1               5                   10                  15

Leu Leu Met Leu Gly Val Thr Leu Pro Asn Ser Tyr Trp Arg Val Ser
            20                  25                  30

Thr Val His Gly Asn Val Ile Thr Thr Asn Thr Ile Phe Glu Asn Leu
        35                  40                  45

Trp Phe Ser Cys Ala Thr Asp Ser Leu Gly Val Tyr Asn Cys Trp Glu
    50                  55                  60

Phe Pro Ser Met Leu Ala Leu Ser Gly Tyr Ile Gln Ala Cys Arg Ala
65                  70                  75                  80

Leu Met Ile Thr Ala Ile Leu Leu Gly Phe Leu Gly Leu Leu Leu Xaa
                    85                  90                  95

Ile Xaa Gly Leu Arg Cys Thr Asn Ile Gly Gly Leu Glu Leu Ser Arg
            100                 105                 110

Lys Ala Lys Leu Ala Ala Xaa Ala Gly Ala Leu His Ile Leu Ala Gly
        115                 120                 125

Ile Cys Gly Met Val Ala Ile Ser Trp Tyr Ala Ser Thr Ser Pro Gly
    130                 135                 140

Thr Ser Ser Thr Pro Cys Thr Pro Glu Pro Ser Thr Ser Trp Ala Pro
145                 150                 155                 160

Xaa Ser Thr Trp Gly Gly Ala Pro His Xaa Ser Pro Ser Trp Val Ala
                165                 170                 175

Ser Ala Ser Ala Pro Pro Ala Ala Ala Ala Leu Thr Xaa Thr Ser Arg
            180                 185                 190

Gln Arg Pro Ala Xaa Leu Pro Xaa Ser Arg Val Arg Asp Ala Arg Arg
        195                 200                 205

His Leu Gly Pro Arg Arg Arg Gln Gln Leu Trp Gln Ile Arg Gln Lys
    210                 215                 220

Arg Leu Arg Val Ala Xaa Leu Ala Arg Gly Xaa Arg Cys Leu Pro Thr
225                 230                 235                 240

Ala Pro Arg Xaa Xaa Asp Xaa Ala Gly Ala His Ser Pro Ile Val Thr
                245                 250                 255

Ser Gly Ala Gly His Ala Pro Leu Pro Xaa
            260                 265

<210> 126
<211> 39
<212> PRT
<213> Homo sapiens

<220>
<221> SITE
<222> (39)
<223> Xaa equals stop translation

<400> 126
Met Leu Phe Ile Tyr Leu Phe Val Phe Pro Ile Arg Ile Gly Ser Glu
  1               5                  10                  15

Lys Ala Lys Thr Val Ser Val Leu Leu Ile Ile Val Ser Leu Thr Ala
            20                  25                  30

Arg Pro Leu Ala Gly Phe Xaa
        35

<210> 127
<211> 93
<212> PRT
<213> Homo sapiens

<220>
<221> SITE
<222> (93)
<223> Xaa equals stop translation

<400> 127
Met Leu Leu Tyr Leu Tyr Ser Leu Gly Ile Ser Val Leu Ile Ile Ser
1               5                   10                  15

Phe Pro Thr Asn Ser Ser Ile His Val Arg Lys Asn Met Ala Asn Gln
            20                  25                  30

Tyr Leu Lys Gly Ala Ile Phe Gln Ser Ser Gly Phe Gln Ser Val Ala
        35                  40                  45

Gly Gln His Trp Gln His Leu Asn Leu Leu Gly Thr Leu Leu Lys Met
    50                  55                  60

Gln Ile Leu Ser Pro Thr Leu Val Leu Leu Asn Trp Glu Thr Gly Val
65                  70                  75                  80

Gly Pro Ser Ser Leu Cys Phe Asn Met Phe Ser Lys Xaa
                85                  90


<210> 128
<211> 196
<212> PRT
<213> Homo sapiens

<220>
<221> SITE
<222> (196)
<223> Xaa equals stop translation

<400> 128
Met Glu Leu Ser Glu Ser Val Gln Lys Gly Phe Gln Met Leu Ala Asp
1               5                   10                  15

Pro Arg Ser Phe Asp Ser Asn Ala Phe Thr Leu Leu Leu Arg Ala Ala
            20                  25                  30

Phe Gln Ser Leu Leu Asp Ala Gln Ala Asp Glu Ala Val Leu Asp His
        35                  40                  45

Pro Asp Leu Lys His Ile Asp Pro Val Val Leu Lys His Cys His Ala
    50                  55                  60

Ala Ala Ala Thr Tyr Ile Leu Glu Ala Gly Lys His Arg Ala Asp Lys
65                  70                  75                  80

Ser Thr Leu Ser Thr Tyr Leu Glu Asp Cys Lys Phe Asp Arg Glu Arg
                85                  90                  95

Ile Glu Leu Phe Cys Thr Glu Tyr Gln Asn Asn Lys Asn Ser Leu Glu
            100                 105                 110

Ile Leu Leu Gly Ser Ile Gly Arg Ser Leu Pro His Ile Thr Asp Val
        115                 120                 125

Ser Trp Arg Leu Glu Tyr Gln Ile Lys Thr Asn Gln Leu His Arg Met
    130                 135                 140

Tyr Arg Pro Ala Tyr Leu Val Thr Leu Ser Val Gln Asn Thr Asp Ser
145                 150                 155                 160

Pro Ser Tyr Pro Glu Ile Ser Phe Ser Cys Ser Met Glu Gln Leu Gln
                165                 170                 175

Asp Leu Val Gly Lys Leu Lys Asp Ala Ser Lys Ser Leu Glu Arg Ala
            180                 185                 190

Thr Gln Leu Xaa
        195

<210> 129
<211> 49
<212> PRT
<213> Homo sapiens

<400> 129
Met Ala Ser Ile Leu Leu Leu Leu Val Leu Ser His Ser Cys Cys Cys
1               5                   10                  15

Lys Asn Thr Cys Leu Gln Val Leu Cys Asn Phe Asp Ser Val His Asn
            20                  25                  30

Leu Ser Thr Leu Ile Leu Lys Ile Ile Ile Arg Val Asp Val Leu Val
        35                  40                  45

Tyr

<210> 130
<211> 55
<212> PRT
<213> Homo sapiens

<220>
<221> SITE
<222> (55)
<223> Xaa equals stop translation

<400> 130
Met Val Tyr Cys Val His Leu Asn Pro Phe Thr Asp Leu Cys Cys Ile
1               5                   10                  15

Phe Phe Met Pro Leu Leu Cys Phe Leu Leu Arg Ser Arg Val Asp Ser
            20                  25                  30

Ile Ser Ile Pro Ser Leu Thr Leu Leu Glu Ala Cys Asn Ser Ile Tyr
        35                  40                  45

Cys Ser Gly Ser Ser Ala Xaa
    50                  55


<210> 131
<211> 33
<212> PRT
<213> Homo sapiens

<220>
<221> SITE
<222> (33)
<223> Xaa equals stop translation

<400> 131
Met Gly Val Asn Lys Val Leu Phe Thr Phe Phe Phe Phe Ser Ser Leu
  1               5                  10                  15

Leu Asp Gly Val Gly Thr Ser His Ser Leu Ala Ser Phe Pro His Thr
             20                  25                  30

Xaa



<210> 132
<211> 24
<212> PRT
<213> Homo sapiens

<220>
<221> SITE
<222> (24)
<223> Xaa equals stop translation

<400> 132
Met Trp Pro Leu Leu Leu Arg Leu Leu Phe Leu His Leu Phe Leu Ala
  1               5                  10                  15

Lys Asn Lys Leu Ile Phe Lys Xaa
             20


<210> 133
<211> 220
<212> PRT
<213> Homo sapiens

<220>
<221> SITE
<222> (68)
<223> Xaa equals any of the naturally occurring L-amino acids

<220>

<221> SITE
<222> (87)
<223> Xaa equals any of the naturally occurring L-amino acids

<220>
<221> SITE
<222> (98)
<223> Xaa equals any of the naturally occurring L-amino acids

<220>
<221> SITE
<222> (220)
<223> Xaa equals stop translation

<400> 133
Met Ala Glu Ile His Thr Pro Tyr Ser Ser Leu Lys Lys Leu Leu Ser
1               5                   10                  15

Leu Leu Asn Gly Phe Val Ala Val Ser Gly Ile Ile Leu Val Gly Leu
            20                  25                  30

Gly Ile Gly Gly Lys Cys Gly Gly Ala Ser Leu Thr Asn Val Leu Gly
        35                  40                  45

Leu Ser Ser Ala Tyr Leu Leu His Val Gly Asn Leu Cys Leu Val Met
    50                  55                  60

Gly Cys Ile Xaa Val Leu Leu Gly Cys Ala Gly Trp Tyr Gly Ala Thr
65                  70                  75                  80

Lys Glu Ser Arg Gly Thr Xaa Leu Phe Val Gly Asp Val Ala Leu Glu
                85                  90                  95

His Xaa Phe Val Thr Leu Arg Lys Asn Tyr Arg Gly Tyr Asn Glu Pro
            100                 105                 110

Asp Asp Tyr Ser Thr Gln Trp Asn Leu Val Met Glu Lys Leu Lys Cys
        115                 120                 125

Cys Gly Val Asn Asn Tyr Thr Asp Phe Ser Gly Ser Ser Phe Glu Met
    130                 135                 140

Thr Thr Gly His Thr Tyr Pro Arg Ser Cys Cys Lys Ser Ile Gly Ser
145                 150                 155                 160

Val Ser Cys Asp Gly Arg Asp Val Ser Pro Asn Val Ile His Gln Lys
                165                 170                 175

Gly Cys Phe His Lys Leu Leu Lys Ile Thr Lys Thr Gln Ser Phe Thr
            180                 185                 190

Leu Ser Gly Ser Ser Leu Gly Ala Ala Val Ile Gln Leu Pro Gly Ile
        195                 200                 205

Leu Ala Thr Leu Leu Leu Phe Ile Lys Leu Gly Xaa
    210                 215                 220

<210> 134
<211> 303
<212> PRT
<213> Homo sapiens

<220>
<221> SITE
<222> (303)
<223> Xaa equals stop translation

<400> 134
Met Ile Gly Ile Ser Ala Ser Phe Ser Ala Leu Leu Glu Gln Ile Leu
1               5                   10                  15

Cys Ala Ser Gly His Ser Ser Gly Phe Ser Gly Leu Cys Gly Ala Leu
            20                  25                  30

Phe Ile Thr Phe Gly Ile Leu Gly Ala Leu Ala Leu Gly Pro Tyr Val
        35                  40                  45

Asp Arg Thr Lys His Phe Thr Glu Ala Thr Lys Ile Gly Leu Cys Leu
    50                  55                  60

Phe Ser Leu Ala Cys Val Pro Phe Ala Leu Val Ser Gln Leu Gln Gly
65                  70                  75                  80

Gln Thr Leu Ala Leu Ala Ala Thr Cys Ser Leu Leu Gly Leu Phe Gly
                85                  90                  95

Phe Ser Val Gly Pro Val Ala Met Glu Leu Ala Val Glu Cys Ser Phe
            100                 105                 110

Pro Val Gly Glu Gly Ala Ala Thr Gly Met Ile Phe Val Leu Gly Gln
        115                 120                 125

Ala Glu Gly Ile Leu Ile Met Leu Ala Met Thr Ala Leu Thr Val Arg
    130                 135                 140

Arg Ser Glu Pro Ser Leu Ser Thr Cys Gln Gln Gly Glu Asp Pro Leu
145                 150                 155                 160

Asp Trp Thr Val Ser Leu Leu Leu Met Ala Gly Leu Cys Thr Phe Phe
                165                 170                 175

Ser Cys Ile Leu Ala Val Phe Phe His Thr Pro Tyr Arg Arg Leu Gln
            180                 185                 190

Ala Glu Ser Gly Glu Pro Pro Ser Thr Arg Asn Ala Val Gly Gly Ala
        195                 200                 205

Asp Ser Gly Pro Gly Val Asp Arg Gly Gly Ala Gly Arg Ala Gly Val
    210                 215                 220

Leu Gly Pro Ser Thr Ala Thr Pro Glu Cys Thr Ala Arg Gly Ala Ser
225                 230                 235                 240

Leu Glu Asp Pro Arg Gly Pro Gly Ser Pro His Pro Ala Cys His Arg
                245                 250                 255

Ala Thr Pro Arg Ala Gln Gly Pro Ala Ala Thr Asp Ala Pro Ser Arg
            260                 265                 270

Pro Gly Arg Leu Ala Gly Arg Val Gln Ala Ser Arg Phe Ile Asp Pro
        275                 280                 285

Ala Gly Ser His Ser Ser Phe Ser Ser Pro Trp Val Ile Thr Xaa
    290                 295                 300

<210> 135
<211> 41
<212> PRT
<213> Homo sapiens

<220>
<221> SITE
<222> (41)
<223> Xaa equals stop translation

<400> 135
Met Arg Leu Val Pro Ser His Leu Leu Ala Ile Leu Ile Asn Ile Lys
  1               5                  10                  15

Asp Gln Met Met Cys Phe Cys Ile Ala Leu Met Met Arg Leu Ser Ser
             20                  25                  30

Cys Ile Ala Ser Ser Gly Pro Trp Xaa
         35                  40

<210> 136
<211> 278
<212> PRT
<213> Homo sapiens

<220>
<221> SITE
<222> (278)
<223> Xaa equals stop translation

<400> 136
Met Ser Phe Asn Leu Gln Ser Ser Lys Lys Leu Phe Ile Phe Leu Gly
  1               5                  10                  15

Lys Ser Leu Phe Ser Leu Leu Glu Ala Met Ile Phe Ala Leu Leu Pro
             20                  25                  30

Lys Pro Arg Lys Asn Val Ala Gly Glu Ile Val Leu Ile Thr Gly Ala
         35                  40                  45

Gly Ser Gly Leu Gly Arg Leu Leu Ala Leu Gln Phe Ala Arg Leu Gly
     50                  55                  60

Ser Val Leu Val Leu Trp Asp Ile Asn Lys Glu Gly Asn Glu Glu Thr
 65                  70                  75                  80

Cys Lys Met Ala Arg Glu Ala Gly Ala Thr Arg Val His Ala Tyr Thr
                85                  90                  95

Cys Asp Cys Ser Gln Lys Glu Gly Val Tyr Arg Val Ala Asp Gln Val
            100                 105                 110

Lys Lys Glu Val Gly Asp Val Ser Ile Leu Ile Asn Asn Ala Gly Ile
        115                 120                 125

Val Thr Gly Lys Lys Phe Leu Asp Cys Pro Asp Glu Leu Met Glu Lys
    130                 135                 140

Ser Phe Asp Val Asn Phe Lys Ala His Leu Trp Thr Tyr Lys Ala Phe
145                 150                 155                 160

Leu Pro Ala Met Ile Ala Asn Asp His Gly His Leu Val Cys Ile Ser
                165                 170                 175

Ser Ser Ala Gly Leu Ser Gly Val Asn Gly Leu Ala Asp Tyr Cys Ala
            180                 185                 190

Ser Lys Phe Ala Ala Phe Gly Phe Ala Glu Ser Val Phe Val Glu Thr
        195                 200                 205

Phe Val Gln Lys Gln Lys Gly Ile Lys Thr Thr Ile Val Cys Pro Phe
    210                 215                 220

Phe Ile Lys Thr Gly Met Phe Glu Gly Cys Thr Thr Gly Cys Pro Ser
225                 230                 235                 240

Leu Leu Pro Ile Leu Glu Pro Lys Tyr Ala Val Glu Lys Ile Val Glu
                245                 250                 255

Ala Ile Leu Gln Glu Lys Met Tyr Leu Tyr Met Pro Lys Val Val Ile
            260                 265                 270

Leu His Asp Val Ser Xaa
        275

<210> 137
<211> 111
<212> PRT
<213> Homo sapiens

<220>
<221> SITE
<222> (111)
<223> Xaa equals stop translation

<400> 137
Met Leu Thr Phe Leu Met Leu Val Arg Leu Ser Thr Leu Cys Pro Ser
  1               5                  10                  15

Ala Val Leu Gln Arg Leu Asp Arg Leu Val Glu Pro Leu Arg Ala Thr
            20                  25                  30

Cys Thr Thr Lys Val Lys Ala Asn Ser Val Lys Gln Glu Phe Glu Lys

        35                  40                  45

Gln Asp Glu Leu Lys Arg Ser Ala Met Arg Ala Val Ala Ala Leu Leu
    50                  55                  60

Thr Ile Pro Glu Ala Glu Lys Ser Pro Leu Met Ser Glu Phe Gln Ser
65                  70                  75                  80

Gln Ile Ser Ser Asn Pro Glu Leu Ala Ala Ile Phe Glu Ser Ile Gln
                85                  90                  95

Lys Asp Ser Ser Ser Thr Asn Leu Glu Ser Met Asp Thr Ser Xaa
            100                 105                 110

<210> 138
<211> 133
<212> PRT
<213> Homo sapiens

<220>
<221> SITE
<222> (133)
<223> Xaa equals stop translation

<400> 138
Met Arg Ala Leu His Phe Ser Ser Arg His Asn Lys Asp Ile Ala Leu
1               5                   10                  15

Val Asn Leu Ala Asn Val Leu His Arg Ala His Phe Ser Ala Asp Ala
            20                  25                  30

Ala Val Val Val His Ala Ala Leu Asp Asp Ser Asp Phe Phe Thr Ser
        35                  40                  45

Tyr Tyr Thr Leu Gly Asn Ile Tyr Ala Met Leu Gly Glu Tyr Asn His
    50                  55                  60

Ser Val Leu Cys Tyr Asp His Ala Leu Gln Ala Arg Pro Gly Phe Glu
65                  70                  75                  80

Gln Ala Ile Lys Arg Lys His Ala Val Leu Cys Gln Gln Lys Leu Glu
                85                  90                  95

Gln Lys Leu Glu Ala Gln His Arg Ser Leu Gln Arg Thr Leu Asn Glu
            100                 105                 110

Leu Lys Glu Tyr Gln Lys Gln His Asp His Tyr Leu Arg Pro Gly Asn
        115                 120                 125

Pro Arg Lys Thr Xaa
    130

<210> 139
<211> 131
<212> PRT
<213> Homo sapiens

<220>
<221> SITE
<222> (131)
<223> Xaa equals stop translation

<400> 139
Met Glu Thr Leu Gly Ala Leu Leu Val Leu Glu Phe Leu Leu Leu Ser
1               5                   10                  15

Pro Val Glu Ala Gln Gln Ala Thr Glu His Arg Leu Lys Pro Trp Leu
            20                  25                  30

Val Gly Leu Ala Ala Val Val Gly Phe Leu Phe Ile Val Tyr Leu Val
        35                  40                  45

Leu Leu Ala Asn Arg Leu Trp Cys Ser Lys Ala Arg Ala Glu Asp Glu
    50                  55                  60

Glu Glu Thr Thr Phe Arg Met Glu Ser Asn Leu Tyr Gln Asp Gln Ser
65                  70                  75                  80

Glu Asp Lys Arg Glu Lys Lys Glu Ala Lys Glu Lys Glu Glu Lys Arg
                85                  90                  95

Lys Lys Glu Lys Lys Thr Ala Lys Glu Gly Glu Ser Asn Leu Gly Leu
            100                 105                 110

Asp Leu Glu Glu Lys Glu Pro Gly Asp His Glu Arg Ala Lys Ser Thr
        115                 120                 125

Val Met Xaa
    130


<210> 140
<211> 106
<212> PRT
<213> Homo sapiens

<220>
<221> SITE
<222> (106)
<223> Xaa equals stop translation

<400> 140
Met Thr His Arg Arg His Cys Gly Leu Ala Arg Trp Ile Leu Met Lys
1               5                   10                  15

Ile Phe Cys Trp Arg Val Ser Thr Val Thr Ser Thr Ala Gly Ala Leu
            20                  25                  30

Thr Asn Pro His Ser Cys Tyr Thr Ser Val Leu Lys Val Gly Ala Thr
        35                  40                  45

Gly Val Gly Gln Ser Leu Ser Val Trp Thr Met Pro Gly Leu Leu Leu
    50                  55                  60

Glu Gln Phe Ser Thr Gly Val Glu Leu Leu Leu Ser Ser Ser Arg Phe
65                  70                  75                  80

Ser Asn Ser Met Glu Tyr Lys Asn Arg Leu Ser Ser Val Glu Asp Arg
                85                  90                  95

Ser Ser Val Val Thr Cys Leu Lys Ala Xaa
            100                 105

<210> 141
<211> 62
<212> PRT
<213> Homo sapiens

<220>
<221> SITE
<222> (62)
<223> Xaa equals stop translation

<400> 141
Met Pro Leu Ala Leu Leu Ala Thr Trp Leu Ser Cys Leu Pro Ser Leu
 1               5                  10                  15

Val Leu Thr Tyr Tyr Ser Arg Ser Asn Gln Lys Met Pro Trp Thr Leu
            20                  25                  30

Ala Ser Pro Phe Ser Ser Met Ala Ser Thr Met Glu Phe Trp Asn Gly
        35                  40                  45

Thr Leu Gln Lys Cys Val Gln Thr Thr Trp His Leu Pro Xaa
    50                  55                  60

<210> 142
<211> 38
<212> PRT
<213> Homo sapiens

<220>
<221> SITE
<222> (38)
<223> Xaa equals stop translation

<400> 142
Met Lys Ala Thr Leu Lys Leu Leu Pro Thr Ile Val Val Ile Tyr Cys
 1               5                  10                  15

Leu Leu Cys Pro Val Pro Arg Gln Ile Leu Gly Val Pro Ser Trp Ala
            20                  25                  30

Pro Gly Lys Cys Leu Xaa
        35

<210> 143
<211> 64
<212> PRT

<213> Homo sapiens

<220>
<221> SITE
<222> (64)
<223> Xaa equals stop translation

<400> 143
Met Leu Thr Ser Ser Ser Asn Leu Ile Ser Trp Val Leu Pro Glu Leu
1               5                   10                  15

Ser Ser Leu Leu Trp Val Phe Leu Phe Trp Lys Arg Gln Cys Gly Asp
            20                  25                  30

Trp Ala Gly Arg Lys Thr Arg Ser Arg Val Ser Gly Val Val Thr Asn
        35                  40                  45

Phe Pro Leu His Ser Pro Ser Leu Arg Tyr Ser Ser Phe Leu Glu Xaa
    50                  55                  60

<210> 144
<211> 105
<212> PRT
<213> Homo sapiens

<220>
<221> SITE
<222> (105)
<223> Xaa equals stop translation

<400> 144
Met Leu Phe Cys Ile Leu Leu Tyr Thr Leu Gly Ser Ala Arg Cys His
1               5                   10                  15

His Leu Ser Phe Phe Leu Trp Gly Trp Ser Asn Pro Pro Glu Lys Thr
            20                  25                  30

Pro Leu Ala Ser Trp Arg Gly Val Lys Ala Arg Leu Pro Gly Pro Gly
        35                  40                  45

Cys Gln Leu Leu Gly Ala Ala Gly Ala Glu Ala Gly Ser Cys Gln Ala
    50                  55                  60

Phe Ser Gln Gln Asp Ala Leu Ser Thr His Leu Gly Phe Arg Ile Pro
65                  70                  75                  80

Leu Pro His Leu Gln Met Gly Gln Met Ser Pro Lys Pro Ala Ala Pro
                85                  90                  95

Phe Cys Phe Thr Leu Ser Thr Glu Xaa
            100                 105

<210> 145

<211> 61
<212> PRT
<213> Homo sapiens

<220>
<221> SITE
<222> (61)
<223> Xaa equals stop translation

<400> 145
Met Gly Pro Trp Cys Leu Thr Leu Leu Ser Thr Thr Ser Gly Phe Phe
1               5                   10                  15

Ser Glu Asn Leu Tyr Leu Thr Leu Ile Leu Ser Phe Leu Leu Ser Ile
            20                  25                  30

Glu Ser Val Asn Thr Asp Pro Phe Ile Phe Gln Phe Pro Lys Ser Cys
        35                  40                  45

Phe Ala Ile Ala Ser Ile Leu Leu Ser Gly Gly Val Xaa
    50                  55                  60


<210> 146
<211> 37
<212> PRT
<213> Homo sapiens

<220>
<221> SITE
<222> (37)
<223> Xaa equals stop translation

<400> 146
Met Gly Cys Thr Ala Leu Leu Leu Leu Phe His Leu Cys Val Pro Cys
1               5                   10                  15

Glu Pro Tyr Gly Thr His Glu Lys Glu Leu Val Pro Gly Leu Tyr Phe
            20                  25                  30

Leu Val Tyr Arg Xaa
        35


<210> 147
<211> 32
<212> PRT
<213> Homo sapiens

<220>
<221> SITE
<222> (32)
<223> Xaa equals stop translation

<400> 147
Met Cys Lys Phe Ile Tyr Val Pro His Ser Val Leu Leu Val Tyr Val
1               5                   10                  15

Phe Thr Phe Val Leu Ile Pro Asn Cys Tyr Asn Ser Val Ala Leu Xaa
            20                  25                  30

<210> 148
<211> 16
<212> PRT
<213> Homo sapiens

<220>
<221> SITE
<222> (16)
<223> Xaa equals stop translation

<400> 148
Met Ser Leu Ala Leu Cys Leu Val Pro Leu Val Arg Glu Gly His Xaa
  1               5                  10                  15

<210> 149
<211> 59
<212> PRT
<213> Homo sapiens

<220>
<221> SITE
<222> (59)
<223> Xaa equals stop translation

<400> 149
Met Ile Ile Ser Ser Ile Arg Cys Leu Val Leu Gly Ile Glu Cys Val
  1               5                  10                  15

Ser Ala Val Cys Gln Asn Leu Leu Leu Gly Glu Phe Pro His Trp Glu
             20                  25                  30

Arg Asp Pro Gly Asn Gly Met Val Leu Glu Gly Leu Leu Asn Thr Phe
         35                  40                  45

Pro Trp Glu Gly Ser Cys Tyr Leu Gln Gly Xaa
     50                  55

<210> 150
<211> 87
<212> PRT
<213> Homo sapiens

<220>
<221> SITE
<222> (87)
<223> Xaa equals stop translation

<400> 150
Met Leu Lys Thr Trp Phe Phe Val Met Ala Val Ile Gly Val Ile Ile
1               5                   10                  15

Pro Thr Val Phe Asp Gln Ser Ser Arg Leu Cys Leu Lys Glu Thr Gly
            20                  25                  30

Phe Val Gln Asn Val Asp Gln Ser Asn Val Leu Glu Asp Ser Pro Leu
        35                  40                  45

Asp Arg Asp His Pro Trp Lys Val Met Lys Met Trp Lys Thr Val Trp
    50                  55                  60

Glu Val Arg Met Met Lys Leu Met Ala Met Lys Lys Lys Val Lys Val
65                  70                  75                  80

Arg Arg Lys Ser Met Arg Xaa
                85

<210> 151
<211> 53
<212> PRT
<213> Homo sapiens

<220>
<221> SITE
<222> (51)
<223> Xaa equals any of the naturally occurring L-amino acids

<220>
<221> SITE
<222> (53)
<223> Xaa equals stop translation

<400> 151
Met Asp Ile Cys Ser Pro Val Ala Leu Tyr Phe Leu Leu Thr Ala Ala
1               5                   10                  15

His Ile Thr Ala Val Ser Lys Pro Thr Val Met Leu Arg Glu Arg Pro
            20                  25                  30

Cys Ser Gly Pro Ser Arg Ser Ala Pro Gln Ser Arg Leu Ile Gly Pro
        35                  40                  45

Trp Asp Xaa Cys Xaa
    50

<210> 152
<211> 78
<212> PRT
<213> Homo sapiens

<220>
<221> SITE
<222> (78)

<223> Xaa equals stop translation

<400> 152
Met Ala Leu Lys Asn Lys Phe Ser Cys Leu Trp Ile Leu Gly Leu Cys
1               5                   10                  15

Leu Val Ala Thr Thr Ser Ser Lys Ile Pro Ser Ile Thr Asp Pro His
            20                  25                  30

Phe Ile Asp Asn Cys Ile Glu Ala His Asn Glu Trp Arg Gly Lys Val
        35                  40                  45

Asn Pro Pro Ala Ala Asp Met Lys Tyr Met Ile Trp Asp Lys Gly Leu
    50                  55                  60

Ala Lys Met Ala Lys Ala Trp Gly Lys Pro Val Gln Ile Xaa
65                  70                  75


<210> 153
<211> 72
<212> PRT
<213> Homo sapiens

<220>
<221> SITE
<222> (72)
<223> Xaa equals stop translation

<400> 153
Met Leu Gln Ala Ala Ser Leu Ser Leu Val Thr Trp Val Val Cys Thr
1               5                   10                  15

Val Trp Leu Glu Thr Thr Val Pro Pro Ser Leu Pro Glu Pro Pro Met
            20                  25                  30

Trp Pro Leu Ser Ser Asp Ser Ser Trp Ser Leu Trp Ile Ser Thr Gly
        35                  40                  45

Met Ala Pro Ala Pro Ser Ser Ser Thr Arg Ser Phe Ser Val Leu Pro
    50                  55                  60

Glu Ile Cys Phe Cys Leu Trp Xaa
65                  70


<210> 154
<211> 41
<212> PRT
<213> Homo sapiens

<220>
<221> SITE
<222> (41)
<223> Xaa equals stop translation

<400> 154
Met Leu Gln Glu Val Lys Leu Asp Phe Leu Trp Leu Leu Asn Leu Pro

1                   5                   10                  15

Leu Ile Leu Leu Phe Ser Ile Leu Glu Ser Ser Met Lys Ile Cys Thr
            20                  25                  30

Asn Ala Met Phe Thr Arg Thr Gly Xaa
        35                  40

<210> 155
<211> 85
<212> PRT
<213> Homo sapiens

<220>
<221> SITE
<222> (85)
<223> Xaa equals stop translation

<400> 155
Met Glu Val Trp His Gly Leu Val Ile Ala Val Val Ser Leu Phe Leu
1               5                   10                  15

Gln Ala Cys Phe Leu Thr Ala Ile Asn Tyr Leu Leu Ser Arg His Met
            20                  25                  30

Gly Asn Trp Leu Ser Ile Leu Phe Pro Pro Ser His Ser Gln Arg Pro
        35                  40                  45

Phe Ser Ser Leu Gln Gln Asp Arg Pro Phe Gly Val Pro Lys Arg His
    50                  55                  60

Ser Lys Thr Thr Arg Gly Pro Thr Gly Gln Ile Pro Ser His Arg Ser
65                  70                  75                  80

Pro Ser Pro Gln Xaa
                85

<210> 156
<211> 96
<212> PRT
<213> Homo sapiens

<220>
<221> SITE
<222> (96)
<223> Xaa equals stop translation

<400> 156
Met Ala Glu Pro Ile Ala Cys Leu Cys Leu Ile Cys Cys Ile Ile Ile
1               5                   10                  15

Ser Ala Thr Thr Gln Met Pro Phe Glu Gly Ser Cys Phe Cys Leu Val
            20                  25                  30

Pro Cys Asn Phe Gln Pro Tyr Phe Arg His Phe Arg Pro Asn Asp Leu
        35                  40                  45

Arg His Met Val Phe Thr His Gly Leu Trp Ala Leu Glu Lys Leu Ser
    50                  55                  60

Pro Leu Lys Glu Asn Gln Asn Val Ala Cys Ile Cys Ile Phe Cys Leu
65                  70                  75                  80

Arg Phe His Leu Ile Leu Lys Trp Ile Leu Asp Ser Pro Lys Val Xaa
                85                  90                  95

<210> 157
<211> 89
<212> PRT
<213> Homo sapiens

<220>
<221> SITE
<222> (89)
<223> Xaa equals stop translation

<400> 157
Met Trp Ala Val Leu Pro Ala Trp Phe Pro Phe Pro Gly Thr Cys His
1               5                   10                  15

Cys Leu Pro Val Ser Leu Arg Gly His Phe Trp Glu Val Arg Pro Trp
            20                  25                  30

Pro Pro Gly Pro Leu Phe Arg Ser Glu Ala Pro Thr Cys Leu Gly Ser
        35                  40                  45

Gly Ser Ser Gly Val Arg Pro Cys Pro Pro Gln Asp Ile Pro Ser Lys
    50                  55                  60

Pro Ala Met Ser Gly Asp Gly Pro Leu Pro Gly Lys Val Leu Phe Leu
65                  70                  75                  80

Leu Val Thr Glu Lys Asn Leu Pro Xaa
                85

<210> 158
<211> 44
<212> PRT
<213> Homo sapiens

<220>
<221> SITE
<222> (44)
<223> Xaa equals stop translation

<400> 158
Met Ser Ala Leu Ser Phe Thr Ser Tyr Phe Leu Leu Leu Leu Arg Val
1               5                   10                  15

Lys Pro Val Glu Val Ser Gly Ser Ile Pro His Pro Glu Gln Pro Asn
            20                  25                  30

Val Leu Cys Leu Val Leu Pro Thr Phe Gly Tyr Xaa
        35                  40

<210> 159
<211> 46
<212> PRT
<213> Homo sapiens

<220>
<221> SITE
<222> (46)
<223> Xaa equals stop translation

<400> 159
Met Cys Cys Phe Phe Leu Lys Thr Leu Asn Leu Trp Leu Gly Tyr Phe
  1               5                  10                  15

Cys Gln Phe Ile Cys Leu Pro Cys Gln Val Thr Leu Cys Leu Ile Asp
            20                  25                  30

Val Leu Cys Val Phe His Ser Val His Ala Glu Leu Ser Xaa
        35                  40                  45

<210> 160
<211> 62
<212> PRT
<213> Homo sapiens

<220>
<221> SITE
<222> (62)
<223> Xaa equals stop translation

<400> 160
Met Tyr Leu Phe Leu Lys Thr Leu Leu Ser Phe Ser Thr Leu Met Met
  1               5                  10                  15

Thr Thr Ala Leu Ser Phe Met Val Ile Thr Val Leu Trp Val Leu Leu
            20                  25                  30

Leu His Leu Leu Ala Asn Ile Cys Ile Pro Arg Lys Cys Ser Phe Ala
        35                  40                  45

Cys Phe Tyr Ile Asn Gly Ile Leu Leu His Ala Val Phe Xaa
    50                  55                  60

<210> 161
<211> 31
<212> PRT
<213> Homo sapiens

<220>

<221> SITE
<222> (31)
<223> Xaa equals stop translation

<400> 161
Met Val Ser Leu Leu Ser Leu Thr Phe His Gln Phe Val Ser Ser Leu
1               5                   10                  15

Lys Tyr Phe Lys Leu Leu Ser Thr Ser Arg Gln Glu Ile Leu Xaa
            20                  25                  30


<210> 162
<211> 25
<212> PRT
<213> Homo sapiens

<220>
<221> SITE
<222> (25)
<223> Xaa equals stop translation

<400> 162
Met Ala Gly Asn Gln Gln Phe Val Asn Leu Leu Leu Arg Ser Val Ile
1               5                   10                  15

His Ser Val Ala Tyr Phe Leu Ser Xaa
            20                  25


<210> 163
<211> 71
<212> PRT
<213> Homo sapiens

<220>
<221> SITE
<222> (71)
<223> Xaa equals stop translation

<400> 163
Met Glu Asn Pro Thr Ser Thr Pro Thr Leu Pro Cys Phe Leu Phe Phe
1               5                   10                  15

Phe Ser Pro Arg Ser Leu Asp Val Leu Thr Pro Pro His Cys Leu Leu
            20                  25                  30

Ser Gly Thr Gly Trp Asp Leu Glu Glu Asp Lys Ala Phe Leu Asp Tyr
        35                  40                  45

Pro Ser Tyr Ser Val Ser Leu Phe Leu Thr Gln Arg Gly Arg Gln Asn
    50                  55                  60

Gln Ser Gly Leu Phe Gln Xaa
65                  70


<210> 164

<211> 43
<212> PRT
<213> Homo sapiens

<220>
<221> SITE
<222> (43)
<223> Xaa equals stop translation

<400> 164
Met Arg Ile His Pro Ile Phe Arg Leu Gly Asn Val Tyr Ser Leu Leu
1               5                   10                  15

Ser Phe Leu Ile Leu Gly Arg Val Ser Thr Lys Asn Ser Ile Glu Glu
            20                  25                  30

Lys Gln Tyr Asn Ile Lys Ile Lys Lys Ile Xaa
        35                  40


<210> 165
<211> 65
<212> PRT
<213> Homo sapiens

<220>
<221> SITE
<222> (65)
<223> Xaa equals stop translation

<400> 165
Met Glu Lys Leu Leu Thr Leu Tyr Leu Leu Leu Tyr Val Ser Tyr Trp
1               5                   10                  15

Ser Val Ser Pro Thr Gly Gln Gly Ala Gly Leu Phe Ile Ala Gln Ser
            20                  25                  30

Ser Ala Pro Gly Leu Arg Gln Thr His Ser Arg His Leu Gly Asn Ala
        35                  40                  45

Trp Glu Arg Lys Glu Gly Arg Arg Glu Glu Gly Leu His Gly His Val
    50                  55                  60

Xaa
65


<210> 166
<211> 68
<212> PRT
<213> Homo sapiens

<220>
<221> SITE
<222> (68)
<223> Xaa equals stop translation

<400> 166

Met Leu Phe Ser Leu Pro Arg Thr Phe Ser Ser His Ser Ser Pro Ala
1               5                   10                  15

Gln Leu Ile Phe Ile His Ala Ala Ser Val Leu Met Ala Phe Pro Pro
            20                  25                  30

Arg Pro Ser Lys Thr Thr Leu Pro Gln Ala Ala Phe Leu Thr Ser Leu
        35                  40                  45

Ala Cys Pro Leu Met Leu Ser Thr Phe Phe Leu Tyr Gln Asn Ala Phe
    50                  55                  60

Val Cys Lys Xaa
65

<210> 167
<211> 59
<212> PRT
<213> Homo sapiens

<220>
<221> SITE
<222> (59)
<223> Xaa equals stop translation

<400> 167
Met Ser Ser Phe Pro Gly Pro Gln Cys Val Gln Leu Ile Asn Leu Leu
1               5                   10                  15

His Leu Ile Cys Pro Val Ser Gly Leu Val Cys Ser Ala Ile Thr Ile
            20                  25                  30

Ala Leu Arg Gln Lys Ser Ile Pro His Gln Gln Gly Arg Glu Ala Val
        35                  40                  45

Ile Lys Thr Pro Pro Pro Gly Ser Leu Pro Xaa
    50                  55

<210> 168
<211> 54
<212> PRT
<213> Homo sapiens

<220>
<221> SITE
<222> (30)
<223> Xaa equals any of the naturally occurring L-amino acids

<220>
<221> SITE
<222> (34)
<223> Xaa equals any of the naturally occurring L-amino acids

<220>
<221> SITE
<222> (38)

<223> Xaa equals any of the naturally occurring L-amino acids

<220>
<221> SITE
<222> (54)
<223> Xaa equals stop translation

<400> 168
Met Leu Val Leu Ala Trp Ile Thr Phe Pro Pro Cys Lys Ala Cys Cys
1               5                   10                  15

Met Met Cys Ile Phe Ser Ser Arg Leu Leu Gln Gln Glu Xaa Val Cys
            20                  25                  30

Thr Xaa Val Gln Gly Xaa Glu Pro Arg Gly Met Ala Gln Arg Asp Arg
        35                  40                  45

Gly Phe Glu Ser Leu Xaa
    50


<210> 169
<211> 20
<212> PRT
<213> Homo sapiens

<220>
<221> SITE
<222> (20)
<223> Xaa equals stop translation

<400> 169
Met Val Tyr His Gly Tyr Asn Ile Tyr Leu Val Val Phe Leu Leu Leu
1               5                   10                  15

Tyr Leu Asp Xaa
            20


<210> 170
<211> 39
<212> PRT
<213> Homo sapiens

<220>
<221> SITE
<222> (39)
<223> Xaa equals stop translation

<400> 170
Met Gly Pro Ala Val Cys Phe Arg Ala Cys Glu Met Cys Ser Leu Ser
1               5                   10                  15

Gly Leu Leu Leu Asn Leu Cys Phe Gln Ser Cys Leu Ser Val Pro Leu
            20                  25                  30

Ser Gly Val Pro Arg Val Xaa
        35

<210> 171
<211> 54
<212> PRT
<213> Homo sapiens

<220>
<221> SITE
<222> (54)
<223> Xaa equals stop translation

<400> 171
Met Asn Leu Glu Thr Val Leu Leu Ser Arg Thr Ser Ser Leu Gly Phe
 1               5                  10                  15

Ala Val Cys Leu Pro Cys Phe Phe Cys Trp Phe Tyr Leu Val Leu Phe
            20                  25                  30

Leu Glu Leu Thr Ser Ile Thr Phe Ala Met Tyr Asp Ile Ile Pro Cys
        35                  40                  45

Met Thr Leu Gly Lys Xaa
    50


<210> 172
<211> 55
<212> PRT
<213> Homo sapiens

<220>
<221> SITE
<222> (55)
<223> Xaa equals stop translation

<400> 172
Met Ser Trp Ala Leu Pro Ser Leu Phe Phe Leu Leu Phe Ser Pro Phe
 1               5                  10                  15

Leu Leu Pro Ser Gly Leu Thr Val Ile Arg Arg Tyr Thr Asn Asn Ser
            20                  25                  30

Asn Asn Tyr Leu Lys Asn His Thr His Gln Lys Asn Lys Arg Gln Gln
        35                  40                  45

Lys Ile Thr Arg Tyr Ser Xaa
    50                  55


<210> 173
<211> 47
<212> PRT
<213> Homo sapiens

<220>
<221> SITE
<222> (47)

<223> Xaa equals stop translation

<400> 173
Met Leu Ser Pro Leu Asn His Leu Tyr Phe Pro Phe Arg Phe Leu Cys
1               5                   10                  15

Met Leu Cys Ser Leu Pro Arg Val Val Phe Gln Leu Thr Pro Ile Lys
            20                  25                  30

Glu Ala Phe Pro Ser Gln Glu Leu Thr Phe Pro Cys Thr His Xaa
        35                  40                  45


<210> 174
<211> 55
<212> PRT
<213> Homo sapiens

<220>
<221> SITE
<222> (55)
<223> Xaa equals stop translation

<400> 174
Met Leu Leu Gly Phe Leu Cys Leu Trp Tyr Gln Val Tyr Val Cys Met
1               5                   10                  15

Tyr Val Cys Thr Tyr Leu Phe Ile Tyr Leu Leu Phe Ser Leu Phe Ser
            20                  25                  30

Leu Pro His Met Ile Cys Lys Lys Ser Val Lys Phe Ile Met Ser Ser
        35                  40                  45

Pro Lys Pro Pro Ser Gly Xaa
    50                  55


<210> 175
<211> 27
<212> PRT
<213> Homo sapiens

<220>
<221> SITE
<222> (27)
<223> Xaa equals stop translation

<400> 175
Met Lys Trp Ser Leu Leu Lys Val Val Leu Val Phe Val Phe Val Cys
1               5                   10                  15

Gly Phe Leu Lys Arg Ala Tyr Pro Ala Thr Xaa
            20                  25


<210> 176
<211> 50
<212> PRT

<213> Homo sapiens

<220>
<221> SITE
<222> (50)
<223> Xaa equals stop translation

<400> 176
Met Ile Asp Ile Cys His Ser Leu Arg Arg Glu His Phe Leu Leu Trp
1               5                   10                  15

Ser Phe Leu Gly Leu Phe Tyr Trp Ala Val Asn Gly Lys Ser Val Cys
            20                  25                  30

Val Ser Leu Leu His Pro Lys His Leu Gly Lys Asn Glu Ser Leu Leu
        35                  40                  45

Ile Xaa
    50


<210> 177
<211> 27
<212> PRT
<213> Homo sapiens

<220>
<221> SITE
<222> (27)
<223> Xaa equals stop translation

<400> 177
Met Phe His Ser Ser Leu Leu Val Phe Leu Ser Leu Leu Ser Gln Glu
1               5                   10                  15

Ile Phe Thr Glu Tyr Asp Cys Met His Lys Xaa
            20                  25


<210> 178
<211> 41
<212> PRT
<213> Homo sapiens

<220>
<221> SITE
<222> (41)
<223> Xaa equals stop translation

<400> 178
Met Val His Val Ser Asn Leu Pro Trp Cys Leu Met Thr Leu Ser Ile
1               5                   10                  15

Phe Ala Leu Leu Cys Asn His His Cys His Pro Ser Thr Glu Arg Leu
            20                  25                  30

Ser Ser Cys Lys Thr Glu Thr Pro Xaa
        35                  40

<210> 179
<211> 65
<212> PRT
<213> Homo sapiens

<400> 179


<210> 180
<211> 20
<212> PRT
<213> Homo sapiens

<220>
<221> SITE
<222> (20)
<223> Xaa equals stop translation

<400> 180
Met Val Gly Thr Trp Arg Met Leu Phe Leu Leu Pro Ser Tyr Ser Ser
1               5                   10                  15

Gly Gln Val Xaa
            20


<210> 181
<211> 15
<212> PRT
<213> Homo sapiens

<220>
<221> SITE
<222> (15)
<223> Xaa equals stop translation

<400> 181
Met Trp Asp Tyr Lys Thr Val Leu Leu Ala Phe Lys Gln Leu Xaa
1               5                   10                  15


<210> 182

<211> 46
<212> PRT
<213> Homo sapiens

<220>
<221> SITE
<222> (46)
<223> Xaa equals stop translation

<400> 182
Met Val Lys Trp Ile Ile Leu Ser Cys Leu Ile Leu Lys Gly Lys Arg
1               5                   10                  15

Thr Leu Asn Ser Ser Thr Phe Tyr Ala Ala Asn Lys Ser Ser Thr Ile
            20                  25                  30

Asn Arg Asn Leu Ser Trp Gln Ala Leu Pro Phe Thr His Xaa
        35                  40                  45


<210> 183
<211> 72
<212> PRT
<213> Homo sapiens

<220>
<221> SITE
<222> (19)
<223> Xaa equals any of the naturally occurring L-amino acids

<220>
<221> SITE
<222> (22)
<223> Xaa equals any of the naturally occurring L-amino acids

<220>
<221> SITE
<222> (57)
<223> Xaa equals any of the naturally occurring L-amino acids

<220>
<221> SITE
<222> (70)
<223> Xaa equals any of the naturally occurring L-amino acids

<220>
<221> SITE
<222> (72)
<223> Xaa equals stop translation

<400> 183
Met Ser Leu Leu Leu Pro Pro Leu Ala Leu Leu Leu Leu Leu Ala Ala
1               5                   10                  15

Leu Val Xaa Pro Ala Xaa Ala Ala Thr Ala Tyr Arg Pro Asp Trp Asn
            20                  25                  30

Arg Leu Ser Gly Leu Thr Arg Ala Arg Val Glu Thr Cys Gly Gly Met

        35                  40                  45

Thr Ala Glu Pro Pro Lys Gly Glu Xaa Arg Leu Ser Ser Arg Arg Thr
    50                  55                  60

Phe His Ser Ile Thr Xaa Trp Xaa
65                  70

<210> 184
<211> 78
<212> PRT
<213> Homo sapiens

<220>
<221> SITE
<222> (78)
<223> Xaa equals stop translation

<400> 184
Met Gly Leu Trp Phe Pro Met Leu Ile Leu Thr Gln Arg Phe Val Ser
1               5                   10                  15

Cys Asp Ser His Pro Asp Pro Lys His Thr His Thr His Ala His Ile
            20                  25                  30

Asn Thr His Thr His Arg His Val His Thr Gln Thr His Met His Thr
        35                  40                  45

His Ile His Thr Pro Trp Phe Glu Glu Lys Arg Asp Gly Asn Arg His
    50                  55                  60

Ser Thr His Ala Tyr Ser Ala Pro Leu Cys Ile Gly Asn Xaa
65                  70                  75

<210> 185
<211> 26
<212> PRT
<213> Homo sapiens

<220>
<221> SITE
<222> (26)
<223> Xaa equals stop translation

<400> 185
Met Leu Asn Lys Cys Gln Thr Ile Phe Tyr Ile Thr Leu Leu Leu Phe
1               5                   10                  15

Asn Phe Val Thr Phe Arg Gly Gly Gly Xaa
            20                  25

<210> 186
<211> 63
<212> PRT
<213> Homo sapiens

<220>
<221> SITE
<222> (63)
<223> Xaa equals stop translation

<400> 186
Met Glu Asn Val Cys Gln Ala Gly Phe Pro Ser Leu Leu His Leu Asn
 1               5                  10                  15

Ile Thr Leu Thr Leu Leu Gly Leu Ala Gln Cys Tyr Leu Ala Asn Phe
            20                  25                  30

Ser Ser Cys Arg Glu Gly Ser Glu His Tyr Leu Phe Phe Phe Phe Phe
        35                  40                  45

Leu Leu Glu Pro Gly Leu His Lys Ala Met Ala Lys Phe Ser Xaa
    50                  55                  60


<210> 187
<211> 92
<212> PRT
<213> Homo sapiens

<220>
<221> SITE
<222> (92)
<223> Xaa equals stop translation

<400> 187
Met Cys Pro Leu His Val Pro Leu Pro Gly His Met Gly Pro Phe Trp
 1               5                  10                  15

Pro Leu Pro Ser Leu Tyr Ser Val Arg Ser Ser Gln Ser Pro Cys Pro
            20                  25                  30

Leu Cys Phe Ser Leu Leu Pro Leu Gln Ala His Leu Ser Leu Leu His
        35                  40                  45

Thr Leu Phe Arg Ser Ala Ser Gln Ser Pro Ala Ser Gly Val Phe Trp
    50                  55                  60

Gly Cys Leu Arg Glu Arg His Glu Tyr Met Ser Pro Cys Leu Pro His
65                  70                  75                  80

Met Tyr Gln Lys Phe Asp Phe Phe Phe Phe Phe Xaa
                85                  90


<210> 188
<211> 48
<212> PRT
<213> Homo sapiens

<220>
<221> SITE
<222> (48)

<223> Xaa equals stop translation

<400> 188
Met Ala Pro Pro Arg Gly Thr Trp Phe Leu Leu Leu Ser Leu Arg Leu
1               5                   10                  15

Pro Tyr Gly Ala Ala Cys Trp Val Phe Leu Pro Phe Pro Ala Ser Cys
            20                  25                  30

Arg Ala Glu Gly Val Ala Ala Pro Ile Lys Cys Ser Arg Asn Glu Xaa
        35                  40                  45

<210> 189
<211> 45
<212> PRT
<213> Homo sapiens

<220>
<221> SITE
<222> (45)
<223> Xaa equals stop translation

<400> 189
Met Cys Leu Gly His Ala Phe Cys Leu Leu Leu Ser His Ser Cys Arg
1               5                   10                  15

Met His Cys Thr Cys Tyr Leu Cys Leu Phe Thr Val Gln Val Leu Pro
            20                  25                  30

Gly Lys Tyr Asn Glu Gly Gly Glu Gly Gln Arg Asn Xaa
        35                  40                  45

<210> 190
<211> 48
<212> PRT
<213> Homo sapiens

<400> 190
Met Phe Pro Gly Cys Ile Leu Leu Cys Asn Leu Cys Met Phe Phe Val
1               5                   10                  15

Leu Ser Phe Ser Met Gly Ile Phe Ala Phe Tyr Ser Leu Ile Arg Ala
            20                  25                  30

Met His Val Ser Arg Leu Asp Phe Asn Phe Ala Thr Tyr Phe Val Ala
        35                  40                  45

<210> 191
<211> 82

<212> PRT
<213> Homo sapiens

<220>
<221> SITE
<222> (2)
<223> Xaa equals any of the naturally occurring L-amino acids

<220>
<221> SITE
<222> (74)
<223> Xaa equals any of the naturally occurring L-amino acids

<220>
<221> SITE
<222> (82)
<223> Xaa equals stop translation

<400> 191
Met Xaa Glu Gly Gly Arg Cys Gly Tyr Val Leu Leu Pro Val Ser Leu
1               5                   10                  15

Leu Gln Cys Leu Ala Met Gly His Lys His Tyr Pro Ala Val Gly Arg
            20                  25                  30

Leu Ala Lys Arg Ser Gln Leu Ala Ser Pro Ala Ser Ser Arg Glu Trp
        35                  40                  45

Asn His Gly Ser Asn Thr Leu Leu Arg Lys Gln Lys Leu Tyr Gly His
    50                  55                  60

Ile Phe His Leu Leu Ser Pro Arg Asn Xaa Met Tyr Cys Asp Pro Ala
65                  70                  75                  80

His Xaa

<210> 192
<211> 40
<212> PRT
<213> Homo sapiens

<220>
<221> SITE
<222> (40)
<223> Xaa equals stop translation

<400> 192
Met Trp Leu Thr Gln Pro Glu Ser Leu Ser Leu Cys Val Ser Val Ser
1               5                   10                  15

Gln Asp Trp Ala His Ile Leu Ala Leu Ser Ile Thr Met Leu Trp Asp
            20                  25                  30

Phe Arg Glu Phe Pro His Leu Xaa
        35                  40

<210> 193
<211> 182
<212> PRT
<213> Homo sapiens

<220>
<221> SITE
<222> (182)
<223> Xaa equals stop translation

<400> 193
Met Ala Ser Phe Leu Lys Gly Ile Thr Ala Thr Val Leu Ile Asn Ala
1               5                   10                  15

Cys Val Ala Asn Thr Val Ala Pro Leu His Tyr Lys Asp Met Ile Ile
            20                  25                  30

Pro Lys Leu Val Asp Asp Leu Gly Lys Val Lys Ile Thr Lys Ser Gly
        35                  40                  45

Phe Leu Thr Phe Met Asp Thr Trp Ser Asn Pro Leu Glu Glu His Asn
    50                  55                  60

His Gln Ser Leu Val Pro Leu Glu Lys Ala Gln Val Pro Phe Leu Phe
65                  70                  75                  80

Ile Val Gly Met Asp Asp Gln Ser Trp Lys Ser Glu Phe Tyr Ala Gln
                85                  90                  95

Ile Ala Ser Glu Arg Leu Gln Ala His Gly Lys Glu Arg Pro Gln Ile
            100                 105                 110

Ile Cys Tyr Pro Glu Thr Gly His Cys Ile Asp Pro Pro Tyr Phe Pro
        115                 120                 125

Pro Ser Arg Ala Ser Val His Ala Val Leu Gly Glu Ala Ile Phe Tyr
    130                 135                 140

Gly Gly Glu Pro Lys Ala His Ser Lys Ala Gln Val Asp Ala Trp Gln
145                 150                 155                 160

Gln Ile Gln Thr Phe Phe His Lys His Leu Asn Gly Lys Lys Ser Val
                165                 170                 175

Lys His Ser Lys Ile Xaa
            180


<210> 194
<211> 40
<212> PRT
<213> Homo sapiens

<220>
<221> SITE
<222> (40)
<223> Xaa equals stop translation

<400> 194
Met Tyr Tyr Thr Ala Ala Cys Leu Phe Ile Ser Val Leu Phe Leu Gly
1               5                   10                  15

Leu Ser Val Leu Ile Ser Val Ala Val Val His Ser Phe Phe Lys His
            20                  25                  30

Cys Ile Val Phe His Asp Asp Xaa
        35                  40


<210> 195
<211> 73
<212> PRT
<213> Homo sapiens

<220>
<221> SITE
<222> (73)
<223> Xaa equals stop translation

<400> 195
Met Ala Ile Ala Leu Gly Pro Leu Val Leu Ser Trp Leu Cys Tyr Leu
1               5                   10                  15

Trp Leu Thr Leu Glu Ser Leu Cys Thr Asn Lys Met Ala Ser Asp Glu
            20                  25                  30

Pro Val Ser His His Cys Leu Pro Arg Leu Ser Glu Pro Pro Leu Thr
        35                  40                  45

Phe Cys Leu Glu Ala Gly Gly Leu Val Glu Val Gly Asp Leu Leu Lys
    50                  55                  60

Ser Arg Ala Arg Pro Val Ile Leu Xaa
65                  70


<210> 196
<211> 56
<212> PRT
<213> Homo sapiens

<220>
<221> SITE
<222> (56)
<223> Xaa equals stop translation

<400> 196
Met Ala Gly His Pro Val Phe Phe Leu Leu Ile His Leu Leu Pro Leu
1               5                   10                  15

Asp Phe Ser Met Gly Trp Thr Gln Thr Pro Gly Ser Asn Asn Trp Arg
            20                  25                  30

Arg Gly Trp Lys Glu Val Ser Gly Ser Ser Ala Pro Glu Gly Ser Arg
        35                  40                  45

Asp Gly Tyr Val Ala Ala Ala Xaa
    50                  55

<210> 197
<211> 70
<212> PRT
<213> Homo sapiens

<220>
<221> SITE
<222> (70)
<223> Xaa equals stop translation

<400> 197
Met Ala Gly Ser Tyr Ser Ser Asp Ile Leu Val Leu Ala Arg Ser Trp
 1               5                  10                  15

Thr Leu Leu Leu Leu Ser Val Leu Arg Leu Gln Thr Val Gly Ser Ser
            20                  25                  30

Val Thr Leu Asp Ser Gln Val Gly Ile Ile Trp Pro Ala Val Phe Lys
        35                  40                  45

Ile Gly Asn Arg Val Lys Lys Gln Asn Gln Ile Lys Glu Lys Arg Gln
    50                  55                  60

Gln Gln Asn Gln Asn Xaa
65                  70

<210> 198
<211> 47
<212> PRT
<213> Homo sapiens

<220>
<221> SITE
<222> (47)
<223> Xaa equals stop translation

<400> 198
Met Trp Ile Tyr Thr Leu Thr Tyr Ile Leu Ile Asn Ser Ser Met Leu
 1               5                  10                  15

Ala Leu Val Leu Ser Lys Leu Tyr Leu Asn Lys Phe Val Ala Arg Asn
            20                  25                  30

Val Leu Lys Ser Tyr Ser Pro Phe Leu Leu Glu Val Ser Lys Xaa
        35                  40                  45

<210> 199
<211> 55
<212> PRT
<213> Homo sapiens

<220>
<221> SITE
<222> (55)
<223> Xaa equals stop translation

<400> 199
Met Leu Glu Trp Pro Ile Ser Met Tyr Phe Val Ala Phe Leu His Cys
 1               5                  10                  15

Phe Leu Cys Ser Gly Gly Asn Leu Gly Asp Ser Phe Gln Ala Leu Pro
            20                  25                  30

Glu Leu Cys Ala Asn Cys Ser Ser Ser Pro Arg Val Leu Cys Cys Val
        35                  40                  45

Val Met Ser Pro Leu Pro Xaa
    50                  55


<210> 200
<211> 38
<212> PRT
<213> Homo sapiens

<220>
<221> SITE
<222> (38)
<223> Xaa equals stop translation

<400> 200
Met Ala Ser Glu Trp Val Gly Leu Ser Ser Leu Ile Thr Leu Leu Leu
 1               5                  10                  15

Leu Ser Cys Val Leu Ser Cys Ile Thr Leu Glu Glu Gly Glu Lys Glu
            20                  25                  30

Leu Val Phe Gly Pro Xaa
        35


<210> 201
<211> 34
<212> PRT
<213> Homo sapiens

<220>
<221> SITE
<222> (21)
<223> Xaa equals any of the naturally occurring L-amino acids

<220>
<221> SITE
<222> (34)
<223> Xaa equals stop translation

<400> 201
Met Cys Leu Leu Ala His Leu Phe Cys His His Leu Leu Ile Leu Leu
 1               5                  10                  15

Pro Val Ile Glu Xaa Leu Leu Cys Thr Arg His Trp Ala Arg Gly Ile
            20                  25                  30

Leu Xaa

<210> 202
<211> 22
<212> PRT
<213> Homo sapiens

<220>
<221> SITE
<222> (22)
<223> Xaa equals stop translation

<400> 202
Met Gln Leu Val Leu Phe His Arg Leu Ile Met Pro Leu Phe Phe Ala
  1               5                  10                  15

Arg Thr Leu Val Asp Xaa
            20

<210> 203
<211> 56
<212> PRT
<213> Homo sapiens

<220>
<221> SITE
<222> (56)
<223> Xaa equals stop translation

<400> 203
Met Lys Gln Arg Gly Glu Gln Val Pro Leu Leu Leu Pro Pro Leu Leu
  1               5                  10                  15

Leu Ser Thr Arg Leu Trp Pro Cys Trp Gly Val Pro Thr Glu Ser Val
            20                  25                  30

Gly Ser Gly Leu Ala Arg Lys Ser Val Gly Ala Ser Gln Gly His Asn
        35                  40                  45

Tyr Pro Met Pro His Arg Val Xaa
    50                  55

<210> 204
<211> 116
<212> PRT
<213> Homo sapiens

<400> 204


<210> 205
<211> 84
<212> PRT
<213> Homo sapiens

<220>
<221> SITE
<222> (84)
<223> Xaa equals stop translation

<400> 205
Met Lys Cys Leu Ala Pro Met Trp Val Ser Leu Trp Asp Ser Asp Pro
1               5                   10                  15

Leu Arg Ser Cys Leu Leu Leu Leu Ile Pro His Phe Ser Val Phe Leu
            20                  25                  30

Ile Leu Ala Ala Val Ser Cys Leu Pro Leu Ser Thr Ala Thr Arg Trp
        35                  40                  45

Arg Gly Arg Asp Pro Val Leu Leu Ile Ile Cys Leu Leu Lys Asn Leu
    50                  55                  60

Gln Asn Gly Lys Ile Thr Ile Cys Ala Glu Leu Ile Ile Ser Leu Lys
65                  70                  75                  80

Phe Lys Thr Xaa


<210> 206
<211> 46
<212> PRT
<213> Homo sapiens

<220>
<221> SITE
<222> (46)
<223> Xaa equals stop translation

<400> 206
Met Leu Phe Ser Phe Leu Phe Thr Arg Ala Thr Pro Ala Thr Phe Leu
1               5                   10                  15

Ser Leu Leu Val Arg Leu Ile Ser Ala Leu Glu His Pro Cys Cys Cys
            20                  25                  30

His His Leu Lys Cys Phe Ser Ser Gly Ile Leu Phe Trp Xaa
        35                  40                  45


<210> 207
<211> 42
<212> PRT
<213> Homo sapiens

<220>
<221> SITE
<222> (42)
<223> Xaa equals stop translation

<400> 207
Met Ala Asn Thr Ala Arg Ile Phe Leu Leu Leu Pro Ile Phe Ile Ile
1               5                   10                  15

Glu Gly Asn Ala Asn Met Lys Ile Lys Met Ser Leu Phe Pro Gln Ser
            20                  25                  30

Met Gln Phe Pro Pro Lys Leu Tyr Pro Xaa
        35                  40


<210> 208
<211> 41
<212> PRT
<213> Homo sapiens

<220>
<221> SITE
<222> (41)
<223> Xaa equals stop translation

<400> 208
Met Glu Thr Gln Ile Cys Leu Thr Gln Ile Val Ala Leu Phe Phe Leu
1               5                   10                  15

Arg Leu Val Leu Gly Lys Leu Thr Cys Phe Leu Tyr Gly Lys Leu Val
            20                  25                  30

Leu Val Glu Ala Phe Ile Leu Ala Xaa
        35                  40

<210> 209
<211> 31
<212> PRT
<213> Homo sapiens

<220>
<221> SITE
<222> (31)
<223> Xaa equals stop translation

<400> 209
Met Ala Ser His Cys Trp Met Gly Ala Val Cys Val Leu Phe Leu Gly
 1               5                  10                  15

Ile Ile Phe Leu Ala Ala Leu Phe Pro Tyr Ile Ser Phe Tyr Xaa
            20                  25                  30


<210> 210
<211> 12
<212> PRT
<213> Homo sapiens

<220>
<221> SITE
<222> (12)
<223> Xaa equals stop translation

<400> 210
Met Leu Arg Ala Leu Cys Leu Ser Thr Cys Pro Xaa
 1               5                  10


<210> 211
<211> 100
<212> PRT
<213> Homo sapiens

<220>
<221> SITE
<222> (5)
<223> Xaa equals any of the naturally occurring L-amino acids

<220>
<221> SITE
<222> (100)
<223> Xaa equals stop translation

<400> 211
Met Leu Trp Tyr Xaa Phe Pro Thr Thr Pro Leu Pro Ala Gln Val Gln
 1               5                  10                  15

Phe Trp Trp Cys Leu Cys Cys Cys Tyr Ile His Gly Ser Trp Trp Gly
            20                  25                  30

Pro Leu Ser Gln Ser Ser Ser Ser Cys Asn Ala Ser Val Thr Ala Leu
        35                  40                  45

Ser Ser Gly Cys Cys Arg Pro Arg Ala Ser Ser Pro Thr Val Pro His
    50                  55                  60

His Arg Leu Phe Pro Met Pro Ala His Thr Ser Val Asn Ser Pro Phe
65                  70                  75                  80

Ile Ser His Pro Ser Val Arg Pro Phe Glu Tyr Ala Ile Cys Phe Arg
                85                  90                  95

Ser Gly Gln Xaa
            100

<210> 212
<211> 29
<212> PRT
<213> Homo sapiens

<220>
<221> SITE
<222> (3)
<223> Xaa equals any of the naturally occurring L-amino acids

<220>
<221> SITE
<222> (29)
<223> Xaa equals stop translation

<400> 212
Met Leu Xaa Gln Phe Phe Leu Phe Val Cys Phe His Phe Ile Thr Tyr
1               5                   10                  15

Gly Phe Leu Cys His Thr Thr Arg Asn Phe Glu Lys Xaa
            20                  25

<210> 213
<211> 47
<212> PRT
<213> Homo sapiens

<220>
<221> SITE
<222> (47)
<223> Xaa equals stop translation

<400> 213
Met Gln Pro Ser Cys Val Asn Phe Arg Leu Lys Leu Phe Tyr Ser His
1               5                   10                  15

Thr Phe Met Leu Arg Leu Gly Phe Leu Phe Gly Leu Leu Asp Ala His
            20                  25                  30

Phe Asp Ile Asp Ile Arg Gly Phe Lys Pro Ser Leu Lys Gly Xaa
        35                  40                  45

<210> 214

<211> 86
<212> PRT
<213> Homo sapiens

<220>
<221> SITE
<222> (86)
<223> Xaa equals stop translation

<400> 214
Glu Leu Gln Pro Asn Pro His Ala Arg Ala Lys Pro Cys Cys Tyr Leu
1               5                   10                  15

Leu Phe Leu Ser Cys Leu Ile Pro Ser Met Phe Ser Leu Ser Val Asp
            20                  25                  30

Pro Val Ser Pro Val Leu Arg Ile Val Pro Gly Ser Asp His Phe Ser
        35                  40                  45

Leu Pro Leu Leu Leu Pro Pro Pro Leu Ala Trp Ile Ile Ala Ala Ala
    50                  55                  60

Ser Gln Leu Ala Leu Leu Cys Pro Ser Leu Phe Ser Pro Ser Val Cys
65                  70                  75                  80

Ser Gln Gln Arg Ser Xaa
                85


<210> 215
<211> 82
<212> PRT
<213> Homo sapiens

<220>
<221> SITE
<222> (49)
<223> Xaa equals any of the naturally occurring L-amino acids

<400> 215
Met Leu Met Lys Ile Asn Phe Tyr Pro Leu Pro Lys Pro Lys Leu His
1               5                   10                  15

Thr Ser Ile Ser Asn Cys Leu Leu Asp Ile Ser Ile Tyr Lys Pro Ser
            20                  25                  30

Ser Leu Ile Ser Ile Thr Ser Asp Leu Pro Gly Leu Thr Leu Lys Ser
        35                  40                  45

Xaa Asn Phe Ser Pro Thr Pro Met Pro Gly Gln Asn Leu Val Val Thr
    50                  55                  60

Ser Tyr Ser Ser Leu Ala Ser Ser His Pro Cys Ser Val Cys Gln Trp
65                  70                  75                  80

Ile Leu

<210> 216
<211> 70
<212> PRT
<213> Homo sapiens

<400> 216
Leu Ala Pro Arg Phe Ala Phe Ser Gln Cys Ser Leu Ala Ile Met Leu
1               5                   10                  15

Thr Leu Leu Phe Gln Ile His Phe Leu Met Ile Leu Ser Ser Asn Trp
            20                  25                  30

Ala Tyr Leu Lys Asp Ala Ser Lys Met Gln Ala Tyr Gln Asp Ile Lys
        35                  40                  45

Ala Lys Glu Glu Gln Glu Leu Gln Asp Ile Gln Ser Arg Ser Lys Glu
    50                  55                  60

Gln Leu Asn Ser Tyr Thr
65                  70


<210> 217
<211> 56
<212> PRT
<213> Homo sapiens

<220>
<221> SITE
<222> (13)
<223> Xaa equals any of the naturally occurring L-amino acids

<400> 217
Ile Arg His Glu Gly Gly Gly Gln Pro Phe Thr Ser Xaa Pro Leu Glu
1               5                   10                  15

Ile Leu Phe Phe Leu Asn Gly Trp Tyr Asn Ala Thr Tyr Phe Leu Leu
            20                  25                  30

Glu Leu Phe Ile Phe Leu Tyr Lys Gly Val Leu Leu Pro Tyr Pro Thr
        35                  40                  45

Ala Asn Leu Val Leu Asp Val Val
    50                  55


<210> 218
<211> 89
<212> PRT
<213> Homo sapiens

<400> 218

<210> 219
<211> 276
<212> PRT
<213> Homo sapiens

<220>
<221> SITE
<222> (7)
<223> Xaa equals any of the naturally occurring L-amino acids

<400> 219
Arg Val Ile Arg Leu Thr Xaa Arg Ala Asn Trp Ser Ser Thr Ala Val
 1               5                  10                  15

Ala Ala Ala Leu Glu Leu Val Asp Pro Pro Gly Cys Arg Asn Ser Ala
            20                  25                  30

Arg Val Lys Tyr Cys Val Val Tyr Asp Asn Asn Ser Ser Thr Leu Glu
        35                  40                  45

Ile Leu Leu Lys Asp Asp Asp Asp Asp Ser Asp Ser Asp Gly Asp Gly
    50                  55                  60

Lys Asp Leu Val Pro Gln Ala Ala Ile Glu Tyr Gly Arg Ile Leu Thr
65                  70                  75                  80

Arg Leu Thr His His Pro Val Tyr Ile Leu Lys Gly Gly Tyr Glu Arg
                85                  90                  95

Phe Ser Gly Thr Tyr His Phe Leu Arg Thr Gln Lys Ile Ile Trp Met
            100                 105                 110

Pro Gln Glu Leu Asp Ala Phe Gln Pro Tyr Pro Ile Glu Ile Val Pro
        115                 120                 125

Gly Lys Val Phe Val Gly Asn Phe Ser Gln Ala Cys Asp Pro Lys Ile
    130                 135                 140

Gln Lys Asp Leu Lys Ile Lys Ala His Val Asn Val Ser Met Asp Thr
145                 150                 155                 160

Gly Pro Phe Phe Ala Gly Asp Ala Asp Lys Leu Leu His Ile Arg Ile
                165                 170                 175

Glu Asp Ser Pro Glu Ala Gln Ile Leu Pro Phe Leu Arg His Met Cys
            180                 185                 190

His Phe Ile Glu Ile His His His Leu Gly Ser Val Ile Leu Ile Phe
        195                 200                 205

Ser Thr Gln Gly Ile Ser Arg Ser Cys Ala Ala Ile Ile Ala Tyr Leu
    210                 215                 220

Met His Ser Asn Glu Gln Thr Leu Gln Arg Ser Trp Ala Tyr Val Lys
225                 230                 235                 240

Lys Cys Lys Asn Asn Met Cys Pro Asn Arg Gly Leu Val Ser Gln Leu
                245                 250                 255

Leu Glu Trp Glu Lys Thr Ile Leu Gly Asp Ser Ile Thr Asn Ile Met
            260                 265                 270

Asp Pro Leu Tyr
        275


<210> 220
<211> 196
<212> PRT
<213> Homo sapiens

<220>
<221> SITE
<222> (98)
<223> Xaa equals any of the naturally occurring L-amino acids

<400> 220
Ile Arg His Glu Phe Thr Ser Glu Lys Ser Trp Lys Ser Ser Cys Asn
  1               5                  10                  15

Glu Gly Glu Ser Ser Ser Thr Ser Tyr Met His Gln Arg Ser Pro Gly
             20                  25                  30

Gly Pro Thr Lys Leu Ile Glu Ile Ile Ser Asp Cys Asn Trp Glu Glu
         35                  40                  45

Asp Arg Asn Lys Ile Leu Ser Ile Leu Ser Gln His Ile Asn Ser Asn
     50                  55                  60

Met Pro Gln Ser Leu Lys Val Gly Ser Phe Ile Ile Glu Leu Ala Ser
 65                  70                  75                  80

Gln Arg Lys Ser Arg Gly Glu Lys Asn Pro Pro Val Tyr Ser Ser Arg
                 85                  90                  95

Val Xaa Ile Ser Met Pro Ser Cys Gln Asp Gln Asp Asp Met Ala Glu
            100                 105                 110

Lys Ser Gly Ser Glu Thr Pro Asp Gly Pro Leu Ser Pro Gly Lys Met
        115                 120                 125

Glu Asp Ile Ser Pro Val Gln Thr Asp Ala Leu Asp Ser Val Arg Glu

130                    135                    140

Arg Leu His Gly Gly Lys Gly Leu Pro Phe Tyr Ala Gly Leu Ser Pro
145                 150                 155                 160

Ala Gly Lys Leu Val Ala Tyr Lys Arg Lys Pro Ser Ser Ser Thr Ser
                165                 170                 175

Gly Leu Ile Gln Val Arg Ile Ile Phe Asn Leu Gly Ile Ala Pro Leu
            180                 185                 190

Tyr Thr Pro Arg
        195

<210> 221
<211> 24
<212> PRT
<213> Homo sapiens

<400> 221
Cys Asn Ile Glu Tyr Ile Arg Ser Asp Lys Cys Met Phe Lys His Glu
 1               5                  10                  15

Leu Glu Glu Leu Arg Thr Thr Ile
            20

<210> 222
<211> 127
<212> PRT
<213> Homo sapiens

<220>
<221> SITE
<222> (8)
<223> Xaa equals any of the naturally occurring L-amino acids

<220>
<221> SITE
<222> (20)
<223> Xaa equals any of the naturally occurring L-amino acids

<220>
<221> SITE
<222> (21)
<223> Xaa equals any of the naturally occurring L-amino acids

<220>
<221> SITE
<222> (126)
<223> Xaa equals any of the naturally occurring L-amino acids

<400> 222
His His Gln Gln Val Pro Glu Xaa Asp Arg Glu Asp Ser Pro Glu Arg
 1               5                  10                  15

Cys Ser Asp Xaa Xaa Glu Glu Lys Lys Ala Arg Arg Gly Arg Ser Pro

            20                  25                  30

Lys Gly Glu Phe Lys Asp Glu Glu Glu Thr Val Thr Thr Lys His Ile
        35                  40                  45

His Ile Thr Gln Ala Thr Glu Thr Thr Thr Thr Arg His Lys Arg Thr
    50                  55                  60

Ala Asn Pro Ser Lys Thr Ile Asp Leu Gly Ala Ala Ala His Tyr Thr
65                  70                  75                  80

Gly Asp Lys Ala Ser Pro Asp Gln Asn Ala Ser Thr His Thr Pro Gln
                85                  90                  95

Ser Ser Val Lys Thr Ser Val Pro Ser Ser Lys Ser Ser Gly Asp Leu
            100                 105                 110

Val Asp Leu Phe Asp Gly Thr Ser Gln Cys Asn Arg Arg Xaa Ser
        115                 120                 125

<210> 223
<211> 95
<212> PRT
<213> Homo sapiens

<220>
<221> SITE
<222> (60)
<223> Xaa equals any of the naturally occurring L-amino acids

<220>
<221> SITE
<222> (73)
<223> Xaa equals any of the naturally occurring L-amino acids

<220>
<221> SITE
<222> (74)
<223> Xaa equals any of the naturally occurring L-amino acids

<400> 223
Val Ser Ser Asp Ser Val Gly Gly Phe Arg Tyr Ser Glu Arg Tyr Asp
1               5                   10                  15

Pro Glu Pro Lys Ser Lys Trp Asp Glu Glu Trp Asp Lys Asn Lys Ser
            20                  25                  30

Ala Phe Pro Phe Ser Asp Lys Leu Gly Glu Leu Ser Asp Lys Ile Gly
        35                  40                  45

Ser Thr Ile Asp Asp Thr Ile Ser Lys Phe Arg Xaa Lys Ile Glu Lys
    50                  55                  60

Thr Leu Gln Lys Asp Ala Ala Thr Xaa Xaa Arg Lys Arg Lys Arg Glu
65                  70                  75                  80

Glu Ala Asp Leu Pro Lys Val Asn Ser Lys Met Lys Arg Arg Leu

85                    90                    95

<210> 224
<211> 45
<212> PRT
<213> Homo sapiens

<400> 224
Arg Gln Ser Ile Phe Ile Ser His Arg Pro Gln Arg Pro Pro Gln Pro
1               5                   10                  15

Asp Thr Ser Ala Gln Gln Ile Leu Pro Lys Pro Leu Ile Leu Glu Gln
            20                  25                  30

Gln His Ile Thr Gln Gly Thr Lys Gln Val Gln Ile Arg
        35                  40                  45

<210> 225
<211> 190
<212> PRT
<213> Homo sapiens

<220>
<221> SITE
<222> (72)
<223> Xaa equals any of the naturally occurring L-amino acids

<220>
<221> SITE
<222> (163)
<223> Xaa equals any of the naturally occurring L-amino acids

<220>
<221> SITE
<222> (180)
<223> Xaa equals any of the naturally occurring L-amino acids

<400> 225
Asp Gln Asp Gly Leu Arg Ala Val Ala Ala Leu Thr Leu His Gln Gly
1               5                   10                  15

Arg Gln Leu Leu Tyr Arg Lys Phe Val His Pro Ser Leu Ser Arg His
            20                  25                  30

Glu Lys Glu Ile Asp Ala Tyr Ile Val Gln Ala Lys Glu Arg Ser Tyr
        35                  40                  45

Glu Thr Val Leu Ser Phe Gly Lys Arg Gly Leu Asn Ile Ala Ala Ser
    50                  55                  60

Ala Ala Val Gln Ala Ala Thr Xaa Ser Gln Gly Ala Leu Ala Gly Arg
65                  70                  75                  80

Leu Arg Ser Phe Ser Met Gln Asp Leu Arg Ser Ile Ser Asp Ala Pro
                85                  90                  95

Ala Pro Ala Tyr His Asp Pro Leu Tyr Leu Glu Asp Gln Val Ser His
            100                 105                 110

Arg Arg Pro Pro Ile Gly Tyr Arg Ala Gly Gly Leu Gln Asp Ser Asp
        115                 120                 125

Thr Glu Asp Glu Cys Trp Ser Asp Thr Glu Ala Val Pro Arg Ala Pro
    130                 135                 140

Ala Arg Pro Arg Glu Lys Pro Leu Ile Arg Ser Gln Ser Leu Arg Val
145                 150                 155                 160

Val Lys Xaa Lys Pro Pro Val Arg Glu Gly Thr Ser Arg Ser Leu Lys
                165                 170                 175

Val Arg Thr Xaa Lys Lys Thr Val Pro Ser Asp Val Asp Ser
            180                 185                 190

<210> 226
<211> 153
<212> PRT
<213> Homo sapiens

<220>
<221> SITE
<222> (45)
<223> Xaa equals any of the naturally occurring L-amino acids

<220>
<221> SITE
<222> (47)
<223> Xaa equals any of the naturally occurring L-amino acids

<220>
<221> SITE
<222> (68)
<223> Xaa equals any of the naturally occurring L-amino acids

<220>
<221> SITE
<222> (84)
<223> Xaa equals any of the naturally occurring L-amino acids

<220>
<221> SITE
<222> (110)
<223> Xaa equals any of the naturally occurring L-amino acids

<220>
<221> SITE
<222> (120)
<223> Xaa equals any of the naturally occurring L-amino acids

<220>
<221> SITE
<222> (149)
<223> Xaa equals any of the naturally occurring L-amino acids

<400> 226
Leu Cys His Arg Leu Pro Gly Arg Leu Gln Leu Leu Gly Val Pro Val
1               5                   10                  15

His Ala Gly Pro Leu Trp Val Tyr Ser Gly Leu Pro Gly Thr His Asp
            20                  25                  30

His Arg His Pro Pro Gly Leu Pro Arg Pro Leu Ala Xaa His Xaa Gly
        35                  40                  45

Pro Ala Leu His Gln His Trp Gly Pro Gly Ala Leu Gln Glu Ser Gln
    50                  55                  60

Ala Gly Gly Xaa Arg Arg Gly Pro Pro His Ser Gly Arg Tyr Leu Arg
65                  70                  75                  80

Asp Gly Gly Xaa Leu Leu Val Arg Phe Asn Ile Thr Arg Asp Phe Phe
                85                  90                  95

Asp Pro Leu Tyr Pro Gly Thr Lys Tyr Glu Leu Gly Pro Xaa Leu Tyr
            100                 105                 110

Leu Gly Trp Ser Ala Ser Leu Xaa Ser Ile Leu Gly Gly Leu Cys Leu
        115                 120                 125

Cys Ser Ala Cys Cys Cys Gly Ser Asp Glu Asp Gln Pro Pro Ala Pro
    130                 135                 140

Gly Gly Pro Thr Xaa Leu Pro Cys Pro
145                 150


<210> 227
<211> 33
<212> PRT
<213> Homo sapiens

<400> 227
Val Asp Gln Met Phe Gln Phe Ala Ser Ile Asp Val Ala Gly Asn Leu
1               5                   10                  15

Asp Tyr Lys Ala Leu Ser Tyr Val Ile Thr His Gly Glu Glu Lys Glu
            20                  25                  30

Glu


<210> 228
<211> 15
<212> PRT
<213> Homo sapiens

<400> 228
Ile Arg His Glu Ala Tyr Val Ile Leu Ala Val Cys Leu Gly Gly
1               5                   10                  15

<210> 229
<211> 185
<212> PRT
<213> Homo sapiens

<220>
<221> SITE
<222> (105)
<223> Xaa equals any of the naturally occurring L-amino acids

<400> 229
Trp Ile Gln Arg Ile Arg His Glu Thr Asn Pro Lys Cys Ser Tyr Ile
1               5                   10                  15

Pro Pro Cys Lys Arg Glu Asn Gln Lys Asn Leu Glu Ser Val Met Asn
            20                  25                  30

Trp Gln Gln Tyr Trp Lys Asp Glu Ile Gly Ser Gln Pro Phe Thr Cys
        35                  40                  45

Tyr Phe Asn Gln His Gln Arg Pro Asp Asp Val Leu Leu His Arg Thr
    50                  55                  60

His Asp Glu Ile Val Leu Leu His Cys Phe Leu Trp Pro Leu Val Thr
65                  70                  75                  80

Phe Val Val Gly Val Leu Ile Val Val Leu Thr Ile Cys Ala Lys Ser
                85                  90                  95

Leu Ala Val Lys Ala Glu Ala Met Xaa Glu Ala Gln Val Leu Leu Lys
            100                 105                 110

Gly Lys Glu Ala Cys Arg Lys Gln Ser Thr Glu Ala Val Leu Ile Gly
        115                 120                 125

Thr Arg Pro Pro Ala Glu Pro Val Phe Pro Gly Ala Gly Asp Gly Gln
    130                 135                 140

Gly His Asp Arg Ala Leu Arg Gly Ser Ser Leu Ser Gly Asn Arg Asn
145                 150                 155                 160

Arg His Asn Trp Lys Thr Trp Asn Leu Lys Ala Cys Ile Pro Ser Ala
                165                 170                 175

Val Ala Met Ala Lys Gly Ser Arg Ser
            180                 185


<210> 230
<211> 152
<212> PRT
<213> Homo sapiens

<220>
<221> SITE
<222> (21)
<223> Xaa equals any of the naturally occurring L-amino acids

<400> 230
His Tyr Glu Lys Val Arg Leu Gln Val Pro Ile Arg Asn Ser Arg Val
1               5                   10                  15

Asp Pro Arg Val Xaa Lys Phe Thr Ile Ser Asp His Pro Gln Pro Ile
            20                  25                  30

Asp Pro Leu Leu Lys Asn Cys Ile Gly Asp Phe Leu Lys Thr Leu Glu
        35                  40                  45

Asp Pro Asp Leu Asn Val Arg Arg Val Ala Leu Val Thr Phe Asn Ser
    50                  55                  60

Ala Ala His Asn Lys Pro Ser Leu Ile Arg Asp Leu Leu Asp Thr Val
65                  70                  75                  80

Leu Pro His Leu Tyr Asn Glu Thr Lys Val Arg Lys Glu Leu Ile Arg
                85                  90                  95

Glu Val Glu Met Gly Pro Phe Lys His Thr Val Asp Asp Gly Leu Asp
            100                 105                 110

Ile Arg Lys Ala Ala Phe Glu Cys Met Tyr Thr Leu Leu Asp Ser Cys
        115                 120                 125

Leu Asp Arg Leu Asp Ile Phe Glu Phe Leu Asn His Val Glu Asp Gly
    130                 135                 140

Leu Lys Asp His Tyr Asp Ile Lys
145                 150


<210> 231
<211> 79
<212> PRT
<213> Homo sapiens

<400> 231
Ile Arg His Glu His Leu Arg Gly Val Gln Glu Arg Val Asn Leu Ser
1               5                   10                  15

Ala Pro Leu Leu Pro Lys Glu Asp Pro Ile Phe Thr Tyr Leu Ser Lys
            20                  25                  30

Arg Leu Gly Arg Ser Ile Asp Asp Ile Gly His Leu Ile His Glu Gly
        35                  40                  45

Leu Gln Lys Asn Thr Ser Ser Trp Val Leu Tyr Asn Met Ala Ser Phe
    50                  55                  60

Tyr Trp Arg Ile Lys Asn Glu Pro Tyr Gln Val Val Glu Cys Ala
65                  70                  75


<210> 232
<211> 27
<212> PRT

<213> Homo sapiens

<400> 232
Glu Phe Gly Thr Ser Pro His Gln Thr Cys Gly Arg Arg Pro Gly Thr
1               5                   10                  15

Ala Ala Gly Trp Leu Leu Ala His Ser Thr Val
            20                  25


<210> 233
<211> 296
<212> PRT
<213> Homo sapiens

<400> 233
Asn Ser Ala Arg Asp Ser Leu Asn Thr Ala Ile Gln Ala Trp Gln Gln
1               5                   10                  15

Asn Lys Cys Pro Glu Val Glu Glu Leu Val Phe Ser His Phe Val Ile
            20                  25                  30

Cys Asn Asp Thr Gln Glu Thr Leu Arg Phe Gly Gln Val Asp Thr Asp
        35                  40                  45

Glu Asn Ile Leu Leu Ala Ser Leu His Ser His Gln Tyr Ser Trp Arg
    50                  55                  60

Ser His Lys Ser Pro Gln Leu Leu His Ile Cys Ile Glu Gly Trp Gly
65                  70                  75                  80

Asn Trp Arg Trp Ser Glu Pro Phe Ser Val Asp His Ala Gly Thr Phe
                85                  90                  95

Ile Arg Thr Ile Gln Tyr Arg Gly Arg Thr Ala Ser Leu Ile Ile Lys
            100                 105                 110

Val Gln Gln Leu Asn Gly Val Gln Lys Gln Ile Ile Ile Cys Gly Arg
        115                 120                 125

Gln Ile Ile Cys Ser Tyr Leu Ser Gln Ser Ile Glu Leu Lys Val Val
    130                 135                 140

Gln His Tyr Ile Gly Gln Asp Gly Gln Ala Val Val Arg Glu His Phe
145                 150                 155                 160

Asp Cys Leu Thr Ala Lys Gln Lys Leu Pro Ser Tyr Ile Leu Glu Asn
                165                 170                 175

Asn Glu Leu Thr Glu Leu Cys Val Lys Ala Lys Gly Asp Glu Asp Trp
            180                 185                 190

Ser Arg Asp Val Cys Leu Glu Ser Lys Ala Pro Glu Tyr Ser Ile Val
        195                 200                 205

Ile Gln Val Pro Ser Ser Asn Ser Ser Ile Ile Tyr Val Trp Cys Thr
    210                 215                 220

Val Leu Thr Leu Glu Pro Asn Ser Gln Val Gln Gln Arg Met Ile Val
225                 230                 235                 240

Phe Ser Pro Leu Phe Ile Met Arg Ser His Leu Pro Asp Pro Ile Ile
                245                 250                 255

Ile His Leu Glu Lys Arg Ser Leu Gly Leu Ser Glu Thr Gln Ile Ile
            260                 265                 270

Pro Gly Lys Gly Gln Glu Lys Pro Leu Gln Asn Ile Glu Pro Asp Leu
        275                 280                 285

Val His His Leu Thr Phe Gln Ala
    290                 295

<210> 234
<211> 26
<212> PRT
<213> Homo sapiens

<400> 234
Asn Lys Cys Pro Glu Val Glu Glu Leu Val Phe Ser His Phe Val Ile
  1               5                  10                  15

Cys Asn Asp Thr Gln Glu Thr Leu Arg Phe
             20                  25

<210> 235
<211> 25
<212> PRT
<213> Homo sapiens

<400> 235
His Ile Cys Ile Glu Gly Trp Gly Asn Trp Arg Trp Ser Glu Pro Phe
  1               5                  10                  15

Ser Val Asp His Ala Gly Thr Phe Ile
             20                  25

<210> 236
<211> 27
<212> PRT
<213> Homo sapiens

<400> 236
Val Val Arg Glu His Phe Asp Cys Leu Thr Ala Lys Gln Lys Leu Pro
  1               5                  10                  15

Ser Tyr Ile Leu Glu Asn Asn Glu Leu Thr Glu
             20                  25

<210> 237
<211> 27
<212> PRT

<213> Homo sapiens

<400> 237
Glu Asp Trp Ser Arg Asp Val Cys Leu Glu Ser Lys Ala Pro Glu Tyr
1               5                   10                  15

Ser Ile Val Ile Gln Val Pro Ser Ser Asn Ser
            20                  25


<210> 238
<211> 27
<212> PRT
<213> Homo sapiens

<400> 238
Ile Ile His Leu Glu Lys Arg Ser Leu Gly Leu Ser Glu Thr Gln Ile
1               5                   10                  15

Ile Pro Gly Lys Gly Gln Glu Lys Pro Leu Gln
            20                  25


<210> 239
<211> 162
<212> PRT
<213> Homo sapiens

<220>
<221> SITE
<222> (44)
<223> Xaa equals any of the naturally occurring L-amino acids

<220>
<221> SITE
<222> (47)
<223> Xaa equals any of the naturally occurring L-amino acids

<220>
<221> SITE
<222> (60)
<223> Xaa equals any of the naturally occurring L-amino acids

<220>
<221> SITE
<222> (63)
<223> Xaa equals any of the naturally occurring L-amino acids

<220>
<221> SITE
<222> (64)
<223> Xaa equals any of the naturally occurring L-amino acids

<400> 239
Leu Ile Ile Gln Asp Gln Thr Arg Arg Cys His Gly Leu Trp His Leu
1               5                   10                  15

Pro Ser Leu Leu Trp Pro Leu Leu Trp Ser Ser Gly Thr Gly Leu Cys

                20                  25                  30

Arg Asn Val Cys Arg Leu His Gly Ile Tyr His Xaa Val Leu Xaa Arg
        35                  40                  45

Val Gly His Ala Tyr Gln Thr Ser Phe Arg Gln Xaa Val Cys Xaa Xaa
    50                  55                  60

Trp Ala Ala Asp Leu Cys Gly Arg His Glu Glu Gly Ile Ile Glu Asn
65                  70                  75                  80

Thr Tyr Arg Leu Ser Cys Asn His Val Phe His Glu Phe Cys Ile Arg
                85                  90                  95

Gly Trp Cys Ile Val Gly Lys Lys Gln Thr Cys Pro Tyr Cys Lys Glu
            100                 105                 110

Lys Val Asp Leu Lys Arg Met Phe Ser Asn Pro Trp Glu Arg Pro His
        115                 120                 125

Val Met Tyr Gly Gln Leu Leu Asp Trp Leu Arg Tyr Leu Val Ala Trp
    130                 135                 140

Gln Pro Val Ile Ile Gly Val Val Gln Gly Ile Asn Tyr Ile Leu Gly
145                 150                 155                 160

Leu Glu

<210> 240
<211> 164
<212> PRT
<213> Homo sapiens

<220>
<221> SITE
<222> (95)
<223> Xaa equals any of the naturally occurring L-amino acids

<220>
<221> SITE
<222> (97)
<223> Xaa equals any of the naturally occurring L-amino acids

<220>
<221> SITE
<222> (111)
<223> Xaa equals any of the naturally occurring L-amino acids

<220>
<221> SITE
<222> (114)
<223> Xaa equals any of the naturally occurring L-amino acids

<400> 240
Thr Ala Phe Val Thr Phe Arg Ala Thr Arg Lys Pro Leu Val Gln Thr
1               5                   10                  15

Thr Pro Arg Leu Val Tyr Lys Trp Phe Leu Leu Ile Tyr Lys Ile Ser
            20                  25                  30

Tyr Ala Thr Gly Ile Val Gly Tyr Met Ala Val Met Phe Thr Leu Phe
        35                  40                  45

Gly Leu Asn Leu Leu Phe Lys Ile Lys Pro Glu Asp Ala Met Asp Phe
    50                  55                  60

Gly Ile Ser Leu Leu Phe Tyr Gly Leu Tyr Tyr Gly Val Leu Glu Arg
65                  70                  75                  80

Asp Phe Ala Glu Met Cys Ala Asp Tyr Met Ala Ser Thr Ile Xaa Phe
                85                  90                  95

Xaa Ser Glu Ser Gly Met Pro Thr Lys His Leu Ser Asp Ser Xaa Cys
            100                 105                 110

Ala Xaa Cys Gly Gln Gln Ile Phe Val Asp Val Met Lys Arg Gly Ser
        115                 120                 125

Leu Arg Thr Arg Ile Gly Cys Pro Ala Ile Met Ser Ser Thr Ser Ser
    130                 135                 140

Ala Ser Val Ala Gly Ala Ser Trp Glu Arg Ser Lys Arg Val Pro Thr
145                 150                 155                 160

Ala Lys Arg Arg

<210> 241
<211> 28
<212> PRT
<213> Homo sapiens

<400> 241

<210> 242
<211> 116
<212> PRT
<213> Homo sapiens

<400> 242

<210> 243
<211> 149
<212> PRT
<213> Homo sapiens

<220>
<221> SITE
<222> (128)
<223> Xaa equals any of the naturally occurring L-amino acids

<220>
<221> SITE
<222> (133)
<223> Xaa equals any of the naturally occurring L-amino acids

<220>
<221> SITE
<222> (136)
<223> Xaa equals any of the naturally occurring L-amino acids

<220>
<221> SITE
<222> (140)
<223> Xaa equals any of the naturally occurring L-amino acids

<220>
<221> SITE
<222> (143)
<223> Xaa equals any of the naturally occurring L-amino acids

<400> 243
Trp Ile Pro Arg Ala Ala Gly Ile Arg His Glu His Leu Ser Thr Leu
1               5                   10                  15

Asp Arg Ser Val Ile Trp Ser Lys Ser Ile Leu Asn Ala Arg Cys Lys
            20                  25                  30

Ile Cys Arg Lys Lys Gly Asp Ala Glu Asn Met Val Leu Cys Asp Gly
        35                  40                  45

Cys Asp Arg Gly His His Thr Tyr Cys Val Arg Pro Lys Leu Lys Thr
    50                  55                  60

Val Pro Glu Gly Asp Trp Phe Cys Pro Glu Cys Arg Pro Lys Gln Arg
65                  70                  75                  80

Ser Arg Arg Leu Ser Ser Arg Gln Arg Pro Ser Leu Glu Ser Asp Glu
                85                  90                  95

Asp Val Glu Asp Ser Met Gly Gly Glu Asp Asp Glu Val Asp Gly Asp
            100                 105                 110

Glu Glu Glu Gly Gln Ser Glu Glu Glu Glu Tyr Glu Val Glu Gln Xaa
        115                 120                 125

Glu Asp Asp Ser Xaa Glu Glu Xaa Glu Val Arg Xaa Val Leu Xaa Cys
    130                 135                 140

Asn Lys Met Ser Gln
145


<210> 244
<211> 11
<212> PRT
<213> Homo sapiens

<400> 244
Met Arg Val Ala Arg Tyr Val Glu Arg Lys Ala
 1               5                  10


<210> 245
<211> 183
<212> PRT
<213> Homo sapiens

<220>
<221> SITE
<222> (29)
<223> Xaa equals any of the naturally occurring L-amino acids

<220>
<221> SITE
<222> (31)
<223> Xaa equals any of the naturally occurring L-amino acids

<220>
<221> SITE
<222> (87)
<223> Xaa equals any of the naturally occurring L-amino acids

<220>
<221> SITE
<222> (89)
<223> Xaa equals any of the naturally occurring L-amino acids

<220>

<221> SITE
<222> (159)
<223> Xaa equals any of the naturally occurring L-amino acids

<400> 245
Gln Arg Trp Leu Lys His Gly Ala Asn Gln Cys Lys Phe Glu His Asn
1               5                   10                  15

Asp Cys Leu Asp Lys Ser Tyr Lys Cys Tyr Ala Ala Xaa Glu Xaa Val
            20                  25                  30

Gly Glu Asn Ile Trp Leu Gly Gly Ile Lys Ser Phe Thr Pro Arg His
        35                  40                  45

Ala Ile Thr Ala Trp Tyr Asn Glu Thr Gln Phe Tyr Asp Phe Asp Ser
    50                  55                  60

Leu Ser Cys Ser Arg Val Cys Gly His Tyr Thr Gln Leu Val Trp Ala
65                  70                  75                  80

Asn Ser Phe Tyr Val Gly Xaa Ala Xaa Ala Met Cys Pro Asn Leu Gly
                85                  90                  95

Gly Ala Ser Thr Ala Ile Phe Val Cys Asn Tyr Gly Pro Ala Gly Asn
            100                 105                 110

Phe Ala Asn Met Pro Pro Tyr Val Arg Gly Glu Ser Cys Ser Leu Cys
        115                 120                 125

Ser Lys Glu Glu Lys Cys Val Lys Asn Leu Cys Lys Asn Pro Phe Leu
    130                 135                 140

Lys Pro Thr Gly Arg Ala Pro Gln Gln Thr Ala Phe Asn Pro Xaa Gln
145                 150                 155                 160

Leu Arg Phe Ser Ser Ser Glu Asn Leu Leu Met Ser Phe Ile Tyr Lys
                165                 170                 175

Arg Asn Ser Gln Met Leu Lys
            180


<210> 246
<211> 164
<212> PRT
<213> Homo sapiens

<400> 246
Thr Glu Gly Gly Cys Ala Leu Val Pro Asn Asp Met Glu Ser Leu Lys
1               5                   10                  15

Gln Lys Leu Val Arg Val Leu Glu Glu Asn Leu Ile Leu Ser Glu Lys
            20                  25                  30

Ile Gln Gln Leu Glu Glu Gly Ala Ala Ile Ser Ile Val Ser Gly Gln
        35                  40                  45

Gln Ser His Thr Tyr Asp Asp Leu Leu His Lys Asn Gln Gln Leu Thr

50                    55                    60

Met Gln Val Ala Cys Leu Asn Gln Glu Leu Ala Gln Leu Lys Lys Leu
65                  70                  75                  80

Glu Lys Thr Val Ala Ile Leu His Glu Ser Gln Arg Ser Leu Val Val
                85                  90                  95

Thr Asn Glu Tyr Leu Leu Gln Gln Leu Asn Lys Glu Pro Lys Gly Tyr
            100                 105                 110

Ser Gly Lys Ala Leu Leu Pro Pro Glu Lys Gly His His Leu Gly Arg
        115                 120                 125

Ser Ser Pro Phe Gly Lys Ser Thr Leu Ser Ser Ser Ser Pro Val Ala
    130                 135                 140

His Glu Thr Gly Gln Tyr Leu Ile Gln Ser Val Leu Asp Ala Ala Pro
145                 150                 155                 160

Glu Pro Gly Leu

<210> 247
<211> 5
<212> PRT
<213> Homo sapiens

<400> 247
Ser Met Val Ser Lys
1               5

<210> 248
<211> 50
<212> PRT
<213> Homo sapiens

<220>
<221> SITE
<222> (34)
<223> Xaa equals any of the naturally occurring L-amino acids

<400> 248
Asn Thr Asp Trp Asp Gln Thr Val Leu Ile Val Leu Arg Ile Ser Ser
1               5                   10                  15

Thr Leu Pro Val Ala Leu Leu Arg Asp Glu Val Pro Gly Trp Phe Leu
            20                  25                  30

Lys Xaa Pro Glu Pro Gln Leu Ile Ser Lys Glu Leu Ile Met Leu Thr
        35                  40                  45

Glu Val
    50

<210> 249
<211> 44
<212> PRT
<213> Homo sapiens

<400> 249
Val Ala Glu Ser Thr Glu Glu Pro Ala Gly Ser Asn Arg Gly Gln Tyr
1               5                   10                  15

Pro Glu Asp Ser Ser Ser Asp Gly Leu Arg Gln Arg Glu Val Leu Arg
            20                  25                  30

Asn Leu Ser Ser Pro Gly Trp Glu Asn Ile Ser Arg
        35                  40


<210> 250
<211> 30
<212> PRT
<213> Homo sapiens

<400> 250
Ala Arg Glu Pro Leu Gly Leu Thr Gln Asp Pro Leu Val Phe Gly Met
1               5                   10                  15

Thr Ser Phe Leu Gln Thr Ser Ser Pro Ile Pro Asn Ser Cys
            20                  25                  30


<210> 251
<211> 15
<212> PRT
<213> Homo sapiens

<400> 251
Phe Gln Ala Pro Ala Ser Ala Arg Thr Ala Cys Ser Thr Leu Leu
1               5                   10                  15


<210> 252
<211> 37
<212> PRT
<213> Homo sapiens

<400> 252


<210> 253
<211> 121
<212> PRT

<213> Homo sapiens

<220>
<221> SITE
<222> (43)
<223> Xaa equals any of the naturally occurring L-amino acids

<220>
<221> SITE
<222> (104)
<223> Xaa equals any of the naturally occurring L-amino acids

<220>
<221> SITE
<222> (115)
<223> Xaa equals any of the naturally occurring L-amino acids

<400> 253
His Ser Leu Pro Pro Ala Leu Pro Ala Trp Leu Thr Pro Gly His Pro
1               5                   10                  15

Ser Asp Ser Ser Leu Cys Leu Leu Gln Leu Ala Pro His Leu Val Met
            20                  25                  30

Ala Val Ser Val Pro Trp Pro Leu Pro Glu Xaa Leu Gly Phe Ser Cys
        35                  40                  45

Cys His Cys Val Ser Leu Thr Gly Pro His Ala Gly Phe Ser Tyr His
    50                  55                  60

Phe Leu His Pro Ala Glu Pro Arg Ala Trp Gln His Gln Ser Ser Val
65                  70                  75                  80

Val Gly Met Ser Arg Lys Gln Ala Ser Phe Ser Met Ala Gln Lys Gly
                85                  90                  95

Val Cys His Leu Gly Lys Ser Xaa Lys Arg Gly Ser Lys Lys Ala Ser
            100                 105                 110

Cys Pro Xaa Tyr Pro Ser Phe Ser Lys
        115                 120


<210> 254
<211> 24
<212> PRT
<213> Homo sapiens

<400> 254
Ile Gly Ile Arg Val Trp Tyr Tyr Arg Asn Gln Lys Asn Ser Lys Gln
1               5                   10                  15

Met Trp Ile Lys Cys Leu Gly Ser
            20